(19) Europäisches Patentamt
European Patent Office
Office européen des brevets

(11) EP 0 951 464 B1

(12) **EUROPEAN PATENT SPECIFICATION**

(45) Date of publication and mention of the grant of the patent:
**11.05.2005 Bulletin 2005/19**

(21) Application number: **97947410.3**

(22) Date of filing: **20.11.1997**

(51) Int Cl.7: **C07C 233/51**, C07C 233/46, C07C 235/34, C07C 233/39, C07C 233/37, A61K 31/16, A61K 31/33, C07D 317/60, C07D 213/56, C07D 333/24, C07D 277/62, C07C 333/24, C07C 323/59, C07D 333/16, C07D 263/54

(86) International application number:
**PCT/US1997/020355**

(87) International publication number:
**WO 1998/022430 (28.05.1998 Gazette 1998/21)**

(54) **N-(ARYL/HETEROARYLACETYL) AMINO ACID ESTERS, PHARMACEUTICAL COMPOSITIONS COMPRISING SAME, AND METHODS FOR INHIBITING BETA-AMYLOID PEPTIDE RELEASE AND/OR ITS SYNTHESIS BY USE OF SUCH COMPOUNDS**

N-(ARYL/HETEROARYLACETYL)AMINOSÄUREESTER, DIESE ENTHALTENDE PHARMAZEUTISCHE ZUSAMMENSETZUNGEN UND METHODEN ZUR INHIBIERUNG DER BETA-AMYLOIDFREISETZUNG UND/ODER DEREN SYNTHESE UNTER VERWENDUNG DIESER VERBINDUNGEN

ESTERS DE N-(ARYL/HETEROARYLACETYL) AMINOACIDE, COMPOSITIONS PHARMACEUTIQUES LES CONTENANT ET METHODES POUR INHIBER LA LIBERATION DU PEPTIDE DE LA PROTEINE BETA-AMYLOIDE ET/OU SA SYNTHESE AU MOYEN DESDITS COMPOSES

(84) Designated Contracting States:
**AT BE CH DE DK ES FI FR GB GR IE IT LI LU MC NL PT SE**
Designated Extension States:
**AL LT LV MK RO SI**

(30) Priority: **22.11.1996 US 754895**

(43) Date of publication of application:
**27.10.1999 Bulletin 1999/43**

(73) Proprietors:
- **Elan Pharmaceuticals, Inc.**
  **South San Francisco, CA 94080 (US)**
- **ELI LILLY AND COMPANY**
  **Indianapolis, Indiana 46285 (US)**

(72) Inventors:
- **WU, Jing**
  **San Mateo, CA 94402 (US)**
- **THORSETT, Eugene, D.**
  **Moss Beach, CA 94038 (US)**
- **NISSEN, Jeffrey, S.**
  **Indianapolis, IN 46268 (US)**
- **MABRY, Thomas, E.**
  **Indianapolis, IN 46227 (US)**
- **LATIMER, Lee, H.**
  **Oakland, CA 94618 (US)**
- **JOHN, Varghese**
  **San Francisco, CA 94122 (US)**
- **FANG, Lawrence, Y.**
  **Foster City, CA 94404 (US)**
- **AUDIA, James, E.**
  **Indianapolis, IN 46278 (US)**

(74) Representative: **Senior, Janet et al**
**Abel & Imray**
**20 Red Lion Street**
**London WC1R 4PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 951 464 B1

(56) References cited:

**EP-A- 0 652 009**
**DE-A- 1 927 692**
**DE-A- 2 321 392**

- **H.T. CLARKE: "The chemistry of penicillin" , PRINCETON UNIVERSITY PRESS , 1949 XP002058308 see page 240, left-hand column, line 23 see page 708, left-hand column; table see column 1, line 4 see page 708, right-hand column, line 21 - line 22 see page 708, right-hand column, line 35 - line 36 see page 708, right-hand column, line 42 - line 43 see page 786, right-hand column, paragraph 2 see page 786, right-hand column, paragraph 3 see page 786, right-hand column, last paragraph see page 795, right-hand column, paragraph 3 see page 875, left-hand column, paragraph 9-12**

## Description

## BACKGROUND OF THE INVENTION

### Field of the Invention

This invention relates to compounds which inhibit β-amyloid peptide release and/or its synthesis, and, accordingly, have utility in treating Alzheimer's disease. This invention also relates to pharmaceutical compositions comprising such compounds as well as methods for inhibiting release of β-amyloid peptide.

### References

The following publications, patents and patent applications are cited in this application as superscript numbers:

[1] Glenner, et al., "Alzheimer's Disease: Initial Report of the Purification and Characterization of a Novel Cerebrovascular Amyloid Protein", *Biochem. Biophys. Res. Commun.*, 120:885-890 (1984).

[2] Glenner, et al., "Polypeptide Marker for Alzheimer's Disease and its Use for Diagnosis", *U.S. Patent No. 4,666, 829* issued May 19, 1987.

[3] Selkoe, "The Molecular Pathology of Alzheimer's Disease", *Neuron,* 6:487-498 (1991).

[4] Goate, et al., "Segregation of a Missense Mutation in the Amyloid Precursor Protein Gene with Familial Alzheimer's Disease", *Nature*, 349:704-706 (1990).

[5] Chartier-Harlan, et al., "Early-Onset Alzheimer's Disease Caused by Mutations at Codon 717 of the β-Amyloid Precursor Proteing Gene", *Nature,* 353:844-846 (1989).

[6] Murrell, et al., "A Mutation in the Amyloid Precursor Protein Associated with Hereditary Alzheimer's Disease", *Science,* 254:97-99 (1991).

[7] Mullan, et al., "A Pathogenic Mutation for Probable Alzheimer's Disease in the APP Gene at the N-Terminus of β-Amyloid, *Nature Genet. ,* 1:345-347 (1992).

[8] Schenk, et al., "Methods and Compositions for the Detection of Soluble β-Amyloid Peptide", *International Patent Application Publication No. WO 94/10569*, published 11 May 1994.

[9] Selkoe, "Amyloid Protein and Alzheimer's Disease", *Scientific American*, pp. 2-8, November, 1991.

[10] Losse, et al., Tetrahedron, 27:1423-1434 (1971).

[11] Citron, et al., "Mutation of the β-Amyloid Precursor Protein in Familial Alzheimer's Disease Increases β-Protein Production, *Nature,* 360:672-674 (1992).

[12] Hansen, et al., "Reexamination and Further Development of a Precise and Rapid Dye Method for Measuring Cell Growth/Cell Kill", J. *Immun. Meth.,* 119:203-210 (1989).

[13] P. Seubert, *Nature* (1992) **359**:325-327

[14] Johnson-Wood et al., *PNAS* USA (1997) **94:**1550-1555

[15] *Tetrahedron Letters*, **34**(48), 7685 (1993))

### State of the Art

Alzheimer's Disease (AD) is a degenerative brain disorder characterized clinically by progressive loss of memory, cognition, reasoning, judgment and emotional stability that gradually leads to profound mental deterioration and ultimately death. AD is a very common cause of progressive mental failure (dementia) in aged humans and is believed

to represent the fourth most common medical cause of death in the United States. AD has been observed in races and ethnic groups worldwide and presents a major present and future public health problem. The disease is currently estimated to affect about two to three million individuals in the United States alone. AD is at present incurable. No treatment that effectively prevents AD or reverses its symptoms and course is currently known.

The brains of individuals with AD exhibit characteristic lesions termed senile (or amyloid) plaques, amyloid angiopathy (amyloid deposits in blood vessels) and neurofibrillary tangles. Large numbers of these lesions, particularly amyloid plaques and neurofibrillary tangles, are generally found in several areas of the human brain important for memory and cognitive function in patients with AD. Smaller numbers of these lesions in a more restrictive anatomical distribution are also found in the brains of most aged humans who do not have clinical AD. Amyloid plaques and amyloid angiopathy also characterize the brains of individuals with Trisomy 21 (Down's Syndrome) and Hereditary Cerebral Hemorrhage with Amyloidosis of the Dutch Type (HCHWA-D). At present, a definitive diagnosis of AD usually requires observing the aforementioned lesions in the brain tissue of patients who have died with the disease or, rarely, in small biopsied samples of brain tissue taken during an invasive neurosurgical procedure.

The principal chemical constituent of the amyloid plaques and vascular amyloid deposits (amyloid angiopathy) characteristic of AD and the other disorders mentioned above is an approximately 4.2 kilodalton (kD) protein of about 39-43 amino acids designated the β-amyloid peptide (βAP) or sometimes Aβ, AβP or β/A4. β-Amyloid peptide was first purified and a partial amino acid sequence was provided by Glenner, et al.[1] The isolation procedure and the sequence data for the first 28 amino acids are described in U.S. Patent No. 4,666,829[2].

Molecular biological and protein chemical analyses have shown that the β-amyloid peptide is a small fragment of a much larger precursor protein (APP), that is normally produced by cells in many tissues of various animals, including humans. Knowledge of the structure of the gene encoding the APP has demonstrated that β-amyloid peptide arises as a peptide fragment that is cleaved from APP by protease enzyme(s). The precise biochemical mechanism by which the β-amyloid peptide fragment is cleaved from APP and subsequently deposited as amyloid plaques in the cerebral tissue and in the walls of the cerebral and meningeal blood vessels is currently unknown.

Several lines of evidence indicate that progressive cerebral deposition of β-amyloid peptide plays a seminal role in the pathogenesis of AD and can precede cognitive symptoms by years or decades. See, for example, Selkoe[3]. The most important line of evidence is the discovery that missense DNA mutations at amino acid 717 of the 770-amino acid isoform of APP can be found in affected members but not unaffected members of several families with a genetically determined (familial) form of AD (Goate, et al.[4]; Chartier-Harlan, et al.[5]; and Murrell, et al.[6]) and is referred to as the Swedish variant. A double mutation changing lysine$^{595}$-methionine$^{596}$ to asparagine$^{595}$-leucine$^{596}$ (with reference to the 695 isoform) found in a Swedish family was reported in 1992 (Mullan, et al.[7]). Genetic linkage analyses have demonstrated that these mutations, as well as certain other mutations in the APP gene, are the specific molecular cause of AD in the affected members of such families. In addition, a mutation at amino acid 693 of the 770-amino acid isoform of APP has been identified as the cause of the β-amyloid peptide deposition disease. HCHWA-D, and a change from alanine to glycine at amino acid 692 appears to cause a phenotype that resembles AD is some patients but HCHWA-D in others. The discovery of these and other mutations in APP in genetically based cases of AD prove that alteration of APP and subsequent deposition of its β-amyloid peptide fragment can cause AD.

Despite the progress which has been made in understanding the underlying mechanisms of AD and other β-amyloid peptide related diseases, there remains a need to develop methods and compositions for treatment of the disease(s). Ideally, the treatment methods would advantageously be based on drugs which are capable of inhibiting β-amyloid peptide release and/or its synthesis *in vivo*.

There are know from EP 0 652 009 aspartyl protease inhibitors that inhibit β-amyloid peptide production in cell culture and *in vivo*.

## SUMMARY OF THE INVENTION

The invention is directed to the discovery of a class of compounds which inhibit β-amyloid peptide release and/or its synthesis and, therefore, are useful in the prevention of AD in patients susceptible to AD and/or in the treatment of patients with AD in order to inhibit further deterioration in their condition. Accordingly the invention provides a compound of formula I or a mixture of compounds of formula I for use as a medicament, wherein formula I is:

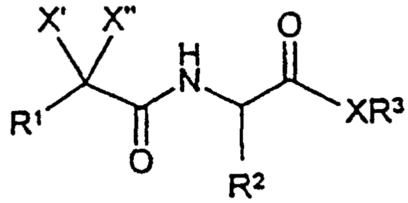

I

wherein $R^1$ is selected from the group consisting of

a) alkyl, alkenyl, alkcycloalkyl, naphthyl-$(R)_m$- wherein R is an alkylene group of from 1 to 8 carbon atoms and *m* is an integer equal to 0 or 1, cycloalkyl, cycloalkenyl, 3-pyridyl, 4-pyridyl and heteroaryl, of 3 to 10 atoms and 1 to 4 heteroatoms selected from oxygen, sulfur and nitrogen wherein the heteroaryl group is optionally substituted with 1 to 3 substituents selected from the group consisting of alkyl, alkoxy, aryl, aryloxy, halo, nitro, thioalkoxy, and thioaryloxy with the proviso that for such heteroaryls when there is at least one nitrogen heteroatom, there is also at least one oxygen and/or sulfur heteroatom;
b) a substituted phenyl group of formula II:

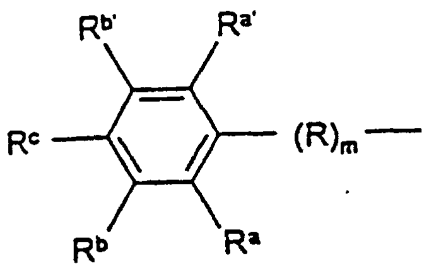

II

wherein R is alkylene of from 1 to 8 carbon atoms,
*m* is an integer equal to 0 or 1,
$R^a$ and $R^{a'}$ are independently selected from the group consisting of hydrogen, hydroxy, fluoro and methyl;
$R^b$ and $R^{b'}$ are independently selected from the group consisting of hydrogen, alkyl, alkoxy, aryl, cyano, cycloalkyl, halo, heteroaryl, heterocyclic, nitro, trihalomethyl, thioalkoxy, thioaryloxy, thioheteroaryloxy, and -C(O)$R^4$ where $R^4$ is selected from the group consisting of alkyl, aryl, alkoxy and aryloxy; and
$R^c$ is selected from the group consisting of hydrogen, alkyl, aryl, cyano, halo, nitro, and where $R^b$ and $R^c$ are fused to form a methylenedioxy ring with the phenyl ring; and
when $R^b$ and/or $R^{b'}$ and/or $R^c$ is fluoro, chloro, bromo and/or nitro, then $R^a$ and/or $R^{a'}$ can also be chloro; and
whereby $R^a$, $R^{a'}$, $R^b$, $R^{b'}$ and $R^c$ are not all simultaneously hydrogen; and
c) 1- or 2-naphthyl-$(R)_m$- substituted at the 5, 6, 7 and/or 8 positions with 1 to 4 substituents selected from the group consisting alkyl, alkoxy, halo, cyano, nitro, trihalomethyl, and thioalkoxy wherein R is an alkylene group of from 1 to 8 carbon atoms and *m* is an integer equal to 0 or 1;
$R^2$ is selected from the group consisting of alkyl, phenyl, alkylalkoxy, alkylthioalkoxy; and
$R^3$ is selected from the group consisting of $-(CH_2)_nCR^{10}R^5R^6$ wherein *n* is an integer equal to 0,1 or 2, $R^5$ and $R^6$ are independently selected from hydrogen, alkyl, alkenyl, aryl, heteroaryl, heterocyclic, $-NR^7R^8$ where $R^7$ and $R^8$ are independently hydrogen or alkyl and $-COOR^9$ where $R^9$ is alkyl, and further wherein $R^5$ and $R^6$ can be joined to form a cycloalkyl group, a cycloalkenyl group, an aryl group, a heteroaryl group, and a heterocyclic group, and when $R^5$ and $R^6$ do not join to form an aryl or heteroaryl group, then $R^{10}$ is selected from hydrogen and alkyl with the proviso that when n is zero, then $R^{10}$ is hydrogen and when *n* is greater than zero and $R^5$ and $R^6$ are joined to form an aryl or heteroaryl group, then $R^{10}$ becomes a bond within that group;
X is oxygen or sulfur;
X' is hydrogen, hydroxy or fluoro;
X" is hydrogen, hydroxy or fluoro, or X' and X" together form an oxo group, and pharmaceutically acceptable salts thereof
with the provisos that:

when $R^1$ is pyrid-3-yl, $R^2$ is ethyl, X is oxygen, and X' and X" are hydrogen, then $R^3$ is not $-CH_2CH(CH_3)_2$, and when $R^1$ is indoxazin-3-yl, 2,4-dimethylthiazol-5-yl, 4-methyl-1,2,5- thiooxadizol-3-yl or 3,5-di(trifluoromethyl) phenyl, $R^2$ is methyl, X is oxygen, and X' and X" are hydrogen, then $R^3$ is not $-CH_2CH(CH_3)_2$.

Surprisingly, the substituents at the 2 and/or 6 positions of the phenyl group are limited to those recited above and larger substituents, other than those specifically specified above, eliminate the ability of the resulting compounds to inhibit β-amyloid peptide release and/or its synthesis.

Accordingly, in one of its aspects, this invention is directed to the compound of formula I or mixture of compounds of formula I described above for inhibiting β-amyloid peptide release and/or its synthesis in a cell.

Because the *in vivo* generation of β-amyloid peptide is associated with the pathogenesis of AD[8,9], the compounds of formula I can also be employed in conjunction with a pharmaceutical composition to prophylactically and/or therapeutically prevent and/or treat AD. Accordingly, in another of its aspects, this invention is directed to the compound of formula I of mixture of compounds of formula I described above for preventing the onset of AD in a patient at risk for developing AD.

In yet another of its method aspects, this invention is directed to the compound of formula I or mixture of compounds according to formula I described above for treating a patient with AD in order to inhibit further deterioration in the condition of that patient.

The invention also provides a pharmaceutical composition comprising a pharmaceutically inert carrier and a pharmaceutically effective amount of a compound of formula I

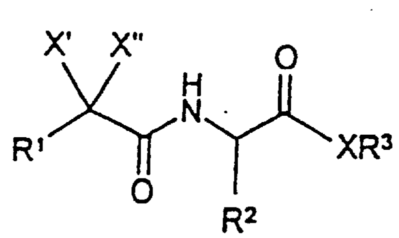

I

wherein $R^1$ is selected from the group consisting of

a) alkyl, alkenyl, alkcycloalkyl, naphthyl-$(R)_m$- wherein R is an alkylene group of from 1 to 8 carbon atoms and *m* is an integer equal to 0 or 1, cycloalkyl, cycloalkenyl, 3-pyridyl, 4-pyridyl and heteroaryl, of 3 to 10 atoms and 1 to 4 heteroatoms selected from oxygen, sulfur and nitrogen wherein the heteroaryl group is optionally substituted with 1 to 3 substituents selected from the group consisting of alkyl, alkoxy, aryl, aryloxy, halo, nitro, thioalkoxy, and thioaryloxy with the proviso that for such heteroaryls when there is at least one nitrogen heteroatom, there is also at least one oxygen and/or sulfur heteroatom;

(b) a substituted phenyl group of formula II:

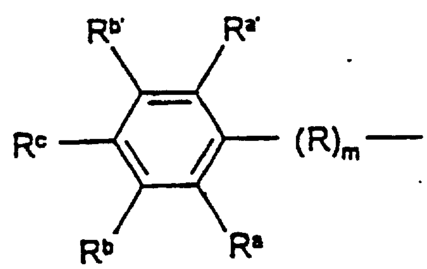

II

wherein R is alkylene of from 1 to 8 carbon atoms,

*m* is an integer equal to 0 or 1,

$R^a$ and $R^{a'}$ are independently selected from the group consisting of hydrogen, hydroxy, fluoro and methyl;

$R^b$ and $R^{b'}$ are independently selected from the group consisting of hydrogen, alkyl, alkoxy, aryl, cyano, cycloalkyl, halo, heteroaryl, heterocyclic, nitro, trihalomethyl, thioalkoxy, thioaryloxy, thioheteroaryloxy, and -C(O)$R^4$ where $R^4$ is selected from the group consisting of alkyl, aryl, alkoxy and aryloxy; and

$R^c$ is selected from the group consisting of hydrogen, alkyl, aryl, cyano, halo, nitro, and where $R^b$ and $R^c$ are

fused to form a methylenedioxy ring with the phenyl ring; and

when $R^b$ and/or $R^{b'}$ and/or $R^c$ is fluoro, chloro, bromo and/or nitro, then $R^a$ and/or $R^{a'}$ can also be chloro; and whereby $R^a$, $R^{a'}$, $R^b$, $R^{b'}$ and $R^c$ are not all simultaneously hydrogen; and

(c) 1- or 2-naphthyl-$(R)_m$- substituted at the 5, 6, 7 and/or 8 positions with 1 to 4 substituents selected from the group consisting alkyl, alkoxy, halo, cyano, nitro, trihalomethyl, and thioalkoxy wherein R is an alkylene group of from 1 to 8 carbon atoms and *m* is an integer equal to 0 or 1;

$R^2$ is selected from the group consisting of alkyl, phenyl, alkylalkoxy, alkylthioalkoxy; and

$R^3$ is selected from the group consisting of $-(CH_2)_nCR^{10}R^5R^6$ wherein *n* is an integer equal to 0, 1 or 2, $R^5$ and $R^6$ are independently selected from hydrogen, alkyl, alkenyl, aryl, heteroaryl, heterocyclic, $-NR^7R^8$ where $R^7$ and $R^8$ are independently hydrogen or alkyl and $-COOR^9$ where $R^9$ is alkyl, and further wherein $R^5$ and $R^6$ can be joined to form a cycloalkyl group, a cycloalkenyl group, an aryl group, a heteroaryl group, and a heterocyclic group, and when $R^5$ and $R^6$ do not join to form an aryl or heteroaryl group, then $R^{10}$ is selected from hydrogen and alkyl with the proviso that when *n* is zero, then $R^{10}$ is hydrogen and when *n* is greater than zero and $R^5$ and $R^6$ are joined to form an aryl or heteroaryl group, then $R^{10}$ becomes a bond within that group;

X is oxygen or sulfur;

X' is hydrogen, hydroxy or fluoro;

X" is hydrogen, hydroxy or fluoro, or X' and X" together form an oxo group, and

pharmaceutically acceptable salts thereof

with the provisos that:

when $R^1$ is pyrid-3-yl, $R^2$ is ethyl, X is oxygen, and X' and X" are hydrogen, then $R^3$ is not $-CH_2CH(CH_3)_2$, and

when $R^1$ is indoxazin-3-yl, 2,4-dimethylthiazol-5-yl, 4-methyl-1,2,5-thiooxadizol-3-yl or 3,5-di(trifluoromethyl)phenyl, $R^2$ is methyl, X is oxygen, and X' and X" are hydrogen, then $R^3$ is not $-CH_2CH(CH_3)_2$.

The invention further provides the use of a compound of formula I' or a mixture of compounds of formula I' in the manufacture of a medicament for inhibiting β-amyloid release and/or its synthesis in a cell, wherein formula I' is:

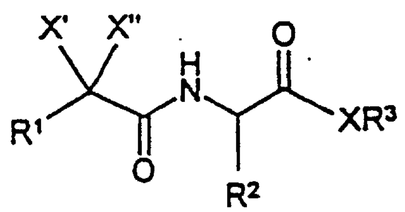

I'

wherein $R^1$ is selected from the group consisting of

a) alkyl, alkenyl, alkcycloalkyl, phenyl-$(R)_m$-, naphthyl-$(R)_m$- wherein R is an alkylene group of from 1 to 8 carbon atoms and *m* is an integer equal to 0 or 1, cycloalkyl, cycloalkenyl, 3-pyridyl, 4-pyridyl and heteroaryl, of 3 to 10 atoms and 1 to 4 heteroatoms selected from oxygen, sulfur and nitrogen wherein the heteroaryl group is optionally substituted with 1 to 3 substituents selected from the group consisting of alkyl, alkoxy, aryl, aryloxy, halo, nitro, thioalkoxy, and thioaryloxy with the proviso that for such heteroaryls when there is at least one nitrogen heteroatom, there is also at least one oxygen and/or sulfur heteroatom;

b) a substituted phenyl group of formula II:

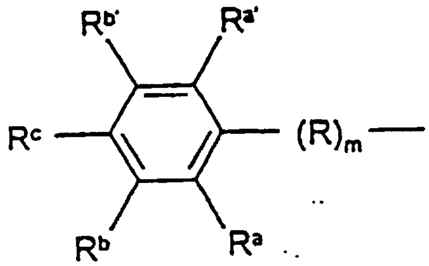

II

wherein R is alkylene of from 1 to 8 carbon atoms,

*m* is an integer equal to 0 or 1 ,

$R^a$ and $R^{a'}$ are independently selected from the group consisting of hydrogen, hydroxy, fluoro and methyl;

$R^b$ and $R^{b'}$ are independently selected from the group consisting of hydrogen, alkyl, alkoxy, aryl, cyano, cycloalkyl, halo, heteroaryl, heterocyclic, nitro, trihalomethyl, thioalkoxy, thioaryloxy, thioheteroaryloxy, and -C(O) $R^4$ where $R^4$ is selected from the group consisting of alkyl, aryl, alkoxy and aryloxy; and

$R^c$ is selected from the group consisting of hydrogen, alkyl, aryl, cyano, halo, nitro, and where $R^b$ and $R^c$ are fused to form a methylenedioxy ring with the phenyl ring; and

when $R^b$ and/or $R^{b'}$ and/or $R^c$ is fluoro, chloro, bromo and/or nitro, then $R^a$ and/or $R^{a'}$ can also be chloro; and

c) 1- or 2-naphthyl-$(R)_m$- substituted at the 5, 6, 7 and/or 8 positions with 1 to 4 substituents selected from the group consisting alkyl, alkoxy, halo, cyano, nitro, trihalomethyl, and thioalkoxy wherein R is an alkylene group of from 1 to 8 carbon atoms and *m* is an integer equal to 0 or 1;

$R^2$ is selected from the group consisting of hydrogen, alkyl, phenyl, alkylalkoxy, alkylthioalkoxy; and

$R^3$ is selected from the group consisting of $-(CH_2)_nCR^{10}R^5R^6$ wherein *n* is an integer equal to 0,1 or 2, $R^5$ and $R^6$ are independently selected from hydrogen, alkyl, alkenyl, aryl, heteroaryl, heterocyclic, $-NR^7R^8$ where $R^7$ and $R^8$ are independently hydrogen or alkyl and $-COOR^9$ where $R^9$ is alkyl, and further wherein $R^5$ and $R^6$ can be joined to form a cycloalkyl group, a cycloalkenyl group, an aryl group, a heteroaryl group, and a heterocyclic group, and when $R^5$ and $R^6$ do not join to form an aryl or heteroaryl group, then $R^{10}$ is selected from hydrogen and alkyl with the proviso that when n is zero, then $R^{10}$ is hydrogen and when *n* is greater than zero and $R^5$ and $R^6$ are joined to form an aryl or heteroaryl group, then $R^{10}$ becomes a bond within that group;

X is oxygen or sulfur;

X' is hydrogen, hydroxy or fluoro;

X" is hydrogen, hydroxy or fluoro, or X' and X" together form an oxo group, and pharmaceutically acceptable salts thereof with the provisos that:

when $R^1$ is phenyl, $R^2$ is $-CH(CH_3)CH_2CH_3$, X is oxygen, and X' and X" are hydrogen, then $R^3$ is not $-CH_2CH_3$ or $-CH_2CH(CH_3)_2$

when $R^1$ is phenyl, $R^3$ is $-CH_2CH(CH_3)_2$, X is oxygen, and X' and X" are hydrogen, then $R^2$ is not $-CH(CH_3)_2$

when $R^1$ is pyrid-3-yl, $R^2$ is ethyl, X is oxygen, and X' and X" are hydrogen, then $R^3$ is not $-CH_2CH(CH_3)_2$,

when $R^1$ is indoxazin-3-yl, 2,4-dimethylthiazol-5-yl, 4-methyl-1,2,5- thiooxadizol-3-yl or 3,5-di(trifluoromethyl) phenyl, $R^2$ is methyl, X is oxygen, and X' and X" are hydrogen, then $R^3$ is not $-CH_2CH(CH_3)_2$, and

when $R^1$ is $-CH_2$-phenyl, $R^3$ is $-CH_2CH_3$, X is oxygen, and X' and X" are hydrogen, then $R^2$ is not $-CH_2CH(CH_3)_2$.

The invention also provides the use of a compound of formula I' or a mixture of compounds of formula I' in the manufacture of a medicament comprising the compound and a pharmaceutically inert carrier for preventing the onset of Alzheimer's Disease in a patient at risk for developing Alzheimer's Disease wherein formula I' is:

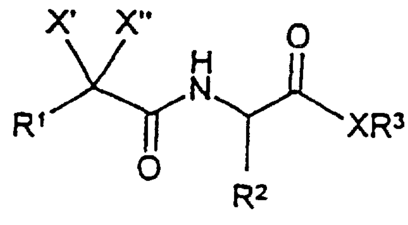

I'

wherein formula I' is as defined above.

The invention also provides the use of a compound of formula I' or a mixture of compounds of formula I' in the manufacture of a medicament comprising the compound and a pharmaceutically inert carrier for inhibiting further deterioration of the condition of a patient with Alzheimer's Disease wherein formula I' is:

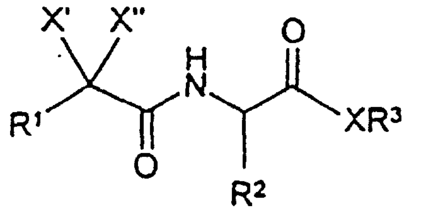

I'

wherein formula I' is as defined above.

In formula I or formula I' above, preferred $R^1$ unsubstituted aryl groups include, for example, 1-naphthyl, 2-naphthyl, and the like.

Preferred $R^1$ substituted aryl groups include, for example, monosubstituted phenyls having a single substitution at the 2, 3 or 4 positions where each of the particular subsituents is governed by the respective $R^a/R^{a'}$, $R^b/R^{b'}$ and $R^c$ groups; disubstituted phenyls which include those having two substituents at the 2,3-positions, 2,4-positions, 2,5-positions, 2,6-positions. 3,4-positions, 3,5-positions or 3,6-positions where each of these substituents is governed by the respective $R^3$, $R^{a'}$, $R^b$, $R^{b'}$ and $R^c$ groups; and trisubstituted phenyls which include those having three substituents at the 2,3,4-positions, 2,3,5-positions, 2,3,6-positions, 3,4,5-positions and 3,4,6-positions again where each of these substituents is governed by the respective $R^a$, $R^{a'}$, $R^b$, $R^{b'}$ and $R^c$ groups. Preferably, the substituted phenyl groups do not include more than 3 substituents.

Examples of substituted phenyls include, for instance, 4-fluorophenyl, 4-chlorophenyl, 4-bromophenyl, 4-nitrophenyl. 4-methylphenyl, 3-methoxy-phenyl, 3-nitrophenyl, 3-fluorophenyl, 3-chlorophenyl, 3-bromophenyl, 3-thiomethoxyphenyl, 3-methylphenyl, 3-trifluoromethylphenyl, 2-hydroxyphenyl, 2-methylphenyl, 2-fluorophenyl, 3,4-dichlorophenyl, 3,4-methylene-dioxyphenyl, 3,5-difluorophenyl, 3,5-dichlorophenyl, 2,4-dichlorophenyl, and 2,5-difluorophenyl.

Preferred $R^1$ groups represented by phenyl-R- include, by way of example, benzyl, 3-phenylethyl, 4-phenyl-*n*-propyl, and the like.

Preferred $R^1$ alkyl, alkcycloalkyl, cycloalkyl and cycloalkenyl groups include, by way of example, *sec*-butyl, cyclopropyl, cyclobutyl, cyclohexyl, cyclopentyl, cyclohex-1-enyl, $-CH_2$-cyclopropyl, $-CH_2$-cyclobutyl, $-CH_2$-cyclohexyl, $-CH_2$-cyclopentyl, $-CH_2CH_2$-cyclopropyl, $-CH_2CH_2$-cyclobutyl, $-CH_2CH_2$-cyclohexyl, $-CH_2CH_2$-cyclopentyl, and the like.

Preferred $R^1$ heteroaryls and substituted heteroaryls include, by way of example, pyrid-3-yl, pyrid-4-yl, thien-2-yl. thien-3-yl, benzothiazol-4-yl, 2-phenylbenzoxazol-5-yl, furan-2-yl, benzofuran-2-yl, benzothiophen-3-yl, 2-chlorothien-5-yl, 3-methylisoxazol-5-yl, 2-(phenylthio)thien-5-yl, 6-methoxythiophen-2-yl, 3-phenyl-1,2,4-thiooxadiazol-5-yl, 2-phenyloxazol-4-yl, and the like.

Preferably $R^2$ is selected from the group consisting of alkyl of from 1 to 4 carbon atoms, phenyl, alkylalkoxy of from 1 to 4 carbon atoms and alkylthioalkoxy of from 1 to 4 carbon atoms. Particularly preferred $R^2$ substituents include, by way of example, methyl, ethyl, *n*-propyl, *iso*-propyl, *n*-butyl, *iso*-butyl, $-CH_2CH_2SCH_3$, cyclohexyl and phenyl.

When X is oxygen, preferred $R^3$ substituents include, for example, methyl, ethyl, *iso*-propyl, *n*-propyl, *n*-butyl, *iso*-butyl, cyclopentyl, allyl, *iso*-but-2-enyl, 3-methylpentyl, $-CH_2$-cyclopropyl, $-CH_2$-cyclohexyl, $-CH_2$-(3-tetrahydrofuranyl), $-CH_2$-thien-2-yl, $-CH_2$(1-methyl)cyclopropyl, $-CH_2$-thien-3-yl, $-CH_2$-C(O)O-*t*-butyl, $-CH_2-C(CH_3)_3$, $-CH_2CH(CH_2CH_3)_2$, -2-methylcyclopentyl, -cyclohex-2-enyl, $-CH[CH(CH_3)_2]COOCH_3$, $-CH_2CH_2N(CH_3)_2$, $-CH_2C(CH_3)=CH_2$, $-CH_2CH=C(CH_3)_2$ and the like.

When X is sulfur, preferred $R^3$ substituents include, for example, *iso*-but-2-enyl and *iso*-butyl.

Particularly preferred compounds for use as a medicament, for use in the manufacture of a medicament, or in compositions of this invention include, by way of example, the following wherein the stereochemistry of the $R^2$ group (where appropriate) is preferably derived from the L-amino acid:

*N*-(phenylacetyl)alanine *iso*-butyl ester
*N*-(3-phenylpropionyl)alanine *iso*-butyl ester
*N*-(3-methylpentanoyl)alanine *iso*-butyl ester
*N*-[(4-chlorophenyl)acetyl]alanine *iso*-butyl ester

*N*-[(3,4-dichlorophenyl)acetyl]alanine *iso*-butyl ester
*N*-[(3-pyridyl)acetyl]alanine *iso*-butyl ester
*N*-[(1-naphthyl)acetyl]alanine *iso*-butyl ester
*N*-[(2-naphthyl)acetyl]alanine *iso*-butyl ester
*N*-(4-phenylbutanoyl)alanine *iso*-butyl ester
*N*-(5-phenylpentanoyl)alanine *iso*-butyl ester
*N*-[(4-pyridyl)acetyl]alanine *iso*-butyl ester
2-[(3,4-dichlorophenyl)acetamido]butyric acid *iso*-butyl ester
2-[(3-methoxyphenyl)acetamido]butyric acid *iso*-butyl ester
2-[(4-nitrophenyl)acetamido]butyric acid *iso*-butyl ester
2-[(3,4-methylenedioxyphenyl)acetamido]butyric acid *iso*-butyl ester
2-[(thien-3-yl)acetamido]butyric acid *iso*-butyl ester
2-[(4-chlorophenyl)acetamido]butyric acid *iso*-butyl ester
2-[(3-nitrophenyl)acetamido]butyric acid *iso*-butyl ester
2-[(2-hydroxyphenyl)acetamido]butyric acid *iso*-butyl ester
2-[(2-naphthyl)acetamido]butyric acid *iso*-butyl ester
2-[(2,4-dichlorophenyl)acetamido]butyric acid *iso*-butyl ester
2-[(4-bromophenyl)acetamido]butyric acid *iso*-butyl ester
2-[(3-chlorophenyl)acetamido])butyric acid *iso*-butyl ester
2-[(3-fluorophenyl)acetamido]butyric acid *iso*-butyl ester
2-[(benzothiazol-4-yl)acetamido]butyric acid *iso*-butyl ester
2-[(2-methylphenyl)acetamido]butyric acid *iso*-butyl ester
2-[(2-fluorophenyl)acetamido]butyric acid *iso*-butyl ester
2-[(4-fluorophenyl)acetamido]butyric acid *iso*-butyl ester
2-[(3-bromophenyl)acetamido]butyric acid *iso*-butyl ester
2-[(3-trifluoromethylphenyl)acetamido]butyric acid *iso*-butyl ester
2-[(2-thienyl)acetamido]butyric acid *iso*-butyl ester
2-(phenylacetamido)butyric acid *iso*-butyl ester
*N*-(phenylacetyl)valine 2-methylbutyl ester
*N*-(phenylacetyl)methionine *iso*-butyl ester
*N*-(phenylacetyl)leucine *iso*-butyl ester
*N*-[(3-chlorophenyl)acetyl]alanine 3-methylbut-2-enyl ester
*N*-[(3-chlorophenyl)acetyl]alanine cyclopropylmethyl ester
*N*-[(3-chlorophenyl)acetyl]alanine 2-thienylmethyl ester
*N*-[(3-chlorophenyl)acetyl]alanine (1-methylcyclopropyl)methyl ester
*N*-[(3-chlorophenyl)acetyl]alanine 3-thienylmethyl ester
*N*-[(3-chlorophenyl)acetyl]alanine 2-methylcyclopentyl ester
*N*-[(3-chlorophenyl)acetyl]alanine 2-methylprop-2-enyl ester
*N*-[(3-chlorophenyl)acetyl]alanine cyclohex-2-enyl ester
*N*-[(2-phenylbenzoxazol-5-yl)acetyl]alanine *iso*-butyl ester
*N*-[(3-methylthiophenyl)acetyl]alanine *iso*-butyl ester
*N*-4-[(2-furyl)acetyl]alanine *iso*-butyl ester
*N*-[(benzofuran-2-yl)acetyl]alanine *iso*-butyl ester
*N*-[(benzothiophen-3-yl)acetyl]alanine *iso*-butyl ester
*N*-[(2-chloro-5-thienyl)acetyl]alanine iso-butyl ester
*N*-[(3-methyl-isoxazol-5-yl)acetyl]alanine *iso*-butyl ester
*N*-[(2-phenylthiothienyl)acetyl]alanine *iso*-butyl ester
*N*-[(6-methoxybenzothiophen-2-yl)acetyl]alanine *iso*-butyl ester
*N*-[(3-phenyl-1,2,4-thiadiazol-5-yl)acetyl]alanine *iso*-butyl ester
*N*-[(2-phenyloxazol-4-yl)acetyl]alanine *iso*-butyl ester
*N*-[(3-methylphenyl)acetyl]alanine *iso*-butyl ester
*N*-[(2,5-difluorophenyl)acetyl]alanine iso-butyl ester
*N*-[(3,5-diflurophenyl)acetyl]alanine iso-butyl ester
*N*-[(3-thienyl)acetyl]alanine *iso*-butyl ester
*N*-[(4-methylphenyl)acetyl]alanine *iso*-butyl ester
*N*-(phenylacetyl)alanine (1-methoxycarbonyl)*iso*-butyl ester
*N*-[(3-nitrophenyl)acetyl]alanine iso-butyl ester
*N*-[(3,5-difluorophenyl)acetyl]alanine ethyl ester

*N*-[(3-nitrophenyl)acetyl]methionine ethyl ester
*N*-[(3-chlorophenyl)acetyl]alanine *iso*-butyl ester
*N*-[(3-chlorophenyl)acetyl]alanine 2-(*N*,*N*-dimethylamino)ethyl ester
2-[(3,5-dichlorophenyl)acetamido]hexanoic acid methyl ester
*N*-[(3,5-dichlorophenyl)acetyl]alanine *iso*-butyl ester
*N*-(cyclohexylacetyl)alanine *iso*-butyl ester
*N*-(cyclopentylacetyl)alanine *iso*-butyl ester
*N*-[(cyclohex-1-enyl)acetyl]alanine *iso*-butyl ester
*N*-[(3-chlorophenyl)acetyl)alanine 3-methylbut-2-enyl thioester
*N*-[(2-phenyl)-2-fluoroacetyl]alanine ethyl ester
*N*-(3,5-difluorophenylacetyl)phenylglycine methyl ester
*N*-(3,5-difluorophenylacetyl)phenylglycine *iso*-butyl ester
*N*-(cyclopentylacetyl)phenylglycine methyl ester
*N*-(cyclopentylacetyl)alanine methyl ester
*N*-(cyclopropylacetyl)phenylglycine methyl ester
*N*-(cyclopropylacetyl)alanine methyl ester
*N*-[(3-nitrophenyl)acetyl]methionine *iso*-butyl ester

Still further, this invention provides for novel compounds of the formula III:

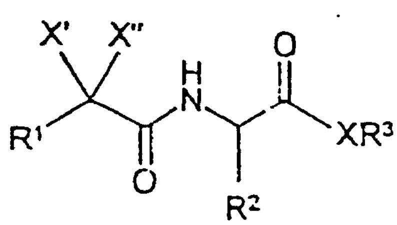

III

wherein $R^1$ is selected from the group consisting of

a) alkyl, alkenyl, alkcycloalkyl, naphthyl-$(R)_m$- wherein R is an alkylene group of from 1 to 8 carbon atoms and *m* is an integer equal to 0 or 1, cycloalkyl, cycloalkenyl, 3-pyridyl, 4-pyridyl and heteroaryl, of 3 to 10 atoms and 1 to 4 heteroatoms selected from oxygen, sulfur and nitrogen wherein the heteroaryl group is optionally substituted with 1 to 3 substituents selected from the group consisting of alkyl, alkoxy, aryl, aryloxy, halo, nitro, thioalkoxy, and thioaryloxy with the proviso that for such heteroaryls when there is at least one nitrogen heteroatom, there is also at least one oxygen and/or sulfur heteroatom;
(b) a substituted phenyl group of formula II:

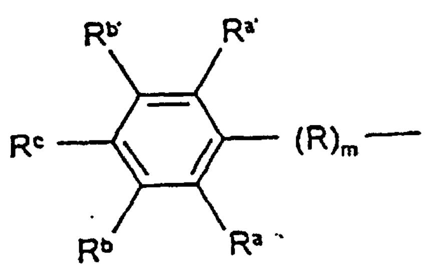

II

wherein R is alkylene of from 1 to 8 carbon atoms,
*m* is an integer equal to 0 or 1,
$R^a$ and $R^{a'}$ are independently selected from the group consisting of hydrogen, hydroxy, fluoro and methyl;
$R^b$ and $R^{b'}$ are independently selected from the group consisting of hydrogen, alkyl, alkoxy, aryl, cyano, cycloalkyl, halo, heteroaryl, heterocyclic, nitro, trihalomethyl, thioalkoxy, thioaryloxy, thioheteroaryloxy, and -C(O)

$R^4$ where $R^4$ is selected from the group consisting of alkyl, aryl, alkoxy and aryloxy; and

$R^c$ is selected from the group consisting of hydrogen, alkyl, aryl, cyano, halo, nitro, and where $R^b$ and $R^c$ are fused to form a methylenedioxy ring with the phenyl ring; and

when $R^b$ and/or $R^{b'}$ and/or $R^c$ is fluoro, chloro, bromo and/or nitro, then $R^a$ and/or $R^{a'}$ can also be chloro; and whereby $R^a$, $R^{a'}$, $R^b$, $R^{b'}$ and $R^c$ are not all simultaneously hydrogen; and

(c) 1- or 2-naphthyl-$(R)_m$- wherein R is an alkylene group of from 1 to 8 carbon atoms and *m* is an integer equal to 0 or 1 substituted at the 5, 6, 7 and/or 8 positions with 1 to 4 substituents selected from the group consisting alkyl, alkoxy, halo, cyano, nitro, trihalomethyl, and thioalkoxy;

$R^2$ is selected from the group consisting of alkyl, phenyl, alkylalkoxy, alkylthioalkoxy; and

$R^3$ is selected from the group consisting of $-(CH_2)_nCR^{10}R^5R^6$ wherein *n* is an integer equal to 0, 1 or 2, $R^5$ and $R^6$ are independently selected from hydrogen, alkyl, alkenyl, aryl, heteroaryl, heterocyclic, $-NR^7R^8$ where $R^7$ and $R^8$ are independently hydrogen or alkyl, and $-COOR^9$ where $R^9$ is alkyl, and further wherein $R^5$ and $R^6$ can be joined to form a cycloalkyl group, a cycloalkenyl group, an aryl group, a heteroaryl group, and a heterocyclic group, and when $R^5$ and $R^6$ do not join to form an aryl or heteroaryl group,

then $R^{10}$ is selected from hydrogen and alkyl with the proviso that when *n* is zero, then $R^{10}$ is hydrogen and when *n* is greater than zero and $R^5$ and $R^6$ are joined to form an aryl or heteroaryl group, then $R^{10}$ becomes a bond within that group;

X is oxygen or sulfur;

X' is hydrogen, hydroxy or fluoro;

X" is hydrogen, hydroxy or fluoro, or X' and X" together form an oxo group, and

pharmaceutically acceptable salts thereof

with the provisos that:

when $R^1$ is pyrid-3-yl, $R^2$ is ethyl, X is oxygen, and X' and X" are hydrogen, then $R^3$ is not $-CH_2CH(CH_3)_2$,

when $R^1$ is butyl, $R^2$ is methyl, X is oxygen, and X' and X" are hydrogen, then $R^3$ is not ethyl,

when $R^1$ is methyl, $-CH(CH_3)_2$, or cyclohexyl, $R^2$ is $-CH(CH_3)_2$, X is oxygen, X' is hydrogen and X" is hydroxyl then $R^3$ is not methyl,

when $R^1$ is ethyl or $C_6H_4Cl$-*p*, $R^2$ is phenyl, methyl or $-CH(CH_3)_2$, X is oxygen, X' is hydrogen and X" is fluorine, then $R^3$ is not methyl, and

when $R^1$ is indoxazin-3-yl, 2,4-dimethylthiazol-5-yl, 4-methyl-1,2,5-thiooxadizol-3-yl or 3,5-di(trifluoromethyl) phenyl, $R_2$ is methyl, X is oxygen, and X' and X" are hydrogen, then $R^3$ is not $-CH_2CH(CH_3)_2$.

Preferred compounds of formula III above include those set forth in Formula IV below:

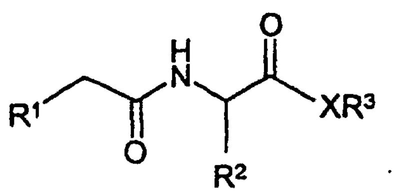

IV

| $R^1$ | $R^2$ | $R^3$ | X |
|---|---|---|---|
| | | | |
| | | | |
| $-CH(CH_3)CH_2CH_3$ | $-CH_3$ | $-CH_2CH(CH_3)_2$ | O |
| 4-Cl-$\phi$- | $-CH_3$ | $-CH_2CH(CH_3)_2$ | O |
| 3,4-Cl-$\phi$- | $-CH_3$ | $-CH_2CH(CH_3)_2$ | O |
| pyrid-3-yl | $-CH_3$ | $-CH_2CH(CH_3)_2$ | O |
| 1-naphthyl | $-CH_3$ | $-CH_2CH(CH_3)_2$ | O |
| 2-naphthyl | $-CH_3$ | $-CH_2CH(CH_3)_2$ | O |
| $-(CH_2)_2-\phi$ | $-CH_3$ | $-CH_2CH(CH_3)_2$ | O |
| $-(CH_2)_3-\phi$ | $-CH_3$ | $-CH_2CH(CH_3)_2$ | O |
| pyrid-4-yl | $-CH_3$ | $-CH_2CH(CH_3)_2$ | O |
| 3,4-di-Cl-$\phi$- | $-CH_2CH_3$ | $-CH_2CH(CH_3)_2$ | O |
| $3-CH_3O-\phi$- | $-CH_2CH_3$ | $-CH_2CH(CH_3)_2$ | O |
| $4-NO_2-\phi$- | $-CH_2CH_3$ | $-CH_2CH(CH_3)_2$ | O |
| 3,4-methylene-dioxyphenyl- | $-CH_2CH_3$ | $-CH_2CH(CH_3)_2$ | O |

| $R^1$ | $R^2$ | $R^3$ | X |
|---|---|---|---|
| thien-3-yl | $-CH_2CH_3$ | $-CH_2CH(CH_3)_2$ | O |
| 4-Cl-$\phi$- | $-CH_2CH_3$ | $-CH_2CH(CH_3)_2$ | O |
| 3-$NO_2$-$\phi$- | $-CH_2CH_3$ | $-CH_2CH(CH_3)_2$ | O |
| 2-HO-$\phi$- | $-CH_2CH_3$ | $-CH_2CH(CH_3)_2$ | O |
| 2-naphthyl | $-CH_2CH_3$ | $-CH_2CH(CH_3)_2$ | O |
| 2,4-di-Cl-$\phi$- | $-CH_2CH_3$ | $-CH_2CH(CH_3)_2$ | O |
| 4-Br-$\phi$- | $-CH_2CH_3$ | $-CH_2CH(CH_3)_2$ | O |
| 3-Cl-$\phi$- | $-CH_2CH_3$ | $-CH_2CH(CH_3)_2$ | O |
| 3-F-$\phi$- | $-CH_2CH_3$ | $-CH_2CH(CH_3)_2$ | O |
| benzothiazol-4-yl | $-CH_2CH_3$ | $-CH_2CH(CH_3)_2$ | O |
| 2-$CH_3$-$\phi$- | $-CH_2CH_3$ | $-CH_2CH(CH_3)_2$ | O |
| 2-F-$\phi$- | $-CH_2CH_3$ | $-CH_2CH(CH_3)_2$ | O |
| 4-F-$\phi$- | $-CH_2CH_3$ | $-CH_2CH(CH_3)_2$ | O |
| 3-Br-$\phi$- | $-CH_2CH_3$ | $-CH_2CH(CH_3)_2$ | O |
| 3-$CF_3$-$\phi$- | $-CH_2CH_3$ | $-CH_2CH(CH_3)_2$ | O |
| thien-2-yl | $-CH_2CH_3$ | $-CH_2CH(CH_3)_2$ | O |
| | | | |
| | | | |
| | | | |
| | | | |
| 3-Cl-$\phi$- | $-CH_3$ | $-CH_2CH=C(CH_3)_2$ | O |
| 3-Cl-$\phi$- | $-CH_3$ | $-CH_2$-cyclopropyl | O |
| 3-Cl-$\phi$- | $-CH_3$ | $-CH_2$-2-thienyl | O |
| 3-Cl-$\phi$- | $-CH_3$ | 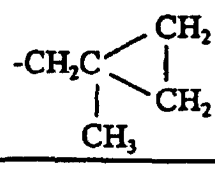 | O |
| 3-Cl-$\phi$- | $-CH_3$ | $-CH_2$-3-thienyl | O |

| $R^1$ | $R^2$ | $R^3$ | X |
|---|---|---|---|
| 3-Cl-ϕ- | $-CH_3$ | -(2-$CH_3$-cyclopentyl) | O |
| 3-Cl-ϕ- | $-CH_3$ | $-CH_2C=CH_2$<br>\|<br>$CH_3$ | O |
| 3-Cl-ϕ- | $-CH_3$ | -cyclohex-2-enyl | O |
| 2-phenyl-benzoxazol-5-yl | $-CH_3$ | $-CH_2CH(CH_3)_2$ | O |
| 3-$CH_3$S-ϕ- | $-CH_3$ | $-CH_2CH(CH_3)_2$ | O |
| furan-2-yl | $-CH_3$ | $-CH_2CH(CH_3)_2$ | O |
| benzofuran-2-yl | $-CH_3$ | $-CH_2CH(CH_3)_2$ | O |
| benzothien-3-yl | $-CH_3$ | $-CH_2CH(CH_3)_2$ | O |
| 2-chloro-thien-5-yl | $-CH_3$ | $-CH_2CH(CH_3)_2$ | O |
| 3-methyl-isoxazol-5-yl | $-CH_3$ | $-CH_2CH(CH_3)_2$ | O |
| 2-ϕ-S-thien-5-yl | $-CH_3$ | $-CH_2CH(CH_3)_2$ | O |
| 6-$CH_3$O-benzothiophen-2-yl | $-CH_3$ | $-CH_2CH(CH_3)_2$ | O |
| 3-phenyl-1,2,4-thiadiazol-5-yl | $-CH_3$ | $-CH_2CH(CH_3)_2$ | O |
| 2-ϕ-oxazol-4-yl | $-CH_3$ | $-CH_2CH(CH_3)_2$ | O |
| 3-$CH_3$-ϕ- | $-CH_3$ | $-CH_2CH(CH_3)_2$ | O |
| 2,5-di-F-ϕ- | $-CH_3$ | $-CH_2CH(CH_3)_2$ | O |
| 3,5-di-F-ϕ- | $-CH_3$ | $-CH_2CH(CH_3)_2$ | O |
| thien-3-yl | $-CH_3$ | $-CH_2CH(CH_3)_2$ | O |
| 4-$CH_3$-ϕ- | $-CH_3$ | $-CH_2CH(CH_3)_2$ | O |
| | | | |
| 3-$NO_2$-ϕ- | $-CH_3$ | $-CH_2CH(CH_3)_2$ | O |

| $R^1$ | $R^2$ | $R^3$ | X |
|---|---|---|---|
| 3,5-di-F-ϕ | $-CH_3$ | $-CH_2CH_3$ | O |
| 3-$NO_2$-ϕ- | $-CH_2CH_2SCH_3$ | $-CH_2CH_3$ | O |
| 3-Cl-ϕ- | $-CH_3$ | $-CH_2CH(CH_3)_2$ | O |
| 3-Cl-ϕ- | $-CH_3$ | $-CH_2CH_2N(CH_3)_2$ | O |
| 3,5-di-Cl-ϕ- | $-CH_2CH_2CH_2CH_3$ | $-CH_3$ | O |
| 3,5-di-Cl-ϕ- | $-CH_3$ | $-CH_2CH(CH_3)_2$ | O |
| cyclohexyl | $-CH_3$ | $-CH_2CH(CH_3)_2$ | O |
| cyclopentyl | $-CH_3$ | $-CH_2CH(CH_3)_2$ | O |
| cyclohex-1-enyl | $-CH_3$ | $-CH_2CH(CH_3)_2$ | O |
| 3-Cl-ϕ- | $-CH_3$ | $-CH_2CH{=}C(CH_3)_2$ | S |
| 3,5-di-F-ϕ- | -ϕ | $-CH_3$ | O |
| 3,5-diF-ϕ | -ϕ | $-CH_2CH(CH_3)_2$ | O |
| cyclopentyl | -ϕ | $-CH_3$ | O |
| cyclopentyl | $-CH_3$ | $-CH_3$ | O |
| cyclopropyl | -ϕ | $-CH_3$ | O |
| cyclopropyl | $-CH_3$ | $-CH_3$ | O |
| 3-$NO_2$-ϕ | $-CH_2CH_2SCH_3$ | $-CH_2CH(CH_3)_2$ | O |

The invention also provides a compound selected from N-(phenylacetyl) alanine *iso*-butyl ester, N-(3-phenylpropionyl) alanine *iso*-butyl ester, 2-(phenylacetamido) butyric acid *iso*-butyl ester, N-(phenylacetyl) valine 2-methylbutyl ester, N-(phenylacetyl) methionine *iso*-butyl ester, N-(phenylacetyl) leucine *iso*-butyl ester, N-(phenylacetyl) alanine (1-methoxycarbonyl)*iso*-butyl ester and N-[(2-phenyl)-2-fluoroacetyl] alanine ethyl ester, those compounds for use as a medicament, and pharmaceutical compositions comprising a pharmaceutically inert carrier and a pharmaceutically effective amount of those compounds.

## DETAILED DESCRIPTION OF THE INVENTION

As above, this invention relates to compounds which inhibit β-amyloid peptide release and/or its synthesis, and, accordingly, have utility in treating Alzheimer's disease. However, prior to describing this invention in further detail, the following terms will first be defined.

Definitions

The term "β-amyloid peptide" refers to a 39-43 amino acid peptide having a molecular weight of about 4.2 kD, which peptide is substantially homologous to the form of the protein described by Glenner, et al.[1] including mutations and post-translational modifications of the normal β-amyloid peptide. In whatever form, the β-amyloid peptide is approximately a 39-43 amino acid fragment of a large membrane-spanning glycoprotein, referred to as the β-amyloid

precursor protein (APP). Its 43-amino acid sequence is:

1

Asp Ala Glu Phe Arg His Asp Ser Gly Tyr

11

Glu Val His His Gln Lys Leu Val Phe Phe

21

Ala Glu Asp Val Gly Ser Asn Lys Gly Ala

31

Ile Ile Gly Leu Met Val Gly Gly Val Val

41

Ile Ala Thr (SEQ ID NO: 1)

or a sequence which is substantially homologous thereto.

"Alkyl" refers to monovalent alkyl groups preferably having from 1 to 10 carbon atoms and more preferably 1 to 6 carbon atoms. This term is exemplified by groups such as methyl, ethyl, *n*-propyl, *iso*-propyl, *n*-butyl, *iso*-butyl, *tert*-butyl, *n*-hexyl, and the like.

"Alkylene" refers to divalent alkylene groups preferably having from 1 to 8 carbon atoms and more preferably 1 to 6 carbon atoms. This term is exemplified by groups such as methylene ($-CH_2-$), ethylene ($-CH_2CH_2-$), the propylene isomers (e.g., $-CH_2CH_2CH_2-$ and $-CH(CH_3)CH_2-$) and the like.

"Alkoxy" refers to the group "alkyl-O-" wherein alkyl is as defined herein. Preferred alkoxy groups include, by way of example, methoxy, ethoxy, *n*-propoxy, *iso*-propoxy, *n*-butoxy, *tert*-butoxy, *sec*-butoxy, *n*-pentoxy, *n*-hexoxy, 1,2-dimethylbutoxy, and the like.

"Alkylalkoxy" refers to the group "-alkylene-O-alkyl" wherein alkylene and alkyl are as defined herein. Such groups include, by way of example, methylenemethoxy ($-CH_2OCH_3$), ethylenemethoxy ($-CH_2CH_2OCH_3$), *n*-propylene-*iso*-propoxy ($-CH_2CH_2CH_2OCH(CH_3)_2$), methylene-*tert*-butoxy ($-CH_2-O-C(CH_3)_3$) and the like.

"Alkylthioalkoxy" refers to the group "-alkylene-S-alkyl" wherein alkylene and alkyl are as defined herein. Such groups include, by way of example, methylenethiomethoxy ($-CH_2SCH_3$), ethylenethiomethoxy ($-CH_2CH_2SCH_3$), *n*-propylene-*iso*-thiopropoxy ($-CH_2CH_2CH_2SCH(CH_3)_2$), methylene-*tert*-thiobutoxy ($-CH_2SC(CH_3)_3$) and the like.

"Alkenyl" refers to alkenyl groups preferably having from 2 to 10 carbon atoms and more preferably 2 to 6 carbon atoms and having at least 1 and preferably from 1-2 sites of alkenyl unsaturation. Preferred alkenyl groups include ethenyl ($-CH=CH_2$), *n*-propenyl ($-CH_2CH=CH_2$), *iso*-propenyl ($-C(CH_3)=CH_2$), and the like.

"Alkynyl" refers to alkynyl groups preferably having from 2 to 10 carbon atoms and more preferably 2 to 6 carbon atoms and having at least 1 and preferably from 1-2 sites of alkynyl unsaturation. Preferred alkynyl groups include ethynyl ($-C\equiv CH$), propargyl ($-CH_2C\equiv CH$) and the like.

"Acyl" refers to the groups alkyl-C(O)-, aryl-C(O)-, and heteroaryl-C(O)-where alkyl, aryl and heteroaryl are as defined herein.

"Acylamino" refers to the group -C(O)NRR where each R is independently hydrogen or alkyl.

"Alkcycloalkyl" refers to the group -alkylene-cycloalkyl wherein alkylene and cycloalkyl are as defined herein.

"Aminoacyl" refers to the group -NRC(O)R where each R is independently hydrogen or alkyl.

"Acyloxy" refers to the groups alkyl-C(O)O-, aryl-C(O)O-, heteroaryl-C(O)O-, and heterocyclic-C(O)O- where alkyl, aryl, heteroaryl and heterocyclic are as defined herein.

"Aryl" refers to an unsaturated aromatic carbocyclic group of from 6 to 14 carbon atoms having a single ring (e.g., phenyl) or multiple condensed rings (e.g., naphthyl or anthryl). Preferred aryls include phenyl, naphthyl and the

like.

Unless otherwise constrained by the definition for the aryl substituent, such aryl groups can optionally be substituted with from 1 to 3 substituents selected from the group consisting of hydroxy, acyl, acyloxy, alkyl, alkoxy, alkenyl, alkynyl, amino, aminoacyl, aryl, aryloxy, carboxyl, carboxylalkyl, acylamino, cyano, halo, nitro, heteroaryl, trihalomethyl, thioalkoxy, and the like. Preferred substituents include alkyl, alkoxy, halo, cyano, nitro, trihalomethyl, and thioalkoxy.

"Aryloxy" refers to the group aryl-O- wherein the aryl group is as defined above including optionally substituted aryl groups as also defined above.

"Carboxylalkyl" refers to the group -C(O)O-alkyl where alkyl is as defined herein.

"Cycloalkyl" refers to cyclic alkyl groups of from 3 to 10 carbon atoms having a single cyclic ring or multiple condensed rings which can be optionally substituted with from 1 to 3 alkyl groups. Such cycloalkyl groups include, by way of example, single ring structures such as cyclopropyl, cyclobutyl, cyclopentyl, cyclooctyl, 1-methylcyclopropyl, 2-methylcyclopentyl, 2-methylcyclooctyl, and the like, or multiple ring structures such as adamantanyl, and the like.

"Cycloalkenyl" refers to cyclic alkenyl groups of from 4 to 8 carbon atoms having a single cyclic ring and at least one point of internal unsaturation which can be optionally substituted with from 1 to 3 alkyl groups. Examples of suitable cycloalkenyl groups include, for instance, cyclobut-2-enyl, cyclopent-3-enyl, cyclooct-3-enyl and the like.

"Halo" or "halogen" refers to fluoro, chloro, bromo and iodo and preferably is either fluoro or chloro.

"Heteroaryl" refers to a monovalent aromatic group of from 2 to 8 carbon atoms and 1 to 4 heteroatoms selected from oxygen, nitrogen and sulfur within the ring.

Unless otherwise constrained by the definition for the heteroaryl substituent, such heteroaryl groups can be optionally substituted with 1 to 3 substituents selected from the group consisting of alkyl, alkoxy, aryl, aryloxy, halo, nitro, heteroaryl, thioalkoxy, thioaryloxy. Such heteroaryl groups can have a single ring (e.g., pyridyl or furyl) or multiple condensed rings (e.g.. indolizinyl or benzothienyl). Preferred heteroaryls include pyridyl and furyl.

"Heterocycle" or "heterocyclic" refers to a monovalent (i.e. one point of attachment) saturated or unsaturated group having a single ring or multiple condensed rings, from 1 to 8 carbon atoms and from to 4 hetero atoms selected from nitrogen, sulfur or oxygen within the ring.

Unless otherwise constrained by the definition for the heterocyclic substituent, such heterocyclic groups can be optionally substituted with 1 to 3 substituents selected from the group consisting of alkyl, alkoxy, aryl, aryloxy, halo, nitro, heteroaryl, thioalkoxy, thioaryloxy and the like. Such heterocyclic groups can have a single ring (e.g., piperidinyl or tetrahydrofuryl) or multiple condensed rings (e.g., indolinyl, dihydrobenzofuran or quinuclidinyl). Preferred heterocycles include piperidinyl, pyrrolidinyl and tetrahydrofuryl.

Examples of heterocycles and heteroaryls include, but are not limited to, furan, thiophene, thiazole, oxazole, benzothiazole, benzofuran, benzothiophene, pyrrole, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, pyrrolidine, indoline and the like.

"Thiol" refers to the group -SH.

"Thioalkoxy" refers to the groups -S-alkyl where alkyl is as defined herein.

"Thioaryloxy" refers to the group aryl-S- wherein the aryl group is as defined above including optionally substituted aryl groups as also defined above.

"Thioheteroaryloxy" refers to the group heteroaryl-S- wherein the heteroaryl group is as defined above including optionally substituted aryl groups as also defined above.

"Pharmaceutically acceptable salt" refers to pharmaceutically acceptable salts of a compound of Formula I which salts are derived from a variety of organic and inorganic counter ions well known in the art and include, by way of example only, sodium, potassium, calcium, magnesium, ammonium, tetraalkylammonium, and the like; and when the molecule contains a basic functionality, salts of organic or inorganic acids, such as hydrochloride, hydrobromide, tartrate, mesylate, acetate, maleate, oxalate and the like.

## Compound Preparation

The compounds of formula I above are readily prepared via several divergent synthetic routes with the particular route selected relative to the ease of compound preparation, the commercial availability of starting materials, and the like.

A first synthetic method involves conventional coupling of an acetic acid derivative with a primary amine of an esterified amino acid as shown in reaction (1):

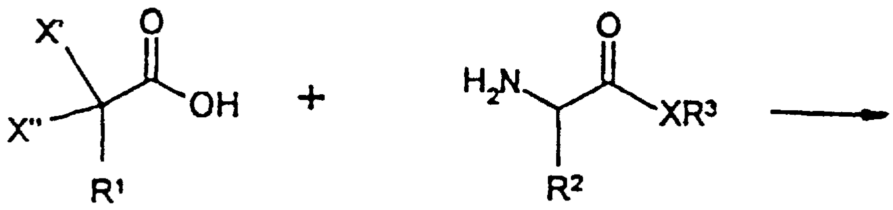

1 2 (1)

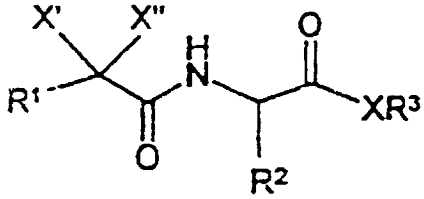

3

wherein $R^1$, $R^2$, $R^3$, X, X' and X" are as defined above.

Reaction (1) merely involves coupling of a suitable acetic acid derivative 1 with the primary amine of amino acid ester 2 under conditions which provide for the N-acetyl derivative 3. This reaction is conventionally conducted for peptide synthesis and synthetic methods used therein can also be employed to prepare the N-acetyl amino acid esters 3 of this invention. For example, well known coupling reagents such as carbodiimides or BOP (benzotriazol-1-yloxy-tris(dimethylamino)phosphonium hexafluorophosphate) with or without the use of well known additives such as N-hydroxysuccinimide, 1-hydroxy-benzotriazole, etc. can be used to facilitate coupling. The reaction is conventionally conducted in an inert aprotic diluent such as dimethylformamide, dichloromethane, chloroform, acetonitrile, tetrahydrofuran and the like. Alternatively, the acid halide of compound 1 can be employed in reaction (1) and, when so employed, it is typically employed in the presence of a suitable base to scavenge the acid generated during the reaction. Suitable bases include, by way of example, triethylamine, diisopropylethylamine, N-methylmorpholine and the like.

Reaction (1) is preferably conducted at from about 0°C to about 60°C until reaction completion which typically occurs within 1 to about 24 hours. Upon reaction completion, N-acetyl amino acid ester **3** is recovered by conventional methods including precipitation, chromatography, filtration and the like.

In reaction (1), each of the reagents (acetic acid derivative **1** and amino acid ester **2**) are well known in the art with a plurality of each being commercially available.

Alternatively, the synthesis described above in reaction (1) can be conducted on the amino acid ($XR^3$ = OH) and subsequent to N-acetyl formation as described above, the carboxylic acid is then esterified with either the alcohol ($HOR^3$) or the thioalcohol ($HSR^3$) under conventional conditions to provide for the N-acetyl amino acid ester **3** which is a compound of formula I. For example, esterification procedures for $R^3$ groups containing an ester group can be achieved by using the methods of Losse, et al.[10]

In still a further embodiment, conventional transesterification techniques can be used to prepare a variety of different ester groups on the N-acetyl amino acid esters **3**. Numerous techniques are known in the art to effect transesterification and each technique merely replaces the $-OR^3$ group on the ester of the N-acetyl amino acid ester **3** with a different $-OR^3$/$-SR^3$ group derived from the corresponding alcohol (i.e., $HOR^3$) or thioalcohol (i.e., $HSR^3$) and, in some cases, a catalyst such as titanium (IV) *iso*-propoxide is used to facilitate reaction completion. In one technique, the alcohol $HOR^3$ or thioalcohol $HSR^3$ is first treated with sodium hydride in a suitable diluent such as toluene to form the corresponding sodium alkoxide or thioalkoxide which is then employed to effect transesterification with the N-acetyl amino acid ester **3.** The efficiency of this technique makes it particularly useful with high boiling and/or expensive alcohols.

In another transesterification technique, the N-acetyl amino acid ester **3** to be transesterified is placed in a large excess of the alcohol or thioalcohol which effects transesterification. A catalytic amount of sodium hydride is then added and the reaction proceeds quickly under conventional conditions to provide the desired transesterified product. Because this protocol requires the use of a large excess of alcohol or thioalcohol, this procedure is particularly useful

when the alcohol is inexpensive.

Transesterification provides a facile means to provide for a multiplicity of $R^3$ substituents on the compounds of formula I above. In all cases, the alcohols and thioalcohols employed to effect transesterification are well known in the art with a significant number being commercially available.

Other methods for preparing the esters of this invention include, by way of example, first hydrolyzing the ester to the free acid followed by O-alkylation with a halo-$R^3$ group in the presence of a base such as potassium carbonate.

The compounds described herein can also be prepared by use of polymer supported forms of carbodiimide peptide coupling reagents. A polymer supported form of EDC, for example, has been described (*Tetrahedron Letters*, **34**(48), 7685 (1993))[15]. Additionally, a new carbodiimide coupling reagent, PEPC, and its corresponding polymer supported forms have been discovered and are very useful for the preparation of the compounds of the present invention.

Polymers suitable for use in making a polymer supported coupling reagent are either commercially available or may be prepared by methods well known to the artisan skilled in the polymer arts. A suitable polymer must possess pendant sidechains bearing moieties reactive with the terminal amine of the carbodiimide. Such reactive moieties include chloro, bromo, iodo and methanesulfonyl. Preferably, the reactive moiety is a chloromethyl group. Additionally, the polymer's backbone must be inert to both the carbodiimide and reaction conditions under which the ultimate polymer bound coupling reagents will be used.

Certain hydroxymethylated resins may be converted into chloromethylated resins useful for the preparation of polymer supported coupling reagents. Examples of these hydroxylated resins include the 4-hydroxymethyl-phenylacetamidomethyl resin (Pam Resin) and 4-benzyloxybenzyl alcohol resin (Wang Resin) available from Advanced Chemtech of Louisville. Kentucky, USA (see Advanced Chemtech 1993-1994 catalog, page 115). The hydroxymethyl groups of these resins may be converted into the desired chloromethyl groups by any of a number of methods well known to the skilled artisan.

Preferred resins are the chloromethylated styrene/divinylbenzene resins because of their ready commercial availability. As the name suggests, these resins are already chloromethylated and require no chemical modification prior to use. These resins are commercially known as Merrifield's resins and are available from Aldrich Chemical Company of Milwaukee, Wisconsin, USA (see Aldrich 1994-1995 catalog, page 899). Methods for the preparation of PEPC and its polymer supported forms are outlined in the following scheme.

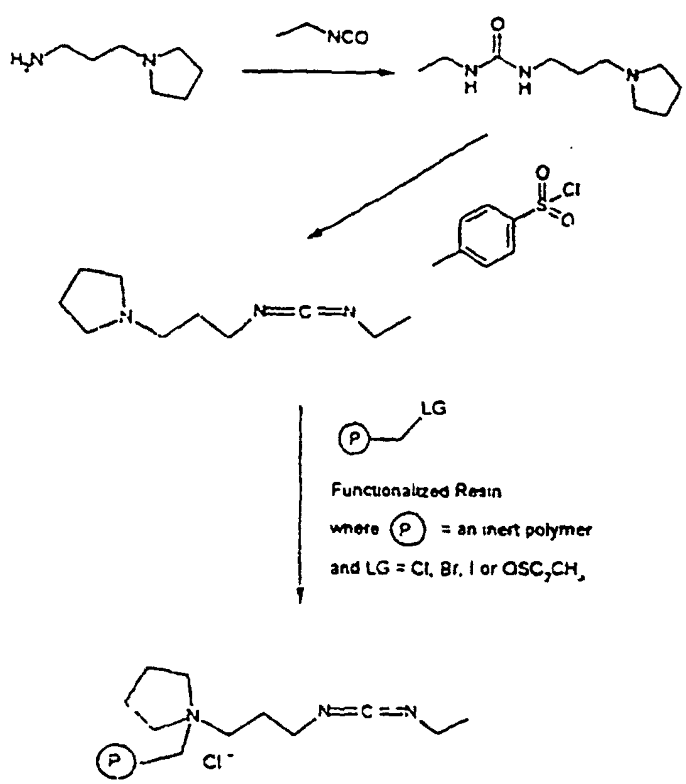


Such methods are described more fully in U.S. Provisional Patent Application 60/019,790, filed June 14, 1996, the disclosure of which is incorporated herein by reference in its entirety. Briefly PEPC is prepared by first reacting ethyl isocyanate with 1-(3-aminopropyl)pyrrolidine. The resulting urea is treated with 4-toluenesulfonyl chloride to provide PEPC. The polymer supported form is prepared by reaction of PEPC with an appropriate resin under standard conditions to give the desired reagent.

The carboxylic acid coupling reactions employing these reagents are performed at about ambient temperature to about 45°C, for from about 3 to 120 hours. Typically, the product may be isolated by washing the reaction with $CHCl_3$ and concentrating the remaining organics under reduced pressure. As discussed *supra*, isolation of products from reactions where a polymer bound reagent has been used is greatly simplified, requiring only filtration of the reaction mixture and then concentration of the filtrate under reduced pressure.

Still other methods for the preparation of esters are provided in the examples below.

In these synthetic methods, the starting materials can contain a chiral center (e.g., alanine) and, when a racemic starting material is employed, the resulting product is a mixture of R,S enantiomers. Alternatively, a chiral isomer of the starting material can be employed and, if the reaction protocol employed does not racemize this starting material, a chiral product is obtained. Such reaction protocols can involve inversion of the chiral center during synthesis.

Accordingly, unless otherwise indicated, the products of this invention are a mixture of R,S enantiomers. Preferably, however, when a chiral product is desired, the chiral product corresponds to the L-amino acid derivative. Alternatively, chiral products can be obtained via purification techniques which separate enantiomers from a R,S mixture to provide for one or the other stereoisomer. Such techniques are well known in the art.

## Pharmaceutical Formulations

When employed as pharmaceuticals, the compounds of formula I are usually administered in the form of pharmaceutical compositions. These compounds can be administered by a variety of routes including oral, rectal, transdermal, subcutaneous, intravenous, intramuscular, and intranasal. These compounds are effective as both injectable and oral compositions. Such compositions are prepared in a manner well known in the pharmaceutical art and comprise at least one active compound.

This invention also includes pharmaceutical compositions which contain, as the active ingredient, one or more of the compounds of formula I above associated with pharmaceutically acceptable carriers. In making the compositions of this invention, the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from about 5 to about 100 mg, more usually about 10 to about 30 mg, of the active ingredient. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Preferably, the compound of formula I above is employed at no more than about 20 weight percent of the pharmaceutical composition, more preferably no more than about 15 weight percent, with the balance being pharmaceutically inert carrier(s).

The active compound is effective over a wide dosage range and is generally administered in a pharmaceutically effective amount. It, will be understood, however, that the amount of the compound actually administered will be determined by a physician, in the light of the relevant circumstances, including the condition to be treated, the chosen route of administration, the actual compound administered, the age, weight, and response of the individual patient, the severity of the patient's symptoms, and the like.

For preparing solid compositions such as tablets, the principal active ingredient is mixed with a pharmaceutical excipient to form a solid preformulation composition containing a homogeneous mixture of a compound of the present

invention. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation is then subdivided into unit dosage forms of the type described above containing from, for example, 0.1 to about 500 mg of the active ingredient of the present invention.

The tablets or pills of the present invention may be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as corn oil, cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra.* Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices which deliver the formulation in an appropriate manner.

The following formulation examples illustrate representative pharmaceutical compositions of the present invention.

Formulation Example 1

Hard gelatin capsules containing the following ingredients are prepared:

| Ingredient | Quantity (mg/capsule) |
|---|---|
| Active Ingredient | 30.0 |
| Starch | 305.0 |
| Magnesium stearate | 5.0 |

The above ingredients are mixed and filled into hard gelatin capsules in 340 mg quantities.

Formulation Example 2

A tablet formula is prepared using the ingredients below:

| Ingredient | Quantity (mg/tablet) |
|---|---|
| Active Ingredient | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

Formulation Example 3

A dry powder inhaler formulation is prepared containing the following components:

| Ingredient | Weight % |
| --- | --- |
| Active Ingredient | 5 |
| Lactose | 95 |

The active ingredient is mixed with the lactose and the mixture is added to a dry powder inhaling appliance.

Formulation Example 4

Tablets, each containing 30 mg of active ingredient, are prepared as follows:

| Ingredient | Quantity (mg/tablet) |
| --- | --- |
| Active Ingredient | 30.0 mg |
| Starch | 45.0 mg |
| Microcrystalline cellulose | 35.0 mg |
| Polyvinylpyrrolidone (as 10% solution in sterile water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U. S. sieve. The granules so produced are dried at 50° to 60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 120 mg.

Formulation Example 5

Capsules, each containing 40 mg of medicament are made as follows:

| Ingredient | Quantity (mg/capsule) |
| --- | --- |
| Active Ingredient | 40.0 mg |
| Starch | 109.0 mg |
| Magnesium stearate | 1.0 mg |
| Total | 150.0 mg |

The active ingredient, starch and magnesium stearate are blended, passed through a No. 20 mesh U.S. sieve, and filled into hard gelatin capsules in 150 mg quantities.

Formulation Example 6

Suppositories, each containing 25 mg of active ingredient are made as follows:

| Ingredient | Amount |
| --- | --- |
| Active Ingredient | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

Formulation Example 7

Suspensions, each containing 50 mg of medicament per 5.0 mL dose are made as follows:

| Ingredient | Amount |
|---|---|
| Active Ingredient | 50.0 mg |
| Xanthan gum | 4.0 mg |
| Sodium carboxymethyl cellulose (11 %) Microcrystalline cellulose (89%) | 50.0 mg |
| Sucrose | 1.75 g |
| Sodium benzoate | 10.0 mg |
| Flavor and Color | q.v. |
| Purified water to | 5.0 mL |

The active ingredient, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Formulation Example 8

| Ingredient | Quantity (mg/capsule) |
|---|---|
| Active Ingredient | 15.0 mg |
| Starch | 407.0 mg |
| Magnesium stearate | 3.0 mg |
| Total | 425.0 mg |

The active ingredient, starch, and magnesium stearate are blended, passed through a No. 20 mesh U.S. sieve, and filled into hard gelatin capsules in 425.0 mg quantities.

Formulation Example 9

A subcutaneous formulation may be prepared as follows:

| Ingredient | Quantity |
|---|---|
| Active Ingredient | 5.0 mg |
| Corn Oil | 1.0 mL |

Formulation Example 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See, e.g., U.S. Patent 5,023,252, issued June 11, 1991, herein incorporated by reference. Such patches may be constructed for continuous, pulsatile, or on demand delivery of pharmaceutical agents.

Frequently, it will be desirable or necessary to introduce the pharmaceutical composition to the brain, either directly or indirectly. Direct techniques usually involve placement of a drug delivery catheter into the host's ventricular system to bypass the blood-brain barrier. One such implantable delivery system used for the transport of biological factors to specific anatomical regions of the body is described in U.S. Patent 5,011,472 which is herein incorporated by reference.

Indirect techniques, which are generally preferred, usually involve formulating the compositions to provide for drug latentiation by the conversion of hydrophilic drugs into lipid-soluble drugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

Other suitable formulations for use in the present invention can be found in *Remington's Pharmaceutical Sciences,* Mace Publishing Company, Philadelphia, PA, 17th ed. (1985).

Utility

The compounds and pharmaceutical compositions of the invention are useful in inhibiting β-amyloid peptide release and/or its synthesis, and, accordingly, have utility in treating Alzheimer's disease in mammals including humans.

As noted above, the compounds described herein are suitable for use in a variety of drug delivery systems described above. Additionally, in order to enhance the *in vivo* serum half-life of the administered compound, the compounds may be encapsulated, introduced into the lumen of liposomes, prepared as a colloid, or other conventional techniques may be employed which provide an extended serum half-life of the compounds. A variety of methods are available for preparing liposomes, as described in, e.g., Szoka, et al., U.S. Patent Nos. 4,235,871, 4,501,728 and 4,837,028 each of which is incorporated herein by reference.

The amount of compound administered to the patient will vary depending upon what is being administered, the purpose of the administration, such as prophylaxis or therapy, the state of the patient, the manner of administration, and the like. In therapeutic applications, compositions are administered to a patient already suffering from AD in an amount sufficient to at least partially arrest further onset of the symptoms of the disease and its complications. An amount adequate to accomplish this is defined as "therapeutically effective dose." Amounts effective for this use will depend on the judgment of the attending clinician depending upon factors such as the degree or severity of AD in the patient, the age, weight and general condition of the patient, and the like. Preferably, for use as therapeutics, the compounds described herein are administered at dosages ranging from about 0.1 to about 500 mg/kg/day.

In prophylactic applications, compositions are administered to a patient at risk of developing AD (determined for example by genetic screening or familial trait) in an amount sufficient to inhibit the onset of symptoms of the disease. An amount adequate to accomplish this is defined as "prophylactically effective dose." Amounts effective for this use will depend on the judgment of the attending clinician depending upon factors such as the age, weight and general condition of the patient, and the like. Preferably, for use as prophylactics, the compounds described herein are administered at dosages ranging from about 0.1 to about 500 mg/kg/day.

As noted above, the compounds administered to a patient are in the form of pharmaceutical compositions described above. These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the compound preparations typically will be between 3 and 11, more preferably from 5 to 9 and most preferably from 7 and 8. It will be understood that use of certain of the foregoing excipients, carriers, or stabilizers will result in the formation of pharmaceutical salts.

The following synthetic and biological examples are offered to illustrate this invention and are not to be construed in any way as limiting the scope of this invention.

## EXAMPLES

In the discussion above and in the examples below, the following abbreviations have the following meanings. If an abbreviation is not defined, it has its generally accepted meaning.

| | |
|---|---|
| bm = | broad multiplet |
| BOC = | *tert*-butoxycarbonyl |
| BOP = | benzotriazol-1-yloxy-tris(dimethylamino)phosphonium hexafluorophosphate |
| bd = | broad doublet |
| bs = | broad singlet |
| CDI = | 1,1" -carbodiimidazole |
| d = | doublet |
| dd = | doublet of doublets |
| dq = | doublet of quartets |
| dt = | doublet of triplets |
| DMF = | dimethylformamide |
| DMAP = | dimethylaminopyridine |
| DMSO = | dimethyl sulfoxide |
| EDC = | 1-(3-dimethyaminopropyl)-ethylcarbodiimide hydrochloride |
| eq. = | equivalents |
| EtOAc = | ethyl acetate |
| g = | grams |
| h = | hours |
| Hunig's base = | diisopropylethylamine |
| kg = | kilogram |
| L = | liter |
| m = | multiplet |
| M = | molar |
| M % = | mole percent |
| max = | maximum |
| meq = | milliequivalent |
| mg = | milligram |
| mL = | milliliter |
| mm = | millimeter |
| mmol = | millimole |
| N = | normal |
| ng = | nanogram |
| nm = | nanometers |
| OD = | optical density |
| P-EPC = | 1-(3-(1-pyrrolidinyl)propyl)-3-ethylcarbodiimide |
| psi = | pounds per square inch |
| $\phi$ = | phenyl |
| q = | quartet |
| quint. = | quintet |
| rpm = | rotations per minute |
| s = | singlet |
| t = | triplet |
| TFA = | trifluoroacetic acid |
| THF = | tetrahydrofuran |
| tlc = | thin layer chromatography |
| $\mu$L = | microliter |
| UV = | ultraviolet |

Additionally, the term "Aldrich" indicates that the compound or reagent used in the following procedures is commercially available from Aldrich Chemical Company, Inc., 1001 West Saint Paul Avenue, Milwaukee, WI 53233 USA; the term "Fluka" indicates that the compound or reagent is commercially available from Fluka Chemical Corp., 980 South 2nd Street, Ronkonkoma NY 11779 USA; the term "Lancaster" indicates that the compound or reagent is commercially available from Lancaster Synthesis, Inc., P.O. Box 100 Windham, NH 03087 USA; the term "Sigma" indicates that the compound or reagent is commercially available from Sigma, P.O. Box 14508, St. Louis MO 63178 USA; the term "Chemservice" indicates that the compound or reagent is commercially available from Chemservice Inc., Westchester, PA. USA; the term "Bachem" indicates that the compound or reagent is commercially available from Bachem Bioscience Inc., 3700 Horizon Drive, Renaissance at Gulph Mills, King of Prussia, PA 19406 USA; the term "Maybridge" indicates that the compound or reagent is commercially available from Maybridge Chemical Co. Trevillett,

Tintagel, Cornwall PL34 OHW United Kingdom; and the term "TCI" indicates that the compound or reagent is commercially available from TCI America, 9211 North Harborgate St., Portland, Oregon, 97203, OR, USA; the term "Alfa" indicates that the compound or reagent is commercially available from Johnson Matthey Catalog Company, Inc. 30 Bond Street, Ward Hill, MA 01835-0747; and the term "Nova Biochem" indicates that the compound or reagent is commercially available from NovaBiochem USA, 10933 North Torrey Pines Road, P.O. Box 12087, La Jolla CA 92039-2087.

In the examples below, all temperatures are in degrees Celsius (unless otherwise indicated) and the following general procedures were used to prepare the compounds as indicated.

GENERAL PROCEDURE A

Coupline of $R^1C(X')(X")C(O)Cl$ with $H_2NCH(R^2)C(O)XR^3$

To a stirred solution of (D,L)-alanine *iso*-butyl ester hydrochloride (from Example B below) (4.6 mmol) in 5 mL of pyridine was added 4.6 mmol of an acid chloride. Precipitation occurred immediately. The mixture was stirred for 3.5 h, diluted with 100 mL of diethyl ether, washed with 10% HCl three times, brine once, 20% potassium carbonate once and brine once. The solution was dried over magnesium sulfate, filtered, and evaporated at reduced pressure to yield the product. Other amino acid esters may also be employed in this procedure.

GENERAL PROCEDURE B

Coupling of $R^1C(X')(X")C(O)OH$ with $H_2NCH(R^2)C(O)XR^3$

A solution of the acid (3.3 mmol) and CDI in 20 mL THF was stirred for 2 h. L-alanine *iso*-butyl ester hydrochloride (from Example B below) (3.6 mmol) was added, followed by 1.5 mL (10.8 mmol) of triethylamine. The reaction mixture was stirred overnight. The reaction mixture was diluted with 100 mL of diethyl ether, washed with 10% HCl three times, brine once, 20% potassium carbonate once and brine once. The solution was dried over magnesium sulfate, filtered, and evaporated at reduced pressure to yield the product. Other amino acid esters may also be employed in this procedure.

GENERAL PROCEDURE C

Esterification of $R^1C(X')(X")C(O)NHCH(R^2)C(O)OH$ With $HOR^3$

To a stirred solution of phenylacetylvaline (1.6470 g, 7.0 mmol) in 20 mL THF was added CDI (1.05 g, 6.5 mmol) and the mixture was stirred for 1.5 h. 2-Methylbutanol (0.53 g, 6 mmol) was added the mixture, followed by addition of NaH (0.16 g, 6.5 mmol). Bubbling occurred immediately. The reaction mixture was stirred overnight. The reaction mixture was diluted with 100 mL of diethyl ether, washed with 10% HCl three times, brine once, 20% potassium carbonate once and brine once. The solution was dried over magnesium sulfate, filtered, and evaporated at reduced pressure to yield the product. Other N-acyl amino acids and alcohols may also be employed in this procedure.

GENERAL PROCEDURE D

Ester Hydrolysis to the Free Acid

Ester hydrolysis to the free acid was conducted by conventional methods. Below are two examples of such conventional de-esterification methods.

To the ester in a 1:1 mixture of $CH_3OH/H_2O$ was added 2-5 equivalents of $K_2CO_3$. The mixture was heated to about 50°C for about 0.5 to 1.5 hours until tlc showed complete reaction. The reaction was cooled to room temperature and the methanol was removed at reduced pressure. The pH of the remaining aqueous solution was adjusted to about 2, and ethyl acetate was added to extract the product. The organic phase was then washed with saturated aqueous NaCl and dried over $MgSO_4$. The solution was stripped free of solvent at reduced pressure to yield the product.

The amino acid ester was dissolved in dioxane/water (4: 1) to which was added LiOH (~ 2 eq.) that was dissolved in water such that the total solvent after addition was about 2:1 dioxane:water. The reaction mixture was stirred until reaction completion and the dioxane was removed under reduced pressure. The residue was diluted with EtOAc, the layers were separated and the aqueous layer acidified to pH 2. The aqueous layer was back extracted with EtOAc, the combined organics were dried over $Na_2SO_4$ and the solvent was removed under reduced pressure after filtration. The residue was purified by conventional methods (e.g., recrystallization).

The following exemplifies this later example. The methyl ester of 3-$NO_2$ phenylacetyl alanine 9.27 g (0.0348 mols) was dissolved in 60 mL dioxane and 15 mL of $H_2O$ and adding LiOH (3.06 g, 0.0731 mol) that has been dissolved in 15 mL of $H_2O$. After stirring for 4 hours, the dioxane was removed under reduced pressure and the residue diluted with EtOAc, the layers were separated and the aqueous layer acidified to pH 2. The aqueous layer was back extracted with EtOAc (4 X 100 mL), the combined organics were dried over $Na_2SO_4$ and the solvent was removed under reduced pressure after filtration. The residue was recrystallized from EtOAc/isooctane giving 7.5 g (85%) of 3-nitrophenylacetyl alanine. $C_{11}H_{12}N_2O_5$ requires C = 52.38, H = 4.80, and N = 11.11. Analysis found C = 52.54, H = 4.85, and N = 11.08. $[\alpha]_{23}$ = -29.9 @ 589 nm.

GENERAL PROCEDURE E

Low Temperature BOP Coupling of Acid and Alcohol

A solution of methylene chloride containing the carboxylic acid (100M%) and N-methyl morpholine (150 M%) was cooled to -20°C under nitrogen. BOP (105 M%) was added in one portion and the reaction mixture was maintained at -20°C for 15 minutes. The corresponding alcohol (120 M%) was added and the reaction mixture was allowed to warm to room temperature and stirred for 12 hours. The reaction mixture was then poured into water and extracted with ethyl acetate (3x). The combined ethyl acetate portions were backwashed with saturated aqueous citric acid (2x), saturated aqueous sodium bicarbonate (2x), brine (1x), dried over anhydrous magnesium sulfate or sodium sulfate and the solvent removed under reduced pressure to yield the crude product.

GENERAL PROCEDURE F

EDC Coupling of Acid and Amine

The acid derivative was dissolved in methylene chloride. The amine (1 eq.), N-methylmorpholine (5 eq.), and hydroxybenzotriazole monohydrate (1.2 eq.) were added in sequence. The reaction was cooled to about 0°C and then 1.2 eq. of 1-(3-dimethylaminopropyl)-3-ethylcarbodiimide hydrochloride was added. The solution was allowed to stir overnight and come to room temperature under $N_2$. pressure. The reaction mix was worked up by washing the solution with saturated, aqueous $Na_2CO_3$, 0.1M citric acid, and brine before drying with $Na_2SO_4$ and removal of solvents to yield crude product. Pure products were obtained by flash chromatography in an appropriate solvent.

GENERAL PROCEDURE G

EDC Coupling of Acid and Amine

A round bottom flask was charged with carboxylic acid (1.0 eq.), hydroxybenzotriazole hydrate (1.1 eq.) and amine (1.0 eq.) in THF under nitrogen atmosphere. An appropriate amount (1.1 eq. for free amines and 2.2 eq. for hydrochloride amine salts) of base, such as Hunig's base was added to the well stirred mixture followed by EDC (1.1 eq.). After stirring from 4 to 17 hours at room temperature the solvent was removed at reduced pressure, the residue taken up in EtOAc (or similar solvent)/water. The organic layer was washed with saturated aqueous sodium bicarbonate solution, 1N HCl, brine and dried over anhydrous sodium sulfate. In some cases, the isolated product was analytically pure at this stage while, in other cases, purification via chromatography and/or recrystallization was required prior to biological evaluation.

GENERAL PROCEDURE H

Coupling of $R^1C(X')(X'')C(O)Cl$ with $H_2NCH(R^2)C(O)XR^3$

An excess of oxalyl chloride in dichloromethane was added to the acid derivative together with one drop of DMF. The resulting mixture was stirred for about 2 hours or until bubbling ceases. The solvent was then removed under reduced pressure and rediluted with dry methylene chloride. To the resulting solution was added about 1.1 eq. of the appropriate amino acid ester and triethylamine (1.1 eq. in methylene chloride). The system was stirred at room temperature for 2 hours and then the solvent was removed under reduced pressure. The residue was dissolved in ethyl acetate, washed with 1N HCl followed by 1N NaOH. The organic layer was dried over anhydrous soldium sulfate, filtered and the solvent removed under reduced pressure to provide for the desired product.

GENERAL PROCEDURE I

P-EPC coupling

P-EPC coupling employs an amino acid ester and a substituted acetic acid compound. The acetic acid derivative is well known in the art and is typically commercially available. The amino acid ester is prepared by conventional methods from the known and typically commercially available N-BOC amino acid as described in GENERAL PROCEDURE J below.

Specifically, the appropriate amino ester free base (0.0346 mmols) and substituted phenylacetic acid (0.069 mmols) were dissolved in 2.0 mL $CHCl_3$ (EtOH free), treated with 150 mg of P-EPC (0.87 meq./g) and the reaction was mixed for 4 days at 23°C. The reaction was filtered through a plug of cotton, rinsed with 2.0 mL of $CHCl_3$ and the filtrate evaporated under a stream of nitrogen. The purity of each sample was determined by $^1$H NMR and ranged from 50% to >95%. Between 8.0 and 15.0 mg of final product was obtained from each reaction and was tested without additional purification.

GENERAL PROCEDURE J

Synthesis of Amino Acid Esters From the Corresponding N-BOC Amino Acid

A. Esterification of the Acid.

The N-BOC amino acid was dissolved in dioxane and treated with an excess of alcohol (~ 1.5 eq.) and catalytic DMAP (100 mg) at 0°C. Stirring was continued until reaction completion whereupon the product was recovered by conventional methods.

B. Removal of N-BOC Group.

The N-BOC protected amino acid was dissolved in methylene chloride (0.05M) and treated with 10 eq. of TFA at room temperature under a nitrogen atmosphere. The reaction was monitored by tlc until starting material was consumed usually within 1-5 hours. An additional 10 eq. of TFA was added to the reaction if the starting material was still present after 5 hours. The reaction was carefully neutralized with $Na_2CO_3$, separated, the organic layer washed with brine and dried over anhydrous $Na_2SO_4$. The crude amine was then used without purification.

Specific exemplification of these procedures are as follows:

1. Racemic (+/-)-N-BOC-α-amino butyric acid (Aldrich) (9.29 g, 0.0457 mol) was dissolved in 100 mL of dioxane and treated with *iso*-butyl alcohol (6.26 mL, 0.0686 mol), EDC (8.72 g, 0.0457) and catalytic DMAP (100 mg) at 0°C. After stirring for 17 hours, the organics were evaporated at reduced pressure, the residue diluted with EtOAc washed with $NaHCO_3$, brine and dried over $Na_2SO_4$. Evaporation yields 8.42 g (71 %) of an oil. $C_{13}H_{25}NO_4$ requires: C = 60.21, H = 9.72, and N = 5.40. Anal found: C = 59.91, H = 9.89, and N = 5.67.

The above N-BOC amino acid ester (8.00 g, 0.032 mol) was deprotected as above giving 3.12 g (61 %) of the free base as a colorless oil which solidifies upon standing.

2. L-N-BOC-alanine (Aldrich) (8.97 g, 0.047 mol) was dissolved in 100 mL of $CH_2Cl_2$, *iso*-butyl alcohol (21.9 mL, 0.238 mol) and treated with DMAP (100 mg) and EDC (10.0 g, 0.52 mol) at O°C. The mixture was stirred for 17 hours, diluted with $H_2O$, washed with 1.0 N HCl, $NaHCO_3$, then brine and the organics were dried over $Na_2SO_4$. Filtration and evaporation yields 11.8 g (quantitative) of L-N-BOC alanine *iso*-butyl ester which is contaminated with a small amount of solvent. A sample was vacuum dried for analytical analysis. $C_{12}H_{23}NO_4$ requires: C = 58.79, H = 9.38, and N = 5.71. Anal found: C = 58.73, H = 9.55, and N = 5.96.

The above N-BOC amino acid ester (11.8 g, 0.0481 mol) was deprotected as above. The free base was converted to the corresponding HCl salt using saturated HCl (g)/EtOAc to give L-N-alanine *iso*-butyl ester hydrochloride. Obtained 4.2 g (48%) of a colorless solid. $C_7H_{15}NO_2$. HCl requires: C = 46.28, H = 8.88, and N = 7.71. Anal found: C = 46.01, H = 8.85, and N = 7.68.

GENERAL PROCEDURE K

Methyl ester formation from amino acids

The amino acid (amino acid or amino acid hydrochloride) is suspended in methanol and chilled to 0°C. HCl gas is bubbled through this solution for 5 minutes. The reaction is allowed to warm to room temperature then stirred for 4 hours. The solvents are then removed at reduced pressure to afford the desired amino acid methyl ester hydrochloride. This product is usually used without further purification.

Example A

**Synthesis of free and polymer bound PEPC**

N-ethyl-N'-3-(1-pyrrolidinyl)propylurea

To a solution of 27.7 g (0.39 mol) ethyl isocyanate in 250 mL chloroform was added 50 g (0.39 mol) 3-(1-pyrrolidinyl)propylamine dropwise with cooling. Once the addition was complete, the cooling bath was removed and the reaction mixture stirred at room temperature for 4 hours. The reaction mixture was then concentrated under reduced pressure to give 74.5 g (96.4%) of the desired urea as a clear oil.

1-(3-(1-pyrrolidinyl)propyl)-3-ethylcarbodiimide (P-EPC)

To a solution of 31.0 g (0.156 mol) N-ethyl-N'-3-(1-pyrrolidinyl)propylurea in 500 mL dichloromethane was added 62.6 g (0.62 mol) triethylamine and the solution was cooled to 0°C. To this solution were then added 59.17 g (0.31 mol) 4-toluenesulfonyl chloride in 400 mL dichloromethane dropwise at such a rate as to maintain the reaction at 0-5°C. After the addition was complete, the reaction mixture was warmed to room temperature and then heated to reflux for 4 hours. After cooling to room temperature, the reaction mixture was washed with saturated aqueous potassium carbonate (3 x 150 mL). The aqueous phases were combined and extracted with dichloromethane. All organic phases were combined and concentrated under reduced pressure. The resultant orange slurry was suspended in 250 mL diethyl ether and the solution decanted off from the solid. The slurry/decantation process was repeated 3 more times. The ether solutions were combined and concentrated under reduced pressure to give 18.9 g (67%) of the desired product as a crude orange oil. A portion of the oil was distilled under vacuum to give a colorless oil distilling at 78-82°C (0.4 mm Hg).

Preparation of a polymer supported form of 1-(3-(1-pyrrolidinyl)propyl)-3-ethylcarbodiimide (P-EPC)

A suspension of 8.75 g (48.3 mmol) 1-(3-(1-pyrrolidin-yl)propyl)-3-ethylcarbodiimide and 24.17 g (24.17 mmol) Merrifield's resin (2% crosslinked, 200-400 mesh, chloromethylated styrene/divinylbenzene copolymer, 1 meq. Cl/g) in dimethylformamide was heated at 100°C for 2 days. The reaction was cooled and filtered and the resulting resin washed sequentially with 1L DMF, 1L THF and 1L diethyl ether. The remaining resin was then dried under vacuum for 18 hours.

Example B

**Preparation of alanine *iso*-butyl ester hydrochloride**

A mixture of 35.64 g (0.4 mol) of (D,L)-alanine (Aldrich) (or L-alanine (Aldrich)); 44 mL (0.6 mol) of thionyl chloride (Aldrich) and 200 mL of isobutanol was refluxed for 1.5 hours and the volatiles were removed completely on a rotavapor of 90°C under reduced pressure to give (D,L)-alanine *iso*-butyl ester hydrochloride (or L-alanine *iso*-butyl ester hydrochloride), which was pure enough to be used for further transformations.

Example C

**Preparation of 3,5-dichlorophenylacetic acid**

To a solution of 3.5 g of 3,5-dichlorobenzyl alcohol (Aldrich) in 75 mL of dichloromethane at 0°C was added 1.8 mL of methane sulfonylchloride followed by 3.5 mL of triethylamine added dropwise. After 2 hours the solution was diluted to 150 mL with dichloromethane, washed with 3N HCl, saturated aqueous $NaHCO_3$ dried with $Na_2SO_4$ and the

solvents removed to yield the desired 3,5-dichlorobenzyl methanesulfonate as a yellow oil that was used without purification.

The crude sulfonate was dissolved in 50 mL of DMF at 0°C and then 3 g of KCN was added. After 2 hours an additional 50 mL of DMF was added and the solution was stirred for 16 hours. The red solution was diluted with 1 L of $H_2O$ and acidified to pH 3 with 3N HCl. The aqueous solution was extracted with dichloromethane. The combined organics were washed with 3N HCl, dried with $Na_2SO_4$ and the solvents removed at reduced pressure to yield crude 3,5-dichlorophenylacetonitrile which was used without purification.

The nitrile was added to a mixture of 40 mL of concentrated sulfuric acid and 50 mL $H_2O$ and heated to reflux for 48 hours, cooled to room temperature and stirred for 48 hours. The reaction was diluted into 1 L of crushed ice, warmed to toom temperature and extracted with 2 x 200 mL of dichloromethane and 2 x 200 mL of ethylacetate. Both sets of organics were combined and washed with saturated aqueous $NaHCO_3$. The $NaHCO_3$ fractions were combined and acidified to pH I with 3N HCl. The white solid was too fine to filter and was extracted out with 2 X 200 mL of dichloromethane. The combined organics were dried with $Na_2SO_4$ and the solvents removed at reduced presure to yield crude 3,5-dichlorophenylacetic acid as a white solid. The solid was slurried with hexane and filtered to get 1.75g of white solid.

NMR ($CDCl_3$): (in ppm) 3.61 (s, 2H), 7.19 (s,1H), 7.30 (s, 1H)

Example D

**Synthesis of N-(3-chlorophenylacetyl)alanine**

The title compound was prepared using L-alanine (Nova Biochem) and 3-chlorophenyl acetic acid (Aldrich) by following General Procedures F or G, followed by hydrolysis using General Procedure D.

Example 1

**Synthesis of N-(phenylacetyl)-D,L-alanine *iso*-butyl ester**

Following General Procedure A above and using phenylacetyl chloride (Aldrich) and D,L-alanine *iso*-butyl ester hydrochloride (from Example B above), the title compound was prepared. The reaction was monitored by tlc on silica gel and purification was by extraction with $Et_2O$ followed by washes with aqueous $K_2CO_3$ and aqueous HCl.

NMR data was as follows:

$^1$H-nmr ($CDCl_3$): $\delta$ = 7.23-7.36 (m, 5H), 6.18 (d, 1H), 4.58 (t, *J* = 7.3 Hz, 1H), 3.87 (m, 2H), 3.57 (s, 2H), 1.90 (m, 1H), 1.34 (d, *J* = 7.2 Hz, 3H), 0.89 (d, *J* = 6.8 Hz, 6H).

$^{13}$C-nmr ($CDCl_3$): $\delta$ = 172.7, 170.3, 134.5, 129.2, 128.8, 127.2, 71.3, 48.1, 43.4, 27.5, 18.8, 18.3.

$C_{15}H_{21}NO_3$ (MW = 263.34; Mass Spectroscopy ($MH^+$ = 264))

Example 2

**Synthesis of *N*-(3-phenylpropionyl)-D,L-alanine *iso*-butyl ester**

Following General Procedure A above and using 3-phenylpropionyl chloride (Aldrich) and D,L-alanine *iso*-butyl ester hydrochloride (from Example B above), the title compound was prepared as a solid having a melting point of from 51°-54°C. The reaction was monitored by tlc on silica gel and purification was by extraction with $Et_2O$ followed by washes with aqueous $K_2CO_3$ and aqueous HCl.

NMR data was as follows:

$^1$H-nmr ($CDCl_3$): $\delta$ = 7.25 (m, 2H), 7.19 (m, 3H), 6.28 (d, *J* = 7.2 Hz, 1H), 4.58 (quint., *J* = 7.2 Hz, 1H), 3.89 (m, 2H), 2.95 (t, *J* = 7.7 Hz, 2H), 2.50 (m, 2H), 1.92 (m, 1H), 1.33 (d, *J* = 7.1 Hz, 3H), 0.91 (d, *J* = 6.7 Hz, 6H).

$^{13}$C-nmr ($CDCl_3$): $\delta$ = 173.0, 171.5, 140.6, 128.3, 128.1, 126.0, 71.2, 47.8, 37.9, 31.4, 27.5, 18.79, 18.77, 18.3.

$C_{16}H_{23}NO_3$ (MW = 277.37, Mass Spectroscopy ($MH^+$ 278))

Example 3

**Synthesis of *N*-(3-methylpentanoyl)-L-alanine *iso*-butyl ester**

Following General Procedure B and using 3-methylpentanoic acid (Aldrich) and L-alanine *iso*-butyl ester hydrochloride (from Example B above), the title compound was prepared as an oil. The reaction was monitored by tlc on silica gel and purification was by extraction with $Et_2O$ followed by washes with aqueous $K_2CO_3$ and aqueous HCl.

NMR data was as follows:

$^1$H-nmr ($CDCl_3$): δ = 6.08 (d, *J* = 5.9 Hz, 1H), 4.62 (quint., *J* = 7.3 Hz, 1H), 3.92 (m, 2H), 2.22 (m, 1H), 1.84-2.00 (m, 3H), 1.40 (d, *J* = 7.2 Hz, 3H), 1.35 (m, 1H), 1.20 (m, 1H), 0.85-0.96 (m, 12H).

$^{13}$C-nmr ($CDCl_3$): δ = 173.3, 172.1, 71.4, 47.9, 43.9, 32.3, 29.38, 29.35, 27.6, 19.10, 19.06, 18.93, 18.91, 18.72, 18.67, 11.3.

$C_{13}H_{25}NO_3$ (MW = 243.35, Mass Spectroscopy ($MH^+$ 244))

Example 4

**Synthesis of *N*-[(4-chlorophenyl)acetyl]-L-alanine *iso*-butyl ester**

Following General Procedure B and using 4-chlorophenylacetic acid (Aldrich) and L-alanine iso-butyl ester hydrochloride (from Example B above), the title compound was prepared as a solid having a melting point of 111°-113°C. The reaction was monitored by tlc on silica gel and purification was by extraction with $Et_2O$ followed by washes with aqueous $K_2CO_3$ and aqueous HCl.

NMR data was as follows:

$^1$H-nmr ($CDCl_3$): δ = 7.30 (d, *J* = 8.2 Hz, 2H), 7.21 (d, *J* = 8.3 Hz, 2H), 6.18 (d, *J* = 5.5 Hz, 1H), 4.57 (quint., *J* = 7.2 Hz, 1H), 3.88 (m, 2H), 3.53 (s, 2H), 1.91 (m, 1H), 1.36 (d, *J* = 7.1 Hz, 3H), 0.90 (d, *J* = 6.8 Hz, 6H).

$^{13}$C-nmr ($CDCl_3$): δ = 172.8, 169.8, 133.1, 133.0, 130.6, 128.9, 71.4, 48.2, 42.6, 27.6, 18.85, 18.82, 18.4.

$C_{15}H_{20}NO_3Cl$ (MW = 297.78, Mass Spectroscopy ($MH^+$ 298))

Example 5

**Synthesis of *N*-[(3,4-dichlorophenyl)acetyl]-L-alanine *iso*-butyl ester**

Following General Procedure B and using 3,4-dichlorophenylacetic acid (Aldrich) and L-alanine *iso*-butyl ester hydrochloride (from Example B above), the title compound was prepared as a solid having a melting point of 81°-83°C. The reaction was monitored by tlc on silica gel and purification was by extraction with $Et_2O$ followed by washes with aqueous $K_2CO_3$ and aqueous HCl.

NMR data was as follows:

$^1$H-nmr ($CDCl_3$): δ = 0.90 (d, *J* = 6.8 Hz, 6H), 1.38 (d, *J* = 7.1 Hz, 3H), 1.91 (m, 1H), 3.50 (s, 2H), 3.90 (m, 2H), 4.57 (quint., *J* = 7.1 Hz, 1H), 6.31 (d, *J* = 4.9 Hz, 1H), 7.12 (m, 1H), 7.38 (m, 2H).

$^{13}$C-nmr ($CDCl_3$): δ = 18.4, 18.8, 18.9, 27.6, 42.2, 48.3, 71.5, 128.6, 130.6, 131.2, 131.3, 132.6, 134.7, 169.2, 172.8.

$C_{15}H_{19}NO_3Cl_2$ (MW = 332.23, Mass Spectroscopy ($MH^+$ 332))

Example 6

**Synthesis of *N*-[(4-methylphenyl)acetyl]-D,L-alanine *iso*-butyl ester**

Following General Procedure B and using 4-methylphenylacetic acid (Aldrich) and D,L-alanine *iso*-butyl ester hydrochloride (from Example B above), the title compound was prepared as a solid having a melting point of 102°-104°C. The reaction was monitored by tlc on silica gel (Rf =0.6 in 33% ethyl acetate/hexanes) and purification was by extraction with $Et_2O$ followed by washes with aqueous $K_2CO_3$ and aqueous HCl.

NMR data was as follows:

$^1$H-nmr ($CDCl_3$): δ = 0.90 (d, *J* = 6.7 Hz, 6H), 1.35 (d, *J* = 7.2 Hz, 3H), 1.91 (m, 1H), 2.34 (s, 3H), 3.55 (s, 2H), 3.88 (m, 2H), 4.58 (m, 1H), 6.05 (bd, 1H), 7.16 (s, 4H).

$^{13}$C-nmr ($CDCl_3$): δ = 18.5, 18.85, 18.87, 21.0, 27.6, 43.1, 48.1, 71.3, 129.2, 129.6, 131.3, 136.9, 170.6, 172.8.

$C_{16}H_{23}NO_3$ (MW = 277.37, Mass Spectroscopy ($MH^+$ 278))

Example 7

**Synthesis of *N*-[(3-pyridyl)acetyl]-D,L-alanine *iso*-butyl ester**

Following General Procedure F and using 3-pyridylacetic acid hydrochloride (Aldrich) and D,L-alanine *iso*-butyl ester hydrochloride (from Example B above), the title compound was prepared as a solid having a melting point of 62°-64°C. The reaction was monitored by tlc on silica gel (Rf = 0.48 10% methanol/dichloromethane) and purification was by silica gel chromatography.

NMR data was as follows:

$^1$H-nmr ($CDCl_3$): δ = 8.40 (d, *J* = 2.8, 2H); 7.6 (m, 1H): 7.16 (m, 2H); 4.5 (quint., *J* = 7.2, 7.2, 1H); 3.8 (m, 2H); 3.48 (s, 2H); 1.8 (m, 1H); 1.30 (d, *J* = 7.2, 3H); 0.81 (d, *J* = 6.7, 6H).

$^{13}$C-nmr ($CDCl_3$): δ = 173.4, 170.1, 150.6, 148.8, 137.4, 131.4, 124.1, 71.9, 48.9, 40.6, 28.1, 19.5, 19.4, 18.6.

$C_{14}H_{20}N_2O_3$ (MW = 264, Mass Spectroscopy ($MH^+$ 265))

Example 8

**Synthesis of *N*-[(1-naphthyl)acetyl]-L-alanine *iso*-butyl ester**

Following General Procedure B and using 1-naphthylacetic acid (Aldrich) and L-alanine *iso*-butyl ester hydrochloride (from Example B above), the title compound was prepared as a solid having a melting point of 69°-73°C. The reaction was monitored by tlc on silica gel and purification was by extraction with $Et_2O$ followed by washes with aqueous $K_2CO_3$ and aqueous HCl.

NMR data was as follows:

$^1$H-nmr ($CDCl_3$): δ = 0.83 (m, 6H), 1.25 (d, *J* = 7.1 Hz, 3H), 1.81 (m, 1H), 3.79 (m, 2H), 4.04 (2s, 2H), 4.57 (quint., *J* = 7.3 Hz, 1H), 5.99 (d, *J* = 7.1 Hz, 1H), 7.44 (m, 2H), 7.53 (m, 2H), 7.85 (m, 2H), 7.98 (m, 1H).

$^{13}$C-nmr ($CDCl_3$): δ = 18.2, 18.81, 18.83, 27.5, 41.5, 48.2, 71.3, 123.7, 125.6, 126.1, 126.6, 128.2, 128.5, 128.7, 130.7, 132.0, 133.9, 170.3, 172.5.

$C_{19}H_{23}NO_3$ (MW = 313.40, Mass Spectroscopy ($MH^+$ 314))

Example 9

**Synthesis of *N*-[(2-naphthyl)acetyl]-L-alanine *iso*-butyl ester**

Following General Procedure B and using 2-naphthylacetic acid (Aldrich) and L-alanine *iso*-butyl ester hydrochloride (from Example B above), the title compound was prepared as a solid having a melting point of 128°-129°C. The reaction was monitored by tlc on silica gel and purification was by extraction with $Et_2O$ followed by washes with aqueous $K_2CO_3$ and aqueous RCl.

NMR data was as follows:

$^1$H-nmr ($CDCl_3$): δ = 0.86 (m, 6H), 1.35 (d, *J* = 7.1 Hz, 3H), 1.78 (m, 1H), 3.76 (s, 2H), 3.87 (m, 2H), 4.62 (quint., *J* = 7.2 Hz, 1H), 6.13 (d, *J* = 7.1 Hz, 1H), 7.41 (m, 1H), 7.48 (m, 2H), 7.74 (s, 1H), 7.83 (m, 3H).

$^{13}$C-nmr ($CDCl_3$): δ = 18.4, 18.82, 18.85, 27.6, 43.7, 48.2, 71.4, 125.9, 126.3, 127.2, 127.6, 127.7, 128.2, 128.7, 132.0, 132.5, 133.5, 170.3, 172.8.

$C_{19}H_{23}NO_3$ (MW = 313.40, Mass Spectroscopy ($MH^+$ 314)).

Example 10

**Synthesis of *N*-(4-phenylbutanoyl)-L-alanine *iso*-butyl ester**

Following General Procedure B and using 4-phenylbutanoic acid (Aldrich) and L-alanine *iso*-butyl ester hydrochloride (from Example B above), the title compound was prepared as an oil. The reaction was monitored by tlc on silica gel and purification was by extraction with $Et_2O$ followed by washes with aqueous $K_2CO_3$ and aqueous HCl.

NMR data was as follows:

$^1$H-nmr ($CDCl_3$): δ = 0.92 (d, *J* = 6.7 Hz, 6H), 1.38 (d, *J* = 7.1 Hz, 3H), 1.96 (m, 3H), 2.21 (t, *J* = 7.1 Hz, 2H), 2.64 (t, *J* = 7.3 Hz, 2H), 3.90 (m, 2H), 4.59 (quint., *J* = 7.2 Hz, 1H), 6.31 (d, 1H), 7.16 (m, 3H), 7.24 (m, 2H).

$^{13}$C-nmr ($CDCl_3$): δ = 18.3, 18.75, 18.78, 26.8, 27.5, 34.9, 35.3, 47.8, 71.2, 125.7, 128.2, 128.3, 141.3, 172.1, 173.0.

$C_{17}H_{25}NO_3$ (MW = 291.39, Mass Spectroscopy ($MH^+$ 292)).

Example 11

**Synthesis of *N*-(5-phenylpentanoyl)-L-alanine *iso*-butyl ester**

Following General Procedure B and using 5-phenylpentanoic acid (Aldrich) and L-alanine *iso*-butyl ester hydrochloride (from Example B above), the title compound was prepared as an oil. The reaction was monitored by tlc on silica gel and purification was by extraction with $Et_2O$ followed by washes with aqueous $K_2CO_3$ and aqueous HCl.

NMR data was as follows:

$^1$H-nmr ($CDCl_3$): δ = 7.23 (m, 2H), 7.17 (m, 3H), 6.30 (d, 1H), 4.59 (quint., *J* = 7.3 Hz, 1H), 3.91 (m, 2H), 2.61 (t, *J* = 7.2 Hz, 2H), 2.22 (t, *J* = 7.2 Hz, 2H), 1.93 (m, 1H), 1.66 (m, 4H), 1.38 (d, *J* = 7.2 Hz, 3H), 0.92 (d, J = 6.7 Hz, 6H).

$^{13}$C-nmr ($CDCl_3$): δ = 173.1, 172.3, 142.0, 128.2, 128.1, 125.6, 71.2, 47.8, 36.1, 35.5, 30.8, 27.5, 25.0, 18.80, 18.77, 18.4.

$C_{18}H_{27}NO_3$ (MW = 305.39, Mass Spectroscopy ($MH^+$ 306)).

Example 12

**Synthesis of *N*-[(4-pyridyl)acetyl]-D,L-alanine *iso*-butyl ester**

Following General Procedure F and using 4-pyridylacetic acid hydrochloride (Aldrich) and (D,L)-alanine *iso*-butyl ester hydrochloride (from Example B above), the title compound was prepared as a solid having a melting point of 64°-66°C. The reaction was monitored by tlc on silica gel (Rf = 0.43 10% methanol/dichloromethane) and purification was by silica gel chromatography.

NMR data was as follows:

$^1$H-nmr ($CDCl_3$): δ = 8.51 (dd, *J* = 1.6, 2.8, 1.6, 2H); 7.23 (dd, *J* = 4.3, 1.6, 4.4, 2H); 6.71 (d, *J* = 6.8, 1H); 4.56 (quint., *J* = 7.3, 7.2, 1H); 3.88 (m, 2H); 3.53 (s, 2H); 1.89 (m, 1H); 1.36 (d, *J* = 7.2, 3H); 0.88 (d, *J* = 6.7, 6H).

$^{13}$C-nmr ($CDCl_3$): δ = 173.5, 169.3, 150.5, 144.4, 125.1, 72.1, 48.9, 43.0, 28.2, 19.5, 19.5, 18.9.

$C_{14}H_{20}N_2O_3$ (MW = 264, Mass Spectroscopy ($MH^+$ 265))

Example 13

**Synthesis of N-(phenylacetyl)-L-alanine *iso*-butyl ester**

Following General Procedure B and using phenylacetyl chloride (Aldrich) and L-alanine *iso*-butyl ester hydrochloride (from Example B above), the title compound was prepared as a solid having a melting point of 45°-47°C. The reaction was monitored by tlc on silica gel and purification was by extraction with $Et_2O$ followed by washes with aqueous $K_2CO_3$ and aqueous HCl.

NMR data was as follows:

$^1$H-nmr ($CDCl_3$): δ = 7.24-7.39 (m, 5H), 6.14 (d, 1H), 4.58 (t, *J* = 7.3 Hz, 1H), 3.88 (m, 2H), 3.58 (s, 2H), 1.90 (m, 1H), 1.35 (d, *J* = 7.2 Hz, 3H), 0.89 (d, *J* = 6.7 Hz, 6H).

$^{13}$C-nmr ($CDCl_3$): δ = 172.8, 170.4, 134.5, 129.3, 128.9, 127.2, 71.3, 48.1, 43.5, 27.5, 18.9, 18.8, 18.4.

$C_{15}H_{21}NO_3$ (MW = 263.34, Mass Spectroscopy ($MH^+$ 264)).

Example 14

**Synthesis of 2-[(3,4-dichlorophenyl)acetamido]butyric acid *iso*-butyl ester**

Following General Procedure I above and using 3,4-dichlorophenylacetic acid (Aldrich) and *iso*-butyl 2-aminobutyrate (prepared following General Procedure J above) the title compound was prepared. The reaction was monitored by tlc on silica gel and purification was by filtration as described in the general procedure.

NMR data was as follows:

$^1$H-nmr ($CDCl_3$): δ = 7.36 (m, 3H), 6.03 (bd, 1H), 4.54 (m, 1H), 3.87 (m, 2H), 3.49 (s, 2H), 1.93 (m, 2H), 1.72 (m, 1H), 0.88 (d, 6H), 0.80 (t, 3H).

Example 15

**Synthesis of 2-[(3-methoxyphenyl)acetamido]butyric acid *iso*-butyl ester**

Following General Procedure I above and using 3-methoxyphenylacetic acid (Aldrich) and *iso*-butyl 2-aminobutyrate (prepared frollowing General Procedure J above), the title compound was prepared. The reaction was monitored by tlc on silica gel and purification was by filtration as described in the general procedure.

NMR data was as follows:

$^1$H-nmr ($CDCl_3$): δ = 6.75 (m, 4H), 5.93 (bd, 1H), 4.51 (m, 1H), 3.83 (m, 2H), 3.75 (s, 2H), 3.52 (s, 2H), 1.82 (m, 2H), 1.60 (m, 1H), 0.84 (d, 6H), 0.74 (t, 3H).

$C_{17}H_{25}NO_4$ (MW = 307.39, Mass Spectroscopy ($MH^+$ 309)).

Example 16

**Synthesis of 2-[(4-nitrophenyl)acetamido]butyric acid *iso*-butyl ester**

Following General Procedure I above and using 4-nitrophenylacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 8.16 (d, 2H), 7.44 (d, 2H), 6.04 (bd, 1H), 4.55 (m, 1H), 3.86 (m, 2H), 3.66 (s, 2H), 1.86 (m, 2H), 1.67 (m, 1H), 0.85 (d, 6H), 0.81 (t, 3H).

$C_{16}H_{22}N_2O_5$ (MW = 322.36, Mass Spectroscopy ($MH^+$ 323)).

Example 17

**Synthesis of 2-[(3,4-methylenedioxyphenyl)acetamido]butyric acid *iso*-butyl ester**

Following General Procedure I above and using 3,4-(methylenedioxy)-phenyl acetic acid (Aldrich) and *iso*-butyl 2-aminobutyrate (prepared following General Procedure J above), the tide compound was prepared. The reaction was monitored by tlc on silica gel and purification was by filtration as described in the general procedure.

**NMR data was as follows:**

$^1$H-nmr ($CDCl_3$): $\delta$ = 6.72 (m, 3H), 5.92 (bd, 1H), 4.54 (m, 1H), 3.86 (m, 2H), 3.66 (s, 2H), 1.86 (m, 2H), 1.66 (m, 1H), 0.89 (d, 6H), 0.79 (t, 3H).

Example 18

**Synthesis of 2-[(thien-3-yl)acetamido]butyric acid *iso*-butyl ester**

Following General Procedure I above and using 3-thiopheneacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.37 (m, 1H), 7.16 (m, 1H), 7.04 (m, 1H), 6.05 (bd, 1H), 4.57 (m, 1H), 3.66 (s, 2H), 1.93 (m, 2H), 1.67 (m, 1H), 0.91 (d, 6H), 0.86 (t, 3H).

Example 19

**Synthesis of 2-[(4-chlorophenyl)acetamido]butyric acid *iso*-butyl ester**

Following General Procedure I above and using 4-chlorophenylacetic acid (Aldrich) and *iso*-butyl 2-aminobutyrate (prepared following General Procedure J above), the tide compound was prepared. The reaction was monitored by tlc on silica gel and purification was by filtration as described in the general procedure.

NMR data was as follows:

$^1$H-nmr ($CDCl_3$): $\delta$ = 7.22 (m, 2H), 7.11 (m, 2H), 5.80 (m, 1H), 4.44 (m, 1H), 3.78 (m, 2H), 3.43 (s, 2H), 1.77 (m, 2H), 1.56 (m, 1H), 0.83 (d, 6H) 0.71 (t, 3H).

Example 20

**Synthesis of 2-[(3-nitrophenyl)acetamido]butyric acid *iso*-butyl ester**

Following General Procedure I above and using 3-nitrophenylacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 8.15 (m, 2H), 7.65 (m, 1H), 6.08 (m, 1H), 4.46 (m, 1H), 3.92 (m, 2H), 3.68 (s, 2H), 1.91 (m, 2H), 1.75 (m, 1H), 0.98 (d, 6H) 0.71 (t, 3H).

Example 21

**Synthesis of 2-[(2-hydroxyphenyl)acetamido]butyric acid *iso*-butyl ester**

Following General Procedure I above and using 2-hydroxyphenylacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): δ = 7.14 (m, 1H), 7.01 (m, 1H), 6.93 (m, 1H), 6.79 (m, 1H), 6.46 (m, 1H), 4.51 (m, 1H), 3.87 (m, 2H), 3.57 (s, 2H), 2.01 (m, 2H). 1.75 (m, 1H), 0.89 (d, 6H), 0.85 (t, 3H).

Example 22

**Synthesis of 2-[(2-naphthyl)acetamido]butyric acid *iso*-butyl ester**

Following General Procedure I above and using 2-naphthylacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): δ = 7.83 (m, 7H), 5.95 (m, 1H), 4.58 (m, 1H), 3.84 (m, 2H), 3.75 (s, 2H), 1.89 (m, 2H), 1.63 (m, 1H), 0.91 (d, 6H), 0.81 (t, 3H).

$C_{20}H_{25}NO_3$ (MW = 327.42, Mass Spectroscopy ($MH^+$ 328)).

Example 23

**Synthesis of 2-[(2,4-dichlorophenyl)acetamido]butyric acid *iso*-butyl ester**

Following General Procedure I above and using 2,4-dichlorophenylacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): δ = 7.49 (m, 1H), 7.22 (m, 2H) 5.98 (m, 1H), 4.52 (m, 1H), 3.86 (m, 2H), 3.61 (s, 2H), 1.84 (m, 2H), 1.62 (m, 1H) 0.87 (d, 6H), 0.80 (t, 3H).

Example 24

**Synthesis of 2-[(4-bromophenyl)acetamido]butyric acid *iso*-butyl ester**

Following General Procedure I above and using 4-bromophenylacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): δ = 7.43 (d, 2H), 7.19 (d, 2H) 5.85 (m, 1H), 4.51 (m, 1H), 3.81 (m, 2H), 3.47 (s, 2H), 1.84 (m, 2H), 1.61 (m, 1H) 0.84 (d, 6H), 0.76 (t, 3H).

$C_{16}H_{22}NO_3Br$ (MW = 356.26, Mass Spectroscopy ($MH^+$ 358)).

Example 25

**Synthesis of 2-[(3-chlorophenyl)acetamido])butyric acid *iso*-butyl ester**

Following General Procedure I above and using 3-chlorophenylacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): δ = 7.25 (m, 3H), 7.12 (m, 1H) 5.80 (m, 1H), 4.52 (m, 1H), 3.86 (m, 2H), 3.50 (s, 2H), 1.87 (m, 2H), 1.67 (m, 1H) 0.88 (d, 6H), 0.77 (t, 3H).

$C_{16}H_{22}NO_3Cl$ (MW = 311.81 Mass Spectroscopy ($MH^+$ 313)).

Example 26

**Synthesis of 2-[(3-fluorophenyl)acetamido]butyric acid *iso*-butyl ester**

Following General Procedure I above and using 3-fluorophenylacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.31 (m, 1H), 7.01 (m, 3H) 5.95 (m, 1H), 4.54 (m, 1H), 3.84 (m, 2H), 3.54 (s, 2H), 1.88 (m, 2H), 1.65 (m, 1H) 0.87 (d, 6H), 0.81 (t, 3H).

$C_{16}H_{22}NO_3F$ (MW = 295.35 Mass Spectroscopy ($MH^+$ 296)).

Example 27

**Synthesis of 2-[(benzothiazol-4-yl)acetamido)butyric acid *iso*-butyl ester**

Following General Procedure I above and using 4-benzothiazol-4-yl acetic acid (Chemservice) and *iso*-butyl 2-aminobutyrate (prepared following General Procedure J above), the title compound was prepared. The reaction was monitored by tlc on silica gel and purification was by filtration as described in the general procedure.

**NMR data was as follows:**

$^1$H-nmr ($CDCl_3$): $\delta$ = 7.82 (m, 1H), 7.51-7.21 (m, 4H) 5.84 (m, 1H), 4.51 (m, 1H), 3.90 (s, 2H), 3.79 (m, 2H), 1.78 (m, 2H), 1.58 (m, 1H) 0.80 (d, 6H), 0.66 (t, 3H).

Example 28

**Synthesis of 2-[(2-methylphenyl)acetamido]butyric acid *iso*-butyl ester**

Following General Procedure I above and using 2-methylphenylacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.18 (m, 4H), 5.79 (m, 1H), 4.54 (m, 1H), 3.85 (m, 2H), 3.59 (s, 2H), 3.29 (s, 3H), 1.81 (m, 2H), 1.59 (m, 1H) 0.87 (d, 6H), 0.77 (t, 3H).

$C_{17}H_{25}NO_3$ (MW = 291.39 Mass Spectroscopy ($M^+$ 291)).

Example 29

**Synthesis of 2-[(2-fluorophenyl)acetamido]butyric acid *iso*-butyl ester**

Following General Procedure I above and using 2-fluorophenylacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.28 (m, 1H), 7.09 (m, 3H) 6.03 (m, 1H), 4.54 (m, 1H), 3.87 (m, 2H), 3.57 (s, 2H), 1.89 (m, 2H), 1.64 (m, 1H) 0.88 (d, 6H), 0.80 (t, 3H).

Example 30

**Synthesis of 2-[(4-fluorophenyl)acetamido]butyric acid *iso*-butyl ester**

Following General Procedure I above and using 4-fluorophenylacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.20 (m, 2H), 6.97 (m, 2H) 5.87 (m, 1H), 4.492 (m, 1H), 3.83 (m, 2H), 3.48 (s, 2H), 1.86 (m, 2H), 1.60 (m, 1H) 0.87 (d, 6H), 0.78 (t, 3H).

$C_{16}H_{22}NO_3F$ (MW = 295.35 Mass Spectroscopy ($MH^+$ 296)).

Example 31

**Synthesis of 2-[(3-bromophenyl)acetamido]butyric acid *iso*-butyl ester**

Following General Procedure I above and using 3-bromophenylacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.45 (m, 2H), 7.23 (m, 2H) 5.95 (m, 1H), 4.55 (m, 1H) 3.84 (m, 2H) 3.55 (s, 2H), 1.89 (m, 2H), 1.68 (m, 1H) 0.91 (d, 6H), 0.81 (t, 3H).

$C_{16}H_{22}NO_3Br$ (MW = 356.26 Mass Spectroscopy ($M^+$ 357)).

Example 32

**Synthesis of 2-[(3-trifluoromethylphenyl)acetamido]butyric acid *iso*-butyl ester**

Following General Procedure I above and using 3-trifluoromethyl-phenylacetic acid (Aldrich) and *iso*-butyl 2-aminobutyrate (prepared following General Procedure J above), the title compound was prepared. The reaction was monitored by tic on silica gel and purification was by filtration as described in the general procedure.

NMR data was as follows:

$^1$H-nmr ($CDCl_3$): $\delta$ = 7.52 (m, 1H), 7.47 (m, 2H) 6.01 (m, 1H), 4.56 (m, 1H), 3.86 (m, 2H), 3.61 (s, 2H), 1.84 (m, 2H), 1.62 (m, 1H) 0.87 (d, 6H), 0.80 (t, 3H).

$C_{17}H_{22}NO_3F_3$ (MW = 345.36 Mass Spectroscopy ($MH^+$ 345)).

Example 33

**Synthesis of 2-[(2-thienyl)acetamido]butyric acid *iso*-butyl ester**

Following General Procedure I above and using 2-thiopheneacetic acid (Aldrich) and *iso*-butyl 2-aminobutyrate (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 6.89 (m, 3H), 6.07 (bd, 1H), 4.50 (m, 1H), 3.82 (m, 2H), 3.71 (s, 2H), 1.85 (m, 2H), 1.62 (m, 1H), 0.81 (d, 6H), 0.75 (t, 3H).

$C_{14}H_{21}NO_3S$ (MW = 283.39, Mass Spectroscopy ($MH^+$ 284)).

Example 34

**Synthesis of 2-(phenylacetamido)butyric acid *iso*-butyl ester**

Following General Procedure H above and using phenylacetic acid (Aldrich) and *iso*-butyl 2-aminobutyrate (prepared following General Procedure J above), the title compound was prepared. The reaction was monitored by tlc on silica gel and purification was by chromatography on silica gel using 9:1 toluene:EtOAc as the eluant.

NMR data was as follows:

$^1$H-nmr ($CDCl_3$): $\delta$ = 7.17-7.28 (m, 5H), 6.23 (bd, 1H), 4.51 (m, 1H), 3.86 (m, 2H), 3.54 (s, 2H), 1.87 (m, 2H), 1.62 (m, 1H), 0.87 (d, 6H), 0.78 (t, 3H).

$C_{16}H_{23}NO_3$ (MW = 277.36, Mass Spectroscopy ($MH^+$ 277)).

Example 35

**Synthesis of *N*-(phenylacetyl)valine 2-methylbutyl ester**

Step A. Preparation of N-(phenylacetyl) valine

To a stirred solution of 5.15 g (44 mmol) of valine (Bachem) in 50 mL (100 mmol) of 2N NaOH cooled to 0°C was added dropwise 5.3 mL (40 mmol) of phenylacetyl chloride (Aldrich). A colorless oil precipitated. The reaction mixture was allowed to warm to room temperature and stirred for 18 hours, washed with 50 mL diethyl ether, acidified to pH 2-3 with aqueous HCl. The white precipitate formed was filtered off, washed thoroughly with water, followed by

diethyl ether to give 7.1 g (30 mmol, 69% yield) of the title compound.

NMR data was as follows:

$^{1}$H-nmr (DMSO-$d_6$): δ = 12.63 (s, 1H), 8.25 (d, *J* = 8.6 Hz, 1H), 7.27 (m, 5H), 4.15 (m, 1H), 3.56 (d, *J* = 13.8 Hz, 1H), 3.47 (d, *J* = 13.8 Hz, 1H), 2.05 (m, 1H), 0.87 (d, *J* = 6.8, Hz, 3H), 0.84 (d, *J* = 6.8 Hz, 3)

$^{13}$C-nmr (DMSO-$d_6$): δ = 173.2, 170.4, 136.6, 129.0, 128.2, 126.3, 57.1, 41.9, 30.0, 19.2, 18.0

$C_{13}H_{17}NO_3$ (MW=235.29; Mass Spectroscopy ($MH^+$ = 236))

Step B. Synthesis of *N*-(phenylacetyl)valine 2-methylbutyl ester

Following General Procedure C and using the N-(phenylacetyl) valine prepared in Step A above and 2-methylbutan-1-ol (Aldrich), the title compound was prepared as a diastereomeric mixture. The reaction was monitored by tlc on silica gel and purification was by filtration as described in the general procedure.

NMR data was as follows:

$^{1}$H-nmr ($CDCl_3$): δ = 7.25-7.40 (m, 5H), 5.95 (d, 1H), 4.56 (m, 1H). 3.84-4.00 (m, 2H), 3.61 (s, 2H), 2.10 (m, 1H), 1.68 (m, 1H), 1.38 (m, 1H), 1.15 (m 1H), 0.82-0.94 (m, 9H), 0.76 (d, 3H).

$^{13}$C-nmr ($CDCl_3$): δ = 171.84, 171.81, 170.7, 134.6, 129.31, 129.27, 128.9, 127.3, 69.8, 57.0, 43.7, 33.9, 31.3, 25.9, 25.8,, 18.9, 17.4, 16.34, 16.27, 11.12, 11.07.

$C_{18}H_{27}NO_3$ (MW = 305.42, Mass Spectroscopy (MH 306)).

Example 36

**Synthesis of *N*-(phenylacetyl)-L-methionine *iso*-butyl ester**

L-Methionine (0. 129g, 0.869 mmols) (Aldrich) was taken-up in dioxane (5.0 mL) and treated with a saturated solution of sodium bicarbonate (5.0 mL) followed by phenylacetyl chloride (Aldrich) (0.114 mL, 0.822 mmols). After stirring for 17 hours at room temperature the mixture was diluted with ethyl acetate, the layers separated and the aqueous layer acidified to pH 2 with 5N HCl. The crude product was extracted into ethyl acetate, dried over sodium sulfate, vacuum dried and used without further purification.

N-phenylacetyl-L-methionine (0.1285 g, 0.447 mmol) was dissolved in 3.0 mL dioxane and *iso*-butyl alcohol (0.2 mL) and treated with EDC (0.094 g, 0.492 mmol), and catalytic DMAP (0.015g). After stirring for 17 hours at 23°C, the mixture was evaporated at reduced pressure to an oil, the residue was diluted in EtOAc and washed with 0.1 N HCl and saturated sodium bicarbonate. Chromatography on silica gel using 98:2 $CHCl_3$/MeOH as eluant provided the pure product.

NMR data was as follows:

$^{1}$H-nmr ($CDCl_3$): δ = 7.4-7.23 (m, 5H), 6.14 (bd, 1H), 4.70 (m, 1H), 3.89 (d, 2H), 3.62 (s, 2H), 2.43 (m, 2H), 2.12 (m, 1H). 1.93 (m, 2H), 0.94 (d, 6H).

$C_{17}H_{25}NO_3S$ (MW = 323.17, Mass Spectroscopy ($M^+$ 323)

Example 37

**Synthesis of *N*-(phenylacetyl)-L-leucine *iso*-butyl ester**

L-Leucine (Aldrich) (0.114g, 0.869 mmols) was taken-up in dioxane (5.0 mL) and treated with a saturated solution of sodium bicarbonate (5.0 mL) followed by phenylacetyl chloride (Aldrich) (0.114 mL, 0.822 mmols). After stirring for 17 hours at room temperature the mixture was diluted with ethyl acetate, the layers separated and the aqueous layer acidified to pH 2 with 5N HCl. The crude product was extracted into ethyl acetate, dried over sodium sulfate, vacuum dried and used without further purification.

N-Phenylacetyl-L-leucine (0.0081 g, 0.038 mmol) was dissolved in 2.0 mL $CHCl_3$ (EtOH free) and *iso*-butyl alcohol (0.055 mL) and treated with P-EPC (100 mg, 0.87 milliequivalents). The mixture was rotated for 4 days, filtered through a plug of cotton and the filtrate evaporated at reduced pressure to an oil

which was sufficiently pure for testing.

NMR data was as follows:

$^{1}$H-nmr ($CDCl_3$): δ = 7.22 (m, 5H), 5.57 (d, 1H), 4.35 (m, 1H), 3.35 (m, 3H), 1.35 (m, 4H), 0.68 (m, 9H).

$C_{18}H_{27}NO_3$ (MW = 305.40, Mass Spectroscopy ($M^+$ 305)).

Example 38

**Synthesis of *N*-[(3-chlorophenyl)acetyl]alanine 3-methylbut-2-enyl ester**

Following General Procedure C above and using N-(3-chlorophenylacetyl alanine (from Example D above) and 3-methylbut-2-en-1-ol (Aldrich), the title compound can be prepared. The reaction was monitored by tlc on silica gel and purification was by liquid chromatography using 30% EtOAc/hexane as the eluant.

NMR data was as follows:

$^1$H-nmr ($CDCl_3$): δ = 7.39-7.16 (m, 4H), 6.06 (bd, 1H), 5.38-5.29 (m, 1H), 4.63 (d, *J* = 9Hz, 2H), 3.56 (s, 2H), 1.79 (s, 3H), 1.7 (s, 3H), 1.39 (d, *J* = 9Hz, 3H).

Example 39

**Synthesis of *N*-[(3-chlorophenyl)acetyl]alanine cyclopropylmethyl ester**

Following General Procedure C above, and using N-(3-chlorophenylacetyl alanine (from Example D above) and cyclopropylmethanol (Aldrich), the title compound can be prepared. The reaction was monitored by tlc on silica gel and purification was by liquid chromatography using 3:7 EtOAc:hexane as the eluant.

NMR data was as follows:

$^1$H-nmr ($CDCl_3$): δ = 7.2-7.1 (m, 4H), 6.09 (bs, 1H), 4.6 (dq, *J* = 9 Hz, 1H), 3.96 (dd, *J* = 9Hz, 2H), 3.59 (s, 2H), 1.2 (d, *J* = 9Hz, 3H), 1.2-1.0 (m, 1H), 0.603-0.503 (m, 2H), 0.300-0.203 (m, 2H).

Example 40

**Synthesis of *N*-[(3-chlorophenyl)acetyl]alanine 2-thienylmethyl ester**

Following General Procedure C above, and using N-(3-chlorophenylacetyl alanine (from Example D above) and 2-thiophenemethanol (Aldrich) the title compound can be prepared. The reaction was monitored by tlc on silica gel and purification was by liquid chromatography using 3:7 EtOAc:hexane as the eluant.

NMR data was as follows:

$^1$H-nmr ($CDCl_3$): δ = 7.37-6.97 (m, 7H), 5.97 (q, *J* = 14 Hz, 2H), 4.6 (dq, *J* = 9 Hz, 1H), 3.76 (s, 2H), 1.38 (d, *J* = 9Hz, 3H).

Example 41

**Synthesis of *N*-[(3-chlorophenyl)acetyl]alanine (1-methylcyclopropyl)methyl ester**

Following General Procedure C above, and using N-(3-chlorophenylacetyl alanine (from Example D above) and (1-methylcyclopropyl)methanol (Aldrich) the title compound can be prepared. The reaction was monitored by tlc on silica gel and purification was by liquid chromatography using 3:7 EtOAc:hexane as the eluant.

NMR data was as follows:

$^1$H-nmr ($CDCl_3$): δ = 8.6 (bd, *J* = 9 Hz, 1H), 3.86 (q, *J* = 14 Hz, 2H), 3.4 (s, 2H), 2.29 (q, *J* = 9 Hz, 1H), 1.3 (d, *J* = 9Hz, 3H), 1.03 (s, 3H), 0.5-0.4 (m, 2H), 0.4-0.28 (m, 2H).

Example 42

**Synthesis of *N*-[(3-chlorophenyl)acetyl]alanine 3-thienylmethyl ester**

Following General Procedure C above, and using N-(3-chlorophenylaceryl alanine (from Example D above) and 3-thiophenemethanol (Aldrich) the title compound can be prepared. The reaction was monitored by tlc on silica gel and purification was by liquid chromatography using 3:7 EtOAc:hexane as the eluant.

**NMR data was as follows:**

$^1$H-nmr ($CDCl_3$): δ = 8.03 (bd, *J* = 9 Hz, 1H), 7.56-7.5 (m, 1H), 7.47 (bs, 1H), 7.4-7.17 (m, 4H), 7.06 (d, *J* = 9 Hz, 1H), 5.1 (s, 2H), 4.3 (dq, 1H), 1.3 (d, *J* = 9 Hz, 3H).

Example 43

**Synthesis of *N*-[(3-chlorophenyl)acetyl]alanine 2-methylcyclopentyl ester**

Following General Procedure C above, and using N-(3-chlorophenylacetyl alanine (from Example D above) and 2-methylcyclopentanol (Aldrich) the title compound can be prepared. The reaction was monitored by tlc on silica gel and purification was by liquid chromatography using 3:7 EtOAc:hexane as the eluant.

NMR data was as follows:

$^1$H-nmr ($CDCl_3$): $\delta$ = 7.39-7.16 (m, 4H), 6.3 (bd, 1H), 4.79-4.7 (m, 1H), 4.6-4.25 (m, *J* = 9 Hz, 1H), 3.577 (s, 2H), 2.09-1.8 (m, 2H), 1.74-1.6 (m, 2H), 1.39 (dd, *J* = 9 Hz, 3H), 1.2 (dt, *J* = 9 Hz, 1H), 0.979 (dd, *J* = 9 Hz, 2H)

$C_{17}H_{22}NO_3Cl$ (MW = 323.82, Mass Spectroscopy ($MH^+$ 323).

Example 44

**Synthesis of *N*-[(3-chlorophenyl)acetyl]alanine 2-methylprop-2-enyl ester**

Following General Procedure C above, and using N-(3-chlorophenylacetyl alanine (from Example D above) and 2-methylprop-2-en-1-ol (Aldrich) the title compound can be prepared. The reaction was monitored by tlc on silica gel and purification was by liquid chromatography using 3:7 EtOAc:hexane as the eluant.

NMR data was as follows:

$^1$H-nmr ($CDCl_3$): $\delta$ = 7.39-7.16 (m, 4H), 6.03 (bs, 1H), 4.77 (s, 2H), 4.7-4.29 (m, 3H), 2.59 (s, 2H), 1.73 (s, 3H), 1.43 (d, *J* = 9 Hz, 3H)

$C_{15}H_{18}NO_3Cl$ (MW = 295.76, Mass Spectroscopy ($MH^+$ 295)).

Example 45

**Synthesis of *N*-[(3-chlorophenyl)acetyl]alanine cyclohex-2-enyl ester**

Following General Procedure C above, and using N-(3-chlorophenylacetyl alanine (from Example D above) and cyclohex-2-en-1-ol (Aldrich) the title compound can be prepared. The reaction was monitored by tlc on silica gel and purification was by liquid chromatography using 3:7 EtOAc:hexane as the eluant.

NMR data was as follows:

$^1$H-nmr ($CDCl_3$): $\delta$ = 8.6 (bd, *J* = 9 Hz, 1H), 7.4-7.2 (m, 4H), 6.0-5.8 (m, 1H), 5.7-5.5 (m, 1H), 5.1 (bs, 1H), 4.13-4.29 (m, 1H), 3.5 (s, 2H), 2.1-1.9 (m, 2H), 1.8-1.69 (m, 1H), 1.69-1.49 (m, 4H), 1.3 (dd, *J* = 9 Hz, 3H)

$C_{17}H_{20}NO_3Cl$ (MW = 321.8, Mass Spectroscopy ($MH^+$ 321.2)).

Example 46

**Synthesis of *N*-[(2-phenylbenzoxazol-5-yl)acetyl]alanine *iso*-butyl ester**

Following General Procedure I above, and using 5-(2-phenylbenzoxazol)-yl-acetic acid (CAS# 62143-69-5) and alanine *iso*-butyl ester (prepared following General Procedure J above), the title compound was prepared.

NMR data was as follows:

$^1$H-nmr ($CDCl_3$): $\delta$ = 8.24 (m, 3H), 7.68 (m, 1H), 7.51 (m, 5H), 6.04 (m, 1H), 4.58 (m, 1H), 3.85 (m, 2H), 3.68 (s, 2H), 1.9 (m, 1H), 1.35 (d, 3H), 0.87 (d, 6H).

$C_{22}H_{24}N_2O_4$ (MW = 380, Mass Spectroscopy ($MH^+$ 381)).

Example 47

**Synthesis of *N*-[(3-methylthiophenyl)acetyl]alanine *iso*-butyl ester**

Following General Procedure I above, and using 3-methylthiophenylacetic acid (CAS# 18698-73-2) and alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.14 (m, 2H), 7.01 (m, 1H), 4.56 (m, 1H), 3.88 (m, 2H), 3.54 (s, 2H), 2.46 (s, 3H), 1.89 (m, 1H), 1.35 (d, 3H) 0.85 (d, 6H).

$C_{16}H_{23}NO_3S$ (MW = 309, Mass Spectroscopy ($MH^+$ 310)).

Example 48

**Synthesis of *N*-4-[(2-furyl)acetyl]alanine *iso*-butyl ester**

Following General Procedure I above, and using 2-furylacetic acid (CAS# 2745-26-8) and alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.36 (m, 1H), 6.34 (m, 1H), 6.21 (m, 1H), 4.56 (m, 1H), 3.91 (m, 2H), 3.61 (s, 2H), 1.92 (m, 1H), 1.38 (d, 3H) 0.89 (d, 6H).

$C_{13}H_{19}NO_4$ (MW = 253, Mass Spectroscopy ($MH^+$ 254)).

Example 49

**Synthesis of *N*-[(benzofuran-2-yl)acetyl]alanine *iso*-butyl ester**

Following General Procedure I above, and using benzofuran-2-ylacetic acid (Maybridge) and alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.51 (m, 1H), 7.44 (m, 1H), 7.25 (m, 2H), 6.67 (s, 1H), 4.60 (m, 1H), 3.87 (m, 2H), 3.77 (s, 2H), 1.88 (m, 1H), 1.38 (d, 3H), 0.87 (d, 6H).

$C_{17}H_{21}NO_4$ (MW = 303, Mass Spectroscopy ($MH^+$ 304)).

Example 50

**Synthesis of *N*-[(benzothiophen-3-yl)acetyl]alanine *iso*-butyl ester**

Following General Procedure I above, and using thianaphthen-3-ylacetic acid (Lancaster) and alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.89 (m, 1H), 7.76 (m, 1H), 7.38 (m, 3H), 6.07 (m, 1H), 4.57 (m, 1H), 3.92 (m, 2H), 3.82 (s, 4H), 1.84 (m, 1H), 1.32 (d, 3H) 0.85 (d, 6H).

$C_{17}H_{21}NO_3S$ (MW = 319, Mass Spectroscopy ($MH^+$ 320)).

Example 51

**Synthesis of *N*-[(2-chloro-5-thienyl)acetyl]alanine *iso*-butyl ester**

Following General Procedure I above, and using 5-chloro-2-thienyl)acetic acid (CAS# 13669-19-7) and alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 6.77 (m, 1H), 6.68 (d, 1H), 6.31 (bm, 1H), 4.59 (m, 1H), 3.91 (m, 2H), 3.38 (s, 2H), 1.90 (m, 1H), 1.39 (d, 3H) 0.89 (d, 6H).

$C_{13}H_{18}NO_3SCl$ (MW = 303, Mass Spectroscopy ($MH^+$ 303)).

Example 52

**Synthesis of *N*-[(3-methylisoxazol-5-yl)acetyl]alanine *iso*-butyl ester**

Following General Procedure I above, and using (3-methyl-isoxazol-5-yl)acetic acid (CAS# 19668-85-0) and alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 6.07 (s, 2H), 4.56 (m, 1H), 3.92 (m, 2H), 3.68 (s, 2H), 2.29 (s, 3H), 1.94 (m, 1H), 1.89 (d, 3H) 0.91 (d, 6H).

$C_{13}H_{20}N_2O_4$ (MW = 268, Mass Spectroscopy ($MH^+$ 269)).

Example 53

**Synthesis of *N*-[(2-phenylthiothienyl)acetyl]alanine *iso*-butyl ester**

Following General Procedure I above, and using (2-phenylthiothienyl)acetic acid and alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): δ = 7.21-7.11 (m, 6H), 6.92 (d, 1H), 4.56(m, 1H), 3.87 (m, 2H), 3.72 (s, 2H), 1.94 (m, 1H), 1.38 (d, 3H) 0.89 (d, 6H).

$C_{19}H_{23}NO_3S_2$ (MW = 377, Mass Spectroscopy ($MH^+$ 378)).

Example 54

**Synthesis of *N*-[(6-methoxybenzothiophen-2-yl)acetyl]alanine *iso*-butyl ester**

Following General Procedure I above, and using (6-methoxythianaphthen-2-yl)acetic acid and alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): δ = 7.59 (d, 1H), 7.33 (d, 1H), 7.16 (s, 1H), 7.03 (dd, 1H), 4.56 (m, 1H), 3.87(s, 3H), 3.84 (m, 2H), 3.76 (s, 2H),1.85 (m, 1H), 1.30 (d, 3H) 0.86 (d, 6H).

$C_{18}H_{23}NO_4S$ (MW = 349, Mass Spectroscopy ($MH^+$ 350)).

Example 55

**Synthesis of *N*-[(3-phenyl-1,2,4-thiadiazol-5-yl)acetyl]alanine *iso*-butyl ester**

Following General Procedure I above, and using (3-phenyl-1,2,4-thiadiazol-5-yl)acetic acid (CAS# 90771-06-5) and alanine iso-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): δ = 7.47 (m, 5H), 4.66 (m, 1H), 4.16 (s, 2H), 3.91 (m, 2H), 1.93 (m, 1H), 1.48 (d, 3H) 0.93 (d, 6H).

$C_{17}H_{21}N_3O_3S$ (MW = 347, Mass Spectroscopy ($MH^+$ 348)).

Example 56

**Synthesis of *N*-[2-phenyloxazol-4-yl)acetyl]alanine *iso*-butyl ester**

Following General Procedure I above, and using (2-phenyloxazol-4-yl)acetic acid (CAS# 22086-89-1) and alanine *iso*-butyl ester (prepared following General Procedure J above), the title compound was prepared. The reaction was monitored by tlc on silica gel and purification was by filtration as described in the general procedure.

Example 57

**Synthesis of N-[(3-methylphenyl)acetyl]alanine *iso*-butyl ester**

Following General Procedure I above, and using 3-methylphenylacetic acid (Aldrich) and alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): δ = 7.21 (m, 1H), 7.07 (m, 3H), 4.54 (m, 1H), 3.83 (m, 2H), 3.52 (s, 2H), 2.35 (s, 3H), 1.87 (m, 1H), 1.32 (d, 3H), 0.88 (d, 6H).

$C_{16}H_{23}NO_3$ (MW = 277, Mass Spectroscopy ($MH^+$ 278)).

Example 58

**Synthesis of *N*-[(2,5-difluorophenyl)acetyl]alanine *iso*-butyl ester**

Following General Procedure I above, and using 2,5-difluorophenylacetic acid (Aldrich) and alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.08-6.94 (m, 3H), 4.57 (m, 1H), 3.91 (m, 2H), 3.56 (s, 2H), 1.92 (m, 1H), 1.41 (d, 3H) 0.91 (d, 6H).

$C_{15}H_{19}NO_3F_2$ (MW = 299, Mass Spectroscopy ($MH^+$ 300)).

Example 59

**Synthesis of *N*-[(3,5-diflurophenyl)acetyl]alanine *iso*-butyl ester**

Following General Procedure I above, and using 3,5-difluorophenylacetic acid (Aldrich) and alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 6.81 (m, 2H), 6.74 (m, 1H), 6.06 (m, 1H), 4.57 (m, 1H), 3.92 (m, 2H), 3.51 (s, 2H), 1.94 (m, 1H), 1.36 (d, 3H) 0.87 (d, 6H).

$C_{15}H_{19}NO_3F_2$ (MW = 299, Mass Spectroscopy ($MH^+$ 300)).

Example 60

**Synthesis of *N*-[(3-thienyl)acetyl]alanine *iso*-butyl ester**

Following General Procedure I above, and using 3-thiopheneacetic acid (Aldrich) and alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.33 (m, 1H), 7.14 (m, 1H), 7.01 (m, 1H), 6.09 (m, 1H), 4.58 (m, 1H), 3.88 (m, 2H), 3.60 (s, 2H), 1.91 (m, 1H), 1.37 (d, 3H) 0.92 (d, 6H).

Optical Rotation: $[\alpha]_{23}$ -52 (c 1 MeOH) @ 589 nm.

$C_{13}H_{19}NO_3S$ (MW = 269, Mass Spectroscopy ($MH^+$ 269)).

Example 61

**Synthesis of *N*-[(4-methylphenyl)acetyl]-L-alanine *iso*-butyl ester**

Following General Procedure I above, and using 4-methylphenylacetic acid (Aldrich) and L-alanine *iso*-butyl ester (prepared following General Procedure J above), the title compound was prepared. The reaction was monitored by tlc on silica gel and purification was by filtration as described in the general procedure.

NMR data was as follows:

$^1$H-nmr ($CDCl_3$): $\delta$ = 7.11 (s, 4H), 5.93 (m, 1H), 4.58 (m, 1H), 3.88 (m, 2H), 3.54 (s, 2H), 2.33 (s, 3H), 1.89 (m, 1H), 1.32 (d, 3H), 0.89 (d, 6H).

$C_{16}H_{23}NO_3$ (MW = 277.35, Mass Spectroscopy ($MH^+$ 278)).

Example 62

**Synthesis of *N*-(phenylacetyl)-L-alanine S-1-(methoxycarbonyl) *iso*-butyl ester**

Following General Procedure K and using (S)-(+)-2-hydroxy-2-methylbutyric acid (Aldrich) in place of the amino acid, methyl (S)-(+)-2-hydroxy-2-methylbutyrate was prepared.

Methyl (S)-(+)-2-hydroxy-2-methylbutyrate was then coupled with carbobenzyloxy-L-alanine (Aldrich) using General Procedure E to provide carbobenzyloxy-L-alanine S-1-(methoxycarbonyl) *iso*-butyl ester.

Carbobenzyloxy-L-alanine S-1-(methoxycarbonyl) *iso*-butyl ester (1.0 g) was then dissolved in 20 mL of methanol and 6N HCl (0.5 mL) and 10% palladium on carbon (0.1 g) were added. This reaction mixture was hydrogenated at 40 psi of hydrogen on a Parr apparatus for 5 hours at room temperature and then filtered through a pad of Celite. The filtrate was concentrated at reduced pressure to provide L-alanine S-1-(methoxycarbonyl) *iso*-butyl ester hydrochloride (98% yield).

L-Alanine S-1-(methoxycarbonyl) *iso*-butyl ester hydrochloride was then coupled to phenylacetic acid using General Procedure G to provide the title compound.

NMR data was as follows:

$^1$H-nmr ($CDCl_3$): $\delta$ = 7.35 - 7.20 (m, 5H), 6.22 (bd, 1H), 4.83 (d, 1H), 4.65 (p, 1H), 3.68 (s, 3H), 3.55 (s, 2H), 2.21 (m, 1H), 1.40 (d, 3H), 0.97 (d, 3H), 0.93 (d, 3H).

$^{13}$C-nmr ($CDCl_3$): $\delta$ = 173.25, 171.18, 170.22, 135.11, 129.94, 129.50, 127.88, 52.67, 48.49, 43.98, 30.53, 19.21, 18.75, 17.58.

Example 63

**Synthesis of *N*-[(3-nitrophenyl)acetyl]-L-alanine *iso*-butyl ester**

Following General Procedure H above and using 3-nitrophenylacetic acid (Aldrich) and L-alanine *iso*-butyl ester hydrochloride (from Example B above), the title compound was prepared. The reaction was monitored by tlc on silica gel and purification was by recrystallization from butyl chloride.

NMR data was as follows:

$^1$H-nmr ($CDCl_3$): $\delta$ = 8.17 (m, 2H), 7.68 (d, 1H), 7.52 (t, 1H), 6.18 (m, 1H), 4.48 (m, 1H), 3.94 (m, 2H), 3.67 (s, 2H), 1.93 (m, 1H), 1.42 (d, 3H), 0.91 (d, 3H).

Optical Rotation: $[\alpha]_{23}$ -49 (c 5, MeOH).

Example 64

**Synthesis of *N*-[(3,5-difluorophenyl)acetyl]alanine ethyl ester**

Following General Procedure G and using 3,5-difluorophenylacetic acid (Aldrich) and alanine ethyl ester (Aldrich), the title compound was prepared as a solid with a melting point of 93°-95°C. The reaction was monitored by tlc on silica gel (Rf = 0.8 in EtOAC) and purification was by chromatography on silica gel using EtOAc as the eluant followed by recrystallization from 1-chlorobutane.

NMR data was as follows:

$^1$H-nmr (DMSO-$d_6$): $\delta$ = 1.30 (d, 3H); 3.52 (s, 2H).

$C_{13}H_{15}NO_3F_2$ (MW = 271.26, Mass Spectroscopy ($MH^+$ 271)).

Example 65

**Synthesis of *N*-[(3-nitrophenyl)acetyl]methionine ethyl ester**

Following General Procedure G above and using 3-nitrophenylacetic acid (Aldrich) and methionine ethyl ester hydrochloride (Aldrich), the title compound was prepared. The reaction was monitored by tlc on silica gel and purification was by recrystallization from butyl chloride.

NMR data was as follows:

$^1$H-nmr ($CDCl_3$): $\delta$ = 8.18 (s, 1H), 8.15 (d, 1H) 7.66 (d, 1H), 7.48 (t, 1H), 6.30 (m, 1H), 4.67 (m, 1H), 4.21 (t, 2H), 3.67 (s, 2H), 2.47 (t, 2H), 2.12 (m, 2 H), 2.08 (s, 3H), 1.27 (t, 3H).

Optical Rotation: $[\alpha]_{23}$ -30 (c 5, MeOH).

Example 66

**Synthesis of *N*-[(3-chlorophenyl)acetyl]alanine *iso*-butyl ester**

Following General Procedure G above and using 3-chlorophenylacetic acid (Aldrich) and alanine *iso*-butyl ester (prepared following General Procedure J above), the title compound was prepared. The reaction was monitored by tlc on silica gel.

NMR data was as follows:

$^1$H-nmr ($CDCl_3$): $\delta$ = 7.29 (m, 3H), 7.18 (m, 1H), 6.0 (m, 1H), 4.56 (m, 1H), 3.89 (m, 2H), 3.53 (s, 2H), 1.91 (m, 1H), 1.39 (d, 3 H), 0.91 (d, 3H).

Optical Rotation: $[\alpha]_{23}$ -45 (c 5, MeOH).
$C_{15}H_{20}NO_3Cl$ (MW = 297.78, Mass Spectroscopy ($MH^+$ 297)).

Example 67

**Synthesis of *N*-[(3-chlorophenyl)acetyl]alanine 2-(*N,N*-dimethylamino)ethyl ester**

Following General Procedure C above, and using N-(3-chlorophenylacetyl)alanine (from Example D above) and 2-(N,N-dimethyl amino) ethanol (Aldrich), the title compound can be prepared. The reaction was monitored by tlc on silica gel and purification was by liquid chromatography using 0. 1:2:0.79 $NH_4OH$:EtOH:$CHCl_3$ as the eluant.

NMR data was as follows:

$^1$H-nmr ($CDCl_3$): 7.37 (s, 1H), 7.33-7.2 (m, 3H), 4.675-4.6 (m, 1H), 4.5-4.37 (m, 1H), 4.25-4.13 (m, 1H), 3.6 (d, *J* = 7 Hz, 2H), 2.86 (bs, 2H), 2.3 (s, 6H), 1.23 (d, *J* = 9 Hz, 3H).

$C_{15}H_{21}N_2O_3Cl$ (MW = 313.799, Mass Spectroscopy ($M^+$ 313)).

Example 68

**Synthesis of 2-[(3,5-dichlorophenyl)acetamido]hexanoic acid methyl ester**

Following General Procedure F above, an using 3,5-dichlorophenylacetic acid (from Example C above) and L-norleucine methyl ester hydrochloride (Bachem), the title compound was prepared as a solid having a melting point of 77°-78°C. The reaction was monitored by tlc on silica gel (Rf = 0.70 in 40% EtOAC/hexanes) and purification was by flash chromatography on silica gel using 40% EtOAc/hexanes as the eluant.

NMR data was as follows:

$^1$H-nmr ($CDCl_3$): δ = 7.20 (s), 7.18 (s), 6.6 (m), 4.55 (m), 3.7 (s), 3.5 (s), 3.4 (s), 2.0 (s), 1.8 (m), 1.6 (m), 1.2 (m), 0.8 (f).

$^{13}$C-nmr ($CDCl_3$): δ = 173.54, 169.67, 138.43, 135.72, 128.33, 128.07, 78.04, 77.62, 77.19, 53.04, 52.90, 43.14, 32.57, 27.87, 22.81, 14.41.

Example 69

**Synthesis of *N*-[(3,5-diclorophenyl)acetyl]-L-alanine *iso*-butyl ester**

Following General Procedure F above, and using 3,5-dichlorophenylacetic acid (from Example C above) and L-alanine *iso*-butyl ester hydrochloride (from Example B above), the title compound was prepared as a solid having a melting point of 115°-116°C. The reaction was monitored by tlc on silica gel (Rf = 0.40 in 3% methanol/dichloromethane) and purification was by flash chromatography on silica gel using 3 % methanol/dichloromethane as the eluant.

NMR data was as follows:

$^1$H-nmr ($CDCl_3$): δ = 7.27 (d, *J* = 2 Hz, 1H), 7.19 (s, 2H), 6.22 (d, *J* = 6 Hz, 1H), 4.59 (quint., *J* = 7 Hz, 1H), 3.9 (q, *J* = 4 Hz, 2H), 3.5 (s, 2H), 1.9 (m, 1H), 1.4 (d, *J* = 7 Hz, 3H), 0.91 (d, *J* = 7 Hz, 6H).

$^{13}$C-nmr ($CDCl_3$): δ = 173.45, 169.37, 138.31, 135.75, 128.39, 128.11, 78.04, 77.61, 77.19, 72.19, 54.03, 48.97, 43.12, 28.24, 19.52, 19.49, 19.09.

$C_{15}H_{19}NO_3Cl_2$ (MW = 331.9, Mass Spectroscopy ($MH^+$ 332)).

Example 70

**Synthesis of *N*-(cyclohexylacetyl)-L-alanine *iso*-butyl ester**

Following General Procedure B above, and using cyclohexylacetic acid (Aldrich) and L-alanine iso-butyl ester hydrochloride (from Example B above), the title compound was prepared as a solid having a melting point of 92°C-93°C. The reaction was monitored by tlc on silica gel (Rf = 0.39 in 1:3 EtOAc:hexane) and purification was by extraction with $Et_2O$ followed by washes with aqueous $K_2CO_3$ and aqueous HCl.

NMR data was as follows:

$^1$H-nmr ($CDCl_3$): δ = 0.93 (d, *J* = 6.7 Hz, 6H), 0.85-1.01 (m, 2H), 1.05-1.35 (m, 3H), 1.40 (d, *J* = 7.1 Hz, 3H), 1.60-1.85 (m, 6H), 1.95 (m, 1H), 2.06 (d, *J* = 7.0 Hz, 2H), 3.92 (m, 2H), 4.61 (m, 1H), 6.08 (bd, 1H).

$^{13}$C-nmr ($CDCl_3$): δ = 18.7, 18.9, 26.0, 26.1, 27.6, 33.0, 35.3, 44.6, 47.9, 71.4, 171.8, 173.3.

$C_{15}H_{27}NO_3$ (MW = 269.39, Mass Spectroscopy ($MH^+$ 270)).

Example 71

**Synthesis of *N*-(cyclopentylacetyl)-L-alanine *iso*-butyl ester**

Following General Procedure B above, and using cyclopentylacetic acid (Aldrich) and L-alanine *iso*-butyl ester hydrochloride (from Example B above), the title compound was prepared as a solid having a melting point of 62°C-64°C. The reaction was monitored by tlc on silica gel (Rf = 0.37 in 1:3 EtOAc:hexane) and purification was by extraction with $Et_2O$ followed by washes with aqueous $K_2CO_3$ and aqueous HCl.

NMR data was as follows:

$^1$H-nmr ($CDCl_3$): $\delta$ = 0.87 (d, *J* = 6.8 Hz, 6H), 1.01-1.17 (m, 2H), 1.34 (d, *J* = 7.2 Hz, 3H), 1.40-1.62 (m, 4H), 1.70-1.83 (m, 2H), 1.89 (m, 1H), 2.15 (m, 3H), 3.86 (m, 2H), 4.55 (m, 1H), 6.30 (d, *J* = 7.1 Hz, 1H).

$^{13}$C-nmr ($CDCl_3$): $\delta$ = 18.4, 18.78, 18.80, 24.8 (very high), 27.5, 32.27, 32.32, 36.9, 42.5, 47.7, 71.2, 172.2, 173.2.

Elemental Analysis-Calc (%): C, 65.85; H, 9.87; N, 5.49; Found (%): C, 66.01; H, 10.08; N, 5.49.

$C_{14}H_{25}NO_3$ (MW = 255.36, Mass Spectroscopy ($MH^+$ 256)).

Example 72

**Synthesis of *N*-[(cyclohex-1-enyl)acetyl]-L-alanine *iso*-butyl ester**

Following General Procedure B above, and using cyclohex-1-enyl acetic acid (Alfa) and L-alanine iso-butyl ester hydrochloride (from Example B above), the title compound was prepared as a solid having a melting point of 49°C-51°C. The reaction was monitored by tlc on silica gel (Rf = 0.40 in 1:3 EtOAc:hexane) and purification was by extraction with $Et_2O$ followed by washes with aqueous $K_2CO_3$ and aqueous HCl.

NMR data was as follows:

$^1$H-nmr ($CDCl_3$): $\delta$ = 0.91 (d, *J* = 4.5 Hz, 3H), 0.93 (d, *J* = 6.7 Hz, 3H), 1.40 (d, *J* = 7.2 Hz, 3H), 1.52-1.70 (m, 4H), 1.97 (m, 3H), 2.06 (bs, 2H), 2.89 (s, 2H), 3.92 (m, 2H), 4.59 (m, 1H), 5.65 (s, 1H), 6.33 (d, *J* = 6.6 Hz, 1H).

$^{13}$C-nmr ($CDCl_3$): $\delta$ = 18.7, 18.91, 18.93, 21.9, 22.7, 25.3, 27.6, 28.3, 46.1, 47.9, 71.4, 127.1, 132.5, 170.6, 173.1.

Elemental Analysis-Calc (%): C, 67.38; H, 9.42; N, 5.24; Found (%): C, 67.34; H, 9.54; N, 5.16.

$C_{15}H_{25}NO_3$ (MW = 267.37, Mass Spectroscopy ($MH^+$ 268)).

Example 73

**Synthesis of *N*-[(3-chlorophenyl)acetyl]alanine 3-methylbut-2-enyl thioester**

Following General Procedure C above, and using *N*-[(3-chlorophenyl)acetyl] alanine and 3-methyl-2-butene thioester (TCI), the title compound can be prepared. The reaction was monitored by tlc on silica gel and purification was by liquid chromatography using 3:7 EtOAc:Hexane as the eluant.

NMR data was as follows:

$^1$H-nmr (DMSO-$d_6$): $\delta$ = 5.2-5.075 (m, 1H), 4.37 (dq, *J* = 9 Hz, 1H), 3.56 (s), 3.43 (d, *J* = 12 Hz, 2H), 1.266 (d, *J* = 12 Hz, 6H) 1.3 (d, *J* = 9 Hz, 3H).

$C_{16}H_{20}NO_2ClS$ (MW = 325.86, Mass Spectroscopy ($M^+$ 325)).

Example 74

**Synthesis of *N*-[(2-phenyl)-2-fluoroacetyl]alanine ethyl ester**

Following General Procedure F above, and using $\alpha$-fluorophenyl acetic acid (Aldrich) and alanine ethyl ester (Aldrich), the title compound was prepared. The reaction was monitored by tlc on silica gel (Rf = 0.75 in 1:1 EtOAc: hexane) and purification was by chromatography on silica gel using 1:2 ethyl acetate/hexanes as the eluent.

NMR data was as follows:

$^1$H-nmr (DMSO-$d_6$): $\delta$ = 1.14 (q, 3H), 1.34 (d, 3H), 4.07 (m, 2H), 4.33 (m, 1H), 5.84 (d, 1H), 6.01 (d, 1H), 7.40-7.55 (m, 5H), 8.87 (m, 1H).

$C_{13}H_{16}NO_3F$ (MW = 253.27, Mass Spectroscopy ($MH^+$ 253)).

Example 75

**Synthesis of *N*-(3,5-difluorophenylacetyl)-L-phenylglycine methyl ester**

Following General Procedure F above, and using 3,5-difluorophenylacetic acid (Aldrich) and L-phenylglycine methyl ester hydrochloride (Bachem), the title compound was prepared.

NMR data was as follows:

$^1$H-nmr ($CDCl_3$): δ =7.4-7.3 (m, 5H), 6.9-6.7 (m, 3H), 6.55 (d 1H, 7.1 Hz), 5.56 (d 1H 7 Hz), 3.72 (s 3H), 3.57 (s 2H)

$^{13}$C-nmr ($CDCl_3$): δ = 197.6, 177.6, 171.8, 169.3, 136.7, 129.6, 129.3, 127.8, 113.0, 112.9, 112.7, 111.4, 103.8, 103.5, 65.1, 57.2, 53.5, 45.1, 43.3, 43.3

$C_{17}H_{15}NO_3F_2$ (MW = 319.31, Mass Spectroscopy (MH +320)).

Example 76

**Synthesis of *N*-(3,5-difluorophenylacetyl)-L-phenylglycine *iso*-butyl ester**

The 3,5-difluorophenylacetic acid (Aldrich) was EDC coupled to L-phenylglycine methyl ester hydrochloride (Bachem) via General Procedure F above.

The resulting compound was placed in a large excess of the desired alcohol. A catalytic amount of dry NaH was added, and the reaction was followed by tlc until the presence of starting material was no longer detected. The reaction was quenched with a few milliliters of 1N HCl, and after a few minutes of stirring saturated aqueous $NaHCO_3$ was added. The volume of the reaction mixture was reduced on a rotary evaporator until the excess alcohol was removed and then the remaining residue was taken up in ethyl acetate and additional water was added. The organic phase was washed with saturated aqueous NaCl and dried over $MgSO_4$. The solution was stripped free of solvent on a rotary evaporator, and the crude product residue was then further purified by chromatography.

NMR data was as follows:

$^1$H-nmr ($CDCl_3$): δ = 7.35-7.3 (m 5H), 6.8-6.7 (m 3H) 6.60 (d 1H, 7 Hz), 5.55 (d 1H 7.1 Hz), 3.9 (m 2H), 3.60 (s 2H), 1.85 (m 1H 7 Hz), 0.8 (q 6H 7 Hz)

$^{13}$C-nmr ($CDCl_3$): δ = 171.3, 169.3, 165.4, 138.5, 137.0, 129.5, 129.2, 127.6, 113.1, 113.0, 112.8, 112.7, 103.8, 103.5, 103.2, 75.5, 57.2, 43.4, 43.3, 28.2, 19.3

$C_{20}H_{21}NO_3F_2$ (MW = 361.39, Mass Spectroscopy (MH +362)).

Example 77

**Synthesis of *N*-(cyclopentylacetyl)-L-phenylglycine methyl ester**

Following General Procedure D above, and using cyclopentylacetic acid (Aldrich) with L-phenylglycine methyl ester hydrochloride (Bachem) the title compound was prepared.

NMR data was as follows:

$^1$H-nmr ($CDCl_3$): δ = 7.35 (s, 5H), 6.44 (bd, 1H), 5.6 (d, 1H), 3.72 (s, 3H), 2.24 (bs, 3H), 1.9-1.4 (m, 6H), 1.2-1.05 (m, 2H)

$^{13}$C-nmr ($CDCl_3$): δ = 172.3, 171.7, 136.7, 129.0, 128.6, 127.3, 56.2, 52.7, 42.5, 36.9, 32.40, 32.38, 24.8

Example 78

**Synthesis of *N*-(cyclopentylacetyl)-L-alanine methyl ester**

Following General Procedure D above, and using cyclopentylacetic acid (Aldrich) with L-alanine methyl ester hydrochloride (Sigma) the title compound was prepared.

NMR data was as follows:

$^1$H-nmr ($CDCl_3$): δ = 6.38 (d, 1H), 4.50 (m, 1H), 3.65 (s, 3H), 2.13 (bs, 3H), 1.80-1.00 (m (includes d at 1.30, 3H), 11H)

$^{13}$C-nmr ($CDCl_3$): δ = 173.7, 172.5, 52.1, 47.6, 42.3, 36.8, 32.15, 32.14, 18.0

$C_{11}H_{19}NO_3$ (MW = 213.28, Mass Spectroscopy ($MH^+$ 214)).

Example 79

**Synthesis of *N*-(cyclopropylacetyl)-L-phenylglycine methyl ester**

Following General Procedure D above, and using cyclopropylacetic acid (Aldrich) with L-phenylglycine methyl ester hydrochloride (Bachem), the title compound was prepared.

NMR data was as follows:

$^1$H-nmr ($CDCl_3$): $\delta$ = 7.35 (m, 5H) 6.97 (bd, *J* = 7.2 Hz, 1H) 5.59 (d, *J* = 7.8 Hz, 1H), 3.71 (s, 3H), 2.17 (m, 2H), 1.05-0.95 (m, 1H), 0.62 (m, 2H), 0.02 (m, 2H)

$^{13}$C-nmr ($CDCl_3$): $\delta$ = 171.9, 174.6, 136.6, 129.0, 128.5, 127.2, 56.1, 52.7, 41.0, 6.9, 4.37, 4.33

Example 80

**Synthesis of *N*-(cyclopropylacetyl)-L-alanine methyl ester**

Following General Procedure D above, and using cyclopropylacetic acid (Aldrich) with L-alanine methyl ester hydrochloride (Sigma), the title compound was prepared.

NMR data was as follows:

$^1$H-nmr ($CDCl_3$): $\delta$ = 6.60 (d, 1H), 4.55 (m, 1H), 3.69 (s, 3H), 2.10 (m, 2H), 1.34 (d, 3H), 0.95 (m, 1H), 0.58 (m, 2H) 0.15 (m, 2H)

$^{13}$C-nmr ($CDCl_3$): $\delta$ = 173.7, 172.3, 52.3, 47.7, 41.0, 18,2, 6.7, 4.27, 4.22

Example 81

**Synthesis of *N*-[(3-nitrophenyl)acetyl]-L-methionine *iso*-butyl ester**

Following General Procedure H above, and using nitrophenylacetic acid (Aldrich) and L-methionine (Aldrich), the title compound was prepared as a tan oil. The reaction was monitored by tlc on silica gel.

NMR data was as follows:

$^1$H-nmr ($CDCl_3$): $\delta$ = 8.16 (m,2H) 7.67 (d,1H) 7.32 (t, 1H), 6.31 (bd, 1H), 4.69 (m, 1H), 3.90 (d, 2H), 3.68 (s, 2H), 2.47 (t, 2H), 2.15 (m, 1H), 2.02 (s, 3H), 1.90 (m, 2H), 0.91 (d, 6H).

$C_{17}H_{24}N_2O_5S$ (MW = 368.4, Mass Spectroscopy ($MH^+$ 368)).

Example 82

**Cellular Screen for the Detection of Inhibitors of β-Amyloid Production**

Numerous compounds of formula I above were assayed for their ability to inhibit β-amyloid production in a cell line possessing the Swedish mutation. This screening assay employed cells (K293 = human kidney cell line) which were stably transfected with the gene for amyloid precursor protein 751 (APP751) containing the double mutation $Lys_{651}Met_{652}$ to $Asn_{651}Leu_{652}$ (APP751 numbering) in the manner described in International Patent Application Publication No. 94/10569[8] and Citron et al.[11]. This mutation is commonly called the Swedish mutation and the cells, designated as "293 751 SWE", were plated in Corning 96-well plates at 1.5-2.5 x $10^4$ cells per well in Dulbecco's minimal essential media plus 10% fetal bovine serum. Cell number is important in order to achieve β-amyloid ELISA results within the linear range of the assay (-0.2 to 2.5 ng per mL).

Following overnight incubation at 37°C in an incubator equilibrated with 10% carbon dioxide, media were removed and replaced with 200 μL of a compound of formula I (drug) containing media per well for a two hour pretreatment period and cells were incubated as above. Drug stocks were prepared in 100% dimethylsulfoxide such that at the final drug concentration used in the treatment, the concentration of dimethylsulfoxide did not exceed 0.5% and, in fact, usually equaled 0.1%.

At the end of the pretreatment period, the media were again removed and replaced with fresh drug containing media as above and cells were incubated for an additional two hours. After treatment, plates were centrifuged in a Beckman GPR at 1200 rpm for five minutes at room temperature to pellet cellular debris from the conditioned media. From each well, 100 μL of conditioned media or appropriate dilutions thereof were transferred into an ELISA plate precoated with antibody 266[13] against amino acids 13-28 of β-amyloid peptide as described in International Patent Application Publication No. 94/10569[8] and stored at 4°C overnight. An ELISA assay employing labelled antibody 6C6[13] against amino acids 1-16 of β-amyloid peptide was run the next day to measure the amount of β-amyloid peptide produced.

Cytotoxic effects of the compounds were measured by a modification of the method of Hansen, et al.[12]. To the cells remaining in the tissue culture plate was added 25 µL of a 3,(4,5-dimethylthiazol-2-yl)2,5-diphenyltetrazolium bromide (MTT) stock solution (5 mg/mL) to a final concentration of 1 mg/mL. Cells were incubated at 37°C for one hour, and cellular activity was stopped by the addition of an equal volume of MTT lysis buffer (20% w/v sodium dodecylsulfate in 50% dimethylformamide, pH 4.7). Complete extraction was achieved by overnight shaking at room temperature. The difference in the $OD_{562nm}$ and the $OD_{650nm}$ was measured in a Molecular Device's $UV_{max}$ microplate reader as an indicator of the cellular viability.

The results of the β-amyloid peptide ELISA were fit to a standard curve and expressed as ng/mL β-amyloid peptide. In order to normalize for cytotoxicity, these results were divided by the MTT results and expressed as a percentage of the results from a drug free control. All results are the mean and standard deviation of at least six replicate assays.

The test compounds were assayed for β-amyloid peptide production inhibition activity in cells using this assay. The results of this assay demonstrate that, each of the compounds within this invention tested reduced β-amyloid peptide production by at least 30 % as compared to control.

Example 83

**In Vivo Suppression of β-Amyloid Release and/or Synthesis**

This example illustrates how the compounds of this invention could be tested for *in vivo* suppression of β-amyloid release and/or synthesis. For these experiments, 3 to 4 month old PDAPP mice are used [Games et al., (1995) *Nature* 373:523-527]. Depending upon which compound is being tested, the compound is usually formulated at either 5 or 10 mg/ml. Because of the low solubility factors of the compounds, they may be formulated with various vehicles, such as corn oil (Safeway, South San Francisco, CA); 10% EtOH in corn oil (Safeway); 2-hydroxypropyl-β-cyclodextrin (Research Biochemicals International, Natick MA); and carboxy-methyl-cellulose (Sigma Chemical Co., St. Louis MO). Specifically, for example 141 the vehicle was carboxy-methyl-cellulose (Sigma).

The mice are dosed subcutaneously with a 26 gauge needle and 3 hours later the animals are euthanized via $CO_2$ narcosis and blood is taken by cardiac puncture using a 1 cc 25G 5/8" tuberculin syringe/needle coated with solution of 0.5 M EDTA, pH 8.0. The blood is placed in a Becton-Dickinson vacutainer tube containing EDTA and spun down for 15 minutes at 1500 xg at 5°C. The brains of the mice are then removed and the cortex and hippocampus are dissected out and placed on ice.

1. Brain Assay

To prepare hippocampal and cortical tissue for enzyme-linked immunosorbent assays (ELISAs) each brain region is homogenized in 10 volumes of ice cold guanidine buffer (5.0 M guanidine-HCl, 50 mM Tris-HCl, pH 8.0) using a Kontes motorized pestle (Fisher, Pittsburgh PA). The homogenates are gently rocked on a rotating platform for three to four hours at room temperature and stored at -20°C prior to quantitation of β-amyloid.

The brain homogenates are diluted 1:10 with ice-cold casein buffer [0.25% casein, phosphate buffered saline (PBS), 0.05% sodium azide, 20 µg/ml aprotinin, 5 mM EDTA, pH 8.0, 10 µg/ml leupeptin], thereby reducing the final concentration of guanidine to 0.5 M, before centrifugation at 16,000 xg for 20 minutes at 4°C. The β-amyloid standards (1-40 or 1-42 amino acids) were prepared such that the final composition equaled 0.5 M guanidine in the presence of 0.1% bovine serum albumin (BSA).

The total β-amyloid sandwich ELISA, quantitating both β-amyloid (aa 1-40) and β-amyloid (aa 1-42) consists of two monoclonal antibodies (mAb) to β-amyloid. The capture antibody, 266[13], is specific to amino acids 13 - 28 of β-amyloid. The antibody 3D6[14], which is specific to amino acids 1 - 5 of β-amyloid, is biotinylated and served as the reporter antibody in the assay. The 3D6 biotinylation procedure employs the manufacturer's (Pierce, Rockford IL) protocol for NHS-biotin labeling of immunoglobulins except that 100 mM sodium bicarbonate, pH 8.5 buffer is used. The 3D6 antibody does not recognize secreted amyloid precursor protein (APP) or full-length APP but detects only β-amyloid species with an amino terminal aspartic acid. The assay has a lower limit of sensitivity of ~ 50 pg/ml (11 pM) and shows no cross-reactivity to the endogenous murine β-amyloid peptide at concentrations up to 1 ng/ml.

The configuration of the sandwich ELISA quantitating the level of β-amyloid (aa 1-42) employs the mAb 21F12[14] (which recognizes amino acids 33-42 of β-amyloid) as the capture antibody. Biotinylated 3D6 is also the reporter antibody in this assay which has a lower limit of sensitivity of ~ 125 pg/ml (28 pM).

The 266 and 21F12 capture mAbs are coated at 10 µg/ml into 96 well immunoassay plates (Costar, Cambidge MA) overnight at room temperature. The plates are then aspirated and blocked with 0.25% human serum albumin in PBS buffer for at least 1 hour at room temperature, then stored desiccated at 4°C until use. The plates are rehydrated with wash buffer (Tris-buffered saline, 0.05% Tween 20) prior to use. The samples and standards are added to the

plates and incubated overnight at 4°C. The plates are washed ≥ 3 times with wash buffer between each step of the assay. The biotinylated 3D6, diluted to 0.5 µg/ml in casein incubation buffer (0.25% casein, PBS, 0.05% Tween 20, pH 7.4) is incubated in the well for 1 hour at room temperature. Avidin-HRP (Vector, Burlingame CA) diluted 1:4000 in casein incubation buffer is added to the wells for 1 hour at room temperature. The colorimetric substrate, Slow TMB-ELISA (Pierce, Cambridge MA), is added and allowed to react for 15 minutes, after which the enzymatic reaction is stopped with addition of 2 N $H_2SO_4$. Reaction product is quantified using a Molecular Devices Vmax (Molecular Devices, Menlo Park CA) measuring the difference in absorbance at 450 nm and 650 nm.

2. Blood Assay

The EDTA plasma is diluted 1:1 in specimen diluent (0.2 gm/l sodium phosphate•$H_2O$ (monobasic), 2.16 gm/l sodium phosphate•$7H_2O$ (dibasic), 0.5gm/l thimerosal, 8.5 gm/l sodium chloride, 0.5 ml TritonX-405, 6.0 g/l globulin-free bovine serum albumin; and water). The samples and standards in specimen diluent are assayed using the total β-amyloid assay (266 capture/3D6 reporter) described above for the brain assay except the specimen diluent was used instead of the casein diluents described.

## Claims

1. A compound of formula I or a mixture of compounds of formula I for use as a medicament, wherein formula I is:

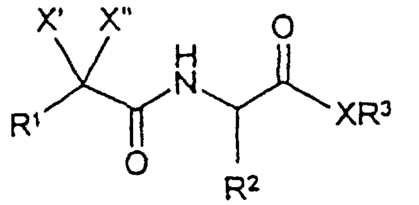

I

wherein $R^1$ is selected from the group consisting of

a) alkyl, alkenyl, alkcycloalkyl, naphthyl-$(R)_m$- wherein R is an alkylene group of from 1 to 8 carbon atoms and *m* is an integer equal to 0 or 1, cycloalkyl, cycloalkenyl, 3-pyridyl, 4-pyridyl and heteroaryl, of 3 to 10 atoms and 1 to 4 heteroatoms selected from oxygen, sulfur and nitrogen wherein the heteroaryl group is optionally substituted with 1 to 3 substituents selected from the group consisting of alkyl, alkoxy, aryl, aryloxy, halo, nitro, thioalkoxy, and thioaryloxy with the proviso that for such heteroaryls when there is at least one nitrogen heteroatom, there is also at least one oxygen and/or sulfur heteroatom;
b) a substituted phenyl group of formula II:

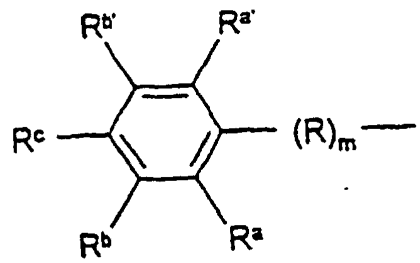

II

wherein R is alkylene of from 1 to 8 carbon atoms,
*m* is an integer equal to 0 or 1 ,
$R^a$ and $R^{a'}$ are independently selected from the group consisting of hydrogen, hydroxy, fluoro and methyl;
$R^b$ and $R^{b'}$ are independently selected from the group consisting of hydrogen, alkyl, alkoxy, aryl, cyano, cycloalkyl, halo, heteroaryl, heterocyclic, nitro, trihalomethyl, thioalkoxy, thioaryloxy, thioheteroaryloxy, and -C(O)$R^4$ where $R^4$ is selected from the group consisting of alkyl, aryl, alkoxy and aryloxy; and
$R^c$ is selected from the group consisting of hydrogen, alkyl, aryl, cyano, halo, nitro, and where $R^b$ and $R^c$ are fused to form a methylenedioxy ring with the phenyl ring; and
when $R^b$ and/or $R^{b'}$ and/or $R^c$ is fluoro, chloro, bromo and/or nitro, then $R^a$ and/or $R^{a'}$ can also be chloro; and
whereby $R^a$, $R^{a'}$, $R^b$, $R^{b'}$ and $R^c$ are not all simultaneously hydrogen; and
c) 1- or 2-naphthyl-$(R)_m$- substituted at the 5, 6, 7 and/or 8 positions with 1 to 4 substituents selected from the group consisting alkyl, alkoxy, halo, cyano, nitro, trihalomethyl, and thioalkoxy wherein R is an alkylene group of from 1 to 8 carbon atoms and *m* is an integer equal to 0 or 1;
$R^2$ is selected from the group consisting of alkyl, phenyl, alkylalkoxy, alkylthioalkoxy; and
$R^3$ is selected from the group consisting of $-(CH_2)_nCR^{10}R^5R^6$ wherein *n* is an integer equal to 0,1 or 2, $R^5$ and $R^6$ are independently selected from hydrogen, alkyl, alkenyl, aryl, heteroaryl, heterocyclic, $-NR^7R^8$ where $R^7$ and $R^8$ are independently hydrogen or alkyl and $-COOR^9$ where $R^9$ is alkyl, and further wherein $R^5$ and $R^6$ can be joined to form a cycloalkyl group, a cycloalkenyl group, an aryl group, a heteroaryl group, and a heterocyclic group, and when $R^5$ and $R^6$ do not join to form an aryl or heteroaryl group, then $R^{10}$ is selected from hydrogen and alkyl with the proviso that when *n* is zero, then $R^{10}$ is hydrogen and when *n* is greater than zero and $R^5$ and $R^6$ are joined to form an aryl or heteroaryl group, then $R^{10}$ becomes a bond within that group;
X is oxygen or sulfur,
X' is hydrogen, hydroxy or fluoro;
X" is hydrogen, hydroxy or fluoro, or X' and X" together form an oxo group, and pharmaceutically acceptable salts thereof
with the provisos that:

when $R^1$ is pyrid-3-yl, $R^2$ is ethyl, X is oxygen, and X' and X" are hydrogen, then $R^3$ is not $-CH_2CH(CH_3)_2$, and

when $R^1$ is indoxazin-3-yl, 2,4-dimethylthiazol-5-yl, 4-methyl-1,2,5- thiooxadizol-3-yl or 3,5-di(trifluoromethyl)phenyl, $R^2$ is methyl, X is oxygen, and X' and X" are hydrogen, then $R^3$ is not $-CH_2CH(CH_3)_2$.

**2.** The compound of formula I or mixture of compounds of formula I according to claim 1 for inhibiting β-amyloid release and/or its synthesis in a cell.

**3.** The compound of formula I or mixture of compounds of formula I according to claim 1 for preventing the onset of Alzheimer's disease in a patient at risk for developing Alzheimer's disease.

**4.** The compound of formula I or a mixture of compounds of formula I according to claim 1 for treating a patient with Alzheimer's disease in order to inhibit further deterioration in the condition of the patient.

**5.** A compound of formula III:

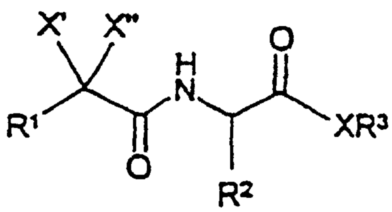

III

wherein $R^1$ is selected from the group consisting of

a) alkyl, alkenyl, alkcycloalkyl, naphthyl-$(R)_m$- wherein R is an alkylene group of from 1 to 8 carbon atoms and *m* is an integer equal to 0 or 1, cycloalkyl, cycloalkenyl, 3-pyridyl, 4-pyridyl and heteroaryl, of 3 to 10 atoms and 1 to 4 heteroatoms selected from oxygen, sulfur and nitrogen wherein the heteroaryl group is optionally substituted with 1 to 3 substituents selected from the group consisting of alkyl alkoxy, aryl, aryloxy, halo, nitro, thioalkoxy, and thioaryloxy with the proviso that for such heteroaryls when there is at least one nitrogen heteroatom, there is also at least one oxygen and/or sulfur heteroatom;

(b) a substituted phenyl group of formula II:

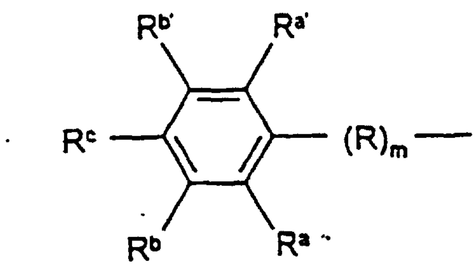

II

wherein R is alkylene of from 1 to 8 carbon atoms,

*m* is an integer equal to 0 or 1,

$R^a$ and $R^{a'}$ are independently selected from the group consisting of hydrogen, hydroxy, fluoro and methyl;

$R^b$ and $R^{b'}$ are independently selected from the group consisting of hydrogen, alkyl, alkoxy, aryl, cyano, cycloalkyl, halo, heteroaryl, heterocyclic, nitro, trihalomethyl, thioalkoxy, thioaryloxy, thioheteroaryloxy, and -C(O) $R^4$ where $R^4$ is selected from the group consisting of alkyl, aryl, alkoxy and aryloxy; and

$R^c$ is selected from the group consisting of hydrogen, alkyl, aryl, cyano, halo, nitro, and where $R^b$ and $R^c$ are fused to form a methylenedioxy ring with the phenyl ring; and

when $R^b$ and/or $R^{b'}$ and/or $R^c$ is fluoro, chloro, bromo and/or nitro, then $R^a$ and/or $R^{a'}$ can also be chloro; and

whereby $R^a$, $R^{a'}$, $R^b$, $R^{b'}$ and $R^c$ are not all simultaneously hydrogen; and

(c) 1- or 2-naphthyl-$(R)_m$- wherein R is an alkylene group of from 1 to 8 carbon atoms and *m* is an integer equal to 0 or 1 substituted at the 5, 6, 7 and/or 8 positions with 1 to 4 substituents selected from the group consisting alkyl, alkoxy, halo, cyano, nitro, trihalomethyl, and thioalkoxy;

$R^2$ is selected from the group consisting of alkyl, phenyl, alkylalkoxy, alkylthioalkoxy; and

$R^3$ is selected from the group consisting of $-(CH_2)_nCR^{10}R^5R^6$ wherein *n* is an integer equal to 0, 1 or 2, $R^5$ and $R^6$ are independently selected from hydrogen, alkyl, alkenyl, aryl, heteroaryl, heterocyclic, $-NR^7R^8$ where $R^7$ and $R^8$ are independently hydrogen or alkyl, and $-COOR^9$ where $R^9$ is alkyl, and further wherein $R^5$ and $R^6$ can be joined to form a cycloalkyl group, a cycloalkenyl group, an aryl group, a heteroaryl group, and a heterocyclic group, and when $R^5$ and $R^6$ do not join to form an aryl or heteroaryl group,

then $R^{10}$ is selected from hydrogen and alkyl with the proviso that when *n* is zero, then $R^{10}$ is hydrogen and when *n* is greater than zero and $R^5$ and $R^6$ are joined to form an aryl or heteroaryl group, then $R^{10}$ becomes a bond within that group;

X is oxygen or sulfur,

X' is hydrogen, hydroxy or fluoro;

X" is hydrogen, hydroxy or fluoro, or X' and X" together form an oxo group, and

pharmaceutically acceptable salts thereof

with the provisos that:

when $R^1$ is pyrid-3-yl, $R^2$ is ethyl, X is oxygen, and X' and X" are hydrogen, then $R^3$ is not $-CH_2CH(CH_3)_2$,
when $R^1$ is butyl, $R^2$ is methyl, X is oxygen, and X' and X" are hydrogen, then $R^3$ is not ethyl,
when $R^1$ is methyl, $-CH(CH_3)_2$, or cyclohexyl, $R^2$ is $-CH(CH_3)_2$, X is oxygen, X' is hydrogen and X" is hydroxyl then $R^3$ is not methyl,
when $R^1$ is ethyl or $C_6H_4Cl$-*p*, $R^2$ is phenyl, methyl or $-CH(CH_3)_2$, X is oxygen, X' is hydrogen and X" is fluorine, then $R^3$ is not methyl, and
when $R^1$ is indoxazin-3-yl, 2,4-dimethylthiazol-5-yl, 4-methyl-1,2,5-thiooxadizol-3-yl or 3,5-di(trifluoromethyl)phenyl, $R_2$ is methyl, X is oxygen, and X' and X" are hydrogen, then $R^3$ is not $-CH_2CH(CH_3)_2$.

**6.** The compound according to any one of claim 1 to 5 wherein $R^1$ is an unsubstituted naphthyl group selected from the group consisting of 1-naphthyl and 2-naphthyl.

**7.** The compound according to any one of claim 1 to 5 wherein $R^1$ is a substituted phenyl group of formula II.

**8.** The compound according to Claim 7 wherein the substituted phenyl group is defined by the following:

(a) monosubstituted phenyls having a single substitution at the 2,3 or 4 positions wherein each of the particular substituents is governed by the respective $R^a$, $R^b$ and $R^c$ groups;
(b) disubstituted phenyls having two substituents at the 2,3-positions, 2,4-positions, 2,5-positions, 2,6-positions, 3,4-positions, 3,5-positions or 3,6-positions wherein each of these substituents is governed by the respective $R^a$, $R^{a'}$, $R^b$, $R^{b'}$ and $R^c$ groups; and
(c) trisubstituted phenyls having three substituents at the 2,3,4-positions, 2,3,5-positions, 2,3,6-positions, 3,4,5-positions and 3,4,6-positions again wherein each of these substituents is governed by the respective $R^a$, $R^{a'}$, $R^b$, $R^{b'}$ and $R^c$ groups.

**9.** The compound according to claim 8 wherein the substituted phenyl groups are selected from the group consisting of 4-fluorophenyl, 4-chlorophenyl, 4-bromophenyl, 4-nitrophenyl, 4-methylphenyl, 3-methoxy-phenyl, 3- nitrophenyl, 3-fluorophenyl, 3-chlorophenyl, 3-bromophenyl, 3-thiomethoxyphenyl, 3-methylphenyl, 3-trifluoromethylphenyl, 2-hydroxyphenyl, 2-methylphenyl, 2-fluorophenyl, 3,4-dichlorophenyl, 3,4-methylene-dioxyphenyl, 3,5-difluorophenyl, 3,5-dichlorophenyl, 2,4-dichlorophenyl, and 2,5-difluorophenyl.

**10.** The compound according to any one of claims 1 to 5 wherein $R^1$ is selected from the group consisting of alkyl, alkenyl, alkcycloalkyl, cycloalkyl and cycloalkenyl groups.

**11.** The compound according to claim 10 wherein $R^1$ is alkyl.

**12.** The compound according to claim 10 wherein $R^1$ is cycloalkyl.

**13.** The compound according to claim 10 wherein $R^1$ is alkenyl.

**14.** The compound according to claim 10 wherein $R^1$ is cycloalkenyl.

**15.** The compound according to claim 10 wherein the $R^1$ alkyl, cycloalkyl, alkcycloalkyl, alkenyl and cycloalkenyl groups are selected from the group consisting of *sec*-butyl, cyclopropyl, cyclobutyl, cyclohexyl, cyclopentyl, cyclohex-1-enyl, $-CH_2$-cyclopropyl, $-CH_2$-cyclobutyl, $-CH_2$-cyclohexyl, $-CH_2$-cyclopentyl, $-CH_2CH_2$-cyclopropyl, $-CH_2CH_2$-cyclobutyl, $-CH_2CH_2$-cyclohexyl, and $-CH_2CH_2$-cyclopentyl.

**16.** The compound according to any one of claims 1 to 5 wherein $R^1$ is selected from the group consisting of heteroaryl and substituted heteroaryl groups.

**17.** The compound according to claim 16 wherein the $R^1$ heteroaryl and substituted heteroaryl groups are selected from the group consisting of pyrid-3-yl, pyrid-4-yl, thien-2-yl, thien-3-yl, benzothiazol-4-yl, 2-phenylbenzoxazol-5-yl, furan-2-yl, benzofuran-2-yl, benzothiophen-3-yl, 2-chlorothien-5-yl, 3-methylisoxazol-5-yl, 2-(phenylthio)thien-5-yl, 6-methoxythiophen-2-yl, 3-phenyl-1,2,4-thiooxadiazol-5-yl and 2-phenyloxazol-4-yl.

**18.** The compound according to any one of claims 1 to 17 wherein $R^2$ is selected from the group consisting of alkyl of from 1 to 4 carbon atoms, alkylalkoxy of from 1 to 4 carbon atoms, phenyl and alkylthioalkoxy of from 1 to 4 carbon atoms.

**19.** The compound according to claim 18 wherein $R^2$ is selected from the group consisting of methyl, ethyl, *n*-propyl, *iso*-propyl, *n*-butyl, *iso*-butyl, $-CH_2CH_2SCH_3$ and phenyl.

**20.** The compound according to any one of claims 1 to 19 wherein X' and X" are both hydrogen and X is oxygen.

**21.** The compound according to claim 20 wherein $R^3$ is selected from the group consisting of methyl, ethyl, *iso*-propyl, *n*-propyl, *n*-butyl, *iso*-butyl, cyclopentyl, allyl, *iso*-but-2-enyl, 3-methylpentyl, $-CH_2$-cyclopropyl, $-CH_2$-cyclohexyl, $-CH_2$-(3-tetrahydrofuranyl), $-CH_2$-thien-2-yl, $-CH_2$(1-methyl)cyclopropyl, $-CH_2$-thien-3-yl, $-CH_2$-C(O)O-*tert*-butyl, $-CH_2-C(CH_3)_3$, $-CH_2CH(CH_2CH_3)_2$, -2-methylcyclopentyl, -cyclohex-2-enyl, $-CH[CH(CH_3)_2]COOCH_3$, $-CH_2CH_2N(CH_3)_2$, $-CH_2C(CH_3)=CH_2$, and - $CH_2CH=C(CH_3)_2$.

**22.** The compound according to any one of claims 1 to 19 wherein X' and X" are both hydrogen and X is sulfur.

**23.** The compound according to claim 22 wherein $R^3$ is selected from the group consisting of *iso*-but-2-enyl and *iso*-butyl.

**24.** The compound according to any one of claim 1 to 5 wherein the compound of formula I is selected from the group consisting of:

*N*-(3-methylpentanoyl)alanine *iso*-butyl ester
*N*-[(4-chlorophenyl)acetyl]alanine *iso*-butyl ester
*N*-[(3,4-dichlorophenyl)acetyl]alanine *iso*-butyl ester
*N*-[(3-pyridyl)acetyl]alanine *iso*-butyl ester
*N*-[(1-naphthyl)acetyl]alanine *iso*-butyl ester
*N*-[(2-naphthyl)acetyl]alanine *iso*-butyl ester
*N*-(4-phenylbutanoyl)alanine *iso*-butyl ester
*N*-(5-phenylpentanoyl)alanine *iso*-butyl ester
*N*-[(4-pyridyl)acetyl]alanine *iso*-butyl ester
2-[(3,4-dichlorophenyl)acetamido]butyric acid *iso*-butyl ester
2-[(3-methoxyphenyl)acetamido]butyric acid *iso*-butyl ester
2-[(4-nitrophenyl)acetamido]butyric acid *iso*-butyl ester
2-[(3,4-methylenedioxyphenyl)acetamido)butyric acid *iso*-butyl ester
2-[(thien-3-yl)acetamido]butyric acid *iso*-butyl ester
2-[(4-chlorophenyl)acetamido]butyric acid *iso*-butyl ester
2-[(3-nitrophenyl)acetamido]butyric acid *iso*-butyl ester
2-[(2-hydroxyphenyl)acetamido]butyric acid *iso*-butyl ester
2-[(2-naphthyl)acetamido]butyric acid *iso*-butyl ester
2-[(2,4-dichlorophenyl)acetamido)butyric acid *iso*-butyl ester
2-[(4-bromophenyl)acetamido]butyric acid *iso*-butyl ester

2-[(3-chlorophenyl)acetamido])butyric acid *iso*-butyl ester
2-[(3-fluorophenyl)acetamido]butyric acid *iso*-butyl ester
2-[(benzothiazol-4-yl)acetamido]butyric acid *iso*-butyl ester
2-[(2-methylphenyl)acetamido]butyric acid *iso*-butyl ester
2-[(2-fluorophenyl)acetamido]butyric acid *iso*-butyl ester
2-[(4-fluorophenyl)acetamido]butyric acid *iso*-butyl ester
2-[(3-bromophenyl)acetamido]butyric acid *iso*-butyl ester
2-[(3-trifluoromethylphenyl)acetamido]butyric acid *iso*-butyl ester
2-[(2-thienyl)acetamido]butyric acid *iso*-butyl ester

*N*-[(3-chlorophenyl)acetyl]alanine 3-methylbut-2-enyl ester
*N*-[(3-chlorophenyl)acetyl]alanine cyclopropylmethyl ester
*N*-[(3-chlorophenyl)acetyl]alanine 2-thienylmethyl ester
*N*-[(3-chlorophenyl)acetyl]alanine (1-methylcyclopropyl)methyl ester
*N*-[(3-chlorophenyl)acetyl]alanine 3-thienylmethyl ester
*N*-[(3-chlorophenyl)acetyl]alanine 2-methylcyclopentyl ester
*N*-[(3-chlorophenyl)acetyl]alanine 2-methylprop-2-enyl ester
*N*-[(3-chlorophenyl)acetyl]alanine cyclohex-2-enyl ester
*N*-[(2-phenylbenzoxazol-5-yl)acetyl]alanine *iso*-butyl ester
*N*-[(3-methylthiophenyl)acetyl]alanine *iso*-butyl ester
*N*-4-[(2-furyl)acetyl]alanine *iso*-butyl ester
*N*-[(benzofuran-2-yl)acetyl]alanine *iso*-butyl ester
*N*-[(benzothiophen-3-yl)acetyl]alanine *iso*-butyl ester
*N*-[(2-chloro-5-thienyl)acetyl]alanine *iso*-butyl ester
*N*-[(3-methyl-isoxazol-5-yl)acetyl]alanine *iso*-butyl ester
*N*-[(2-phenylthiothienyl)acetyl]alanine *iso*-butyl ester
*N*-[(6-methoxybenzothiophen-2-yl)acetyl]alanine *iso*-butyl ester
*N*-[(3-phenyl-1,2,4-thiadiazol-5-yl)acetyl]alanine *iso*-butyl ester
*N*-[(2-phenyloxazol-4-yl)acetyl]alanine *iso*-butyl ester
*N*-[(3-methylphenyl)acetyl]alanine *iso*-butyl ester
*N*-[(2,5-difluorophenyl)acetyl]alanine *iso*-butyl ester
*N*-[(3,5-diflurophenyl)acetyl]alanine *iso*-butyl ester
*N*-[(3-thienyl)acetyl]alanine *iso*-butyl ester
*N*-[(4-methylphenyl)acetyl]alanine *iso*-butyl ester

*N*-[(3-nitrophenyl)acetyl]alanine *iso*-butyl ester
*N*-[(3,5-difluorophenyl)acetyl]alanine ethyl ester
*N*-[(3-nitrophenyl)acetyl]methionine ethyl ester
*N*-[(3-chlorophenyl)acetyl]alanine *iso*-butyl ester
*N*-[(3-chlorophenyl)acetyl]alanine 2-(*N*,*N*-dimethylamino)ethyl ester
2-[(3,5-dichlorophenyl)acetamido]hexanoic acid methyl ester
*N*-[(3,5-dichlorophenyl)acetyl]alanine *iso*-butyl ester
*N*-(cyclohexylacetyl)alanine *iso*-butyl ester
*N*-(cyclopentylacetyl)alanine *iso*-butyl ester
*N*-[(cyclohex-1-enyl)acetyl]alanine *iso*-butyl ester
*N*-[(3-chlorophenyl)acetyl]alanine 3-methylbut-2-enyl thioester

*N*-(3,5-difluorophenylacetyl)phenylglycine methyl ester
*N*-(3,5-difluorophenylacetyl)phenylglycine *iso*-butyl ester
*N*-(cyclopentylacetyl)phenylglycine methyl ester
*N*-(cyclopentylacetyl)alanine methyl ester
*N*-(cyclopropylacetyl)phenylglycine methyl ester
*N*-(cyclopropylacetyl)alanine methyl ester; and
*N*-[(3-nitrophenyl)acetyl]methionine *iso*-butyl ester.

**25.** A pharmaceutical composition comprising a pharmaceutically inert carrier and a pharmaceutically effective amount of a compound of formula I:

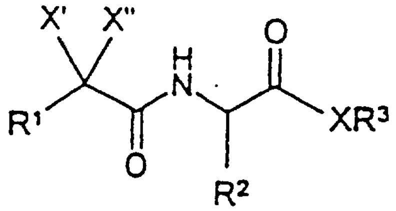

I

wherein $R^1$ is selected from the group consisting of

a) alkyl, alkenyl, alkcycloalkyl, naphthyl-$(R)_m$- wherein R is an alkylene group of from 1 to 8 carbon atoms and *m* is an integer equal to 0 or 1, cycloalkyl, cycloalkenyl, 3-pyridyl, 4-pyridyl and heteroaryl,

of 3 to 10 atoms and 1 to 4 heteroatoms selected from oxygen, sulfur and nitrogen wherein the heteroaryl group is optionally substituted with 1 to 3 substituents selected from the group consisting of alkyl, alkoxy, aryl, aryloxy, halo, nitro, thioalkoxy, and thioaryloxy with the proviso that for such heteroaryls when there is at least one nitrogen heteroatom, there is also at least one oxygen and/or sulfur heteroatom;

(b) a substituted phenyl group of formula II:

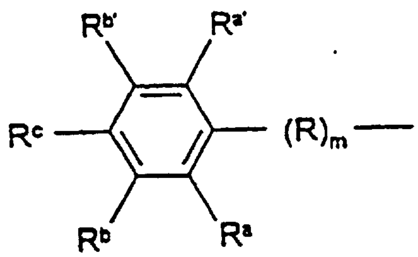

II

wherein R is alkylene of from 1 to 8 carbon atoms,

*m* is an integer equal to 0 or 1,

$R^a$ and $R^{a'}$ are independently selected from the group consisting of hydrogen, hydroxy, fluoro and methyl;

$R^b$ and $R^{b'}$ are independently selected from the group consisting of hydrogen, alkyl, alkoxy, aryl, cyano, cycloalkyl, halo, heteroaryl, heterocyclic, nitro, trihalomethyl, thioalkoxy, thioaryloxy, thioheteroaryloxy, and -C(O)$R^4$ where $R^4$ is selected from the group consisting of alkyl, aryl, alkoxy and aryloxy; and

$R^c$ is selected from the group consisting of hydrogen, alkyl, aryl, cyano, halo, nitro, and where $R^b$ and $R^c$ are fused to form a methylenedioxy ring with the phenyl ring; and

when $R^b$ and/or $R^{b'}$ and/or $R^c$ is fluoro, chloro, bromo and/or nitro, then $R^a$ and/or $R^{a'}$ can also be chloro; and

whereby $R^a$, $R^{a'}$, $R^b$, $R^{b'}$ and $R^c$ are not all simultaneously hydrogen; and

(c) 1- or 2-naphthyl-$(R)_m$- substituted at the 5, 6, 7 and/or 8 positions with 1 to 4 substituents selected from the group consisting alkyl, alkoxy, halo, cyano, nitro, trihalomethyl, and thioalkoxy wherein R is an alkylene group of from 1 to 8 carbon atoms and *m* is an integer equal to 0 or 1;

$R^2$ is selected from the group consisting of alkyl, phenyl, alkylalkoxy, alkylthioalkoxy; and

$R^3$ is selected from the group consisting of $-(CH_2)_nCR^{10}R^5R^6$ wherein *n* is an integer equal to 0, 1 or 2, $R^5$ and $R^6$ are independently selected from hydrogen, alkyl, alkenyl, aryl, heteroaryl, heterocyclic, $-NR^7R^8$ where $R^7$ and $R^8$ are independently hydrogen or alkyl and $-COOR^9$ where $R^9$ is alkyl, and further wherein $R^5$ and $R^6$ can be joined to form a cycloalkyl group, a cycloalkenyl group, an aryl group, a heteroaryl group, and a heterocyclic group, and when $R^5$ and $R^6$ do not join to form an aryl or heteroaryl group, then $R^{10}$ is selected from hydrogen and alkyl with the proviso that when *n* is zero, then $R^{10}$ is hydrogen and when *n* is greater than zero and $R^5$ and $R^6$ are joined to form an aryl or heteroaryl group, then $R^{10}$ becomes a bond within that group;

X is oxygen or sulfur;

X' is hydrogen, hydroxy or fluoro;

X" is hydrogen, hydroxy or fluoro, or X' and X" together form an oxo group, and

pharmaceutically acceptable salts thereof

with the provisos that:

when $R^1$ is pyrid-3-yl, $R^2$ is ethyl, X is oxygen, and X' and X" are hydrogen, then $R^3$ is not $-CH_2CH(CH_3)_2$,

and
when $R^1$ is indoxazin-3-yl, 2,4-dimethylthiazol-5-yl, 4-methyl-1,2,5-thiooxadizol-3-yl or 3,5-di(trifluoromethyl)phenyl, $R^2$ is methyl, X is oxygen, and X' and X" are hydrogen, then $R^3$ is not $-CH_2CH(CH_3)_2$.

**26.** The pharmaceutical composition according to Claim 25 wherein $R^1$ is an unsubstituted naphthyl group selected from the group consisting of 1-naphthyl and 2-naphthyl.

**27.** The pharmaceutical composition according to Claim 25 wherein $R^1$ is a substituted phenyl group of formula II.

**28.** The pharmaceutical composition according to Claim 27 wherein the substituted phenyl group is defined by the following:

(a) monosubstituted phenyls having a single substitution at the 2, 3 or 4 positions wherein each of the particular subsituents is governed by the respective $R^a$, $R^b$ and $R^c$ groups;
(b) disubstituted phenyls having two substituents at the 2,3-positions, 2,4-positions, 2,5-positions, 2,6-positions, 3,4-positions, 3,5-positions or 3,6-positions wherein each of these substituents is governed by the respective $R^a$, $R^{a'}$, $R^b$, $R^{b'}$ and $R^c$ groups; and
(c) trisubstituted phenyls having three substituents at the 2,3,4-positions, 2,3,5-positions, 2,3,6-positions, 3,4,5-positions and 3,4,6-positions again wherein each of these substituents is governed by the respective $R^a$, $R^{a'}$, $R^b$, $R^{b'}$ and $R^c$ groups.

**29.** The pharmaceutical composition according to Claim 28 wherein the substituted phenyl groups is selected from the group consisting of 4-fluorophenyl, 4-chlorophenyl, 4-bromophenyl, 4-nitrophenyl, 4-methylphenyl, 3-methoxyphenyl, 3-nitrophenyl, 3-fluorophenyl, 3-chlorophenyl, 3-bromophenyl, 3-thiomethoxyphenyl, 3-methylphenyl, 3-trifluoromethylphenyl, 2-hydroxy-phenyl, 2-methylphenyl, 2-fluorophenyl, 3,4-dichlorophenyl, 3,4-methylene-dioxyphenyl, 3,5-difluorophenyl, 3,5-dichlorophenyl, 2,4-dichlorophenyl, and 2,5-difluorophenyl.

**30.** The pharmaceutical composition according to Claim 25 wherein $R^1$ is selected from the group consisting of alkyl, alkenyl, alkcycloalkyl, cycloalkyl and cycloalkenyl groups.

**31.** The pharmaceutical composition according to Claim 30 wherein $R^1$ is alkyl.

**32.** The pharmaceutical composition according to Claim 30 wherein $R^1$ is cycloalkyl.

**33.** The pharmaceutical composition according to Claim 30 wherein $R^1$ is alkenyl.

**34.** The pharmaceutical composition according to Claim 30 wherein $R^1$ is cycloalkenyl.

**35.** The pharmaceutical composition according to Claim 30 wherein the $R^1$ alkyl, alkenyl, alkcycloalkyl, cycloalkyl, and cycloalkenyl groups are selected from the group consisting of *sec*-butyl, cyclopropyl, cyclobutyl, cyclohexyl, cyclopentyl, cyclohex-1-enyl, $-CH_2$-cyclopropyl, $-CH_2$-cyclobutyl, $-CH_2$-cyclohexyl, $-CH_2$-cyclopentyl, $-CH_2CH_2$-cyclopropyl, $-CH_2CH_2$-cyclobutyl, $-CH_2CH_2$-cyclohexyl, and $-CH_2CH_2$-cyclopentyl.

**36.** The pharmaceutical composition according to Claim 25 wherein $R^1$ is selected from the group consisting of heteroaryl and substituted heteroaryl groups.

**37.** The pharmaceutical composition according to Claim 36 wherein the $R^1$ heteroaryl and substituted heteroaryl groups are selected from the group consisting of pyrid-3-yl, pyrid-4-yl, thien-2-yl, thien-3-yl, benzothiazol-4-yl, 2-phenylbenzoxazol-5-yl, furan-2-yl, benzofuran-2-yl, benzothiophen-3-yl, 2-chlorothien-5-yl, 3-methylisoxazol-5-yl, 2-(phenylthio)thien-5-yl, 6-methoxythiophen-2-yl, 3-phenyl-1,2,4-thiooxadiazol-5-yl and 2-phenyloxazol-4-yl.

**38.** The pharmaceutical composition according to any one of claims 25 to 37 wherein $R^2$ is selected from the group consisting of alkyl of from 1 to 4 carbon atoms, alkylalkoxy of from 1 to 4 carbon atoms, phenyl and alkylthioalkoxy of from 1 to 4 carbon atoms.

**39.** The pharmaceutical composition according to Claim 38 wherein $R^2$ is selected from the group consisting of methyl, ethyl, *n*-propyl, *iso*-propyl, *n*-butyl, *iso*-butyl, $-CH_2CH_2SCH_3$ and phenyl.

**40.** The pharmaceutical composition according to any one of claims 25 to 39 wherein X' and X" are both hydrogen and X is oxygen.

**41.** The pharmaceutical composition according to Claim 40 wherein $R^3$ is selected from the group consisting of methyl, ethyl, *iso*-propyl, *n*-propyl, *n*-butyl, *iso*-butyl, cyclopentyl, allyl, *iso*-but-2-enyl, 3-methylpentyl, $-CH_2$-cyclopropyl, $-CH_2$-cyclohexyl, $-CH_2$-(3-tetrahydrofuranyl), $-CH_2$-thien-2-yl, $-CH_2$(1-methyl)cyclopropyl, $-CH_2$-thien-3-yl, $-CH_2-C(O)O$-*tert*-butyl, $-CH_2-C(CH_3)_3$, $-CH_2CH(CH_2CH_3)_2$, -2-methylcyclopentyl, -cyclohex-2-enyl, $-CH[CH(CH_3)_2]COOCH_3$, $-CH_2CH_2N(CH_3)_2$, $-CH_2C(CH_3)=CH_2$, and $-CH_2CH=C(CH_3)_2$.

**42.** The pharmaceutical composition according to any one of claim 25 to 39 wherein X' and X" are both hydrogen and X is sulfur.

**43.** The pharmaceutical composition according to Claim 42 wherein $R^3$ is selected from the group consisting of *iso*-but-2-enyl and *iso*-butyl.

**44.** The pharmaceutical composition according to Claim 25 wherein the compound of formula I is selected from the group consisting of:

*N*-(3-methylpentanoyl)alanine *iso*-butyl ester
*N*-[(4-chlorophenyl)acetyl]alanine *iso*-butyl ester
*N*-[(3,4-dichlorophenyl)acetyl]alanine *iso*-butyl ester
*N*-[(3-pyridyl)acetyl]alanine *iso*-butyl ester
*N*-[(1-naphthyl)acetyl]alanine *iso*-butyl ester
*N*-[(2-naphthyl)acetyl]alanine *iso*-butyl ester
*N*-(4-phenylbutanoyl)alanine *iso*-butyl ester
*N*-(5-phenylpentanoyl)alanine *iso*-butyl ester
*N*-[(4-pyridyl)acetyl]alanine *iso*-butyl ester
2-[(3,4-dichlorophenyl)acetamido]butyric acid *iso*-butyl ester
2-[(3-methoxyphenyl)acetamido]butyric acid *iso*-butyl ester
2-[(4-nitrophenyl)acetamido]butyric acid *iso*-butyl ester
2-[(3,4-methylenedioxyphenyl)acetamido]butyric acid *iso*-butyl ester
2-[(thien-3-yl)acetamido]butyric acid *iso*-butyl ester
2-[(4-chlorophenyl)acetamido]butyric acid *iso*-butyl ester
2-[(3-nitrophenyl)acetamido]butyric acid *iso*-butyl ester
2-[(2-hydroxyphenyl)acetamido]butyric acid *iso*-butyl ester
2-[(2-naphthyl)acetamido]butyric acid *iso*-butyl ester
2-[(2,4-dichlorophenyl)acetamido]butyric acid *iso*-butyl ester
2-[(4-bromophenyl)acetamido]butyric acid *iso*-butyl ester
2-[(3-chlorophenyl)acetamido])butyric acid *iso*-butyl ester
2-[(3-fluorophenyl)acetamido]butyric acid *iso*-butyl ester
2-[(benzothiazol-4-yl)acetamido]butyric acid *iso*-butyl ester
2-[(2-methylphenyl)acetamido]butyric acid *iso*-butyl ester
2-[(2-fluorophenyl)acetamido]butyric acid *iso*-butyl ester
2-[(4-fluorophenyl)acetamido]butyric acid *iso*-butyl ester
2-[(3-bromophenyl)acetamido]butyric acid *iso*-butyl ester
2-[(3-trifluoromethylphenyl)acetamido]butyric acid *iso*-butyl ester
2-[(2-thienyl)acetamido]butyric acid *iso*-butyl ester

*N*-[(3-chlorophenyl)acetyl]alanine 3-methylbut-2-enyl ester
*N*-[(3-chlorophenyl)acetyl]alanine cyclopropylmethyl ester
*N*-[(3-chlorophenyl)acetyl]alanine 2-thienylmethyl ester
*N*-[(3-chlorophenyl)acetyl]alanine (1-methylcyclopropyl)methyl ester
*N*-[(3-chlorophenyl)acetyl]alanine 3-thienylmethyl ester
*N*-[(3-chlorophenyl)acetyl]alanine 2-methylcyclopentyl ester
*N*-[(3-chlorophenyl)acetyl]alanine 2-methylprop-2-enyl ester
*N*-[(3-chlorophenyl)acetyl]alanine cyclohex-2-enyl ester
*N*-[(2-phenylbenzoxazol-5-yl)acetyl]alanine *iso*-butyl ester
*N*-[(3-methylthiophenyl)acetyl]alanine *iso*-butyl ester

*N*-4-[(2-furyl)acetyl]alanine *iso*-butyl ester
*N*-[(benzofuran-2-yl)acetyl]alanine *iso*-butyl ester
*N*-[(benzothiophen-3-yl)acetyl]alanine *iso*-butyl ester
*N*-[(2-chloro-5-thienyl)acetyl]alanine *iso*-butyl ester
*N*-[(3-methyl-isoxazol-5-yl)acetyl]alanine *iso*-butyl ester
*N*-[(2-phenylthiothienyl)acetyl]alanine *iso*-butyl ester
*N*-[(6-methoxybenzothiophen-2-yl)acetyl]alanine *iso*-butyl ester
*N*-[(3-phenyl-1,2,4-thiadiazol-5-yl)acetyl]alanine *iso*-butyl ester
*N*-[(2-phenyloxazol-4-yl)acetyl]alanine *iso*-butyl ester
*N*-[(3-methylphenyl)acetyl]alanine *iso*-butyl ester
*N*-[(2,5-difluorophenyl)acetyl]alanine *iso*-butyl ester
*N*-[(3,5-diflurophenyl)acetyl]alanine *iso*-butyl ester
*N*-[(3-thienyl)acetyl]alanine *iso*-butyl ester
*N*-[(4-methylphenyl)acetyl]alanine *iso*-butyl ester

*N*-[(3-nitrophenyl)acetyl]alanine *iso*-butyl ester
*N*-[(3,5-difluorophenyl)acetyl]alanine ethyl ester
*N*-[(3-nitrophenyl)acetyl]methionine ethyl ester
*N*-[(3-chlorophenyl)acetyl]alanine *iso*-butyl ester
*N*-[(3-chlorophenyl)acetyl]alanine 2-(*N*,*N*-dimethylamino)ethyl ester
2-[(3,5-dichlorophenyl)acetamido]hexanoic acid methyl ester
*N*-[(3,5-dichlorophenyl)acetyl]alanine *iso*-butyl ester
*N*-(cyclohexylacetyl)alanine *iso*-butyl ester
*N*-(cyclopentylacetyl)alanine iso-butyl ester
*N*-[(cyclohex-1-enyl)acetyl]alanine *iso*-butyl ester
*N*-[(3-chlorophenyl)acetyl]alanine 3-methylbut-2-enyl thioester

*N*-(3,5-difluorophenylacetyl)phenylglycine methyl ester
*N*-(3,5-difluorophenylacetyl)phenylglycine *iso*-butyl ester
*N*-(cyclopentylacetyl)phenylglycine methyl ester
*N*-(cyclopentylacetyl)alanine methyl ester
*N*-(cyclopropylacetyl)phenylglycine methyl ester
*N*-(cyclopropylacetyl)alanine methyl ester; and
*N*-[(3-nitrophenyl)acetyl]methionine *iso*-butyl ester.

**45.** Use of a compound of formula I' or a mixture of compounds of formula I' in the manufacture of a medicament for inhibiting β-amyloid release and/or its synthesis in a cell, wherein formula I' is:

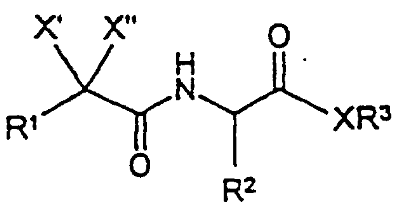

I'

wherein $R^1$ is selected from the group consisting of

a) alkyl, alkenyl, alkcycloalkyl, phenyl-$(R)_m$-, naphthyl-$(R)_m$- wherein R is an alkylene group of from 1 to 8 carbon atoms and *m* is an integer equal to 0 or 1, cycloalkyl, cycloalkenyl, 3-pyridyl, 4-pyridyl and heteroaryl, of 3 to 10 atoms and 1 to 4 heteroatoms selected from oxygen, sulfur and nitrogen wherein the heteroaryl group is optionally substituted with 1 to 3 substituents selected from the group consisting of alkyl, alkoxy, aryl, aryloxy, halo, nitro, thioalkoxy, and thioaryloxy with the proviso that for such heteroaryls when there is at least one nitrogen heteroatom, there is also at least one oxygen and/or sulfur heteroatom;
b) a substituted phenyl group of formula II:

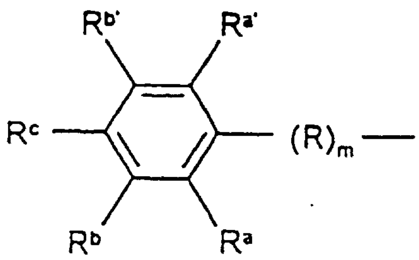

II

wherein R is alkylene of from 1 to 8 carbon atoms,

*m* is an integer equal to 0 or 1 ,

$R^a$ and $R^{a'}$ are independently selected from the group consisting of hydrogen, hydroxy, fluoro and methyl;

$R^b$ and $R^{b'}$ are independently selected from the group consisting of hydrogen, alkyl, alkoxy, aryl, cyano, cycloalkyl, halo, heteroaryl, heterocyclic, nitro, trihalomethyl, thioalkoxy, thioaryloxy, thioheteroaryloxy, and -C(O)$R^4$ where $R^4$ is selected from the group consisting of alkyl, aryl, alkoxy and aryloxy; and

$R^c$ is selected from the group consisting of hydrogen, alkyl, aryl, cyano, halo, nitro, and where $R^b$ and $R^c$ are fused to form a methylenedioxy ring with the phenyl ring; and

when $R^b$ and/or $R^{b'}$ and/or $R^c$ is fluoro, chloro, bromo and/or nitro, then $R^a$ and/or $R^{a'}$ can also be chloro; and

c) 1- or 2-naphthyl-$(R)_m$- substituted at the 5, 6, 7 and/or 8 positions with 1 to 4 substituents selected from the group consisting alkyl, alkoxy, halo, cyano, nitro, trihalomethyl, and thioalkoxy wherein R is an alkylene group of from 1 to 8 carbon atoms and *m* is an integer equal to 0 or 1;

$R^2$ is selected from the group consisting of hydrogen, alkyl, phenyl, alkylalkoxy, alkylthioalkoxy; and

$R^3$ is selected from the group consisting of $-(CH_2)_nCR^{10}R^5R^6$ wherein *n* is an integer equal to 0, 1 or 2, $R^5$ and $R^6$ are independently selected from hydrogen, alkyl, alkenyl, aryl, heteroaryl, heterocyclic, $-NR^7R^8$ where $R^7$ and $R^8$ are independently hydrogen or alkyl and $-COOR^9$ where $R^9$ is alkyl, and further wherein $R^5$ and $R^6$ can be joined to form a cycloalkyl group, a cycloalkenyl group, an aryl group, a heteroaryl group, and a heterocyclic group, and when $R^5$ and $R^6$ do not join to form an aryl or heteroaryl group, then $R^{10}$ is selected from hydrogen and alkyl with the proviso that when n is zero, then $R^{10}$ is hydrogen and when *n* is greater than zero and $R^5$ and $R^6$ are joined to form an aryl or heteroaryl group, then $R^{10}$ becomes a bond within that group;

X is oxygen or sulfur;

X' is hydrogen, hydroxy or fluoro;

X" is hydrogen, hydroxy or fluoro, or X' and X" together form an oxo group, and pharmaceutically acceptable salts thereof with the provisos that:

when $R^1$ is phenyl, $R^2$ is $-CH(CH_3)CH_2CH_3$, X is oxygen, and X' and X" are hydrogen, then $R^3$ is not $-CH_2CH_3$ or $-CH_2CH(CH_3)_2$

when $R^1$ is phenyl, $R^3$ is $-CH_2CH(CH_3)_2$, X is oxygen, and X' and X" are hydrogen, then $R^2$ is not $-CH(CH_3)_2$

when $R^1$ is pyrid-3-yl, $R^2$ is ethyl, X is oxygen, and X' and X" are hydrogen, then $R^3$ is not $-CH_2CH(CH_3)_2$,

when $R^1$ is indoxazin-3-yl, 2,4-dimethylthiazol-5-yl, 4-methyl-1,2,5- thiooxadizol-3-yl or 3,5-di(trifluoromethyl)phenyl, $R^2$ is methyl, X is oxygen, and X' and X" are hydrogen, then $R^3$ is not $-CH_2CH(CH_3)_2$, and

when $R^1$ is $-CH_2$-phenyl, $R^3$ is $-CH_2CH_3$, X is oxygen, and X' and X" are hydrogen, then $R^2$ is not $-CH_2CH(CH_3)_2$.

**46.** Use of a compound of formula I' or a mixture of compounds of formula I' in the manufacture of a medicament comprising the compound and a pharmaceutically inert carrier for preventing the onset of Alzheimer's Disease in a patient at risk for developing Alzheimer's Disease wherein formula I' is:

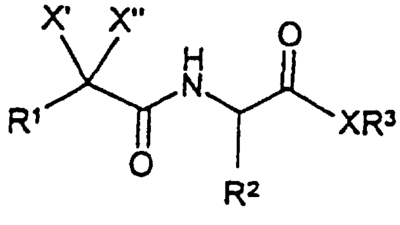

I'

wherein $R^1$ is selected from the group consisting of

a) alkyl, alkenyl, alkcycloalkyl, phenyl-$(R)_m$-, naphthyl-$(R)_m$- wherein R is an alkylene group of from 1 to 8 carbon atoms and *m* is an integer equal to 0 or 1, cycloalkyl, cycloalkenyl, 3-pyridyl, 4-pyridyl and heteroaryl, of 3 to 10 atoms and 1 to 4 heteroatoms selected from oxygen, sulfur and nitrogen wherein the heteroaryl group is optionally substituted with 1 to 3 substituents selected from the group consisting of alkyl, alkoxy, aryl, aryloxy, halo, nitro, thioalkoxy, and thioaryloxy with the proviso that for such heteroaryls when there is at least one nitrogen heteroatom, there is also at least one oxygen and/or sulfur heteroatom;
b) a substituted phenyl group of formula II:

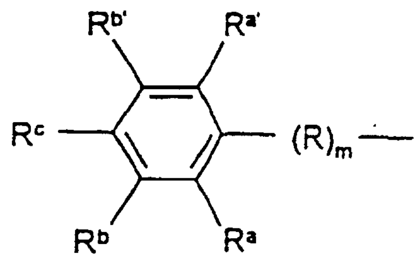

II

wherein R is alkylene of from 1 to 8 carbon atoms,
*m* is an integer equal to 0 or 1 ,
$R^a$ and $R^{a'}$ are independently selected from the group consisting of hydrogen, hydroxy, fluoro and methyl;
$R^b$ and $R^{b'}$ are independently selected from the group consisting of hydrogen, alkyl, alkoxy, aryl, cyano, cycloalkyl, halo, heteroaryl, heterocyclic, nitro, trihalomethyl, thioalkoxy, thioaryloxy, thioheteroaryloxy, and -C(O)$R^4$ where $R^4$ is selected from the group consisting of alkyl, aryl, alkoxy and aryloxy; and
$R^c$ is selected from the group consisting of hydrogen, alkyl, aryl, cyano, halo, nitro, and where $R^b$ and $R^c$ are fused to form a methylenedioxy ring with the phenyl ring; and
when $R^b$ and/or $R^{b'}$ and/or $R^c$ is fluoro, chloro, bromo and/or nitro, then $R^a$ and/or $R^{a'}$ can also be chloro; and
c) 1- or 2-naphthyl-$(R)_m$- substituted at the 5, 6, 7 and/or 8 positions with 1 to 4 substituents selected from the group consisting alkyl, alkoxy, halo, cyano, nitro, trihalomethyl, and thioalkoxy wherein R is an alkylene group of from 1 to 8 carbon atoms and *m* is an integer equal to 0 or 1;
$R^2$ is selected from the group consisting of hydrogen, alkyl, phenyl, alkylalkoxy, alkylthioalkoxy; and
$R^3$ is selected from the group consisting of $-(CH_2)_nCR^{10}R^5R^6$ wherein *n* is an integer equal to 0, 1 or 2, $R^5$ and $R^6$ are independently selected from hydrogen, alkyl, alkenyl, aryl, heteroaryl, heterocyclic, $-NR^7R^8$ where $R^7$ and $R^8$ are independently hydrogen or alkyl and $-COOR^9$ where $R^9$ is alkyl, and further wherein $R^5$ and $R^6$ can be joined to form a cycloalkyl group, a cycloalkenyl group, an aryl group, a heteroaryl group, and a heterocyclic group, and when $R^5$ and $R^6$ do not join to form an aryl or heteroaryl group, then $R^{10}$ is selected from hydrogen and alkyl with the proviso that when n is zero, then $R^{10}$ is hydrogen and when *n* is greater than zero and $R^5$ and $R^6$ are joined to form an aryl or heteroaryl group, then $R^{10}$ becomes a bond within that group;
X is oxygen or sulfur;
X' is hydrogen, hydroxy or fluoro;
X" is hydrogen, hydroxy or fluoro, or X' and X" together form an oxo group, and pharmaceutically acceptable salts thereof with the provisos that:

when $R^1$ is phenyl, $R^2$ is $-CH(CH_3)CH_2CH_3$, X is oxygen, and X' and X" are hydrogen, then $R^3$ is not $-CH_2CH_3$ or $-CH_2CH(CH_3)_2$
when $R^1$ is phenyl, $R^3$ is $-CH_2CH(CH_3)_2$, X is oxygen, and X' and X" are hydrogen, then $R^2$ is not $-CH(CH_3)_2$
when $R^1$ is pyrid-3-yl, $R^2$ is ethyl, X is oxygen, and X' and X" are hydrogen, then $R^3$ is not $-CH_2CH(CH_3)_2$,
when $R^1$ is indoxazin-3-yl, 2,4-dimethylthiazol-5-yl, 4-methyl-1,2,5- thiooxadizol-3-yl or 3,5-di(trifluoromethyl)phenyl, $R^2$ is methyl, X is oxygen, and X' and X" are hydrogen, then $R^3$ is not $-CH_2CH(CH_3)_2$, and
when $R^1$ is $-CH_2$-phenyl, $R^3$ is $-CH_2CH_3$, X is oxygen, and X' and X" are hydrogen, then $R^2$ is not $-CH_2CH(CH_3)_2$.

**47.** Use of a compound of formula I' or a mixture of compounds of formula I' in the manufacture of a medicament

comprising the compound and a pharmaceutically inert carrier for inhibiting further deterioration of the condition of a patient with Alzheimer's Disease wherein formula I' is:

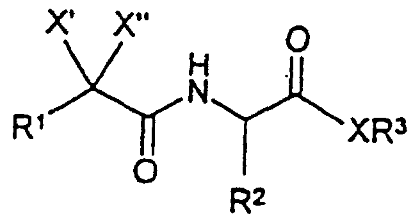

I'

wherein $R^1$ is selected from the group consisting of

a) alkyl, alkenyl, alkcycloalkyl, phenyl-$(R)_m$-, naphthyl-$(R)_m$- wherein R is an alkylene group of from 1 to 8 carbon atoms and *m* is an integer equal to 0 or 1, cycloalkyl, cycloalkenyl, 3-pyridyl, 4-pyridyl and heteroaryl, of 3 to 10 atoms and 1 to 4 heteroatoms selected from oxygen, sulfur and nitrogen wherein the heteroaryl group is optionally substituted with 1 to 3 substituents selected from the group consisting of alkyl, alkoxy, aryl, aryloxy, halo, nitro, thioalkoxy, and thioaryloxy with the proviso that for such heteroaryls when there is at least one nitrogen heteroatom, there is also at least one oxygen and/or sulfur heteroatom;

b) a substituted phenyl group of formula II:

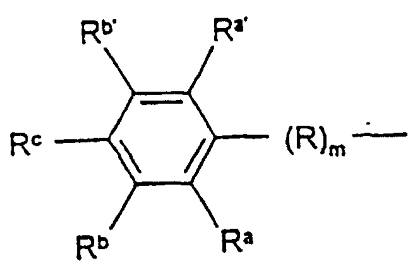

II

wherein R is alkylene of from 1 to 8 carbon atoms,

*m* is an integer equal to 0 or 1 ,

$R^a$ and $R^{a'}$ are independently selected from the group consisting of hydrogen, hydroxy, fluoro and methyl;

$R^b$ and $R^{b'}$ are independently selected from the group consisting of hydrogen, alkyl, alkoxy, aryl, cyano, cycloalkyl, halo, heteroaryl, heterocyclic, nitro, trihalomethyl, thioalkoxy, thioaryloxy, thioheteroaryloxy, and -C(O)$R^4$ where $R^4$ is selected from the group consisting of alkyl, aryl, alkoxy and aryloxy; and

$R^c$ is selected from the group consisting of hydrogen, alkyl, aryl, cyano, halo, nitro, and where $R^b$ and $R^c$ are fused to form a methylenedioxy ring with the phenyl ring; and

when $R^b$ and/or $R^{b'}$ and/or $R^c$ is fluoro, chloro, bromo and/or nitro, then $R^a$ and/or $R^{a'}$ can also be chloro; and

c) 1- or 2-naphthyl-$(R)_m$- substituted at the 5, 6, 7 and/or 8 positions with 1 to 4 substituents selected from the group consisting alkyl, alkoxy, halo, cyano, nitro, trihalomethyl, and thioalkoxy wherein R is an alkylene group of from 1 to 8 carbon atoms and *m* is an integer equal to 0 or 1;

$R^2$ is selected from the group consisting of hydrogen, alkyl, phenyl, alkylalkoxy, alkylthioalkoxy; and

$R^3$ is selected from the group consisting of $-(CH_2)_nCR^{10}R^5R^6$ wherein *n* is an integer equal to 0, 1 or 2, $R^5$ and $R^6$ are independently selected from hydrogen, alkyl, alkenyl, aryl, heteroaryl, heterocyclic, $-NR^7R^8$ where $R^7$ and $R^8$ are independently hydrogen or alkyl and $-COOR^9$ where $R^9$ is alkyl, and further wherein $R^5$ and $R^6$ can be joined to form a cycloalkyl group, a cycloalkenyl group, an aryl group, a heteroaryl group, and a heterocyclic group, and when $R^5$ and $R^6$ do not join to form an aryl or heteroaryl group, then $R^{10}$ is selected from hydrogen and alkyl with the proviso that when n is zero, then $R^{10}$ is hydrogen and when *n* is greater than zero and $R^5$ and $R^6$ are joined to form an aryl or heteroaryl group, then $R^{10}$ becomes a bond within that group;

X is oxygen or sulfur;

X' is hydrogen, hydroxy or fluoro;

X" is hydrogen, hydroxy or fluoro, or X' and X" together form an oxo group, and pharmaceutically acceptable salts thereof with the provisos that:

when $R^1$ is phenyl, $R^2$ is $-CH(CH_3)CH_2CH_3$, X is oxygen, and X' and X" are hydrogen, then $R^3$ is not $-CH_2CH_3$ or $-CH_2CH(CH_3)_2$
when $R^1$ is phenyl, $R^3$ is $-CH_2CH(CH_3)_2$, X is oxygen, and X' and X" are hydrogen, then $R^2$ is not $-CH(CH_3)_2$
when $R^1$ is pyrid-3-yl, $R^2$ is ethyl, X is oxygen, and X' and X" are hydrogen, then $R^3$ is not $-CH_2CH(CH_3)_2$,
when $R^1$ is indoxazin-3-yl, 2,4-dimethylthiazol-5-yl, 4-methyl-1,2,5- thiooxadizol-3-yl or 3,5-di(trifluoromethyl)phenyl, $R^2$ is methyl, X is oxygen, and X' and X" are hydrogen, then $R^3$ is not $-CH_2CH(CH_3)_2$, and
when R' is $-CH_2$-phenyl, $R^3$ is $-CH_2CH_3$, X is oxygen, and X' and X" are hydrogen, then $R^2$ is not $-CH_2CH(CH_3)_2$.

**48.** The use according to any one of claims 45 to 47 wherein $R^1$ is an unsubstituted phenyl group.

**49.** The use according to any one of claims 45 to 47 wherein $R^1$ is an unsubstituted naphthyl group selected from the group consisting of 1-naphthyl and 2-naphthyl.

**50.** The use according to any one of claims 45 to 47 wherein $R^1$ is a substituted phenyl group of formula II.

**51.** The use according to claim 50 wherein the substituted phenyl group is defined by the following:

(a) monosubstituted phenyls having a single substitution at the 2, 3 or 4 positions wherein each of the particular substituents is governed by the respective $R^a$, $R^b$ and $R^c$ groups;
(b) disubstituted phenyls having two substituents at the 2,3-positions, 2,4-positions, 2,5-positions, 2,6-positions, 3,4-positions, 3,5-positions or 3,6-positions wherein each of these substituents is governed by the respective $R^a$, $R^{a'}$, $R^b$, $R^{b'}$ and $R^c$ groups; and
(c) trisubstituted phenyls having three substituents at the 2,3,4-positions, 2,3,5-positions, 2,3,6-positions, 3,4,5-positions and 3,4,6-positions again wherein each of these substituents is governed by the respective $R^a$, $R^{a'}$, $R^b$, $R^{b'}$ and $R^c$ groups.

**52.** The use according to claim 51 wherein the substituted phenyl groups are selected from the group consisting of 4-fluorophenyl, 4-chlorophenyl, 4-bromophenyl, 4-nitrophenyl, 4-methylphenyl, 3-methoxy-phenyl, 3- nitrophenyl, 3-fluorophenyl, 3-chlorophenyl, 3-bromophenyl, 3-thiomethoxyphenyl, 3-methylphenyl, 3-trifluoromethylphenyl, 2-hydroxyphenyl, 2-methylphenyl, 2-fluorophenyl, 3,4-dichlorophenyl, 3,4-methylene-dioxyphenyl, 3,5-difluorophenyl, 3,5-dichlorophenyl, 2,4-dichlorophenyl, and 2,5-difluorophenyl.

**53.** The use according to any one of claims 45 to 47 wherein $R^1$ is a phenyl-$(R)_m$- group where R is alkylene of from 1 to 8 carbon atoms and m is equal to 1.

**54.** The compound according to claim 53 wherein $R^1$ is selected from the group consisting of benzyl, 3-phenyl-n-propyl and 4-phenyl-n-butyl.

**55.** The use according to any one of claims 45 to 47 wherein $R^1$ is selected from the group consisting of alkyl, alkenyl, alkcycloalkyl, cycloalkyl and cycloalkenyl groups.

**56.** The use according to claim 55 wherein $R^1$ is alkyl.

**57.** The compound according to claim 55 wherein $R^1$ is cycloalkyl.

**58.** The compound according to claim 55 wherein $R^1$ is alkenyl.

**59.** The compound according to claim 55 wherein $R^1$ is cycloalkenyl.

**60.** The compound according to claim 55 wherein the $R^1$ alkyl, cycloalkyl, alkcycloalkyl, alkenyl and cycloalkenyl groups are selected from the group consisting of *sec*-butyl, cyclopropyl, cyclobutyl, cyclohexyl, cyclopentyl, cyclohex-1-enyl, $-CH_2$-cyclopropyl, $-CH_2$-cyclobutyl, $-CH_2$-cyclohexyl, $-CH_2$-cyclopentyl, $-CH_2CH_2$-cyclopropyl, $-CH_2CH_2$-cyclobutyl, $-CH_2CH_2$-cyclohexyl, and $-CH_2CH_2$-cyclopentyl.

**61.** The use according to any one of claims 45 to 47 wherein $R^1$ is selected from the group consisting of heteroaryl

and substituted heteroaryl groups.

**62.** The use according to claim 61 wherein the $R^1$ heteroaryl and substituted heteroaryl groups are selected from the group consisting ofpyrid-3-yl, pyrid-4-yl, thien-2-yl, thien-3-yl, benzothiazol-4-yl, 2-phenylbenzoxazol-5-yl, furan-2-yl, benzofuran-2-yl, benzothiophen-3-yl, 2-chlorothien-5-yl, 3-methylisoxazol-5-yl, 2-(phenylthio)thien-5-yl, 6-methoxythiophen-2-yl, 3-phenyl-1,2,4-thiooxadiazol-5-yl and 2-phenyloxazol-4-yl.

**63.** The use according to any one of claims 45 to 62 wherein $R^2$ is selected from the group consisting of alkyl of from 1 to 4 carbon atoms, alkylalkoxy of from 1 to 4 carbon atoms, phenyl and alkylthioalkoxy of from 1 to 4 carbon atoms.

**64.** The use according to claim 63 wherein $R^2$ is selected from the group consisting of methyl, ethyl, *n*-propyl, *iso*-propyl, *n*-butyl, *iso*-butyl, $-CH_2CH_2SCH_3$ and phenyl.

**65.** The use according to any one of claims 45 to 64 wherein X' and X'' are both hydrogen and X is oxygen.

**66.** The use according to claim 65 wherein.$R^3$ is selected from the group consisting of methyl, ethyl, *iso*-propyl, *n*-propyl, *n*-butyl, *iso*-butyl, cyclopentyl, allyl, *iso*-but-2-enyl, 3-methylpentyl, $-CH_2$-cyclopropyl, $-CH_2$-cyclohexyl, $-CH_2$-(3-tetrahydrofuranyl), $-CH_2$-thien-2-yl, $-CH_2$(1-methyl)cyclopropyl, $-CH_2$-thien-3-yl, $-CH_2$-C(O)O-*tert*-butyl, $-CH_2-C(CH_3)_3$, $-CH_2CH(CH_2CH_3)_2$, -2-methylcyclopentyl, -cyclohex-2-enyl, $-CH[CH(CH_3)_2]COOCH_3$, $-CH_2CH_2N(CH_3)_2$, $-CH_2C(CH_3)=CH_2$, and $-CH_2CH=C(CH_3)_2$.

**67.** The use according to any one of claims 45 to 64 wherein X' and X'' are both hydrogen and X is sulfur.

**68.** The use according to claim 67 wherein $R^3$ is selected from the group consisting of *iso*-but-2-enyl and *iso*-butyl.

**69.** The use according to any one of claims 45 to 47 wherein the compound of formula I is selected from the group consisting of:

*N*-(phenylacetyl)alanine *iso*-butyl ester
*N*-(3-phenylpropionyl)alanine *iso*-butyl ester
*N*-(3-methylpentanoyl)alanine *iso*-butyl ester
*N*-[(4-chlorophenyl)acetyl]alanine *iso*-butyl ester
*N*-[(3,4-dichlorophenyl)aceryl]alanine *iso*-butyl ester
*N*-[(3-pyridyl)acetyl]alanine *iso*-butyl ester
*N*-[(1-naphthyl)acetyl]alanine *iso*-butyl ester
*N*-[(2-naphthyl)acetyl]alanine *iso*-butyl ester
*N*-(4-phenylbutanoyl)alanine *iso*-butyl ester
*N*-(5-phenylpentanoyl)alanine *iso*-butyl ester
*N*-[(4-pyridyl)acetyl]alanine *iso*-butyl ester
2-[(3,4-dichlorophenyl)acetamido]butyric acid *iso*-butyl ester
2-[(3-methoxyphenyl)acetamido]butyric acid *iso*-butyl ester
2-[(4-nitrophenyl)acetamido]butyric acid *iso*-butyl ester
2-[(3,4-methylenedioxyphenyl)acetamido]buryric acid *iso*-butyl ester
2-[(thien-3-yl)acetamido]butyric acid *iso*-butyl ester
2-[(4-chlorophenyl)acetamido]butyric acid *iso*-butyl ester
2-[(3-nitrophenyl)acetamido]butyric acid *iso*-butyl ester
2-[(2-hydroxyphenyl)acetamido]butyric acid *iso*-butyl ester
2-[(2-naphthyl)acetamido]butyric acid *iso*-butyl ester
2-[(2,4-dichlorophenyl)acetamido]butyric acid *iso*-butyl ester
2-[(4-bromophenyl)acetamido]butyric acid *iso*-butyl ester
2-[(3-chlorophenyl)acetamido])butyric acid *iso*-butyl ester
2-[(3-fluorophenyl)acetamido]butyric acid *iso*-butyl ester
2-[(benzothiazol-4-yl)acetamido]butyric acid *iso*-butyl ester
2-[(2-methylphenyl)acetamido]butyric acid *iso*-butyl ester
2-[(2-fluorophenyl)acetamido]butyric acid *iso*-butyl ester
2-[(4-fluorophenyl)acetamido]butyric acid *iso*-butyl ester
2-[(3-bromophenyl)acetamido]butyric acid *iso*-butyl ester
2-[(3-trifluoromethylphenyl)acetamido]butyric acid *iso*-butyl ester

2-[(2-thienyl)acetamido]butyric acid *iso*-butyl ester
2-(phenylacetamido)butyric acid *iso*-butyl ester
*N*-(phenylacetyl)valine 2-methylbutyl ester
*N*-(phenylacetyl)methionine *iso*-butyl ester
*N*-(phenylacetyl)leucine *iso*-butyl ester
*N*-[(3-chlorophenyl)acetyl]alanine 3-methylbut-2-enyl ester
*N*-[(3-chlorophenyl)aceryl)alanine cyclopropylmethyl ester
*N*-[(3-chlorophenyl)acetyl]alanine 2-thienylmethyl ester
*N*-[(3-chlorophenyl)acetyl]alanine (1-methylcyclopropyl)methyl ester
*N*-[(3-chlorophenyl)aceryl]alanine 3-thienylmethyl ester
*N*-[(3-chlorophenyl)acetyl]alanine 2-methylcyclopentyl ester
*N*-[(3-chlorophenyl)aceryl]alanine 2-methylprop-2-enyl ester
*N*-[(3-chlorophenyl)acetyl]alanine cyclohex-2-enyl ester
*N*-[(2-phenylbenzoxazol-5-yl)acetyl]alanine *iso*-butyl ester
*N*-[(3-methylthiophenyl)acetyl]alanine *iso*-butyl ester
*N*-4-[(2-furyl)aceryl]alanine *iso*-butyl ester
*N*-[(benzofuran-2-yl)acetyl]alanine *iso*-butyl ester
*N*-[(benzothiophen-3-yl)aceryl]alanine *iso*-butyl ester
*N*-[(2-chloro-5-thienyl)acetyl]alanine *iso*-butyl ester
*N*-[(3-methyl-isoxazol-5-yl)acetyl]alanine *iso*-butyl ester
*N*-[(2-phenylthiothienyl)acetyl]alanine *iso*-butyl ester
*N*-[(6-methoxybenzothiophen-2-yl)acetyl]alanine *iso*-butyl ester
*N*-[(3-phenyl-1,2,4-thiadiazol-5-yl)acetyl]alanine *iso*-butyl ester
*N*-[(2-phenyloxazol-4-yl)acetyl]alanine *iso*-butyl ester
*N*-[(3-methylphenyl)acetyl]alanine *iso*-butyl ester
*N*-[(2,5-difluorophenyl)acetyl]alanine *iso*-butyl ester
*N*-[(3,5-diflurophenyl)acetyl]alanine *iso*-butyl ester
*N*-[(3-thienyl)aceryl]alanine *iso*-butyl ester
*N*-[(4-methylphenyl)acetyl]alanine *iso*-butyl ester
*N*-(phenylacetyl)alanine (1-methoxycarbonyl)*iso*-butyl ester
*N*-[(3-nitrophenyl)acetyl]alanine *iso*-butyl ester
*N*-[(3,5-difluorophenyl)acetyl]alanine ethyl ester
*N*-[(3-nitrophenyl)acetyl]methionine ethyl ester
N-[(3-chlorophenyl)acetyl]alanine *iso*-butyl ester
*N*-[(3-chlorophenyl)acetyl]alanine 2-(*N*,*N*-dimethylamino)ethyl ester
2-[(3,5-dichlorophenyl)acetamido]hexanoic acid methyl ester
*N*-[(3,5-dichlorophenyl)acetyl]alanine *iso*-butyl ester
*N*-(cyclohexylacetyl)alanine *iso*-butyl ester
*N*-(cyclopentylacetyl)alanine *iso*-butyl ester
*N*-[(cyclohex-1-enyl)acetyl]alanine *iso*-butyl ester
*N*-[(3-chlorophenyl)acetyl]alanine 3-methylbut-2-enyl thioester
*N*-[(2-phenyl)-2-fluoroacetyl]alanine ethyl ester
*N*-(3,5-difluorophenylacetyl)phenylglycine methyl ester
*N*-(3,5-difluorophenylacetyl)phenylglycine *iso*-butyl ester
*N*-(cyclopentylacetyl)phenylglycine methyl ester
*N*-(cyclopentylacetyl)alanine methyl ester
*N*-(cyclopropylacetyl)phenylglycine methyl ester
*N*-(cyclopropylacetyl)alanine methyl ester; and
*N*-[(3-nitrophenyl)acetyl]methionine *iso*-butyl ester.

**70.** A method for inhibiting β-amyloid release and/or its synthesis in a cell *in vitro* which method comprises administering to such a cell an amount of a compound of formula I or a mixture of compounds of formula I' effective in inhibiting the cellular release and/or synthesis of β-amyloid peptide wherein formula I' is as defined in any one of claims 45 to 69.

**71.** A compound for use as a medicament selected from the group consisting of:

N-(phenylacetyl)alanine *iso*-butyl ester

N-(3-phenylpropionyl)alanine *iso*-butyl ester
2-(phenylacetamido)butyric acid *iso*-butyl ester
N-(phenylacetyl)valine 2-methylbutyl ester
N-(phenylacetyl)methionine *iso*-butyl ester
N-(phenylacetyl)leucine *iso*-butyl ester
N-(phenylacetyl)alanine (1-methoxycarbonyl)*iso*-butyl ester
N-[(2-phenyl)-2-fluoroacetyl]alanine ethyl ester.

**72.** A compound selected from the group consisting of:

N-(phenylacetyl)alanine *iso*-butyl ester
N-(3-phenylpropionyl)alanine *iso*-butyl ester
2-(phenylacetamido)butyric acid *iso*-butyl ester
N-(phenylacetyl)valine 2-methylbutyl ester
N-(phenylacetyl)methionine *iso*-butyl ester
N-(phenylacetyl)leucine *iso*-butyl ester
N-(phenylacetyl)alanine (1-methoxycarbonyl)*iso*-butyl ester
N-[(2-phenyl)-2-fluoroacetyl]alanine ethyl ester.

**73.** A pharmaceutical composition comprising a pharmaceutically inert carrier and a pharmaceutically effective amount of a compound as claimed in claim 71 or 72.

**Patentansprüche**

**1.** Verbindung der Formel I oder Gemisch aus Verbindungen der Formel I zur Verwendung als Medikament, wobei Formel I ist:

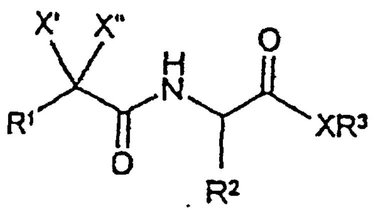

I

wobei $R^1$ aus der Gruppe ausgewählt, die besteht aus:

a) Alkyl, Alkenyl, Alkcycloalkyl, Naphthyl-$(R)_m$-, wobei R eine Alkylengruppe mit 1 bis 8 Kohlenstoffatomen ist und *m* eine ganze Zahl gleich 0 oder 1 ist, Cycloalkyl, Cycloalkenyl, 3-Pyridyl, 4-Pyridyl und Heteroaryl mit 3 bis 10 Atomen und 1 bis 4 Heteroatomen, die aus Sauerstoff, Schwefel und Stickstoff ausgewählt sind, wobei die Heteroarylgruppe fakultativ mit 1 bis 3 Substituenten substituiert ist, die aus der Gruppe ausgewählt sind, die besteht aus Alkyl, Alkoxy, Aryl, Aryloxy, Halo, Nitro, Thioalkoxy und Thioaryloxy, mit der Maßgabe, daß für solche Heteroaryle dann, wenn mindestens ein Stickstoff-Heteroatom vorhanden ist, auch mindestens ein Sauerstoff- und/oder Schwefel-Heteroatom vorhanden ist;
b) einer substituierten Phenylgruppe der Formel II:

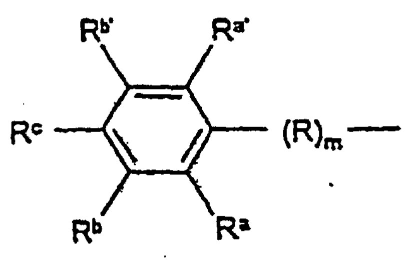

wobei R Alkylen mit 1 bis 8 Kohlenstoffatomen ist,

*m* eine ganze Zahl gleich 0 oder 1 ist,

$R^a$ und $R^{a'}$ jeweils unabhängig aus der Gruppe ausgewählt sind, die aus Wasserstoff, Hydroxy, Fluoro und Methyl besteht;

$R^b$ und $R^{b'}$ jeweils unabhängig aus der Gruppe ausgewählt sind, die besteht aus Wasserstoff, Alkyl, Alkoxy, Aryl, Cyano, Cycloalkyl, Halo, Heteroaryl, heterocyclischer Gruppe, Nitro, Trihalomethyl, Thioalkoxy, Thioaryloxy, Thioheteroaryloxy und $-C(O)R^4$, wobei $R^4$ aus der Gruppe ausgewählt ist, die aus Alkyl, Aryl, Alkoxy und Aryloxy besteht; und

$R^c$ aus der Gruppe ausgewählt ist, die aus Wasserstoff, Alkyl, Aryl, Cyano, Halo, Nitro besteht, und wobei $R^b$ und $R^c$ verschmolzen sind, um einen Methylendioxyring mit dem Phenylring zu bilden; und,

wenn $R^b$ und/oder $R^{b'}$ und/oder $R^c$ Fluoro, Chloro, Bromo und/oder Nitro ist, dann können $R^a$ und/oder $R^{a'}$ auch Chloro sein; und

wobei $R^a$, $R^{a'}$, $R^b$, $R^{b'}$ und $R^c$ nicht sämtlich gleichzeitig Wasserstoff sind; und

c) 1- oder 2-Naphthyl-$(R)_m$-, das an den 5-, 6-, 7- und/oder 8-Positionen mit 1 bis 4 Substituenten substituiert ist, die aus der Gruppe ausgewählt sind, die besteht aus Alkyl, Alkoxy, Halo, Cyano, Nitro, Trihalomethyl und Thioalkoxy, wobei R eine Alkylengruppe mit 1 bis 8 Kohlenstoffatomen ist und *m* eine ganze Zahl gleich 0 oder 1 ist;

$R^2$ aus der Gruppe ausgewählt ist, die aus Alkyl, Phenyl, Alkylalkoxy, Alkylthioalkoxy besteht; und

$R^3$ aus der Gruppe ausgewählt ist, die aus $-(CH_2)_nCR^{10}R^5R^6$ besteht, wobei *n* eine ganze Zahl gleich 0, 1 oder 2 ist, $R^5$ und $R^6$ jeweils unabhängig aus Wasserstoff, Alkyl, Alkenyl, Aryl, Heteroaryl, heteroyclischer Gruppe, $-NR^7R^8$ ausgewählt sind, wobei $R^7$ und $R^8$ jeweils unabhängig Wasserstoff oder Alkyl und $-COOR^9$ sind, wobei $R^9$ Alkyl ist, und wobei ferner $R^5$ und $R^6$ vereinigt sein können, um eine Cycloalkylgruppe, eine Cycloalkenylgruppe, eine Arylgruppe, eine Heteroarylgruppe und eine heterocyclische Gruppe zu bilden, und dann, wenn $R^5$ und $R^6$ sich nicht vereinigen, um eine Aryl- oder Heteroarylgruppe zu bilden, $R^{10}$ aus Wasserstoff und Alkyl ausgewählt wird, mit der Maßgabe, daß dann, wenn *n* null ist, $R^{10}$ Wasserstoff ist, und dann, wenn *n* größer als null ist und $R^5$ und $R^6$ vereinigt sind, um eine Aryloder Heteroarylgruppe zu bilden, $R^{10}$ eine Bindung innerhalb dieser Gruppe wird;

X Sauerstoff oder Schwefel ist;

X' Wasserstoff, Hydroxy oder Fluoro ist;

X" Wasserstoff, Hydroxy oder Fluoro ist oder X' und X" gemeinsam eine Oxogruppe und pharmazeutisch akzeptable Salze davon bilden, mit den Maßgaben, daß:

dann, wenn $R^1$ Pyrid-3-yl ist, $R^2$ Ethyl ist, X Sauerstoff ist und X' und X" Wasserstoff sind, $R^3$ nicht $-CH_2CH(CH_3)_2$ ist, und

dann, wenn $R^1$ Indoxazin-3-yl, 2,4-Dimethylthiazol-5-yl, 4-Methyl-1,2,5-thiooxadizol-3-yl oder 3,5-Di(trifluormethyl)phenyl ist, $R^2$ Methyl ist, X Sauerstoff ist und X' und X" Wasserstoff sind, $R^3$ nicht $-CH_2CH(CH_3)_2$ ist.

2. Verbindung der Formel I oder Gemisch aus Verbindungen der Formel I nach Anspruch 1 zum Hemmen einer β-Amyloid-Freisetzung und/oder seiner Synthese in einer Zelle.

3. Verbindung der Formel I oder Gemisch aus Verbindungen der Formel I nach Anspruch 1 zum Verhindern des Beginns der Alzheimer Krankheit bei einem Patienten, bei dem das Risiko besteht, daß er die Alzheimer Krankheit entwickelt.

4. Verbindung der Formel I oder Gemisch aus Verbindungen der Formel I nach Anspruch 1 zur Behandlung eines Alzheimer-Patienten, um eine weitere Verschlechterung des Zustands des Patienten zu hemmen.

5. Verbindung der Formel III:

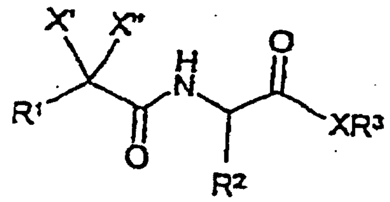

III

wobei $R^1$ aus der Gruppe ausgewählt ist, die besteht aus

a) Alkyl, Alkenyl, Alkcycloalkyl, Naphthyl-$(R)_m$-, wobei R eine Alkylengruppe mit 1 bis 8 Kohlenstoffatomen ist und *m* eine ganze Zahl gleich 0 oder 1 ist, Cycloalkyl, Cycloalkenyl, 3-Pyridyl, 4-Pyridyl und Heteroaryl mit 3 bis 10 Atomen und 1 bis 4 Heteroatomen, die aus Sauerstoff, Schwefel und Stickstoff ausgewählt sind, wobei die Heteroarylgruppe fakultativ mit 1 bis 3 Substituenten substituiert ist, die aus der Gruppe ausgewählt sind, die besteht aus Alkyl, Alkoxy, Aryl, Aryloxy, Halo, Nitro, Thioalkoxy und Thioaryloxy, mit der Maßgabe, daß für solche Heteroaryle dann, wenn mindestens ein Stickstoff-Heteroatom vorhanden ist, auch mindestens ein Sauerstoff- und/oder Schwefel-Heteroatom vorhanden ist;
b) einer substituierten Phenylgruppe der Formel II:

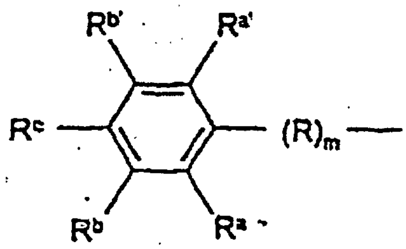

II

wobei R Alkylen mit 1 bis 8 Kohlenstoffatomen ist,
*m* eine ganze Zahl gleich 0 oder 1 ist,
$R^a$ und $R^{a'}$ jeweils unabhängig aus der Gruppe ausgewählt sind, die aus Wasserstoff, Hydroxy, Fluoro und Methyl besteht;
$R^b$ und $R^{b'}$ jeweils unabhängig aus der Gruppe ausgewählt sind, die besteht aus Wasserstoff, Alkyl, Alkoxy, Aryl, Cyano, Cycloalkyl, Halo, Heteroaryl, heterocyclischer Gruppe, Nitro, Trihalomethyl, Thioalkoxy, Thioaryloxy, Thioheteroaryloxy und -C(O)$R^4$, wobei $R^4$ aus der Gruppe ausgewählt ist, die aus Alkyl, Aryl, Alkoxy und Aryloxy besteht; und
$R^c$ aus der Gruppe ausgewählt ist, die aus Wasserstoff, Alkyl, Aryl, Cyano, Halo, Nitro besteht, und wobei $R^b$ und $R^c$ verschmolzen sind, um einen Methylendioxyring mit dem Phenylring zu bilden; und,
wenn $R^b$ und/oder $R^{b'}$ und/oder $R^c$ Fluoro, Chloro, Bromo und/oder Nitro ist, dann kann $R^a$ und/oder $R^{a'}$ auch Chloro sein; und
wobei $R^a$, $R^{a'}$, $R^b$, $R^{b'}$ und $R^c$ nicht sämtlich gleichzeitig Wasserstoff sind; und
c) 1- oder 2-Naphthyl-$(R)_m$-, wobei R eine Alkylengruppe mit 1 bis 8 Kohlenstoffatomen ist und *m* eine ganze Zahl gleich 0 oder 1 ist, das an den 5-, 6-, 7- und/oder 8-Positionen mit 1 bis 4 Substituenten substituiert ist, die aus der Gruppe ausgewählt sind, die besteht aus Alkyl, Alkoxy, Halo, Cyano, Nitro, Trihalomethyl und Thioalkoxy;
$R^2$ aus der Gruppe ausgewählt ist, die aus Alkyl, Phenyl, Alkylalkoxy, Alkylthioalkoxy besteht; und
$R^3$ aus der Gruppe ausgewählt ist, die aus $-(CH_2)_nCR^{10}R^5R^6$ besteht, wobei *n* eine ganze Zahl gleich 0, 1 oder 2 ist, $R^5$ und $R^6$ jeweils unabhängig aus Wasserstoff, Alkyl, Alkenyl, Aryl, Heteroaryl, heteroyclischer Gruppe, $-NR^7R^8$ ausgewählt sind, wobei $R^7$ und $R^8$ jeweils unabhängig Wasserstoff oder Alkyl und $-COOR^9$ sind, wobei $R^9$ Alkyl ist, und wobei ferner $R^5$ und $R^6$ vereinigt sein können, um eine Cycloalkylgruppe, eine Cycloalkenylgruppe, eine Arylgruppe, eine Heteroarylgruppe und eine heterocyclische Gruppe zu bilden, und dann, wenn $R^5$ und $R^6$ sich nicht vereinigen, um eine Aryl- oder Heteroarylgruppe zu bilden, $R^{10}$ aus Wasserstoff und Alkyl ausgewählt wird, mit der Maßgabe, daß dann, wenn *n* null ist, $R^{10}$ Wasserstoff ist, und dann, wenn *n* größer als null ist und $R^5$ und $R^6$ vereinigt sind, um eine Aryloder Heteroarylgruppe zu bilden, $R^{10}$ eine Bindung innerhalb dieser Gruppe wird;

X Sauerstoff oder Schwefel ist;
X' Wasserstoff, Hydroxy oder Fluoro ist;
X" Wasserstoff, Hydroxy oder Fluoro ist oder X' und X" gemeinsam eine Oxogruppe und pharmazeutisch akzeptable Salze davon bilden, mit den Maßgaben, daß:

dann, wenn $R^1$ Pyrid-3-yl ist, $R^2$ Ethyl ist, X Sauerstoff ist und X' und X" Wasserstoff sind, $R^3$ nicht $-CH_2CH(CH_3)_2$ ist,
dann, wenn $R^1$ Butyl ist, $R^2$ Metyhl ist, X Sauerstoff ist und X' und X" Wasserstoff sind, $R^3$ nicht Ethyl ist,
dann, wenn $R^1$ Methyl, $-CH(CH_3)_2$ oder Cyclohexyl ist, $R^2$ $-CH(CH_3)_2$ ist, X Sauerstoff ist, X' Wasserstoff ist und X" Hydroxyl ist, $R^3$ nicht Methyl ist,
dann, wenn $R^1$ Ethyl oder $C_6H_4Cl$-*p* ist, $R^2$ Phenyl, Methyl oder $-CH(CH_3)_2$ ist, X Sauerstoff ist, X' Wasserstoff ist und X" Fluor ist, $R^3$ nicht Methyl ist, und
dann, wenn $R^1$ Indoxazin-3-yl, 2,4-Dimethylthiazol-5-yl, 4-Methyl-1,2,5-thiooxadizol-3-yl oder 3,5-Di(trifluormethyl)phenyl ist, $R^2$ Methyl ist, X Sauerstoff ist und X' und X" Wasserstoff sind, $R^3$ nicht $-CH_2CH(CH_3)_2$ ist.

**6.** Verbindung nach einem der Ansprüche 1 bis 5, wobei $R^1$ eine unsubstituierte Naphthylgruppe ist, die aus der Gruppe ausgewählt ist, die aus 1-Naphthyl und 2-Naphthyl besteht.

**7.** Verbindung nach einem der Ansprüche 1 bis 5, wobei $R^1$ eine substituierte Phenylgruppe der Formel II ist.

**8.** Verbindung nach Anspruch 7, wobei die substituierte Phenylgruppe durch folgendes definiert ist:

(a) monosubstituierte Phenyle, die eine einzige Substitution an den 2-, 3-oder 4-Positionen haben, wobei jeder von den speziellen Substituenten durch die jeweiligen $R^a$-, $R^b$- und $R^c$-Gruppen bestimmt ist;
(b) disubstituierte Phenyle, die zwei Substituenten an den 2,3-Positionen, 2,4-Positionen, 2,5-Positionen, 2,6-Positionen, 3,4-Positionen, 3,5-Positionen oder 3,6-Positionen haben, wobei jeder von diesen Substituenten durch die jeweiligen $R^a$-, $R^{a'}$-, $R^b$-, $R^{b'}$- und $R^c$-Gruppen bestimmt ist; und
(c) trisubstituierte Phenyle, die drei Substituenten an den 2,3,4-Positionen, 2,3,5-Positionen, 2,3,6-Positionen, 3,4,5-Positionen und 3,4,6-Positionen haben, wobei wiederum jeder von diesen Substituenten durch die jeweiligen $R^a$-, $R^{a'}$-, $R^b$-, $R^{b'}$- und $R^c$-Gruppen bestimmt ist.

**9.** Verbindung nach Anspruch 8, wobei die substituierten Phenylgruppen aus der Gruppe ausgewählt sind, die besteht aus 4-Fluorphenyl, 4-Chlorphenyl, 4-Bromphenyl, 4-Nitrophenyl, 4-Methylphenyl, 3-Methoxyphenyl, 3-Nitrophenyl, 3-Fluorphenyl, 3-Chlorphenyl, 3-Bromphenyl, 3-Thiomethoxyphenyl, 3-Methylphenyl, 3-Trifluormethylphenyl, 2-Hydroxyphenyl, 2-Methylphenyl, 2-Fluorphenyl, 3,4-Dichlorphenyl, 3,4-Methylendioxyphenyl, 3,5-Difluorphenyl, 3,5-Dichlorphenyl, 2,4-Dichlorphenyl und 2,5-Difluorphenyl.

**10.** Verbindung nach einem der Ansprüche 1 bis 5, wobei $R^1$ aus der Gruppe ausgewählt ist, die aus Alkyl-, Alkenyl-, Alkcycloalkyl-, Cycloalkyl- und Cycloalkenylgruppen besteht.

**11.** Verbindung nach Anspruch 10, wobei $R^1$ Alkyl ist.

**12.** Verbindung nach Anspruch 10, wobei $R^1$ Cycloalkyl ist.

**13.** Verbindung nach Anspruch 10, wobei $R^1$ Alkenyl ist.

**14.** Verbindung nach Anspruch 10, wobei $R^1$ Cycloalkenyl ist.

**15.** Verbindung nach Anspruch 10, wobei die $R^1$-Alkyl-, -Cycloalkyl-, Alkcycloalkyl-, -Alkenyl- und -Cycloalkenylgruppen aus der Gruppe ausgewählt sind, die besteht aus *sec*-Butyl, Cyclopropyl, Cyclobutyl, Cyclohexyl, Cyclopentyl, Cyclohex-1-enyl, $-CH_2$-Cyclopropyl, $-CH_2$-Cyclobutyl, $-CH_2$-Cyclohexyl, $-CH_2$-Cyclopentyl, $-CH_2CH_2$-Cyclopropyl, $-CH_2CH_2$-Cyclobutyl, $-CH_2CH_2$-Cyclohexyl und $-CH_2CH_2$-Cyclopentyl.

**16.** Verbindung nach einem der Ansprüche 1 bis 5, wobei $R^1$ aus der Gruppe ausgewählt ist, die aus Heteroaryl- und substituierten Heteroarylgruppen besteht.

**17.** Verbindung nach Anspruch 16, wobei die $R^1$-Heteroaryl- und substituierten -Heteroarylgruppen aus der Gruppe

ausgewählt sind, die besteht aus Pyrid-3-yl, Pyrid-4-yl, Thien-2-yl, Thien-3-yl, Benzothiazol-4-yl, 2-Phenylbenzoxazol-5-yl, Furan-2-yl, Benzofuran-2-yl, Benzothiophen-3-yl, 2-Chlorthien-5-yl, 3-Methylisoxazol-5-yl, 2-(Phenylthio)thien-5-yl, 6-Methoxythiophen-2-yl, 3-Phenyl-1,2,4-thiooxadiazol-5-yl und 2-Phenyloxazol-4-yl.

**18.** Verbindung nach einem der Ansprüche 1 bis 17, wobei $R^2$ aus der Gruppe ausgewählt ist, die aus Alkyl mit 1 bis 4 Kohlenstoffatomen, Alkylalkoxy mit 1 bis 4 Kohlenstoffatomen, Phenyl und Alkylthioalkoxy mit 1 bis 4 Kohlenstoffatomen besteht.

**19.** Verbindung nach Anspruch 18, wobei $R^2$ aus der Gruppe ausgewählt ist, die aus Methyl, Ethyl, *n*-Propyl, *iso*-Propyl, *n*-Butyl, *iso*-Butyl, $-CH_2CH_2SCH_3$ und Phenyl besteht.

**20.** Verbindung nach einem der Ansprüche 1 bis 19, wobei X' und X" beide Wasserstoff sind und X Sauerstoff ist.

**21.** Verbindung nach Anspruch 20, wobei $R^3$ aus der Gruppe ausgewählt ist, die besteht aus Methyl, Ethyl, *iso*-Propyl, *n*-Propyl, *n*-Butyl, *iso*-Butyl, Cyclopentyl, Allyl, *iso*-But-2-enyl, 3-Methylpentyl, $-CH_2$-Cyclopropyl, $-CH_2$-Cyclohexyl, $-CH_2$-(3-Tetrahydrofuranyl), $-CH_2$-Thien-2-yl, $-CH_2$(1-Methyl)cyclopropyl, $-CH_2$-Thien-3-yl, $-CH_2$-C(O)O-*tert*-Butyl, $-CH_2-C(CH_3)_3$, $-CH_2CH(CH_2CH_3)_2$, -2-Methylcyclopentyl, -Cyclohex-2-enyl, $-CH[CH(CH_3)_2]COOCH_3$, $-CH_2CH_2N(CH_3)_2$, $-CH_2C(CH_3)=CH_2$ und $-CH_2CH=C(CH_3)_2$.

**22.** Verbindung nach einem der Ansprüche 1 bis 19, wobei X' und X" beide Wasserstoff sind und X Schwefel ist.

**23.** Verbindung nach Anspruch 22, wobei $R^3$ aus der Gruppe ausgewählt ist, die aus *iso*-But-2-enyl und *iso*-Butyl besteht.

**24.** Verbindung nach einem der Ansprüche 1 bis 5, wobei die Verbindung der Formel I aus der Gruppe ausgewählt ist, die besteht aus:

*N*-(3-Methylpentanoyl)alanin-*iso*-Butylester
*N*-[(4-Chlorphenyl)acetyl]alanin-*iso*-Butylester
*N*-[(3,4-Dichlorphenyl)acetyl]alanin-*iso*-Butylester
*N*-[(3-Pyridyl)acetyl]alanin-*iso*-Butylester
*N*-[(1-Naphthyl)acetyl]alanin-*iso*-Butylester
*N*-[(2-Napthyl)acetyl]alanin-*iso*-Butylester
*N*-[(4-Phenylbutanoyl)alanin-*iso*-Butylester
*N*-[(5-Phenylpentanoyl)alanin-*iso*-Butylester
*N*-[(4-Pyridyl)acetyl]alanin-*iso*-Butylester
2-[(3,4-Dichlorphenyl)acetamido]buttersäure-*iso*-Butylester
2-[(3-Methoxyphenyl)acetamido]buttersäure-*iso*-Butylester
2-[(4-Nitrophenyl)acetamido]buttersäure-*iso*-Butylester
2-[(3,4-Methylendioxyphenyl)acetamido]buttersäure-*iso*-Butylester
2-[(Thien-3-yl)acetamido]buttersäure-*iso*-Butylester
2-[(4-Chlorphenyl)acetamido]buttersäure-*iso*-Butylester
2-[(3-Nitrophenyl)acetamido]buttersäure-*iso*-Butylester
2-[(2-Hydroxyphenyl)acetamido]buttersäure-*iso*-Butylester
2-[(2-Naphthyl)acetamido]buttersäure-*iso*-Butylester
2-[(2,4-Dichlorphenyl)acetamido]buttersäure-*iso*-Butylester
2-[(4-Bromphenyl)acetamido]buttersäure-*iso*-Butylester
2-[(3-Chlorphenyl)acetamido]buttersäure-*iso*-Butylester
2-[(3-Fluorphenyl)acetamido]buttersäure-*iso*-Butylester
2-[(Benzothiazol-4-yl)acetamido]buttersäure-*iso*-Butylester
2-[(2-Methylphenyl)acetamido]buttersäure-*iso*-Butylester
2-[(2-Fluorphenyl)acetamido]buttersäure-*iso*-Butylester
2-[(4-Fluorphenyl)acetamido-buttersäure-*iso*-Butylester
2-[(3-Bromphenyl)acetamido]buttersäure-*iso*-Butylester
2-[(3-Trifluormethylphenyl)acetamido]buttersäure-*iso*-Butylester
2-[(2-Thienyl)acetamido]buttersäure-*iso*-Butylester

*N*-[(3-Chlorphenyl)acetyl]alanin-3-Methylbut-2-enylester

*N*-[(3-Chlorphenyl)acetyl]alanin-Cyclopropylmethylester
*N*-[(3-Chlorphenyl)acetyl]alanin-2-Thienylmethylester
*N*-[(3-Chlorphenyl)acetyl]alanin-(1-Methylcyclopropyl)methylester
*N*-[(3-Chlorphenyl)acetyl]alanin-3-Thienylmethylester
*N*-[(3-Chlorphenyl)acetyl]alanin-2-Methylcyclopentylester
*N*-[(3-Chlorphenyl)acetyl]alanin-2-Methylprop-2-enylester
*N*-[(3-Chlorphenyl)acetyl]alanin-Cyclohex-2-enylester
*N*-[(2-Phenylbenzoxazol-5-yl)acetyl]alanin-*iso*-Butylester
*N*-[(3-Methylthiophenyl)acetyl]alanin-*iso*-Butylester
*N*-4-[(2-Furyl)acetyl]alanin-*iso*-Butylester
*N*-[(Benzofuran-2-yl)acetyl]alanin-*iso*-Butylester
*N*-[(Benzothiophen-3-yl)acetyl]alanin-*iso*-Butylester
*N*-[(2-Chlor-5-Thienyl)acetyl]alanin-*iso*-Butylester
*N*-[(3-Methylisoxazol-5-yl)acetyl]alanin-*iso*-Butylester
*N*-[(2-Phenylthiothienyl)acetyl]alanin-*iso*-Butylester
*N*-[(6-Methoxybenzothiophen-2-yl)acetyl]alanin-*iso*-Butylester
*N*-[(3-Phenyl-1,2,4-thiadiazol-5-yl)acetyl]alanin-*iso*-Butylester
*N*-[(2-Phenyloxazol-4-yl)acetyl]alanin-*iso*-Butylester
*N*-[(3-Methylphenyl)acetyl]alanin-*iso*-Butylester
*N*-[(2,5-Difluorphenyl)acetyl]alanin-*iso*-Butylester
*N*-[(3,5-Difluorphenyl)acetyl]alanin-*iso*-Butylester
*N*-[(3-Thienyl)acetyl)alanin-*iso*-Butylester
*N*-[(4-Methylphenyl)acetyl]alanin-*iso*-Butylester

*N*-[(3-Nitrophenyl)acetyl]alanin-*iso*-Butylester
*N*-[(3,5-Difluorphenyl)acetyl]alanin-Ethylester
*N*-[(3-Nitrophenyl)acetyl]methionin-Ethylester
*N*-[(3-Chlorphenyl)acetyl]alanin-*iso*-Butylester
*N*-[(3-Chlorphenyl)acetyl]alanin-2-(*N*,*N*-Dimethylamino)ethylester
2-[(3,5-Dichlorphenyl)acetamido]hexylsäure-Methylester
*N*-[(3,5-Dichlorphenyl)acetyl]alanin-*iso*-Butylester
*N*-(Cyclohexylacetyl)alanin-*iso*-Butylester
*N*-(Cyclopentylacetyl)alanin-*iso*-Butylester
*N*-[(Cyclohex-1-enyl)acetyl]alanin-*iso*-Butylester
*N*-[(3-Chlorphenyl)acetyl]alanin-3-Methylbut-2-enylthioester

*N*-(3,5-Difluorphenylacetyl)phenylglycin-Methylester
*N*-(3,5-Difluorphenylacetyl)phenylglycin-*iso*-Butylester
*N*-(Cyclopentylacetyl)phenylglycin-Methylester
*N*-(Cyclopentylacetyl)alanin-Methylester
*N*-(Cyclopropylacetyl)phenylglycin-Methylester
*N*-(Cyclopropylacetyl)alanin-Mmethylester; und
*N*-[(3-Nitrophenyl)acetyl]methionin-*iso*-Butylester.

**25.** Pharmazeutische Zusammensetzung, die einen pharmazeutisch inerten Träger und eine pharmazeutisch wirksame Menge einer Verbindung der Formel I aufweist:

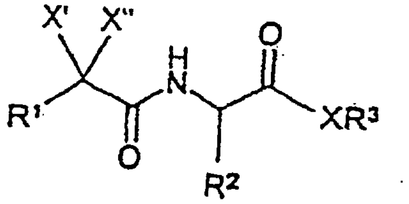
I

wobei $R^1$ aus der Gruppe ausgewählt, die besteht aus:

a) Alkyl, Alkenyl, Alkcycloalkyl, Naphthyl-$(R)_m$-, wobei R eine Alkylengruppe mit 1 bis 8 Kohlenstoffatomen ist und *m* eine ganze Zahl gleich 0 oder 1 ist, Cycloalkyl, Cycloalkenyl, 3-Pyridyl, 4-Pyridyl und Heteroaryl mit 3 bis 10 Atomen und 1 bis 4 Heteroatomen, die aus Sauerstoff, Schwefel und Stickstoff ausgewählt sind, wobei die Heteroarylgruppe fakultativ mit 1 bis 3 Substituenten substituiert ist, die aus der Gruppe ausgewählt sind, die besteht aus Alkyl, Alkoxy, Aryl, Aryloxy, Halo, Nitro, Thioalkoxy und Thioaryloxy, mit der Maßgabe, daß für solche Heteroaryle dann, wenn mindestens ein Stickstoff-Heteroatom vorhanden ist, auch mindestens ein Sauerstoff- und/oder Schwefel-Heteroatom vorhanden ist;
b) einer substituierten Phenylgruppe der Formel II:

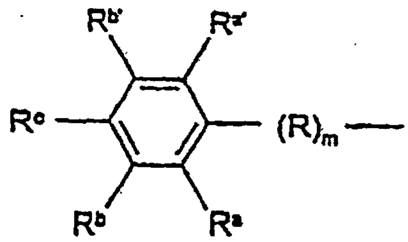

II

wobei R Alkylen mit 1 bis 8 Kohlenstoffatomen ist,
*m* eine ganze Zahl gleich 0 oder 1 ist,
$R^a$ und $R^{a'}$ jeweils unabhängig aus der Gruppe ausgewählt sind, die aus Wasserstoff, Hydroxy, Fluoro und Methyl besteht;
$R^b$ und $R^{b'}$ jeweils unabhängig aus der Gruppe ausgewählt sind, die besteht aus Wasserstoff, Alkyl, Alkoxy, Aryl, Cyano, Cycloalkyl, Halo, Heteroaryl, heterocyclischer Gruppe, Nitro, Trihalomethyl, Thioalkoxy, Thioaryloxy, Thioheteroaryloxy und $-C(O)R^4$, wobei $R^4$ aus der Gruppe ausgewählt ist, die aus Alkyl, Aryl, Alkoxy und Aryloxy besteht; und
$R^c$ aus der Gruppe ausgewählt ist, die aus Wasserstoff, Alkyl, Aryl, Cyano, Halo, Nitro besteht, und wobei $R^b$ und $R^c$ verschmolzen sind, um einen Methylendioxyring mit dem Phenylring zu bilden; und,
wenn $R^b$ und/oder $R^{b'}$ und/oder $R^c$ Fluoro, Chloro, Bromo und/oder Nitro ist, dann kann $R^a$ und/oder $R^{a'}$ auch Chloro sein; und
wobei $R^a$, $R^{a'}$, $R^b$, $R^{b'}$ und $R^c$ nicht sämtlich gleichzeitig Wasserstoff sind; und
c) 1- oder 2-Naphthyl-$(R)_m$-, das an den 5-, 6-, 7- und/oder 8-Positionen mit 1 bis 4 Substituenten substituiert ist, die aus der Gruppe ausgewählt sind, die besteht aus Alkyl, Alkoxy, Halo, Cyano, Nitro, Trihalomethyl und Thioalkoxy, wobei R eine Alkylengruppe mit 1 bis 8 Kohlenstoffatomen ist und *m* eine ganze Zahl gleich 0 oder 1 ist;
$R^2$ aus der Gruppe ausgewählt ist, die aus Alkyl, Phenyl, Alkylalkoxy, Alkylthioalkoxy besteht; und
$R^3$ aus der Gruppe ausgewählt ist, die aus $-(CH_2)_nCR^{10}R^5R^6$ besteht, wobei *n* eine ganze Zahl gleich 0, 1 oder 2 ist, $R^5$ und $R^6$ jeweils unabhängig aus Wasserstoff, Alkyl, Alkenyl, Aryl, Heteroaryl, heteroyclischer Gruppe, $-NR^7R^8$ ausgewählt sind, wobei $R^7$ und $R^8$ jeweils unabhängig Wasserstoff oder Alkyl und $-COOR^9$ sind, wobei $R^9$ Alkyl ist, und wobei ferner $R^5$ und $R^6$ vereinigt sein können, um eine Cycloalkylgruppe, eine Cycloalkenylgruppe, eine Arylgruppe, eine Heteroarylgruppe und eine heterocyclische Gruppe zu bilden, und dann, wenn $R^5$ und $R^6$ sich nicht vereinigen, um eine Aryl- oder Heteroarylgruppe zu bilden, $R^{10}$ aus Wasserstoff und Alkyl ausgewählt ist, mit der Maßgabe, daß dann, wenn *n* null ist, $R^{10}$ Wasserstoff ist, und dann, wenn *n* größer als null ist und $R^5$ und $R^6$ vereinigt sind, um eine Aryloder Heteroarylgruppe zu bilden, $R^{10}$ eine Bindung innerhalb dieser Gruppe wird;
X Sauerstoff oder Schwefel ist;
X' Wasserstoff, Hydroxy oder Fluoro ist;
X" Wasserstoff, Hydroxy oder Fluoro ist oder X' und X" gemeinsam eine Oxogruppe und pharmazeutisch akzeptable Salze davon bilden, mit den Maßgaben, daß:

dann, wenn $R^1$ Pyrid-3-yl ist, $R^2$ Ethyl ist, X Sauerstoff ist und X' und X" Wasserstoff sind, $R^3$ nicht $-CH_2CH(CH_3)_2$ ist, und
dann, wenn $R^1$ Indoxazin-3-yl, 2,4-Dimethylthiazol-5-yl, 4-Methyl-1,2,5-thiooxadizol-3-yl oder 3,5-Di

(trifluormethyl)phenyl ist, $R^2$ Methyl ist, X Sauerstoff ist und X' und X'' Wasserstoff sind, $R^3$ nicht $-CH_2CH(CH_3)_2$ ist.

**26.** Pharmazeutische Zusammensetzung nach Anspruch 25, wobei $R^1$ eine unsubstituierte Naphthylgruppe ist, die aus der Gruppe ausgewählt ist, die aus 1-Naphthyl und 2-Naphthyl besteht.

**27.** Pharmazeutische Zusammensetzung nach Anspruch 25, wobei $R^1$ eine substituierte Phenylgruppe der Formel II ist.

**28.** Pharmazeutische Zusammensetzung nach Anspruch 27, wobei die substituierte Phenylgruppe durch folgendes definiert ist:

(a) monosubstituierte Phenyle, die eine einzige Substitution an den 2-, 3-oder 4-Positionen haben, wobei jeder von den speziellen Substituenten durch die jeweiligen $R^a$-, $R^b$- und $R^c$-Gruppen bestimmt ist;
(b) disubstituierte Phenyle, die zwei Substituenten an den 2,3-Positionen, 2,4-Positionen, 2,5-Positionen, 2,6-Positionen, 3,4-Positionen, 3,5-Positionen oder 3,6-Positionen haben, wobei jeder von diesen Substituenten durch die jeweiligen $R^a$-, $R^{a'}$-, $R^b$-, $R^{b'}$- und $R^c$-Gruppen bestimmt ist; und
(c) trisubstituierte Phenyle, die drei Substituenten an den 2,3,4-Positionen, 2,3,5-Positionen, 2,3,6-Positionen, 3,4,5-Positionen und 3,4,6-Positionen haben, wobei wiederum jeder von diesen Substituenten durch die jeweiligen $R^a$-, $R^{a'}$-, $R^b$-, $R^{b'}$- und $R^c$-Gruppen bestimmt ist.

**29.** Pharmazeutische Zusammensetzung nach Anspruch 28, wobei die substituierten Phenylgruppen aus der Gruppe ausgewählt sind, die besteht aus 4-Fluorphenyl, 4-Chlorphenyl, 4-Bromphenyl, 4-Nitrophenyl, 4-Methylphenyl, 3-Methoxyphenyl, 3-Nitrophenyl, 3-Fluorphenyl, 3-Chlorphenyl, 3-Bromphenyl, 3-Thiomethoxyphenyl, 3-Methylphenyl, 3-Trifluormethylphenyl, 2-Hydroxyphenyl, 2-Methylphenyl, 2-Fluorphenyl, 3,4-Dichlorphenyl. 3,4-Methylendioxyphenyl, 3,5-Difluorphenyl, 3,5-Dichlorphenyl, 2,4-Dichlorphenyl und 2,5-Difluorphenyl.

**30.** Pharmazeutische Zusammensetzung nach Anspruch 25, wobei $R^1$ aus der Gruppe ausgewählt ist, die aus Alkyl-, Alkenyl-, Alkcycloalkyl-, Cycloalkyl- und Cycloalkenylgruppen besteht.

**31.** Pharmazeutische Zusammensetzung nach Anspruch 30, wobei $R^1$ Alkyl ist,

**32.** Pharmazeutische Zusammensetzung nach Anspruch 30, wobei $R^1$ Cycloalkyl ist.

**33.** Pharmazeutische Zusammensetzung nach Anspruch 30, wobei $R^1$ Alkenyl ist.

**34.** Pharmazeutische Zusammensetzung nach Anspruch 30, wobei $R^1$ Cycloalkenyl ist.

**35.** Pharmazeutische Zusammensetzung nach Anspruch 30, wobei die $R^1$-Alkyl-, -Alkenyl-, Alkcycloalkyl-, -Cycloalkyl-, und -Cycloalkenylgruppen aus der Gruppe ausgewählt sind, die besteht aus *sec*-Butyl, Cyclopropyl, Cyclobutyl, Cyclohexyl, Cyclopentyl, Cyclohex-1-enyl, $-CH_2$-Cyclopropyl, $-CH_2$-Cyclobutyl, $-CH_2$-Cyclohexyl, $-CH_2$-Cyclopentyl, $-CH_2CH_2$-Cyclopropyl, $-CH_2CH_2$-Cyclobutyl, $-CH_2CH_2$-Cyclohexyl und $-CH_2CH_2$-Cyclopentyl.

**36.** Pharmazeutische Zusammensetzung nach Anspruch 25, wobei $R^1$ aus der Gruppe ausgewählt ist, die aus Heteroaryl- und substituierten Heteroarylgruppen besteht.

**37.** Pharmazeutische Zusammensetzung nach Anspruch 36, wobei die $R^1$-Heteroaryl- und substituierten -Heteroarylgruppen aus der Gruppe ausgewählt sind, die besteht aus Pyrid-3-yl, Pyrid-4-yl, Thien-2-yl, Thien-3-yl, Benzothiazol-4-yl, 2-Phenylbenzoxazol-5-yl, Furan-2-yl, Benzofuran-2-yl, Benzothiophen-3-yl, 2-Chlorthien-5-yl, 3-Methylisoxazol-5-yl, 2-(Phenylthio)thien-5-yl, 6-Methoxythiophen-2-yl, 3-Phenyl-1,2,4-thiooxadiazol-5-yl und 2-Phenyloxazol-4-yl.

**38.** Pharmazeutische Zusammensetzung nach einem der Ansprüche 25 bis 37, wobei $R^2$ aus der Gruppe ausgewählt ist, die aus Alkyl mit 1 bis 4 Kohlenstoffatomen, Alkylalkoxy mit 1 bis 4 Kohlenstoffatomen, Phenyl und Alkylthioalkoxy mit 1 bis 4 Kohlenstoffatomen besteht.

**39.** Pharmazeutische Zusammensetzung nach Anspruch 38, wobei $R^2$ aus der Gruppe ausgewählt ist, die aus Methyl, Ethyl, *n*-Propyl, *iso*-Propyl, *n*-Butyl, *iso*-Butyl, $-CH_2CH_2SCH_3$ und Phenyl besteht.

**40.** Pharmazeutische Zusammensetzung nach einem der Ansprüche 25 bis 39, wobei X' und X" beide Wasserstoff sind und X Sauerstoff ist.

**41.** Pharmazeutische Zusammensetzung nach Anspruch 40, wobei $R^3$ aus der Gruppe ausgewählt ist, die besteht aus Methyl, Ethyl, *iso*-Propyl, *n*-Propyl, *n*-Butyl, *iso*-Butyl, Cyclopentyl, Allyl, *iso*-But-2-enyl, 3-Methylpentyl, $-CH_2$-Cyclopropyl, $-CH_2$-Cyclohexyl, $-CH_2$-(3-Tetrahydrofuranyl), $-CH_2$-Thien-2-yl, $-CH_2$(1-Methyl)cyclopropyl, $-CH_2$-Thien-3-yl, $-CH_2$-C(O)O-*tert*-Butyl, $-CH_2-C(CH_3)_3$, $-CH_2CH(CH_2CH_3)_2$, -2-Methylcyclopentyl, -Cyclohex-2-enyl, $-CH[CH(CH_3)_2]COOCH_3$, $-CH_2CH_2N(CH_3)_2$, $-CH_2C(CH_3)=CH_2$ und $-CH_2CH=C(CH_3)_2$.

**42.** Pharmazeutische Zusammensetzung nach einem der Ansprüche 25 bis 39, wobei X' und X" beide Wasserstoff sind und X Schwefel ist.

**43.** Pharmazeutische Zusammensetzung nach Anspruch 42, wobei $R^3$ aus der Gruppe ausgewählt ist, die aus *iso*-But-2-enyl und *iso*-Butyl besteht.

**44.** Pharmazeutische Zusammensetzung nach Anspruch 25, wobei die Verbindung der Formel I aus der Gruppe ausgewählt ist, die besteht aus:

*N*-(3-Methylpentanoyl)alanin-*iso*-Butylester
*N*-[(4-Chlorphenyl)acetyl]alanin-*iso*-Butylester
*N*-[(3,4-Dichlorphenyl)acetyl]alanin-*iso*-Butylester
*N*-[(3-Pyridyl)acetyl]alanin-*iso*-Butylester
*N*-[(1-Naphthyl)acetyl]alanin-*iso*-Butylester
*N*-[(2-Napthyl)acetyl]alanin-*iso*-Butylester
*N*-[(4-Phenylbutanoyl)alanin-*iso*-Butylester
*N*-[(5-Phenylpentanoyl)alanin-*iso*-Butylester
*N*-[(4-Pyridyl)acetyl]alanin-*iso*-Butylester
2-[(3,4-Dichlorphenyl)acetamido]buttersäure-*iso*-Butylester
2-[(3-Methoxyphenyl)acetamido]buttersäure-*iso*-Butylester
2-[(4-Nitrophenyl)acetamido]buttersäure-*iso*-Butylester
2-[(3,4-Methylendioxyphenyl)acetamido]buttersäure-*iso*-Butylester
2-[(Thien-3-yl)acetamido]buttersäure-*iso*-Butylester
2-[(4-Chlorphenyl)acetamido]buttersäure-*iso*-Butylester
2-[(3-Nitrophenyl)acetamido]buttersäure-*iso*-Butylester
2-[(2-Hydroxyphenyl)acetamido]buttersäure-*iso*-Butylester
2-[(2-Naphthyl)acetamido]buttersäure-*iso*-Butylester
2-[(2,4-Dichlorphenyl)acetamido]buttersäure-*iso*-Butylester
2-[(4-Bromphenyl)acetamido]buttersäure-*iso*-Butylester
2-[(3-Chlorphenyl)acetamido]buttersäure-*iso*-Butylester
2-[(3-Fluorphenyl)acetamido]buttersäure-*iso*-Butylester
2-[(Benzothiazol-4-yl)acetamido]buttersäure-*iso*-Butylester
2-[(2-Methylphenyl)acetamido]buttersäure-*iso*-Butylester
2-[(2-Fluorphenyl)acetamido]buttersäure-*iso*-Butylester
2-[(4-Fluorphenyl)acetamido]buttersäure-*iso*-Butylester
2-[(3-Bromphenyl)acetamido]buttersäure-*iso*-Butylester
2-[(3-Trifluormethylphenyl)acetamido]buttersäure-*iso*-Butylester
2-[(2-Thienyl)acetamido]buttersäure-*iso*-Butylester

*N*-[(3-Chlorphenyl)acetyl]alanin-3-Methylbut-2-enylester
*N*-[(3-Chlorphenyl)acetyl]alanin-Cyclopropylmethylester
*N*-[(3-Chlorphenyl)acetyl]alanin-2-Thienylmethylester
*N*-[(3-Chlorphenyl)acetyl]alanin-(1-Methylcyclopropyl)methylester
*N*-[(3-Chlorphenyl)acetyl]alanin-3-Thienylmethylester
*N*-[(3-Chlorphenyl)acetyl]alanin-2-Methylcyclopentylester
*N*-[(3-Chlorphenyl)acetyl]alanin-2-Methylprop-2-enylester
*N*-[(3-Chlorphenyl)acetyl]alanin-Cyclohex-2-enylester
*N*-[(2-Phenylbenzoxazol-5-yl)acetyl]alanin-*iso*-Butylester
*N*-[(3-Methylthiophenyl)acetyl]alanin-*iso*-Butylester

*N*-4-[(2-Furyl)acetyl]alanin-*iso*-Butylester
*N*-[(Benzofuran-2-yl)acetyl]alanin-*iso*-Butylester
*N*-[(Benzothiophen-3-yl)acetyl]alanin-*iso*-Butylester
*N*-[(2-Chlor-5-Thienyl)acetyl]alanin-*iso*-Butylester
*N*-[(3-Methylisoxazol-5-yl)acetyl]alanin-*iso*-Butylester
*N*-[(2-Phenylthiothienyl)acetyl]alanin-*iso*-Butylester
*N*-[(6-Methoxybenzothiophen-2-yl)acetyl]alanin-*iso*-Butylester
*N*-[(3-Phenyl-1,2,4-thiadiazol-5-yl)acetyl]alanin-*iso*-Butylester
*N*-[(2-Phenyloxazol-4-yl)acetyl]alanin-*iso*-Butylester
*N*-[(3-Methylphenyl)acetyl]alanin-*iso*-Butylester
*N*-[(2,5-Difluorphenyl)acetyl]alanin-*iso*-Butylester
*N*-[(3,5-Difluorphenyl)acetyl]alanin-*iso*-Butylester
*N*-[(3-Thienyl)acetyl]alanin-*iso*-Butylester
*N*-[(4-Methylphenyl)acetyl]alanin-*iso*-Butylester

*N*-[(3-Nitrophenyl)acetyl]alanin-*iso*-Butylester
*N*-[(3,5-Difluorphenyl)acetyl]alanin-Ethylester
*N*-[(3-Nitrophenyl)acetyl]methionin-Ethylester
*N*-[(3-Chlorphenyl)acetyl]alanin-*iso*-Butylester
*N*-[(3-Chlorphenyl)acetyl]alanin-2-(*N*,*N*-Dimethylamino)ethylester
2-[(3,5-Dichlorphenyl)acetamido]hexylsäure-Methylester
*N*-[(3,5-Dichlorphenyl)acetyl]alanin-*iso*-Butylester
*N*-(Cyclohexylacetyl)alanin-*iso*-Butylester
*N*-(Cyclopentylacetyl)alanin-*iso*-Butylester
*N*-[(Cyclohex-1-enyl)acetyl]alanin-*iso*-Butylester
*N*-[(3-Chlorphenyl)acetyl]alanin-3-Methylbut-2-enylthioester

*N*-(3,5-Difluorphenylacetyl)phenylglycin-Methylester
*N*-(3,5-Difluorphenylacetyl)phenylglycin-*iso*-Butylester
*N*-(Cyclopentylacetyl)phenylglycin-Methylester
*N*-(Cyclopentylacetyl)alanin-Methylester
*N*-(Cyclopropylacetyl)phenylglycin-Methylester
*N*-(Cyclopropylacetyl)alanin-Methylester; und
*N*-[(3-Nitrophenyl)acetyl]methionin-*iso*-Butylester.

**45.** Verwendung einer Verbindung der Formel I' oder eines Gemischs aus Verbindungen der Formel I' bei der Herstellung eines Medikaments zum Hemmen der β-Amyloid-Freisetzung und/oder seiner Synthese in einer Zelle, wobei Formel I' ist:

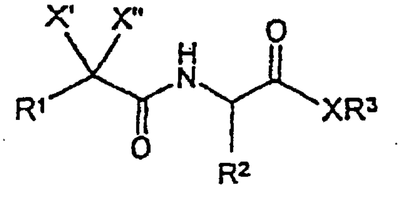

I'

wobei $R^1$ aus der Gruppe ausgewählt, die besteht aus:

a) Alkyl, Alkenyl, Alkcycloalkyl, Phenyl-$(R)_m$-, Naphthyl-$(R)_m$-, wobei R eine Alkylengruppe mit 1 bis 8 Kohlenstoffatomen ist und *m* eine ganze Zahl gleich 0 oder 1 ist, Cycloalkyl, Cycloalkenyl, 3-Pyridyl, 4-Pyridyl und Heteroaryl mit 3 bis 10 Atomen und 1 bis 4 Heteroatomen, die aus Sauerstoff, Schwefel und Stickstoff ausgewählt sind, wobei die Heteroarylgruppe fakultativ mit 1 bis 3 Substituenten substituiert ist, die aus der Gruppe ausgewählt sind, die besteht aus Alkyl, Alkoxy, Aryl, Aryloxy, Halo, Nitro, Thioalkoxy und Thioaryloxy, mit der Maßgabe, daß für solche Heteroaryle dann, wenn mindestens ein Stickstoff-Heteroatom vorhanden ist, auch mindestens ein Sauerstoff- und/oder Schwefel-Heteroatom vorhanden ist;

b) einer substituierten Phenylgruppe der Formel II:

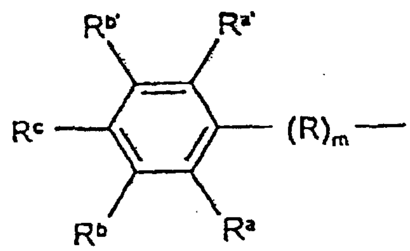

II

wobei R Alkylen mit 1 bis 8 Kohlenstoffatomen ist,

*m* eine ganze Zahl gleich 0 oder 1 ist,

$R^a$ und $R^{a'}$ jeweils unabhängig aus der Gruppe ausgewählt sind, die aus Wasserstoff, Hydroxy, Fluoro und Methyl besteht;

$R^b$ und $R^{b'}$ jeweils unabhängig aus der Gruppe ausgewählt sind, die besteht aus Wasserstoff, Alkyl, Alkoxy, Aryl, Cyano, Cycloalkyl, Halo, Heteroaryl, heterocyclischer Gruppe, Nitro, Trihalomethyl, Thioalkoxy, Thioaryloxy, Thioheteroaryloxy und $-C(O)R^4$, wobei $R^4$ aus der Gruppe ausgewählt ist, die aus Alkyl, Aryl, Alkoxy und Aryloxy besteht; und

$R^c$ aus der Gruppe ausgewählt ist, die aus Wasserstoff, Alkyl, Aryl, Cyano, Halo, Nitro besteht, und wobei $R^b$ und $R^c$ verschmolzen sind, um einen Methylendioxyring mit dem Phenylring zu bilden; und,

wenn $R^b$ und/oder $R^{b'}$ und/oder $R^c$ Fluoro, Chloro, Bromo und/oder Nitro ist, dann kann $R^a$ und/oder $R^{a'}$ auch Chloro sein; und

c) 1- oder 2-Naphthyl-$(R)_m$-, das an den 5-, 6-, 7- und/oder 8-Positionen mit 1 bis 4 Substituenten substituiert ist, die aus der Gruppe ausgewählt sind, die besteht aus Alkyl, Alkoxy, Halo, Cyano, Nitro, Trihalomethyl und Thioalkoxy, wobei R eine Alkylengruppe mit 1 bis 8 Kohlenstoffatomen ist und *m* eine ganze Zahl gleich 0 oder 1 ist;

$R^2$ aus der Gruppe ausgewählt ist, die aus Wasserstoff, Alkyl, Phenyl, Alkylalkoxy, Alkylthioalkoxy besteht; und

$R^3$ aus der Gruppe ausgewählt ist, die aus $-(CH_2)_nCR^{10}R^5R^6$ besteht, wobei *n* eine ganze Zahl gleich 0, 1 oder 2 ist, $R^5$ und $R^6$ jeweils unabhängig aus Wasserstoff, Alkyl, Alkenyl, Aryl, Heteroaryl, heteroyclischer Gruppe, $-NR^7R^8$ ausgewählt sind, wobei $R^7$ und $R^8$ jeweils unabhängig Wasserstoff oder Alkyl und $-COOR^9$ sind, wobei $R^9$ Alkyl ist, und wobei ferner $R^5$ und $R^6$ vereinigt sein können, um eine Cycloalkylgruppe, eine Cycloalkenylgruppe, eine Arylgruppe, eine Heteroarylgruppe und eine heterocyclische Gruppe zu bilden, und dann, wenn $R^5$ und $R^6$ sich nicht vereinigen, um eine Aryl- oder Heteroarylgruppe zu bilden, $R^{10}$ aus Wasserstoff und Alkyl ausgewählt wird, mit der Maßgabe, daß dann, wenn *n* null ist, $R^{10}$ Wasserstoff ist, und dann, wenn *n* größer als null ist und $R^5$ und $R^6$ vereinigt sind, um eine Aryloder Heteroarylgruppe zu bilden, $R^{10}$ eine Bindung innerhalb dieser Gruppe wird;

X Sauerstoff oder Schwefel ist;

X' Wasserstoff, Hydroxy oder Fluoro ist;

X" Wasserstoff, Hydroxy oder Fluoro ist oder X' und X" gemeinsam eine Oxogruppe und pharmazeutisch akzeptable Salze davon bilden, mit den Maßgaben, daß:

dann, wenn $R^1$ Phenyl ist, $R^2$ $-CH(CH_3)CH_2CH_3$ ist, X Sauerstoff ist und X' und X" Wasserstoff sind, $R^3$ nicht $-CH_2CH_3$ oder $-CH_2CH(CH_3)_2$ ist,

dann, wenn $R^1$ Phenyl ist, $R^3$ $-CH_2CH(CH_3)_2$ ist, X Sauerstoff ist und X' und X" Wasserstoff sind, $R^2$ nicht $-CH(CH_3)_2$ ist,

dann, wenn $R^1$ Pyrid-3-yl ist, $R^2$ Ethyl ist, X Sauerstoff ist und X' und X" Wasserstoff sind, $R^3$ nicht $-CH_2CH(CH_3)_2$ ist,

dann, wenn $R^1$ Indoxazin-3-yl, 2,4-Dimethylthiazol-5-yl, 4-Methyl-1,2,5-thiooxadizol-3-yl oder 3,5-Di(trifluormethyl)phenyl ist, $R^2$ Methyl ist, X Sauerstoff ist und X' und X" Wasserstoff sind, $R^3$ nicht $-CH_2CH(CH_3)_2$ ist, und

dann, wenn $R^1$ $-CH_2$-Phenyl ist, $R^3$ $-CH_2CH_3$ ist, X Sauerstoff ist und X' und X" Wasserstoff sind, $R^2$ nicht

$-CH_2CH(CH_3)_2$ ist.

**46.** Verwendung einer Verbindung der Formel I' oder eines Gemisches aus Verbindungen der Formel I' bei der Herstellung eines Medikaments, das die Verbindung und einen pharmazeutisch inerten Träger aufweist, zum Verhindern des Beginns der Alzheimer Krankheit bei einem Patienten, bei dem das Risiko besteht, daß er die Alzheimer Krankheit entwickelt, wobei die Formel I' ist:

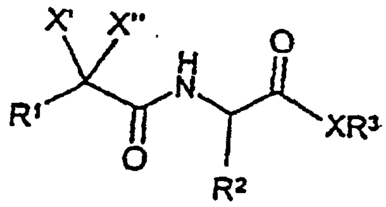

I'

wobei $R^1$ aus der Gruppe ausgewählt, die besteht aus:

a) Alkyl, Alkenyl, Alkcycloalkyl, Phenyl-$(R)_m$-, Naphthyl-$(R)_m$-, wobei R eine Alkylengruppe mit 1 bis 8 Kohlenstoffatomen ist und *m* eine ganze Zahl gleich 0 oder 1 ist, Cycloalkyl, Cycloalkenyl, 3-Pyridyl, 4-Pyridyl und Heteroaryl mit 3 bis 10 Atomen und 1 bis 4 Heteroatomen, die aus Sauerstoff, Schwefel und Stickstoff ausgewählt sind, wobei die Heteroarylgruppe fakultativ mit 1 bis 3 Substituenten substituiert ist, die aus der Gruppe ausgewählt sind, die besteht aus Alkyl, Alkoxy, Aryl, Aryloxy, Halo, Nitro, Thioalkoxy und Thioaryloxy, mit der Maßgabe, daß für solche Heteroaryle dann, wenn mindestens ein Stickstoff-Heteroatom vorhanden ist, auch mindestens ein Sauerstoff- und/oder Schwefel-Heteroatom vorhanden ist;
b) einer substituierten Phenylgruppe der Formel II:

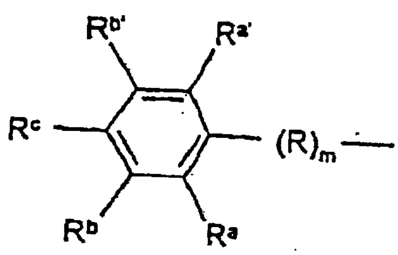

II

wobei R Alkylen mit 1 bis 8 Kohlenstoffatomen ist,
*m* eine ganze Zahl gleich 0 oder 1 ist,
$R^a$ und $R^{a'}$ jeweils unabhängig aus der Gruppe ausgewählt sind, die aus Wasserstoff, Hydroxy, Fluoro und Methyl besteht;
$R^b$ und $R^{b'}$ jeweils unabhängig aus der Gruppe ausgewählt sind, die besteht aus Wasserstoff, Alkyl, Alkoxy, Aryl, Cyano, Cycloalkyl, Halo, Heteroaryl, heterocyclischer Gruppe, Nitro, Trihalometbyl, Thioalkoxy, Thioaryloxy, Thioheteroaryloxy und $-C(O)R^4$, wobei $R^4$ aus der Gruppe ausgewählt ist, die aus Alkyl, Aryl, Alkoxy und Aryloxy besteht; und
$R^c$ aus der Gruppe ausgewählt ist, die aus Wasserstoff, Alkyl, Aryl, Cyano, Halo, Nitro besteht, und wobei $R^b$ und $R^c$ verschmolzen sind, um einen Methylendioxyring mit dem Phenylring zu bilden; und,
wcnn $R^b$ und/oder $R^{b'}$ und/oder $R^c$ Fluoro, Chloro, Bromo und/oder Nitro ist, dann kann $R^a$ und/oder $R^{a'}$ auch Chloro sein; und
c) 1- oder 2-Naphthyl-$(R)_m$-, das an den 5-, 6-, 7- und/oder 8-Positionen mit 1 bis 4 Substituenten substituiert ist, die aus der Gruppe ausgewählt sind, die besteht aus Alkyl, Alkoxy, Halo, Cyano, Nitro, Trihalomethyl und Thioalkoxy, wobei R eine Alkylengruppe mit 1 bis 8 Kohlenstoffatomen ist und *m* eine ganze Zahl gleich 0 oder 1 ist;
$R^2$ aus der Gruppe ausgewählt ist, die aus Wasserstoff, Alkyl, Phenyl, Alkylalkoxy, Alkylthioalkoxy besteht; und

$R^3$ aus der Gruppe ausgewählt ist, die aus $-(CH_2)_nCR^{10}R^5R^6$ besteht, wobei *n* eine ganze Zahl gleich 0, 1 oder 2 ist, $R^5$ und $R^6$ jeweils unabhängig aus Wasserstoff, Alkyl, Alkenyl, Aryl, Heteroaryl, heteroyclischer Gruppe, $-NR^7R^8$ ausgewählt sind, wobei $R^7$ und $R^8$ jeweils unabhängig Wasserstoff oder Alkyl und $-COOR^9$ sind, wobei $R^9$ Alkyl ist, und wobei ferner $R^5$ und $R^6$ vereinigt sein können, um eine Cycloalkylgruppe, eine Cycloalkenylgruppe, eine Arylgruppe, eine Heteroarylgruppe und eine heterocyclische Gruppe zu bilden, und dann, wenn $R^5$ und $R^6$ sich nicht vereinigen, um eine Aryl- oder Heteroarylgruppe zu bilden, $R^{10}$ aus Wasserstoff und Alkyl ausgewählt wird, mit der Maßgabe, daß dann, wenn *n* null ist, $R^{10}$ Wasserstoff ist, und dann, wenn *n* größer als null ist und $R^5$ und $R^6$ vereinigt sind, um eine Aryloder Heteroarylgruppe zu bilden, $R^{10}$ eine Bindung innerhalb dieser Gruppe wird;

X Sauerstoff oder Schwefel ist;

X' Wasserstoff, Hydroxy oder Fluoro ist;

X" Wasserstoff, Hydroxy oder Fluoro ist oder X' und X" gemeinsam eine Oxogruppe und pharmazeutisch akzeptable Salze davon bilden, mit den Maßgaben, daß:

dann, wenn $R^1$ Phenyl ist, $R^2$ $-CH(CH_3)CH_2CH_3$ ist, X Sauerstoff ist und X' und X" Wasserstoff sind, $R^3$ nicht $-CH_2CH_3$ oder $-CH_2CH(CH_3)_2$ ist,

dann, wenn $R^1$ Phenyl ist, $R^3$ $-CH_2CH(CH_3)_2$ ist, X Sauerstoff ist und X' und X" Wasserstoff sind, $R^2$ nicht $-CH(CH_3)_2$ ist,

dann wenn $R^1$ Pyrid-3-yl ist, $R^2$ Ethyl ist, X Sauerstoff ist und X' und X" Wasserstoff sind, $R^3$ nicht $-CH_2CH(CH_3)_2$ ist,

dann wenn $R^1$ Indoxazin-3-yl, 2,4-Dimethylthiazol-5-yl, 4-Methyl-1,2,5-thiooxadizol-3-yl oder 3,5-Di(trifluormethyl)phenyl ist, $R^2$ Methyl ist, X Sauerstoff ist und X' und X" Wasserstoff sind, $R^3$ nicht $-CH_2CH(CH_3)_2$ ist, und

dann, wenn $R^1$ $-CH_2$-Phenyl, $R^3$ $-CH_2CH_3$ ist, X Sauerstoff ist und X' und X" Wasserstoff sind, $R^2$ nicht $-CH_2CH(CH_3)_2$ ist.

**47.** Verwendung einer Verbindung der Formel I' oder eines Gemischs aus Verbindungen der Formel I' bei der Herstellung eines Medikaments, das die Verbindung und einen pharmazeutisch inerten Träger aufweist, zum Hemmen einer weiteren Verschlechterung des Zustands eines Alzheimer-Patienten, wobei Formel I' ist:

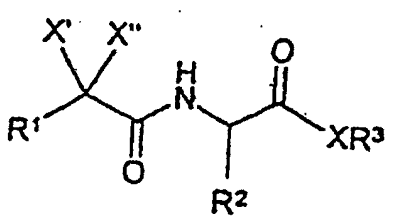

I'

wobei $R^1$ aus der Gruppe ausgewählt, die besteht aus:

a) Alkyl, Alkenyl, Alkcycloalkyl, Phenyl-$(R)_m$-, Naphthyl-$(R)_m$-, wobei R eine Alkylengruppe mit 1 bis 8 Kohlenstoffatomen ist und *m* eine ganze Zahl gleich 0 oder 1 ist, Cycloalkyl, Cycloalkenyl, 3-Pyridyl, 4-Pyridyl und Heteroaryl mit 3 bis 10 Atomen und 1 bis 4 Heteroatomen, die aus Sauerstoff, Schwefel und Stickstoff ausgewählt sind, wobei die Heteroarylgruppe fakultativ mit 1 bis 3 Substituenten substituiert ist, die aus der Gruppe ausgewählt sind, die besteht aus Alkyl; Alkoxy, Aryl, Aryloxy, Halo, Nitro, Thioalkoxy und Thioaryloxy, mit der Maßgabe, daß für solche Heteroaryle dann, wenn mindestens ein Stickstoff-Heteroatom vorhanden ist, auch mindestens ein Sauerstoff- und/oder Schwefel-Heteroatom vorhanden ist;

b) einer substituierten Phenylgruppe der Formel II:

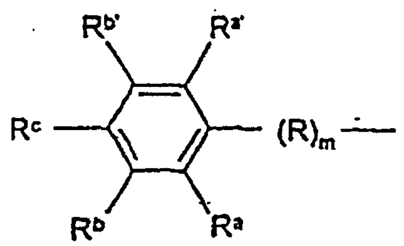

II

wobei R Alkylen mit 1 bis 8 Kohlenstoffatomen ist,

m eine ganze Zahl gleich 0 oder 1 ist,

$R^a$ und $R^{a'}$ jeweils unabhängig aus der Gruppe ausgewählt sind, die aus Wasserstoff, Hydroxy, Fluoro und Methyl besteht;

$R^b$ und $R^{b'}$ jeweils unabhängig aus der Gruppe ausgewählt sind, die besteht aus Wasserstoff, Alkyl, Alkoxy, Aryl, Cyano, Cycloalkyl, Halo, Heteroaryl, heterocyclischer Gruppe, Nitro, Trihalomethyl, Thioalkoxy, Thioaryloxy, Thioheteroaryloxy und -C(O)$R^4$, wobei $R^4$ aus der Gruppe ausgewählt ist, die aus Alkyl, Aryl, Alkoxy und Aryloxy besteht; und

$R^c$ aus der Gruppe ausgewählt ist, die aus Wasserstoff, Alkyl, Aryl, Cyano, Halo, Nitro besteht, und wobei $R^b$ und $R^c$ verschmolzen sind, um einen Methylendioxyring mit dem Phenylring zu bilden; und,

wenn $R^b$ und/oder $R^{b'}$ und/oder $R^c$ Fluoro, Chloro, Bromo und/oder Nitro ist, dann kann $R^a$ und/oder $R^{a'}$ auch Chloro sein; und

c) 1- oder 2-Naphthyl-$(R)_m$-, das an den 5-, 6-, 7-und/oder 8-Positionen mit 1 bis 4 Substituenten substituiert ist, die aus der Gruppe ausgewählt sind, die besteht aus Alkyl, Alkoxy, Halo, Cyano, Nitro, Trihalomethyl und Thioalkoxy, wobei R eine Alkylengruppe mit 1 bis 8 Kohlenstoffatomen ist und *m* eine ganze Zahl gleich 0 oder 1 ist;

$R^2$ aus der Gruppe ausgewählt ist, die aus Wasserstoff, Alkyl, Phenyl, Alkylalkoxy, Alkylthioalkoxy besteht; und

$R^3$ aus der Gruppe ausgewählt ist, die aus $-(CH_2)_nCR^{10}R^5R^6$ besteht, wobei *n* eine ganze Zahl gleich 0, 1 oder 2 ist, $R^5$ und $R^6$ jeweils unabhängig aus Wasserstoff, Alkyl, Alkenyl, Aryl, Heteroaryl, heteroyclischer Gruppe, $-NR^7R^8$ ausgewählt sind, wobei $R^7$ und $R^8$ jeweils unabhängig Wasserstoff oder Alkyl und $-COOR^9$ sind, wobei $R^9$ Alkyl ist, und wobei ferner $R^5$ und $R^6$ vereinigt sein können, um eine Cycloalkylgruppe, eine Cycloalkenylgruppe, eine Arylgruppe, eine Heteroarylgruppe und eine heterocyclische Gruppe zu bilden, und dann, wenn $R^5$ und $R^6$ sich nicht vereinigen, um eine Aryl- oder Heteroarylgruppe zu bilden, $R^{10}$ aus Wasserstoff und Alkyl ausgewählt wird, mit der Maßgabe, daß dann, wenn *n* null ist, $R^{10}$ Wasserstoff ist, und dann, wenn *n* größer als null ist und $R^5$ und $R^6$ vereinigt sind, um eine Aryloder Heteroarylgruppe zu bilden, $R^{10}$ eine Bindung innerhalb dieser Gruppe wird;

X Sauerstoff oder Schwefel ist;

X' Wasserstoff, Hydroxy oder Fluoro ist;

X" Wasserstoff, Hydroxy oder Fluoro ist oder X' und X" gemeinsam eine Oxogruppe und pharmazeutisch akzeptable Salze davon bilden, mit den Maßgaben, daß:

dann, wenn $R^1$ Phenyl ist, $R^2$ $-CH(CH_3)CH_2CH_3$ ist, X Sauerstoff ist und X' und X" Wasserstoff sind, $R^3$ nicht $-CH_2CH_3$ oder $-CH_2CH(CH_3)_2$ ist,

dann, wenn $R^1$ Phenyl ist, $R^3$ $-CH_2CH(CH_3)_2$ ist, X Sauerstoff ist und X' und X" Wasserstoff sind, $R^2$ nicht $-CH(CH_3)_2$ ist,

dann, wenn $R^1$ Pyrid-3-yl ist, $R^2$ Ethyl ist, X Sauerstoff ist und X' und X" Wasserstoff sind, $R^3$ nicht $-CH_2CH(CH_3)_2$ ist,

dann, wcnn $R^1$ Indoxazin-3-yl, 2,4-Dimethylthiazol-5-yl, 4-Methyl-1,2,5-thiooxadizol-3-yl oder 3,5-Di(trifluormethyl)phenyl ist, $R^2$ Methyl ist, X Sauerstoff ist und X' und X" Wasserstoff sind, $R^3$ nicht $-CH_2CH(CH_3)_2$ ist, und

dann, wenn $R^1$ $-CH_2$-Phenyl ist, $R^3$ $-CH_2CH_3$ ist, X Sauerstoff ist und X' und X" Wasserstoff sind, $R^2$ nicht $-CH_2CH(CH_3)_2$ ist.

**48.** Verwendung nach einem der Ansprüche 45 bis 47, wobei $R^1$ eine unsubstituierte Phenylgruppe ist.

**49.** Verwendung nach einem der Ansprüche 45 bis 47, wobei $R^1$ eine unsubstituierte Naphthylgruppe ist, die aus der

Gruppe ausgewählt ist, die aus 1-Naphthyl und 2-Naphthyl besteht.

**50.** Verwendung nach einem der Ansprüche 45 bis 47, wobei $R^1$ eine unsubstituierte Phenylgruppe der Formel II ist.

**51.** Verwendung nach Anspruch 50, wobei die substituierte Phenylgruppe durch folgendes definiert ist:

(a) monosubstituierte Phenyle, die eine einzige Substitution an den 2-, 3-oder 4-Positionen haben, wobei jeder von den speziellen Substituenten durch die jeweiligen $R^a$-, $R^b$- und $R^c$-Gruppen bestimmt ist;
(b) disubstituierte Phenyle, die zwei Substituenten an den 2,3-Positionen, 2,4-Positionen, 2,5-Positionen, 2,6-Positionen, 3,4-Positionen, 3,5-Positionen oder 3,6-Positionen haben, wobei jeder von diesen Substituenten durch die jeweiligen $R^a$-, $R^{a'}$-, $R^b$-, $R^{b'}$- und $R^c$-Gruppen bestimmt ist; und
(c) trisubstituierte Phenyle, die drei Substituenten an den 2,3,4-Positionen, 2,3,5-Positionen, 2,3,6-Positionen, 3,4,5-Positionen und 3,4,6-Positionen haben, wobei wiederum jeder von diesen Substituenten durch die jeweiligen $R^a$-, $R^{a'}$-, $R^b$-, $R^{b'}$- und $R^c$-Gruppen bestimmt ist.

**52.** Verwendung nach Anspruch 51, wobei die substituierten Phenylgruppen aus der Gruppe ausgewählt sind, die besteht aus 4-Fluorphenyl, 4-Chlorphenyl, 4-Bromphenyl, 4-Nitrophenyl, 4-Methylphenyl, 3-Methoxyphenyl, 3-Nitrophenyl, 3-Fluorphenyl, 3-Chlorphenyl, 3-Bromphenyl, 3-Thiomethoxyphenyl, 3-Methylphenyl, 3-Trifluormethylphenyl, 2-Hydroxyphenyl, 2-Methylphenyl, 2-Fluorphenyl, 3,4-Dichlorphenyl, 3,4-Methylendioxyphenyl, 3,5-Difluorphenyl, 3,5-Dichlorphenyl, 2,4-Dichlorphenyl und 2,5-Difluorphenyl.

**53.** Verwendung nach einem der Ansprüche 45 bis 47, wobei $R^1$ eine Phenyl-$(R)_m$-Gruppe ist, wobei R Alkylen mit 1 bis 8 Kohlenstoffatomen ist und *m* gleich 1 ist.

**54.** Verbindung nach Anspruch 53, wobei $R^1$ aus der Gruppe ausgewählt ist, die aus Benzyl, 3-Phenyl-n-Propyl und 4-Phenyl-*n*-Butyl besteht.

**55.** Verwendung nach einem der Ansprüche 45 bis 47, wobei $R^1$ aus der Gruppe ausgewählt ist, die aus Alkyl-, Alkenyl-, Alkcycloalkyl-, Cycloalkyl- und Cycloalkenylgruppen besteht.

**56.** Verwendung nach Anspruch 55, wobei $R^1$ Alkyl ist.

**57.** Verbindung nach Anspruch 55, wobei $R^1$ Cycloalkyl ist.

**58.** Verbindung nach Anspruch 55, wobei $R^1$ Alkenyl ist.

**59.** Verbindung nach Anspruch 55, wobei $R^1$ Cycloalkenyl ist.

**60.** Verbindung nach Anspruch 55, wobei die $R^1$-Alkyl-, -Cycloalkyl-, -Alkcycloalkyl-, -Alkenyl- und -Cycloalkenylgruppen aus der Gruppe ausgewählt sind, die besteht aus *sec*-Butyl, Cyclopropyl, Cyclobutyl, Cyclohexyl, Cyclopentyl, Cyclohex-1-enyl, $-CH_2$-Cyclopropyl, $-CH_2$-Cyclobutyl, $-CH_2$-Cyclohexyl, $-CH_2$-Cyclopentyl, $-CH_2CH_2$-Cyclopropyl, $-CH_2CH_2$-Cyclobutyl, $-CH_2CH_2$-Cyclohexyl und $-CH_2CH_2$-Cyclopentyl.

**61.** Verwendung nach einem der Ansprüche 45 bis 47, wobei $R^1$ aus der Gruppe ausgewählt ist, die aus Heteroaryl- und substituierten Heteroarylgruppen besteht.

**62.** Verwendung nach Anspruch 61, wobei die $R^1$-Heteroaryl- und substituierten -Heteroarylgruppen aus der Gruppe ausgewählt sind, die besteht aus Pyrid-3-yl, Pyrid-4-yl, Thien-2-yl, Thien-3-yl, Benzothiazol-4-yl, 2-Phenylbenzoxazol-5-yl, Furan-2-yl, Benzofuran-2-yl, Benzothiophen-3-yl, 2-Chlorthien-5-yl, 3-Methylisoxazol-5-yl, 2-(Phenylthio)thien-5-yl, 6-Methoxythiophen-2-yl, 3-Phenyl-1,2,4-thiooxadiazol-5-yl und 2-Phenyloxazol-4-yl.

**63.** Verwendung nach einem der Ansprüche 45 bis 62, wobei $R^2$ aus der Gruppe ausgewählt ist, die aus Alkyl mit 1 bis 4 Kohlenstoffatomen, Alkylalkoxy mit 1 bis 4 Kohlenstoffatomen, Phenyl und Alkylthioalkoxy mit 1 bis 4 Kohlenstoffatomen besteht.

**64.** Verwendung nach Anspruch 63, wobei $R^2$ aus der Gruppe ausgewählt ist, die aus Methyl, Ethyl, *n*-Propyl, *iso*-Propyl, *n*-Butyl, *iso*-Butyl, $-CH_2CH_2SCH_3$ und Phenyl besteht.

**65.** Verwendung nach einem der Ansprüche 45 bis 64, wobei X' und X" beide Wasserstoff sind und X Sauerstoff ist.

**66.** Verwendung nach Anspruch 65, wobei $R^3$ aus der Gruppe ausgewählt ist, die besteht aus Methyl, Ethyl, *iso*-Propyl, *n*-Propyl, *n*-Butyl, *iso*-Butyl, Cyclopentyl, Allyl, *iso*-But-2-enyl, 3-Methylpentyl, $-CH_2$-Cyclopropyl, $-CH_2$-Cyclohexyl, $-CH_2$-(3-Tetrahydrofuranyl), $-CH_2$-Thien-2-yl, $-CH_2$(1-Methyl)cyclopropyl, $-CH_2$-Thien-3-yl, $-CH_2-C(O)O$-*tert*-Butyl, $-CH_2-C(CH_3)_3$, $-CH_2CH(CH_2CH_3)_2$, -2-Methylcyclopentyl, -Cyclohex-2-enyl, $-CH\ [CH(CH_3)_2]COOCH_3$, $-CH_2CH_2N(CH_3)_2$, $-CH_2C(CH_3)=CH_2$ und $-CH_2CH=C(CH_3)_2$.

**67.** Verwendung nach einem der Ansprüche 45 bis 64, wobei X' und X" beide Wasserstoff sind und X Schwefel ist.

**68.** Verwendung nach Anspruch 67, wobei $R^3$ aus der Gruppe ausgewählt ist, die aus *iso*-But-2-enyl und *iso*-Butyl besteht.

**69.** Verwendung nach einem der Ansprüche 45 bis 47, wobei die Verbindung der Formel I aus der Gruppe ausgewählt ist, die besteht aus:

*N*-(Phenylacetyl)alanin-*iso*-Butylester
*N*-(Phenylpropionyl)alanin-*iso*-Butylester
*N*-(3-Methylpentanoyl)alanin-*iso*-Butylester
*N*-([(4-Chlorphenyl)acetyl]alanin-*iso*-Butylester
*N*-[(3,4-Dichlorphenyl)acetyl] alanin-*iso*-Butylester
*N*-[(3-Pyridyl)acetyl]alanin-iso-Butylester
*N*-[(1-Naphthyl)acetyl]alanin-*iso*-Butylester
*N*-[(2-Naphthyl)acetyl]alanin-*iso*-Butylester
*N*-(4-Phenylbutanoyl)alanin-*iso*-Butylester
*N*-(5-Phenylpentanoyl)alanin-*iso*-Butylester
*N*-[(4-Pyridyl)acetyl]alanin-*iso*-Butylester
2-[(3,4-Dichlorphenyl)acetamido]buttersäure-*iso*-Butylester
2-[(3-Methoxyphenyl)acetamido]buttersäure-*iso*-Butylester
2-[(4-Nitrophenyl)acetamido]buttersäure-*iso*-Butylester
2-[(3,4-Methylendioxyphenyl)acetamido]buttersäure-*iso*-Butylester
2-[(Thien-3-yl)acetamido]buttersäure-*iso*-Butylester
2-[(4-Chlorphenyl)acetamido]buttersäure-*iso*-Butylester
2-[(3-Nitrophenyl)acetamido]buttersäure-*iso*-Butylester
2-[(2-Hydroxyphenyl)acetamido]buttersäure-*iso*-Butylester
2-[(2-Naphthyl)acetamido]buttersäure-*iso*-Butylester
2-[(2,4-Dichlorphenyl)acetamido]buttersäure-*iso*-Butylester
2-[(4-Bromphenyl)acetamido]buttersäure-*iso*-Butylester
2-[(3-Chlorphenyl)acetamido]buttersäure-*iso*-Butylester
2-[(3-Fluorphenyl)acetamido]buttersäure-*iso*-Butylester
2-[(Benzothiazol-4-yl)acetamido]buttersäure-*iso*-Butylester
2-[(2-Methylphenyl)acetamido]buttersäure-*i*so-Butylester
2-[(2-Fluorphenyl)acetamido]buttersäure-*iso*-Butylester
2-[(4-Fluorphenyl)acetamido]buttersäure-*iso*-Butylester
2-[(3-Bromphenyl)acetamido]buttersäure-*iso*-Butylester
2-[(3-Trifluormethylphenyl)acetamido]buttersäure-*iso*-Butylester
2-[(2-Thienyl)acetamido]buttersäure-*iso*-Butylester
2-(Phenylacetamido)buttersäure-*iso*-Butylester
*N*-(Phenylacetyl)valin-2-Methylbutylester
*N*-(Phenylacetyl)methionin-*iso*-Butylester
*N*-(Phenylacetyl)leucin-*iso*-Butylester
*N*-[(3-Chlorphenyl)acetyl]alanin-3-Methylbut-2-enylester
*N*-[(3-Chlorphenyl)acetyl]alanin-Cyclopropylmethylester
*N*-[(3-Chlorphenyl)acetyl]alanin-2-Thienylmethylester
*N*-[(3-Chlorphenyl)acetyl]alanin-(1-Methylcyclopropyl)methylester
*N*-[(3-Chlorphenyl)acetyl]alanin-3-Thienylmethylester
*N*-[(3-Chlorphenyl)acetyl]alanin-2-Methylcyclopentylester
*N*-[(3-Chlorphenyl)acetyl]alanin-2-Methylprop-2-enylester

*N*-[(3-Chlorphenyl)acetyl]alanin-Cyclohex-2-enylester
*N*-[(2-Phenylbenzoxazol-5-yl)acetyl]alanin-*iso*-Butylester
*N*-[(3-Methylthiophenyl)acetyl]alanin-*iso*-Butylester
*N*-4-[(2-Furyl)acetyl]alanin-*iso*-Butylester
*N*-[(Benzofuran-2-yl)acetyl]alanin-*iso*-Butylester
*N*-[(Benzothiophen-3-yl)acetyl]alanin-*iso*-Butylester
*N*-[(2-Chlor-5-Thienyl)acetyl]alanin-*iso*-Butylester
*N*-[(3-Methylisoxazol-5-yl)acetyl]alanin-*iso*-Butylester
*N*-[(2-Phenylthiothienyl)acetyl]alanin-*iso*-Butylester
*N*-[(6-Methoxybenzothiophen-2-yl)acetyl]alanin-*iso*-Butylester
*N*-[(3-Phenyl-1,2,4-thiadiazol-5-yl)acetyl]alanin-*iso*-Butylester
*N*-[(2-Phenyloxazol-4-yl)acetyl]alanin-*iso*-Butylester
*N*-[(3-Methylphenyl)acetyl]alanin-*iso*-Butylester
*N*-[(2,5-Difluorphenyl)acetyl]alanin-*iso*-Butylester
*N*-[(3,5-Difluorphenyl)acetyl]alanin-*iso*-Butylester
*N*-[(3-Thienyl)acetyl]alanin-*iso*-Butylester
*N*-[(4-Methylphenyl)acetyl]alanin-*iso*-Butylester
*N*-(Phenylacetyl)alanin (1-Methoxycarbonyl)-*iso*-Butylester
*N*-[(3-Nitrophenyl)acetyl]alanin-*iso*-Butylester
*N*-[(3,5-Difluorphenyl)acetyl]alanin-Ethylester
*N*-[(3-Nitrophenyl)acetyl]methionin-Ethylester
*N*-[(3-Chlorphenyl)acetyl]alanin-*iso*-Butylester
*N*-[(3-Chlorphenyl)acetyl]alanin-2-(*N*,*N*-Dimethylamino)ethylester
2-[(3,5-Dichlorphenyl)acetamido]hexylsäure-Methylester
*N*-[(3,5-Dichlorphenyl)acetyl]alanin-*iso*-Butylester
*N*-(Cyclohexylacetyl)alanin-*iso*-Butylester
*N*-(Cyclopentylacetyl)alanin-*iso*-Butylester
*N*-[(Cyclohex-1-enyl)acetyl]alanin-*iso*-Butylester
*N*-[(3-Chlorphenyl)acetyl]alanin-3-Methylbut-2-enylthioester
*N*-[(2-Phenyl)-2-fluoracetyl]alanin-Ethylester
*N*-(3,5-Difluorphenylacetyl)phenylglycin-Methylester
*N*-(3,5-Difluorphenylacetyl)phenylglycin-*iso*-Butylester
*N*-(Cyclopentylacetyl)phenylglycin-Methylester
*N*-(Cyclopentylacetyl)alanin-Methylester
*N*-(Cyclopropylacetyl)phenylglycin-Methylester
*N*-(Cyclopropylacetyl)alanin-Methylester; und
*N*-[(3-Nitrophenyl)acetyl]methionin-*iso*-Butylester,

**70.** Verfahren zum Hemmen einer β-Amyloid-Freisetzung und/oder seiner Synthese in einer Zelle *in vitro*, wobei das Verfahren aufweist: an eine solche Zelle wird eine Menge einer Verbindung der Formel I oder eines Gemischs aus Verbindungen der Formel I' verabreicht, die wirksam ist, um die zelluläre Freisetzung und/oder Synthese von β-Amyloidpeptid zu hemmen, wobei die Formel I' gemäß der Definition in einem der Ansprüche 45 bis 69 ist.

**71.** Verbindung zur Verwendung als Medikament, die aus der Gruppe ausgewählt ist, die besteht aus:

N-(Phenylacetyl)alanin-*iso*-Butylester
N-(3-Phenylpropionyl)alanin-*iso*-Butylester
2-(Phenylacetamido)buttersäure-*iso*-Butylester
N-(Phenylacetyl)valin-2-Methylbutylester
N-(Phenylacetyl)methionin-*iso*-Butylester
N-(Phenalyacetyl)leucin-*iso*-Butylester
N-(Phenylacetyl)alanin-(1-Methoxycarbonyl)-*iso*-Butylester
N-[(2-Phenyl)-2-fluoracetyl]alanin-Ethylester.

**72.** Verbindung, die aus der Gruppe ausgewählt ist, die besteht aus:

N-(Phenylacetyl)alanin-*iso*-Butylester
N-(3-Phenylpropionyl)alanin-*iso*-Butylester

2-(Phenylacetamido)buttersäure-*iso*-Butylester
N-(Phenylacetyl)valin-2-Methylbutylester
N-(Phenylacetyl)methionin-*iso*-Butylester
N-(Phenalyacetyl)leucin-*iso*-Butylester
N-(Phenylacetyl)alanin-(1-Methoxycarbonyl)-*iso*-Butylester
N-[(2-Phenyl)-2-fluoracetyl]alanin-Ethylester.

**73.** Pharmazeutische Zusammensetzung, die einen pharmazeutisch inerten Träger und eine pharmazeutisch wirksame Menge einer Verbindung nach Anspruch 71 oder 72 aufweist.

## Revendications

**1.** Composé de formule I ou mélange de composés de formule I à utiliser comme médicament, où la formule I représente

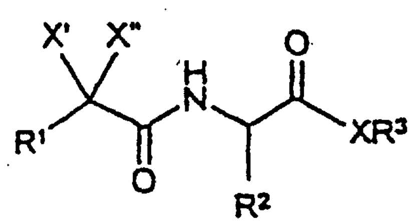

I

où
$R^1$ est pris dans le groupe constitué par

a) un groupe alkyle, alcényle, alkcycloalkyle, naphtyl-$(R)_m$-, où R représente un groupe alkylène présentant de 1 à 8 atomes de carbone et *m* est un entier égal à 0 ou 1, cycloalkyle, cycloalcényle, 3-pyridyle, 4-pyridyle et hétéroaryle présentant de 3 à 10 atomes et de 1 à 4 hétéroatomes pris dans le groupe constitué, par les atomes d'oxygène, de soufre et d'azote, où le groupe hétéroaryle est éventuellement substitué par 1 à 3 substituants pris dans le groupe constitué par un groupe alkyle, alkoxy, aryle, aryloxy, halo, nitro, thioalkoxy et thioaryloxy, à condition que lorsque de tels groupes hétéroaryle présentent au moins un hétéroatome d'azote, au moins un hétéroatome d'oxygène et/ou de soufre soit également présent ;
b) un groupe phényle substitué de formule II :

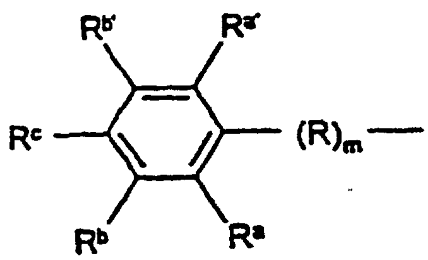

II

où

| | |
|---|---|
| R | représente un groupe alkylène présentant de 1 à 8 atomes de carbone, |
| *m* | est un entier égal à 0 ou 1, |
| $R^a$ et $R^{a'}$ | indépendamment l'un de l'autre sont pris dans le groupe constitué par un atome d'hydrogène, un groupe hydroxy, fluoro et méthyle ; |
| $R^b$ et $R^{b'}$ | indépendamment l'un de l'autre sont pris dans le groupe constitué par un atome d'hydrogène, un groupe alkyle, alkoxy, aryle, cyano, cycloalkyle, halo, hétéroaryle, hétérocyclique, nitro, trihalométhyle, thio-alkoxy, thioaryloxy, thiohétéroaryloxy et -C(O)$R^4$, où $R^4$ est pris dans le groupe constitué par un |

groupe alkyle, aryle, alkoxy et aryloxy ; et

$R^c$ est pris dans le groupe constitué par un atome d'hydrogène, un groupe alkyle, aryle, cyano, halo, nitro, et où $R^b$ et $R^c$ sont condensés pour former un cycle méthylènedioxy avec le cycle phényle ; et

lorsque $R^b$ et/ou $R^{b'}$ et/ou $R^c$ représentent un groupe fluoro, chloro, bromo et/ou nitro, alors $R^a$ et/ou $R^{a'}$ peuvent également représenter un groupe chloro ; et,

par conséquent, $R^a$, $R^{a'}$, $R^b$, $R^{b'}$ et $R^c$ ne sont pas tous simultanément un atome d'hydrogène ; et

c) 1- ou 2-naphtyl-$(R)_m$- substitué à la position 5, 6, 7 et/ou 8 par 1 à 4 substituants pris dans le groupe constitué par des groupes alkyle, alkoxy, halo, cyano, nitro, trihalométhyle et thioalkoxy, où R représente un groupe alkylène de 1 à 8 atomes de carbone et *m* est un entier égal à 0 ou 1 ;

$R^2$ est pris dans le groupe constitué par un groupe alkyle, phényle, alkylalkoxy, alkylthioalkoxy ; et

$R^3$ est pris dans le groupe comprenant $-(CH_2)_nCR^{10}R^5R^6$, où *n* est un entier égal à 0, 1 ou 2, $R^5$ et $R^6$ indépendamment l'un de l'autre sont pris dans un groupe constitué par un atome d'hydrogène, un groupe alkyle, alcényle, aryle, hétéroaryle, hétérocyclique, $-NR^7R^8$, où $R^7$ et $R^8$ indépendamment l'un de l'autre représentent un atome d'hydrogène ou un groupe alkyle, et $-COOR^9$, où $R^9$ représente un groupe alkyle et, de plus, où $R^5$ et $R^6$ peuvent être réunis pour former un groupe cycloalkyle, un groupe cycloalcényle, un groupe aryle, un groupe hétéroaryle et un groupe hétérocyclique, et lorsque $R^5$ et $R^6$ ne sont pas réunis pour former un groupe aryle ou hétéroaryle, alors $R^{10}$ est pris parmi un atome d'hydrogène et un groupe alkyle, à condition que lorsque n vaut zéro, $R^{10}$ représente alors un atome d'hydrogène, et lorsque n est supérieur à zéro et $R^5$ et $R^6$ sont réunis pour former un groupe aryle ou hétéroaryle, $R^{10}$ devienne alors une liaison dans ce groupe ;

X représente un atome d'oxygène ou de soufre ;

X' représente un atome d'hydrogène, un groupe hydroxy ou fluoro ;

X" représente un atome d'hydrogène, un groupe hydroxy ou fluoro, ou X' et X" conjointement forment un groupe oxo,

et leurs sels acceptables du point de vue pharmaceutique,
à condition que :

lorsque $R^1$ représente un groupe pyrid-3-yle, $R^2$ représente un groupe éthyle, X représente un atome d'oxygène, et X' et X" représentent un atome d'hydrogène, $R^3$ ne représente pas alors un groupe $-CH_2CH(CH_3)_2$, et

lorsque $R^1$ représente un groupe indoxazin-3-yle, 2,4-diméthylthiazol-5-yle, 4-méthyl-1,2,5-thiooxadizol-3-yle ou 3,5-di(trifluorométhyl)phényle, $R^2$ représente un groupe méthyle, X représente un atome d'oxygène, et X' et X" représentent un atome d'hydrogène, $R^3$ ne représente pas alors un groupe $-CH_2CH(CH_3)_2$.

2. Composé de formule I ou mélange de composés de formule I selon la revendication 1, pour inhiber la libération du β-amyloïde et/ou sa synthèse dans une cellule.

3. Composé de formule I ou mélange de composés de formule I selon la revendication 1, pour prévenir l'apparition de la maladie d'Alzheimer chez un patient à risque de développer la maladie d'Alzheimer.

4. Composé de formule I ou mélange de composés de formule I selon la revendication 1, pour le traitement d'un patient atteint de la maladie d'Alzheimer afin d'inhiber la dégradation ultérieure de l'état du patient.

5. Composé de formule III

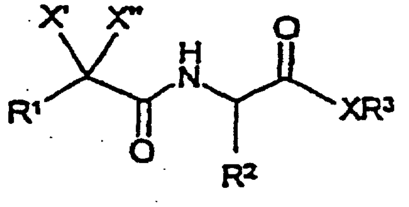

III

où
$R^1$ est pris dans le groupe constitué par

(a) un groupe alkyle, alcényle, alkcycloalkyle, naphtyl-$(R)_m$-, où R représente un groupe alkylène de 1 à 8 atomes de carbone et *m* est un entier égal à 0 ou 1, cycloalkyle, cycloalcényle, 3-pyridyle, 4-pyridyle et hétéroaryle présentant de 3 à 10 atomes et de 1 à 4 hétéroatomes pris dans le groupe comprenant un atome d'oxygène, de soufre et d'azote, où le groupe hétéroaryle est éventuellement substitué par 1 à 3 substituants pris dans le groupe constitué par un groupe alkyle, alkoxy, aryle, aryloxy, halo, nitro, thioalkoxy et thioaryloxy, à condition que lorsque de tels groupes hétéroaryle présentent au moins un hétéroatome d'azote, au moins un hétéroatome d'oxygène et/ou de soufre soit également présent ;
(b) un groupe phényle substitué de formule II :

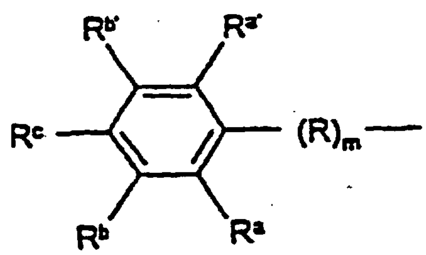

II

où

| | |
|---|---|
| R | représente un groupe alkylène présentant de 1 à 8 atomes de carbone, |
| m | est un entier égal à 0 ou 1, |
| $R^a$ et $R^{a'}$ | indépendamment l'un de l'autre sont pris dans le groupe constitué par un atome d'hydrogène, un groupe hydroxy, fluoro et méthyle ; |
| $R^b$ et $R^{b'}$ | indépendamment l'un de l'autre sont pris dans le groupe constitué par un atome d'hydrogène, un groupe alkyle, alkoxy, aryle, cyano, cycloalkyle, halo, hétéroaryle, hétérocyclique, nitro, trihalométhyle, thio-alkoxy, thioaryloxy, thiohétéroaryloxy et $-C(O)R^4$, où $R^4$ est pris dans le groupe constitué par un groupe alkyle, aryle, alkoxy et aryloxy ; et |
| $R^c$ | est pris dans le groupe constitué par un atome d'hydrogène, un groupe alkyle, aryle, cyano, halo, nitro, et où $R^b$ et $R^c$ sont condensés pour former un cycle méthylènedioxy avec le cycle phényle ; et |
| lorsque $R^b$ et/ou $R^{b'}$ et/ou $R^c$ | représentent un groupe fluoro, chloro, bromo et/ou nitro, alors $R^a$ et/ou $R^{a'}$ peuvent aussi représenter un groupe chloro ; et, |

par conséquent, $R^a$, $R^{a'}$, $R^b$, $R^{b'}$ et $R^c$ ne sont pas tous simultanément un atome d'hydrogène ; et
(c) 1- ou 2-naphtyl-$(R)_m$-, où R représente un groupe alkylène présentant de 1 à 8 atomes de carbone et *m* est un entier égal à 0 ou 1 substitué à la position 5, 6, 7 et/ou 8 par 1 à 4 substituants pris dans le groupe constitué par un groupe alkyle, alkoxy, halo, cyano, nitro, trihalométhyle et thioalkoxy ;

$R^2$ est pris dans le groupe constitué par un groupe alkyle, phényle, alkylalkoxy, alkylthioalkoxy ; et
$R^3$ est pris dans le groupe constitué par $-(CH_2)_nCR^{10}R^5R^6$, où *n* est un entier égal à 0, 1 ou 2, $R^5$ et $R^6$ indépendamment l'un de l'autre sont pris dans un groupe constitué par un atome d'hydrogène, un groupe alkyle, alcényle, aryle, hétéroaryle, hétérocyclique, $-NR^7R^8$, où $R^7$ et $R^8$ indépendamment l'un de l'autre représentent un atome d'hydrogène ou un groupe alkyle, et $-COOR^9$, où $R^9$ représente un groupe alkyle et, de plus, où $R^5$ et $R^6$ peuvent être réunis pour former un groupe cycloalkyle, un groupe cycloalcényle, un groupe aryle, un groupe hétéroaryle et un groupe hétérocyclique, et lorsque $R^5$ et $R^6$ ne sont pas réunis pour former un groupe aryle ou hétéroaryle, dans ce cas $R^{10}$ est pris dans un groupe constitué par un atome d'hydrogène et un groupe alkyle, à condition que lorsque *n* vaut zéro, $R^{10}$ représente alors un atome d'hydrogène et lorsque *n* est supérieur à zéro et $R^5$ et $R^6$ sont réunis pour former un groupe aryle ou hétéroaryle, $R^{10}$ devienne alors une liaison dans ce groupe ;
X représente un atome d'oxygène ou de soufre ;
X' représente un atome d'hydrogène, un groupe hydroxy ou fluoro ;
X" représente un atome d'hydrogène, un groupe hydroxy ou fluoro, ou X' et X" conjointement forment un

groupe oxo,

et leurs sels acceptables du point de vue pharmaceutique,
à condition que :

lorsque $R^1$ représente un groupe pyrid-3-yle, $R^2$ représente un groupe éthyle, X représente un atome d'oxygène, et X' et X" représentent un atome d'hydrogène, $R^3$ ne représente pas alors un groupe $-CH_2CH(CH_3)_2$,
lorsque $R^1$ représente un groupe butyle, $R^2$ représente un groupe méthyle, X représente un atome d'oxygène, et X' et X" représentent un atome d'hydrogène, $R^3$ ne représente pas alors un groupe éthyle,
lorsque $R^1$ représente un groupe méthyle, $-CH(CH_3)_2$ ou cyclohexyle, $R^2$ représente un groupe $-CH_2CH(CH_3)_2$, X représente un atome d'oxygène, et X' représente un atome d'hydrogène et X" représente un groupe hydroxyle, $R^3$ ne représente pas alors un groupe méthyle,
lorsque $R^1$ représente un groupe éthyle ou $C_6H_4Cl$-*p*, $R^2$ représente un groupe phényle, méthyle ou $-CH(CH_3)_2$, X représente un atome d'oxygène, et X' représente un atome d'hydrogène et X" représente un atome de fluor, $R^3$ ne représente pas alors un groupe méthyle, et
lorsque $R^1$ représente un groupe indoxazin-3-yle, 2,4-diméthylthiazol-5-yle, 4-méthyl-1,2,5-thiooxadizol-3-yle ou 3,5-di(trifluorométhyl)phényle, $R_2$ représente un groupe méthyle, X représente un atome d'oxygène, et X' et X" représentent un atome d'hydrogène, $R^3$ ne représente pas alors un groupe $-CH_2CH(CH_3)_2$.

**6.** Composé selon l'une quelconque des revendications 1 à 5, où $R^1$ représente un groupe naphtyle non substitué pris dans le groupe constitué par 1-naphtyle et 2-naphtyle.

**7.** Composé selon l'une quelconque des revendications 1 à 5, où $R^1$ représente un groupe phényle substitué de formule II.

**8.** Composé selon la revendication 7, où le groupe phényle substitué est défini par le suivant :

(a) des groupes phényle monosubstitué présentant une seule substitution à la position 2, 3 ou 4, où chacun des substituants particuliers est régi par les groupes respectifs $R^a$, $R^b$ et $R^c$ ;
(b) des groupes phényle disubstitué présentant deux substituants aux positions 2,3, aux positions 2,4, aux positions 2,5, aux positions 2,6, aux positions 3,4, aux positions 3,5 ou aux positions 3,6, où chacun de ces substituants est régi par les groupes respectifs $R^a$, $R^{a'}$, $R^b$, $R^{b'}$ et $R^c$ ; et
(c) des groupes phényle trisubstitué présentant trois substituants aux positions 2,3,4, aux positions 2,3,5, aux positions 2,3,6, aux positions 3,4,5 et aux positions 3,4,6, où une fois de plus, chacun de ces substituants est régi par les groupes respectifs $R^a$, $R^{a'}$, $R^b$, $R^{b'}$ et $R^c$.

**9.** Composé selon la revendication 8, où les groupes phényle substitué sont pris dans le groupe comprenant 4-fluorophényle, 4-chlorophényle, 4-bromophényle, 4-nitrophényle, 4-méthylphényle, 3-méthoxyphényle, 3-nitrophényle, 3-fluorophényle, 3-chlorophényle, 3-bromophényle, 3-thiométhoxyphényle, 3-méthylphényle, 3-trifluoro-méthylphényle, 2-hydroxyphényle, 2-méthylphényle, 2-fluorophényle, 3,4-dichlorophényle, 3,4-méthylène-dioxyphényle, 3,5-difluorophényle, 3,5-dichloro-phényle, 2,4-dichlorophényle et 2,5-difluorophényle.

**10.** Composé selon l'une quelconque des revendications 1 à 5, où $R^1$ est pris dans le groupe constitué par alkyle, alcényle, alkcycloalkyle, cycloalkyle et cycloalcényle.

**11.** Composé selon la revendication 10, où $R^1$ représente un groupe alkyle.

**12.** Composé selon la revendication 10, où $R^1$ représente un groupe cycloalkyle.

**13.** Composé selon la revendication 10, où $R^1$ représente un groupe alcényle.

**14.** Composé selon la revendication 10, où $R^1$ représente un groupe cycloalcényle.

**15.** Composé selon la revendication 10, où $R^1$ représente un groupe alkyle, cycloalkyle, alkcycloalkyle, alcényle et cycloalcényle qui sont pris dans le groupe constitué par *sec*-butyle, cyclopropyle, cyclobutyle, cyclohexyle, cyclopentyle, cyclohex-1-ényle, $-CH_2$-cyclopropyle, $-CH_2$-cyclobutyle, $-CH_2$-cyclohexyle, $-CH_2$-cyclopentyle, $-CH_2CH_2$-

cyclopropyle, $-CH_2CH_2$-cyclobutyle, $-CH_2CH_2$-cyclohexyle et $-CH_2CH_2$-cyclopentyle.

**16.** Composé selon l'une quelconque des revendications 1 à 5, où $R^1$ est pris dans le groupe constitué par des groupes hétéroaryle et hétéroaryle substitué.

**17.** Composé selon la revendication 16, où les groupes $R^1$ hétéroaryle et hétéroaryle substitué sont pris dans le groupe constitué par pyrid-3-yle, pyrid-4-yle, thién-2-yle, thién-3-yle, benzothiazol-4-yle, 2-phénylbenzoxazol-5-yle, furan-2-yle, benzofuran-2-yle, benzothiophén-3-yle, 2-chlorothién-5-yle, 3-méthylisoxazol-5-yle, 2-(phénylthio)thién-5-yle, 6-méthoxythiophén-2-yle, 3-phényl-1,2,4-thiooxadiazol-5-yle et 2-phényloxazol-4-yle.

**18.** Composé selon l'une quelconque des revendications 1 à 17, où $R^2$ est pris dans le groupe constitué par alkyle présentant de 1 à 4 atomes de carbone, alkylalkoxy présentant de 1 à 4 atomes de carbone, phényle et alkylthioalkoxy présentant de 1 à 4 atomes de carbone.

**19.** Composé selon la revendication 18, où $R^2$ est pris dans le groupe constitué par méthyle, éthyle, *n*-propyle, *iso*-propyle, *n*-butyle, *iso*-butyle, $-CH_2CH_2SCH_3$ et phényle.

**20.** Composé selon l'une quelconque des revendications 1 à 19, où X' et X" représentent chacun un atome d'hydrogène et X représente un atome d'oxygène.

**21.** Composé selon la revendication 20, où $R^3$ est pris dans le groupe constitué par méthyle, éthyle, *iso*-propyle, *n*-propyle, *n*-butyle, *iso*-butyle, cyclopentyle, allyle, *iso*-but-2-ényle, 3-méthylpentyle, $-CH_2$-cyclopropyle, $-CH_2$-cyclohexyle, $-CH_2$-(3-tétrahydrofuranyle), $-CH_2$-thién-2-yle, $-CH_2$(1-méthyl)-cyclopropyle, $-CH_2$-thién-3-yle, $-CH_2$-C(O)O-tert-butyle, $-CH_2-C(CH_3)_3$, $-CH_2CH(CH_2CH_3)_2$, -2-méthylcyclopentyle, -cyclohex-2-ényle, $-CH[CH(CH_3)_2]COOCH_3$, $-CH_2CH_2N(CH_3)_2$, $-CH_2C(CH_3)=CH_2$ et $-CH_2CH=C(CH_3)_2$.

**22.** Composé selon l'une quelconque des revendications 1 à 19, où X' et X" chacun représente un atome d'hydrogène et X représente un atome de soufre.

**23.** Composé selon la revendication 22, où $R^3$ est pris dans le groupe constitué par *iso*-but-2-ényle et *iso*-butyle.

**24.** Composé selon l'une quelconque des revendications 1 à 5, où le composé de formule I est pris dans le groupe constitué par :

N-(3-méthylpentanoyl)alaninate d'*iso*-butyle,
N-[(4-chlorophényl)acétyl]alaninate d'*iso*-butyle,
N-((3,4-dichlorophényl)acétyl]alaninate d'*iso*-butyle,
N-[(3-pyridyl)acétyl]alaninate d'*iso*-butyle,
N-[(1-naphtyl)acétyl]alaninate d'*iso*-butyle,
N-[(2-naphtyl)acétyl]alaninate d'*iso*-butyle,
N-(4-phénylbutanoyl)alaninate d'*iso*-butyle,
N-(5-phénylpentanoyl)alaninate d'*iso*-butyle,
N-[(4-pyridyl)acétyl]alaninate d'*iso*-butyle,
N-[(3,4-dichlorophényl)acétamido]butyrate d'*iso*-butyle,
2-[(3-méthoxyphényl)acétamido]butyrate d'*iso*-butyle,
2-[(4-nitrophényl)acétamido]butyrate d'*iso*-butyle,
2-[(3,4-méthylènedioxyphényl)acétamido]butyrate d'*iso*-butyle,
2-[(thién-3-yl)acétamido]butyrate d'*iso*-butyle,
2-[(4-chlorophényl)acétamido]butyrate d'*iso*-butyle,
2-[(3-nitrophényl)acétamido]butyrate d'*iso*-butyle,
2-[(2-hydroxyphényl)acétamido]butyrate d'*iso*-butyle,
2-[(2-naphtyl)acétamido]butyrate d'*iso*-butyle,
2-[(2,4-dichlorophényl)acétamido]butyrate d'*iso*-butyle,
2-[(4-bromophényl)acétamido]butyrate d'*iso*-butyle,
2-[(3-chlorophényl)acétamido])butyrate d'*iso*-butyle,
2-{(3-fluorophényl)acetamido]butyrate d'*iso*-butyle,
2-[(benzothiazol-4-yl)acétamido]butyrate *d'iso*-butyle,
2-[(2-méthylphényl)acétamido]butyrate d'*iso*-butyle,

2-[(2-fluorophényl)acetamido]butyrate d'*iso*-butyle,
2-[(4-fluorophényl)acétamido]butyrate d'*iso*-butyle,
2-[(3-bromophényl)acétamido]butyrate d'*iso*-butyle,
2-[(3-trifluorométhylphényl)acétamido]butyrate *d'iso*-butyle,
2-[(2-thiényl)acétamido]butyrate d'*iso*-butyle,
N-[(3-chlorophényl)acétyl]alaninate de 3-méthylbut-2-ényle,
N-[(3-chlorophényl)acétyl]alaninate de cyclopropylméthyle,
N-[(3-chlorophényl)acétyl]alaninate de 2-thiénylméthyle,
N[(3-chlorophényl)acétyl]alaninate de (1-méthylcyclopropyl)-méthyle,
N-[(3-chlorophényl)acétyl)alaninate de 3-thiénylméthyle,
N-[(3-chlorophényl)acétyl]alaninate de 2-méthylcyclopentyle,
N-[(3-chlorophényl)acétyl]alaninate de 2-méthylprop-2-ényle,
N-[(3-chlorophényl)acétyl]alaninate de cyclohex-2-ényle,
N-[(2-phénylbenzoxazol-5-yl)acétyl]alaninate *d'iso*-butyle,
N-[(3-méthylthiophényl)acétyl]alaninate d'*iso*-butyle,
N-4-[(2-furyl)acétyl]alaninate d'*iso*-butyle,
N-[(benzofuran-2-yl)acétyl]alaninate d'*iso*-butyle,
N-[(benzothiophén-3-yl)acétyl]alaninate d'*iso*-butyle,
N-[(2-chloro-5-thiényl)acétyl]alaninate d'*iso*-butyle,
N-[(3-méthyl-isoxazol-5-yl)acétyl]alaninate d'*iso*-butyle,
N-[(2-phénylthiothiényl)acétyl]alaninate d'*iso*-butyle,
N-[(6-méthoxybenzothiophén-2-yl)acétyl]alaninate d'*iso*-butyle,
N-[(3-phényl-1,2,4-thiadiazol-5-yl)acétyl]alaninate d'*iso*-butyle,
N-[(2-phényloxazol-4-yl)acétyl]alaninate d'*iso*-butyle,
N-[(3-méthylphényl)acétyl]alaninate d'*iso*-butyle,
N-[(2,5-difluorophényl)acétyl]alaninate d'*iso*-butyle,
N-[(3,5-diflurophényl)acétyl]alaninate d'*iso*-butyle,
N-[(3-thiényl)acétyl]alaninate d'*iso*-butyle,
N-[(4-méthylphényl)acétyl]alaninate d'*iso*-butyle,
N-[(3-nitrophényl)acétyl]alaninate d'*iso*-butyle,
N-[(3,5-difluorophényl)acétyl]alaninate d'éthyle,
N-[(3-nitrophényl)acétyl]méthioninate d'éthyle ,
N-[(3-chlorophényl)acétyl]alaninate d'*iso*-butyle
N-[(3-chlorophényl)acétyl]alaninate de 2-(N,N-diméthylamino)éthyle,
2-[(3,5-dichlorophényl)acétamido]hexanoate de méthyle,
N-[(3,5-dichlorophényl)acétyl]alaninate d'*iso*-butyle,
N-(cyclohexylacétyl)alaninate d'*iso*-butyle,
N-(cyclopentylacétyl)alaninate d'*iso*-butyle,
N-[(cyclohex-1-ényle)acétyl]alaninate d'*iso*-butyle,
N-[(3-chlorophényl)acétyl]alaninate de 3-méthylbut-2-ényle thioester,
N-(3,5-difluorophénylacétyl)phénylglycinate de méthyle, .
N-(3,5-difluorophénylacétyl)phénylglycinate d'*iso*-butyle,
N-(cyclopentylacétyl)phénylglycinate de méthyle,
N-(cyclopentylacétyl)alaninate de méthyle,
N-(cyclopropylacétyl)phénylglycinate de méthyle,
N-(cyclopropylacétyl)alaninate de méthyle, et
N-[(3-nitrophényl)acétyl)méthioninate d'*iso*-butyle.

**25.** Composition pharmaceutique comprenant un véhicule inerte du point de vue pharmaceutique et une quantité efficace du point de vue pharmaceutique d'un composé de formule I :

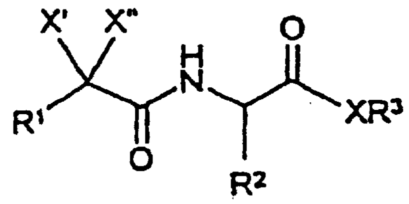

I

où
$R^1$ est pris dans le groupe constitué par

(a) un groupe alkyle, alcényle, alkcycloalkyle, naphtyl-$(R)_m$-, où R représente un groupe alkylène présentant de 1 à 8 atomes de carbone et *m* est un entier égal à 0 ou 1, cycloalkyle, cycloalcényle, 3-pyridyle, 4-pyridyle et hétéroaryle présentant de 3 à 10 atomes et de 1 à 4 hétéroatomes pris dans le groupe constitué par les atomes d'oxygène, de soufre et d'azote, où le groupe hétéroaryle est éventuellement substitué par 1 à 3 substituants pris dans le groupe constitué par un groupe alkyle, alcoxy, aryle, aryloxy, halo, nitro, thioalkoxy et thioaryloxy, à condition que lorsque de tels groupes hétéroaryle présentent au moins un hétéroatome d'azote, au moins un hétéroatome d'oxygène et/ou de soufre soit également présent ;
(b) un groupe phényle substitué de formule II :

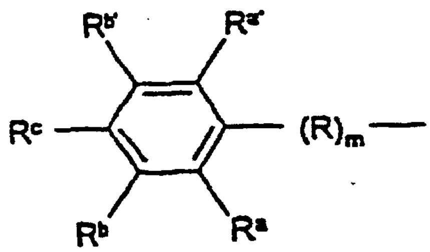

II

où

| | |
|---|---|
| R | représente un groupe alkylène présentant de 1 à 8 atomes de carbone, |
| *m* | est un entier égal à 0 ou 1, |
| $R^a$ et $R^{a'}$ | indépendamment l'un de l'autre sont pris dans le groupe constitué par un atome d'hydrogène, un groupe hydroxy, fluoro et méthyle ; |
| $R^b$ et $R^{b'}$ | indépendamment l'un de l'autre sont pris dans le groupe constitué par un atome d'hydrogène, un groupe alkyle, alkoxy, aryle, cyano, cycloalkyle, halo, hétéroaryle, hétérocyclique, nitro, trihalométhyle, thioalkoxy, thioaryloxy, thiohétéroaryloxy et -C(O)$R^4$, où $R^4$ est pris dans le groupe constitué par un groupe alkyle, aryle, alkoxy et aryloxy ; et |
| $R^c$ | est pris dans le groupe constitué par un atome d'hydrogène, un groupe alkyle, aryle, cyano, halo, nitro, et où $R^b$ et $R^c$ sont condensés pour former un cycle méthylènedioxy avec le cycle phényle ; et lorsque $R^b$ et/ou $R^{b'}$ et/ou $R^c$ représentent un groupe fluoro, chloro, bromo et/ou nitro, alors $R^a$ et/ou $R^{a'}$ peuvent également représenter un groupe chloro ; et, par conséquent, $R^a$, $R^{a'}$, $R^b$, $R^{b'}$ et $R^c$ ne sont pas tous simultanément un atome d'hydrogène ; et |

. (c) 1- ou 2-naphtyl-$(R)_m$- substitué à la position 5, 6, 7 et/ou 8 par 1 à 4 substituants pris dans le groupe constitué par un groupe alkyle, alkoxy, halo, cyano, nitro, trihalométhyle et thioalkoxy, où R représente un groupe alkylène de 1 à 8 atomes de carbone et *m* est un entier égal à 0 ou 1 ;

$R^2$ est pris dans le groupe constitué par un groupe alkyle, phényle, alkylalkoxy, alkylthioalkoxy ; et
$R^3$ est pris dans le groupe constitué par $-(CH_2)_nCR^{10}R^5R^6$, où *n* est un entier égal à 0, 1 ou 2, $R^5$ et $R^6$ indépendamment l'un de l'autre sont pris dans un groupe constitué par un atome d'hydrogène, un groupe alkyle, alcényle, aryle, hétéroaryle, hétérocyclique, $-NR^7R^8$, où $R^7$ et $R^8$ indépendamment l'un de l'autre représentent un atome d'hydrogène ou un groupe alkyle, et $-COOR^9$, où $R^9$ représente un groupe alkyle et, de plus, où $R^5$ et $R^6$ peuvent être réunis pour former un groupe cycloalkyle, un groupe cycloalcényle, un groupe aryle, un groupe hétéroaryle et un groupe hétérocyclique, et lorsque $R^5$ et $R^6$ ne sont pas

réunis pour former un groupe aryle ou hétéroaryle, alors $R^{10}$ est pris dans un groupe constitué par un atome d'hydrogène et un groupe alkyle, à condition que lorsque *n* vaut zéro, $R^{10}$ représente alors un atome d'hydrogène, et lorsque *n* est supérieur à zéro et $R^5$ et R sont réunis pour former un groupe aryle ou hétéroaryle, $R^{10}$ devienne alors une liaison dans ce groupe ;

X représente un atome d'oxygène ou de soufre ;

X' représente un atome d'hydrogène, un groupe hydroxy ou fluoro ;

X" représente un atome d'hydrogène, un groupe hydroxy ou fluoro, ou X' et X" conjointement forment un groupe oxo, et

leurs sels acceptables du point de vue pharmaceutique, à condition que :

lorsque $R^1$ représente un groupe pyrid-3-yle, $R^2$ représente un groupe éthyle, X représente un atome d'oxygène et X' et X" représentent un atome d'hydrogène, $R^3$ ne représente pas alors un groupe $-CH_2CH(CH_3)_2$, et

lorsque $R^1$ représente un groupe indoxazin-3-yle, 2,4-diméthylthiazol-5-yle, 4-méthyl-1,2,5-thiooxadizol-3-yle ou 3,5-di(trifluorométhyl)phényle, $R^2$ représente un groupe méthyle, X représente un atome d'oxygène, et X' et X" représentent un atome d'hydrogène, $R^3$ ne représente pas alors un groupe $-CH_2CH(CH_3)_2$.

**26.** Composition pharmaceutique selon la revendication 25, où $R^1$ représente un groupe naphtyle non substitué pris dans le groupe constitué par 1-naphtyle et 2-naphtyle.

**27.** Composition pharmaceutique selon la revendication 25, où $R^1$ représente un groupe phényle substitué de formule II.

**28.** Composition pharmaceutique selon la revendication 27, où le groupe phényle substitué est défini comme suit :

(a) des groupes phényle monosubstitué présentant une seule substitution à la position 2, 3 ou 4, où chacun des substituants particuliers est régi par les groupes respectifs $R^a$, $R^b$ et $R^c$ ;
(b) des groupes phényle disubstitué présentant deux substituants aux positions 2,3, aux positions 2,4, aux positions 2,5, aux positions 2,6, aux positions 3,4, aux positions 3,5 ou aux positions 3,6, où chacun de ces substituants est régi par les' groupes respectifs $R^a$, $R^{a'}$, $R^b$, $R^{b'}$ et $R^c$ ; et
(c) des groupes phényle trisubstitué présentant trois substituants aux positions 2,3,4, aux positions 2,3,5, aux positions 2,3,6, aux positions 3,4,5 et aux positions 3,4,6, où une fois de plus, chacun de ces substituants est régi par les groupes respectifs $R^a$, $R^{a'}$, $R^b$, $R^{b'}$, et $R^c$.

**29.** Composition pharmaceutique selon la revendication 28, où le groupe phényle substitué est pris dans le groupe constitué par 4-fluorophényle, 4-chlorophényle, 4-bromophényle, 4-nitrophényle, 4-méthylphényle, 3-méthoxyphényle, 3-nitrophényle, 3-fluorophényle, 3-chlorophényle, 3-bromophényle, 3-thiométhoxyphényle, 3-méthylphényle, 3-trifluoro-méthylphényle, 2-hydroxyphényle, 2-méthylphényle, 2-fluorophényle, 3,4-dichlorophényle, 3,4-méthylène-dioxyphényle, 3,5-difluorophényle, 3,5-dichlorophényle, 2,4-dichlorophényle, et 2,5-difluorophényle.

**30.** Composition pharmaceutique selon la revendication 25, où $R^1$ est pris dans le groupe constitué par alkyle, alcényle, alkcycloalkyle, cycloalkyle et cycloalcényle.

**31.** Composition pharmaceutique selon la revendication 30, où $R^1$ représente un groupe alkyle.

**32.** Composition pharmaceutique selon la revendication 30, où $R^1$ représente un groupe cycloalkyle.

**33.** Composition pharmaceutique selon la revendication 30, où $R^1$ représente un groupe alcényle.

**34.** Composition pharmaceutique selon la revendication 30, où $R^1$ représente un groupe cycloalcényle.

**35.** Composition pharmaceutique selon la revendication 30, où $R^1$ qui représente les groupes alkyle, alcényle. alkcycloalkyle, cycloalkyle et cycloalcényle est pris dans le groupe constitué par sec-butyle, cyclopropyle, cyclobutyle, cyclohexyle, cyclopentyle, cyclohex-1-ényle, $-CH_2$-cyclopropyle, $-CH_2$-cyclobutyle, $-CH_2$-cyclohexyle, $-CH_2$-cyclopentyle, $-CH_2CH_2$-cyclopropyle, $-CH_2CH_2$-cyclobutyle, $-CH_2CH_2$-cyclohexyle et $-CH_2CH_2$-cyclopentyle.

**36.** Composition pharmaceutique selon la revendication 25, où $R^1$ est pris dans le groupe constitué par des groupes hétéroaryle et hétéroaryle substitué.

**37.** Composition pharmaceutique selon la revendication 36, où $R^1$ qui représente les groupes hétéroaryle et hétéroaryle substitué est pris dans le groupe constitué par pyrid-3-yle, pyrid-4-yle, thién-2-yle, thién-3-yle, benzothiazol-4-yle, 2-phénylbenzoxazol-5-yle, furan-2-yle, benzofuran-2-yle, benzothiophén-3-yle, 2-chlorothién-5-yle, 3-méthylisoxazol-5-yle, 2-(phénylthio)thién-5-yle, 6-méthoxy-thiophén-2-yle, 3-phényl-1,2,4-thiooxadiazol-5-yle et 2-phényloxazol-4-yle.

**38.** Composition pharmaceutique selon l'une quelconque des revendications 25 à 37, où $R^2$ est pris dans le groupe constitué par alkyle présentant 1 à 4 atomes de carbone, alkylalkoxy présentant 1 à 4 atomes de carbone, phényle et alkylthioalkoxy présentant 1 à 4 atomes de carbone.

**39.** Composition pharmaceutique selon la revendication 38, où $R^2$ est pris dans le groupe constitué par méthyle, éthyle, *n*-propyle, *iso*-propyle, *n*-butyle, *iso*-butyle, $-CH_2CH_2SCH_3$ et phényle.

**40.** Composition pharmaceutique selon l'une quelconque des revendications 25 à 39, où X' et X" représentent chacun un atome d'hydrogène et X un atome d'oxygène.

**41.** Composition pharmaceutique selon la revendication 40, où $R^3$ est pris dans le groupe constitué par méthyle, éthyle, *iso*-propyle, *n*-propyle, *n*-butyle, *iso*-butyle, cyclopentyle, allyle, *iso*-but-2-ényle, 3-méthylpentyle, $-CH_2$-cyclopropyle, $-CH_2$-cyclohexyle, $-CH_2$-(3-tétrahydrofuranyle), $-CH_2$-thién-2-yle, $-CH_2$(1-méthyle)cyclopropyle, $-CH_2$-thién-3-yle, $-CH_2$-C(O)O-*tert*-butyle, $-CH_2-C(CH_3)_3$, $-CH_2CH(CH_2CH_3)_2$, 2-méthylcyclopentyle, -cyclohex-2-ényle, $-CH[CH(CH_3)_2]COOCH_3$, $-CH_2CH_2N(CH_3)_2$, $-CH_2C(CH_3)=CH_2$ et $-CH_2CH=C(CH_3)_2$.

**42.** Composition pharmaceutique selon l'une quelconque des revendications 25 à 39, où X' et X" représentent chacun un atome d'hydrogène et X représente un atome de soufre.

**43.** Composition selon la revendication 42, où $R^3$ est pris dans le groupe constitué par *iso*-but-2-ényle et *iso*-butyle.

**44.** Composition pharmaceutique selon la revendication 25, où le composé de formule I est pris dans le groupe constitué par :

N-(3-méthylpentanoyl)alaninate d'*iso*-butyle,
N-[(4-chlorophényl)acétyl]alaninate d'*iso*-butyle,
N-((3,4-dichlorophényl)acétyl]alaninate d'*iso*-butyle,
N-[(3-pyridyl)acétyl]alaninate d'*iso*-butyle,
N-[(1-naphtyl)acétyl]alaninate d'*iso*-butyle,
N-[(2-naphtyl)acétyl]alaninate d'*iso*-butyle,
N-(4-phénylbutanoyl)alaninate d'*iso*-butyle,
N-(5-phénylpentanoyl)alaninate d'*iso*-butyle,
N-[(4-pyridyl)acétyl]alaninate d'*iso*-butyle,
N-[(3,4-dichlorophényl)acétamido]butyrate d'*iso*-butyle,
2-[(3-méthoxyphényl)acétamido]butyrate d'*iso*-butyle,
2-[(4-nitrophényl)acétamido]butyrate d'*iso*-butyle,
2-[(3,4-méthylènedioxyphényl)acétamido]butyrate d'*iso*-butyle,
2-[(thién-3-yl)acétamido]butyrate d'*iso*-butyle,
2-[(4-chlorophényl)acétamido]butyrate d'*iso*-butyle,
2-[(3-nitrophényl)acétamido]butyrate d'*iso*-butyle,
2-[(2-hydroxyphényl)acétamido]butyrate d'*iso*-butyle,
2-[(2-naphtyl)acétamido]butyrate d'*iso*-butyle,
2-[(2,4-dichlorophényl)acétamido]butyrate d'*iso*-butyle,
2-[(4-bromophényl)acétamido]butyrate d'*iso*-butyle,
2-[(3-chlorophényl)acétamido])butyrate d'*iso*-butyle,
2-[(3-fluorophényl)acetamido]butyrate d'*iso*-butyle,
2-[(benzothiazol-4-yl)acétamido]butyrate d'*iso*-butyle,
2-[(2-méthylphényl)acétamido]butyrate d'*iso*-butyle,
2-[(2-fluorophényl)acetamido]butyrate d'*iso*-butyle,

2-[(4-fluorophényl)acétamido]butyrate d'*iso*-butyle,
2-[(3-bromophényl)acétamido]butyrate d'*iso*-butyle,
2-[(3-trifluorométhylphényl)acétamido]butyrate d'*iso*-butyle,
2-[(2-thiényl)acétamido]butyrate d'*iso*-butyle,
N-[(3-chlorophényl)acétyl]alaninate de 3-méthylbut-2-ényle,
N-[(3-chlorophényl)acétyl]alaninate de cyclopropylméthyle,
N-[(3-chlorophényl)acétyl]alaninate de 2-thiénylméthyle,
N[(3-chlorophényl)acétyl]alaninate de (1-méthylcyclopropyl)-méthyle,
N-[(3-chlorophényl)acétyl)alaninate de 3-thiénylméthyle,
N-[(3-chlorophényl)acétyl]alaninate de 2-méthylcyclopentyle,
N-[(3-chlorophényl)acétyl]alaninate de 2-méthylprop-2-ényle,
N-[(3-chlorophényl)acétyl]alaninate de cyclohex-2-ényle,
N-[(2-phénylbenzoxazol-5-yl)acétyl]alaninate d'*iso* butyle,
N-[(3-méthylthiophényl)acétyl]alaninate d'*iso*-butyle,
N-4-[(2-furyl)acétyl]alaninate d'*iso*-butyle,
N-[(benzofuran-2-yl)acétyl]alaninate d'*iso*-butyle,
N-[(benzothiophén-3-yl)acétyl]alaninate d'*iso*-butyle,
N-[(2-chloro-5-thiényl)acétyl]alaninate d'*iso*-butyle,
N-[(3-méthyl-isoxazol-5-yl)acétyl]alaninate d'*iso*-butyle,
N-[(2-phénylthiothiényl)acétyl]alaninate d'*iso*-butyle,
N-[(6-méthoxybenzothiophén-2-yl)acétyl]alaninate d'*iso*-butyle,
N-[(3-phényl-1,2,4-thiadiazol-5-yl)acétyl]alaninate d'*iso*-butyle,
N-[(2-phényloxazol-4-yl)acétyl]alaninate d'*iso*-butyle,
N-[(3-méthylphényl)acétyl]alaninate d'*iso*-butyle,
N-[(2,5-difluorophényl)acétyl]alaninate d'*iso*-butyle,
N-[(3,5-diflurophényl)acétyl]alaninate d'*iso*-butyle,
N-[(3-thiényl)acétyl]alaninate d'*iso*-butyle,
N-[(4-méthylphényl)acétyl]alaninate d'*iso*-butyle,
N-[(3-nitrophényl)acétyl]alaninate d'*iso*-butyle,
N-[(3,5-difluorophényl)acétyl]alaninate d'éthyle,
N-[(3-nitrophényl)acétyl]méthioninate d'éthyle,
N-[(3-chlorophényl)acétyl]alaninate d'*iso*-butyle,
N-[(3-chlorophényl)acétyl]alaninate de 2-(N,N-diméthylamino)-éthyle,
2-[(3,5-dichlorophényl)acétamido]hexanoate de méthyle,
N-[(3,5-dichlorophényl)acétyl]alaninate d'*iso*-butyle,
N-(cyclohexylacétyl)alaninate d'*iso*-butyle,
N-(cyclopentylacétyl)alaninate d'*iso*-butyle,
N-[(cyclohex-1-ényle)acétyl]alaninate d'*iso*-butyle,
N-[(3-chlorophényl)acétyl]alaninate de 3-méthylbut-2-ényle thioester,
N-(3,5-difluorophénylacétyl)phénylglycinate de méthyle,
N-(3,5-difluorophénylacétyl)phénylglycinate d'*iso*-butyle,
N-(cyclopéntylacétyl)phénylglycinate de méthyle,
N-(cyclopentylacétyl)alaninate de méthyle,
N-(cyclopropylacétyl)phénylglycinate de méthyle,
N-(cyclopropylacétyl)alaninate de méthyle, et
N-[(3-nitrophényl)acétyl]méthioninate d'*iso*-butyle.

**45.** Utilisation d'un composé de formule I' ou d'un mélange de composés de formule I' dans la fabrication d'un médicament pour l'inhibition de la libération du β-amyloïde et/ou de sa synthèse dans une cellule, où la formule I'

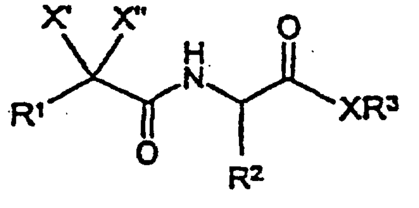

I'

où
$R^1$ est pris dans le groupe constitué par

(a) un groupe alkyle, alcényle, alkcycloalkyle, phényl-$(R)_m$-, naphtyl-$(R)_m$-, où R est un groupe alkylène présentant de 1 à 8 atomes de carbone et *m* est un entier égal à 0 ou 1, cycloalkyle, cycloalcényle, 3-pyridyle, 4-pyridyle et hétéroaryle présentant de 3 à 10 atomes et 1 à 4 hétéroatomes pris dans le groupe constitué par un atome d'oxygène, de soufre et d'azote, où le groupe hétéroaryle est éventuellement substitué par 1 à 3 substituants pris dans le groupe constitué par un groupe alkyle, alkoxy, aryle, aryloxy, halo, nitro, thioalkoxy et thioaryloxy, à condition que lorsque de tels groupes hétéroaryle présentent au moins un hétéroatome d'azote, au moins un hétéroatome d'oxygène et/ou de soufre soit également présent ;
(b) un groupe phényle substitué de formule II:

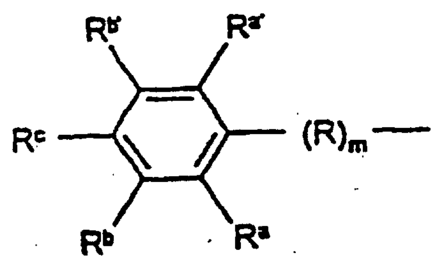

II

où

| | |
|---|---|
| R | représente un groupe alkylène présentant de 1 à 8 atomes de carbone, |
| *m* | est un entier égal à 0 ou 1, |
| $R^a$ et $R^{a'}$ | indépendamment l'un de l'autre sont pris dans le groupe constitué par un atome d'hydrogène, un groupe hydroxy, fluoro et méthyle ; |
| $R^b$ et $R^{b'}$ | indépendamment l'un de l'autre sont pris dans le groupe constitué par un atome d'hydrogène, un groupe alkyle, alkoxy, aryle, cyano, cycloalkyle, halo, hétéroaryle, hétérocyclique, nitro, trihalométhyle, thioalkoxy, thioaryloxy, thiohétéroaryloxy et -C(O)$R^4$, où $R^4$ est pris dans le groupe constitué par un groupe alkyle, aryle, alkoxy et aryloxy ; et |
| $R^c$ | est pris dans le groupe constitué par un atome d'hydrogène, un groupe alkyle, aryle, cyano, halo, nitro, et où $R^b$ et $R^c$ sont condensés pour former un cycle méthylènedioxy avec le cycle phényle ; et |
| lorsque $R^b$ et/ou $R^{b'}$ et/ou $R^c$ | représentent un groupe fluoro, chloro, bromo et/ou nitro, alors $R^a$ et/ou $R^{a'}$ peuvent aussi représenter un groupe chloro ; et |

(c) 1- ou 2-naphtyl-$(R)_m$- substitué à la position 5, 6, 7 et/ou 8 par 1 à 4 substituants pris dans le groupe constitué par un groupe alkyle, alkoxy, halo, cyano, nitro, trihalométhyle et thioalkoxy, où R représente un groupe alkylène présentant de 1 à 8 atomes de carbone et *m* est un entier égal à 0 ou 1 ;

$R^2$ est pris dans le groupe constitué par un atome d'hydrogène, un groupe alkyle, phényle, alkylalkoxy, alkylthioalkoxy ; et
$R^3$ est pris dans le groupe comprenant un groupe $-(CH_2)_nCR^{10}R^5R^6$, où *n* est un entier égal à 0, 1 ou 2, $R^5$ et $R^6$ indépendamment l'un de l'autre sont pris dans un groupe constitué par un atome d'hydrogène, un groupe alkyle, alcényle, aryle, hétéroaryle, hétérocyclique, $-NR^7R^8$, où $R^7$ et $R^8$ indépendamment l'un de l'autre représentent un atome d'hydrogène ou un groupe alkyle, et $-COOR^9$, où $R^9$ représente un groupe alkyle, et de plus, où $R^5$ et $R^6$ peuvent être réunis pour former un groupe cycloalkyle, un groupe cycloalcényle, un groupe aryle, un groupe hétéroaryle et un groupe hétérocyclique, et lorsque $R^5$ et $R^6$ ne sont pas réunis pour former un groupe aryle ou hétéroaryle, alors $R^{10}$ est pris dans un groupe constitué par un atome d'hydrogène et un groupe alkyle à condition que lorsque *n* vaut zéro, $R^{10}$ représente alors un atome d'hydrogène, et lorsque *n* est supérieur à zéro et $R^5$ et $R^6$ sont réunis pour former un groupe aryle ou hétéroaryle, $R^{10}$ devienne alors une liaison dans ce groupe ;
X représente un atome d'oxygène ou de soufre ;
X' représente un atome d'hydrogène, un groupe hydroxy ou fluoro ;

X" représente un atome d'hydrogène, un groupe hydroxy ou fluoro, ou X' et X" conjointement forment un groupe oxo, et leurs sels acceptables du point de vue pharmaceutique, à condition que :

lorsque $R^1$ représente un groupe phényle, $R^2$ représente un groupe $-CH(CH_3)CH_2CH_3$, X représente un atome d'oxygène; et X' et X" représentent un atome d'hydrogène, alors $R^3$ ne représente pas un groupe $-CH_2CH_3$ ou $-CH_2CH(CH_3)_2$,

lorsque $R^1$ représente un groupe phényle, $R^3$ représente un groupe $-CH_2CH(CH_3)_2$, X représente un atome d'oxygène, et X' et X" représentent un atome d'hydrogène, alors $R^2$ ne représente pas un groupe $-CH(CH_3)_2$,

lorsque $R^1$ représente un groupe pyrid-3-yle, $R^2$ représente un groupe éthyle, X représente un atome d'oxygène, et X' et X" représentent un atome d'hydrogène, alors $R^3$ ne représente pas un groupe $-CH_2CH(CH_3)_2$,

lorsque $R^1$ représente un groupe indoxazin-3-yle, 2,4-diméthylthiazol-5-yle, 4-méthyl-1,2,5-thiooxadizol-3-yle ou 3,5-di(trifluorométhyl)phényle, $R^2$ représente un groupe méthyle, X représente un atome d'oxygène, et X' et X" représentent un atome d'hydrogène, alors $R^3$ ne représente pas un groupe $-CH_2CH(CH_3)_2$, et

lorsque $R^1$ représente un groupe $-CH_2$-phényle, $R^3$ représente un groupe $-CH_2CH_3$, X représente un atome d'oxygène, et X' et X" représentent un atome d'hydrogène, alors $R^2$ ne représente pas un groupe $-CH_2CH(CH_3)_2$.

**46.** Utilisation d'un composé de formule l'ou d'un mélange de composés de formule I' dans la fabrication d'un médicament comprenant le composé et un véhicule inerte du point de vue pharmaceutique pour la prévention de l'apparition de la maladie d'Alzheimer chez un patient à risque de développer la maladie d'Alzheimer, où la formule I' est

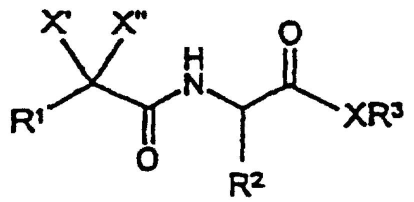

I'

où

$R^1$ est pris dans le groupe constitué par

(a) un groupe alkyle, alcényle, alkcycloalkyle, phényl-$(R)_m$-, naphtyl-$(R)_m$-, où R est un groupe alkylène de 1 à 8 atomes de carbone et *m* est un entier égal à 0 ou 1, cycloalkyle, cycloalcényle, 3-pyridyle, 4-pyridyle et hétéroaryle présentant de 3 à 10 atomes et 1 à 4 hétéroatomes pris dans le groupe constitué par un atome d'oxygène, de soufre et d'azote,
où le groupe hétéroaryle est éventuellement substitué par 1 à 3 substituants pris dans le groupe constitué par un groupe alkyle, alkoxy, aryle, aryloxy, halo, nitro, thioalkoxy et thioaryloxy, à condition que lorsque de tels groupes hétéroaryle présentent au moins un hétéroatome d'azote, au moins un hétéroatome d'oxygène et/ou de soufre soit également présent ;
(b) un groupe phényle substitué de formule II :

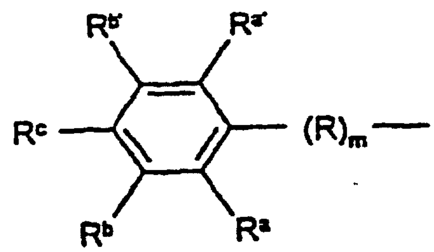

II

où

| | |
|---|---|
| R | représente un groupe alkylène présentant de 1 à 8 atomes de carbone, |
| *m* | est un entier égal à 0 ou 1, |
| $R^a$ et $R^{a'}$ | indépendamment l'un de l'autre sont pris dans le groupe constitué par un atome d'hydrogène, un groupe hydroxy, fluoro et méthyle ; |
| $R^b$ et $R^{b'}$ | indépendamment l'un de l'autre sont pris dans le groupe constitué par un atome d'hydrogène, un groupe alkyle, alkoxy, aryle, cyano, cycloalkyle, halo, hétéroaryle, hétérocyclique, nitro, trihalométhyle, thioalkoxy, thioaryloxy, thiohétéroaryloxy et -C(O)$R^4$, où $R^4$ est pris dans le groupe constitué par un groupe alkyle, aryle, alkoxy et aryloxy ; et |
| $R^c$ | est pris dans le groupe constitué par un atome d'hydrogène, un groupe alkyle, aryle, cyano, halo, nitro, et où $R^b$ et $R^c$ sont condensés pour former un cycle méthylènedioxy avec le cycle phényle ; et |
| lorsque $R^b$ et/ou $R^{b'}$ et/ou $R^c$ | représentent un groupe fluoro, chloro, bromo et/ou nitro, alors $R^a$ et/ou $R^{a'}$ peuvent aussi représenter un groupe chloro ; et |

(c) 1- ou 2-naphtyl-$(R)_m$- substitué à la position 5, 6, 7 et/ou 8 par 1 à 4 substituants pris dans le groupe constitué par un groupe alkyle, alkoxy, halo, cyano, nitro, trihalométhyle et thioalkoxy, où R représente un groupe alkylène présentant de 1 à 8 atomes de carbone et *m* est un entier égal à 0 ou 1 ;

$R^2$ est pris dans le groupe constitué par un atome d'hydrogène, un groupe alkyle, phényle, alkylalkoxy, alkylthioalkoxy ; et

$R^3$ est pris dans le groupe constitué par $-(CH_2)_nCR^{10}R^5R^6$, où n est un entier égal à 0, 1 ou 2, $R^5$ et $R^6$ indépendamment l'un de l'autre sont pris dans un groupe constitué par un atome d'hydrogène, un groupe alkyle, alcényle, aryle, hétéroaryle, hétérocyclique, $-NR^7R^8$, où $R^7$ et $R^8$ indépendamment l'un de l'autre représentent un atome d'hydrogène ou un groupe alkyle, et $-COOR^9$, où $R^9$ représente un groupe alkyle et, de plus, où $R^5$ et $R^6$ peuvent être réunis pour former un groupe cycloalkyle, un groupe cycloalcényle, un groupe aryle, un groupe hétéroaryle et un groupe hétérocyclique, et lorsque $R^5$ et $R^6$ ne sont pas réunis pour former un groupe aryle ou hétéroaryle, dans ce cas $R^{10}$ est pris dans un groupe constitué par un atome d'hydrogène et un groupe alkyle, à condition que lorsque *n* vaut zéro, $R^{10}$ représente alors un atome d'hydrogéné, et lorsque *n* est supérieur à zéro et $R^5$ et $R^6$ sont réunis pour former un groupe aryle ou hétéroaryle, $R^{10}$ devienne alors une liaison dans ce groupe ;

X représente un atome d'oxygène ou de soufre ;

X' représente un atome d'hydrogène, un groupe hydroxy ou fluoro ;

X" représente un atome d'hydrogène, un groupe hydroxy ou fluoro, ou X' et X" conjointement forment un groupe oxo, et leurs sels acceptables du point de vue pharmaceutique, à condition que :

lorsque $R^1$ représente un groupe phényle, $R^2$ représente un groupe $-CH(CH_3)CH_2CH_3$, X représente un atome d'oxygène, et X' et X" représentent un atome d'hydrogène, $R^3$ ne représente pas alors un groupe $-CH_2CH_3$ ou $-CH_2CH(CH_3)_2$,

lorsque $R^1$ représente un groupe phényle, $R^3$ représente un groupe $-CH_2CH(CH_3)_2$, X représente un atome d'oxygène, et X' et X" représentent un atome d'hydrogène, $R^2$ ne représente pas alors un groupe $-CH(CH_3)_2$,

lorsque $R^1$ représente un groupe pyrid-3-yle, $R^2$ représente un groupe éthyle, X représente un atome d'oxygène, et X' et X" représentent un atome d'hydrogène, $R^3$ ne représente pas alors un groupe $-CH_2CH(CH_3)_2$,

lorsque $R^1$ représente un groupe indoxazin-3-yle, 2,4-diméthylthiazol-5-yle, 4-méthyl-1,2,5-thiooxadizol-3-yle ou 3,5-di(trifluorométhyl)phényle, $R^2$ représente un groupe méthyle, X représente un atome d'oxygène, et X' et X" représentent un atome d'hydrogène, $R^3$ ne représente pas alors un groupe $-CH_2CH(CH_3)_2$, et

lorsque $R^1$ représente un groupe $-CH_2$-phényle, $R^3$ représente un groupe $-CH_2CH_3$, X représente un atome d'oxygène, et X' et X" représentent un atome d'hydrogène, $R^2$ ne représente pas alors un groupe $-CH_2CH(CH_3)_2$.

**47.** Utilisation d'un composé de formule I' ou d'un mélange de composés de formule I' dans la fabrication d'un médicament comprenant le composé et un véhicule inerte du point de vue pharmaceutique pour l'inhibition de la détérioration de l'état d'un patient atteint de la maladie d'Alzheimer, où la formule I' est :

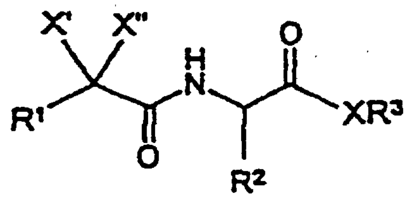

I'

où
$R^1$ est pris dans le groupe constitué par

(a) un groupe alkyle, alcényle, alkcycloalkyle, phényl-$(R)_m$-, naphtyl-$(R)_m$-, où R est un groupe alkylène présentant de 1 à 8 atomes de carbone et *m* est un entier égal à 0 ou 1, cycloalkyle, cycloalcényle, 3-pyridyle, 4-pyridyle et hétéroaryle présentant de 3 à 10 atomes et 1 à 4 hétéroatomes pris dans le groupe constitué par un atome d'oxygène, de soufre et d'azote, où le groupe hétéroaryle est éventuellement substitué par 1 à 3 substituants pris dans le groupe constitué par un groupe alkyle, alkoxy, aryle, aryloxy, halo, nitro, thioalkoxy et thioaryloxy, à condition que lorsque de tels groupes hétéroaryles présentent au moins un hétéroatome d'azote, au moins un hétéroatome d'oxygène et/ou de soufre soit également présent ;
(b) un groupe phényle substitué de formule II :

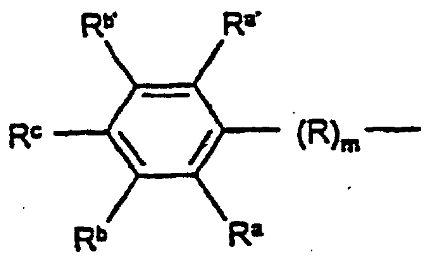

II

où

| | |
|---|---|
| R | représente un groupe alkylène présentant de 1 à 8 atomes de carbone, |
| m | est un entier égal à 0 ou 1, |
| $R^a$ et $R^{a'}$ | indépendamment l'un de l'autre sont pris dans le groupe constitué par un atome d'hydrogène, un groupe hydroxy, fluoro et méthyle ; |
| $R^b$ et $R^{b'}$ | indépendamment l'un de l'autre sont pris dans le groupe constitué par un atome d'hydrogène, un groupe alkyle, alkoxy, aryle, cyano, cycloalkyle, halo, hétéroaryle, hétérocyclique, nitro, trihalométhyle, thioalkoxy, thioaryloxy, thiohétéroaryloxy et -C(O)$R^4$, où $R^4$ est pris dans le groupe constitué par un groupe alkyle, aryle, alkoxy et aryloxy ; et |
| $R^c$ | est pris dans le groupe constitué par un atome d'hydrogène, un groupe alkyle, aryle, cyano, halo, nitro, et où $R^b$ et $R^c$ sont condensés pour former un cycle méthylènedioxy avec le cycle phényle ; et |
| lorsque $R^b$ et/ou $R^{b'}$ et/ou $R^c$ | représentent un groupe fluoro, chloro, bromo et/ou nitro, alors $R^a$ et/ou $R^{a'}$ peuvent aussi représenter un groupe chloro ; et |

(c) 1- ou 2-naphtyl-$(R)_m$- substitué à la position 5, 6, 7 et/ou 8 par 1 à 4 substituants pris dans le groupe constitué par un groupe alkyle, alkoxy, halo, cyano, nitro, trihalométhyle et thioalkoxy, où R représente un groupe alkylène présentant de 1 à 8 atomes de carbone et *m* est un entier égal à 0 ou 1 ;

$R^2$ est pris dans le groupe constitué par un atome d'hydrogène, un groupe alkyle, phényle, alkylalkoxy, alkylthioalkoxy ; et
$R^3$ est pris dans le groupe constitué par $-(CH_2)_nCR^{10}R^5R^6$, où n est un entier égal à 0, 1 ou 2, $R^5$ et $R^6$ indépendamment l'un de l'autre sont pris dans un groupe constitué par un atome d'hydrogène, un groupe alkyle, alcényle, aryle, hétéroaryle, hétérocyclique, $-NR^7R^8$, où $R^7$ et $R^8$ indépendamment l'un de l'autre représentent un atome d'hydrogène ou un groupe alkyle, et $-COOR^9$, où $R^9$ représente un groupe alkyle,

et de plus où $R^5$ et $R^6$ peuvent être réunis pour former un groupe cycloalkyle, un groupe cycloalcényle, un groupe aryle, un groupe hétéroaryle et un groupe hétérocyclique, et lorsque $R^5$ et $R^6$ ne sont pas réunis pour former un groupe aryle ou hétéroaryle, dans ce cas $R^{10}$ est pris dans un groupe constitué par un atome d'hydrogène et un groupe alkyle, à condition que lorsque *n* vaut zéro, $R^{10}$ représente alors un atome d'hydrogène, et lorsque *n* est supérieur à zéro et $R^5$ et $R^6$ sont réunis pour former un groupe aryle ou hétéroaryle, $R^{10}$ devienne alors une liaison dans ce groupe ;

X représente un atome d'oxygène ou de soufre ;

X' représente un atome d'hydrogène, un groupe hydroxy ou fluoro ;

X" représente un atome d'hydrogène, un groupe hydroxy ou fluoro, ou X' et X" conjointement forment un groupe oxo, et leurs sels acceptables du point de vue pharmaceutique, à condition que :

lorsque $R^1$ représente un groupe phényle, $R^2$ représente un groupe $-CH(CH_3)CH_2CH_3$, X représente un atome d'oxygène, et X' et X" représentent un atome d'hydrogène, $R^3$ ne représente pas alors un groupe $-CH_2CH_3$ ou $-CH_2CH(CH_3)_2$,

lorsque $R^1$ représente un groupe phényle, $R^3$ représente un groupe $-CH_2CH(CH_3)_2$, X représente un atome d'oxygène, et X' et X" représentent un atome d'hydrogène, $R^2$ ne représente pas alors un groupe $-CH(CH_3)_2$,

lorsque $R^1$ représente un groupe pyrid-3-yle, $R^2$ représente un groupe éthyle, X représente un atome d'oxygène, et X' et X" représentent un atome d'hydrogène, $R^3$ ne représente pas alors un groupe $-CH_2CH(CH_3)_2$,

lorsque $R^1$ représente un groupe indoxazin-3-yle, 2,4-diméthylthiazol-5-yle, 4-méthyl-1,2,5-thiooxadizol-3-yle ou 3,5-di(trifluorométhyl)phényle, $R^2$ représente un groupe méthyle, X représente un atome d'oxygène, et X' et X" représentent un atome d'hydrogène, $R^3$ ne représente pas alors un groupe $-CH_2CH(CH_3)_2$, et

lorsque $R^1$ représente un groupe $-CH_2$-phényle, $R^3$ représente un groupe $-CH_2CH_3$, X représente un atome d'oxygène, et X' et X" représentent un atome d'hydrogène, $R^2$ ne représente pas alors un groupe $-CH_2CH(CH_3)_2$.

**48.** Utilisation selon l'une quelconque des revendications 45 à 47, où $R^1$ représente un groupe phényle non substitué.

**49.** Utilisation selon l'une quelconque des revendications 45 à 47, où $R^1$ représente un groupe naphtyle non substitué pris dans le groupe constitué par 1-naphtyle et 2-naphtyle.

**50.** Utilisation selon l'une quelconque des revendications 45 à 47, où $R^1$ représente un groupe phényle substitué de formule II.

**51.** Utilisation selon la revendication 50, où le groupe phényle substitué est défini par le suivant :

(a) des groupes phényle monosubstitué présentant une seule substitution à la position 2, 3 ou 4, où chacun des substituants particuliers est régi par les groupes respectifs $R^a$, $R^b$ et $R^c$ ;
(b) des groupes phényle disubstitué présentant deux substituants aux positions 2,3, aux positions 2,4, aux positions 2,5, aux positions 2,6, aux positions 3,4, aux positions 3,5 ou aux positions 3,6, où chacun de ces substituants est régi par les groupes respectifs $R^a$, $R^{a'}$, $R^b$, $R^{b'}$ et $R^c$ ; et
(c) des groupes phényle trisubstitué présentant trois substituants aux positions 2,3,4, aux positions 2,3,5, aux positions 2,3,6, aux positions 3,4,5 et aux positions 3,4,6, où une fois de plus, chacun de ces substituants est. régi par les groupes respectifs $R^a$, $R^{a'}$, $R^b$, $R^{b'}$, et $R^c$.

**52.** Utilisation selon la revendication 51, où les groupes phényle substitué sont pris dans le groupe constitué par 4-fluorophényle, 4-chlorophényle, 4-bromophényle, 4-nitrophényle, 4-méthylphényle, 3-méthoxyphényle, 3-nitrophényle, 3-fluorophényle, 3-chlorophényle, 3-bromophényle, 3-thiométhoxyphényle, 3-méthylphényle, 3-trifluorométhylphényle, 2-hydroxyphényle, 2-méthylphényle, 2-fluorophényle, 3,4-dichlorophényle, 3,4-méthylène-dioxyphényle, 3,5-difluorophényle, 3,5-dichlorophényle, 2,4-dichlorophényle et 2,5-difluorophényle.

**53.** Utilisation selon l'une quelconque des revendications 45 à 47, où $R^1$ représente un groupe phényl-$(R)_m$-, où R représente un groupe alkylène présentant 1 à 8 atomes de carbone et *m* est égal à 1.

**54.** Composé selon la revendication 53, où $R^1$ est pris dans le groupe constitué par benzyle, 3-phényle-*n*-propyle et 4-phényl-*n*-butyle.

**55.** Utilisation selon l'une quelconque des revendications 45 à 47, où $R^1$ est pris dans le groupe constitué par un groupe alkyle, alcényle, alkcycloalkyle, cycloalkyle et cycloalcényle.

**56.** Utilisation selon la revendication 55, où $R^1$ représente un groupe alkyle.

**57.** Utilisation selon la revendication 55, où $R^1$ représente un groupe cycloalkyle.

**58.** Utilisation selon la revendication 55, où $R^1$ représente un groupe alcényle.

**59.** Utilisation selon la revendication 55, où $R^1$ représente un groupe cycloalcényle.

**60.** Composé selon la revendication 55, où les groupes $R^1$ alkyle, cycloalkyle, alkcycloalkyle, alcényle et cycloalcényle sont pris dans le groupe constitué par sec-butyle, cyclopropyle, cyclobutyle, cyclohexyle, cyclopentyle, cyclohex-1-ényle, $-CH_2$-cyclopropyle, $-CH_2$-cyclobutyle, $-CH_2$-cyclohexyle, $-CH_2$-cyclopentyle, $-CH_2CH_2$-cyclopropyle, $-CH_2CH_2$-cyclobutyle, $-CH_2CH_2$-cyclohexyle, et $-CH_2CH_2$-cyclopentyle.

**61.** Utilisation selon l'une quelconque des revendications 45 à 47, où $R^1$ est pris dans le groupe constitué par un groupe hétéroaryle et hétéroaryle substitué.

**62.** Utilisation selon la revendication 61, où les groupes $R^1$ hétéroaryle et hétéroaryle substitué sont pris dans le groupe constitué par pyrid-3-yle, pyrid-4-yle, thién-2-yle, thién-3-yle, benzothiazol-4-yle, 2-phénylbenzoxazol-5-yle, furan-2-yle, benzofuran-2-yle, benzothiophén-3-yle, 2-chlorothién-5-yle, 3-méthylisoxazol-5-yle, 2-(phénylthio)thién-5-yle, 6-méthoxythiophén-2-yle, 3-phényl-1,2,4-thiooxadiazol-5-yle et 2-phényloxazol-4-yle.

**63.** Utilisation selon l'une quelconque des revendications 45 à 62, où $R^2$ est pris dans le groupe constitué par alkyle présentant 1 à 4 atomes de carbone, alkylalkoxy présentant 1 à 4 atomes de carbone, phényle et alkylthioalkoxy présentant 1 à 4 atomes de carbone.

**64.** Utilisation selon la revendication 63, où $R^2$ est pris dans le groupe constitué par méthyle, éthyle, *n*-propyle, *iso*-propyle, *n*-butyle, *iso*-butyle, $-CH_2CH_2SCH_3$ et phényle.

**65.** Utilisation selon l'une quelconque des revendications 45 à 64, où X' et X" chacun représente un atome d'hydrogène et X représente un atome d'oxygène.

**66.** Utilisation selon la revendication 65, où $R^3$ est pris dans le groupe constitué par un groupe méthyle, éthyle, *iso*-propyle, *n*-propyle, *n*-butyle, *iso*-butyle, cyclopentyle, allyle, *iso*-but-2-ényle, 3-méthyl-pentyle, $-CH_2$-cyclopropyle, $-CH_2$-cyclohexyle, $-CH_2$-(3-tétrahydrofuranyle), $-CH_2$-thién-2-yle, $-CH_2$(1-méthyle)cyclopropyle, $-CH_2$-thién-3-yle, $-CH_2$-C (O)O-*tert*-butyle, $-CH_2-C\ (CH_3)_3$, $-CH_2CH\ (CH_2CH_3)_2$, -2-méthyl-cyclopentyle, -cyclohex-2-ényle, $-CH\ [CH\ (CH_3)_2]COOCH_3$, $-CH_2CH_2N\ (CH_3)_2$, $-CH_2C(CH_3)\ =CH_2$ et $-CH_2CH=C(CH_3)_2$.

**67.** Utilisation selon l'une quelconque des revendications 45 à 64, où X' et X" chacun représente un atome d'hydrogène et X représente un atome de soufre.

**68.** Utilisation selon la revendication 67, où $R^3$ est pris dans le groupe constitué par un groupe *iso*-but-2-ényle et *iso*-butyle.

**69.** Utilisation selon l'une quelconque des revendications 45 à 47, où le composé de formule I est pris dans le groupe constitué par :

N-(phénylacétyl)alaninate d'*iso*-butyle,
N-(3-phénylpropionyl)alaninate d'*iso*-butyle,
N-(3-méthylpentanoyl)alaninate d'*iso*-butyle,
N-[(4-chlorophényl)acétyl]alaninate d'*iso*-butyle,
N-[(3,4-dichlorophényl)acétyl]alaninate d'*iso*-butyle,
N-[(3-pyridylacétyl]alaninate d'*iso*-butyle,
N-[(1-naphtyl)acétyl]alaninate d'*iso*-butyle,
N-[(2-naphtyl)acétyl]alaninate d'*iso*-butyle,
N-(4-phénylbutanoyl)alaninate d'*iso*-butyle,

N-(5-phénylpentanoyl)alaninate d'*iso*-butyle,
N-[(4-pyridyl)acétyl]alaninate d'*iso*-butyle,
2-[(3,4-dichlorophényl)acétamido]butyrate d'*iso*-butyle,
2-[(3-méthoxyphényl)acétamido]butyrate d'*iso*-butyle,
2-[(4-nitrophényl)acétamido]butyrate d'*iso*-butyle,
2-[(3,4-méthylènedioxyphényl)acétamido]butyrate d'*iso*-butyle,
2-[(thién-3-yl)acétamido]butyrate d'*iso*-butyle,
2-[(4-chlorophényl)acétamido]butyrate d'*iso*-butyle,
2-[(3-nitrophényl)acétamido]butyrate d'*iso*-butyle,
2-[(2-hydroxyphényl)acétamido]butyrate d'*iso*-butyle,
2-[(2-naphtyl)acétamido]butyrate d'*iso*-butyle,
2-[(2,4-dichlorophényl)acétamido]butyrate d'*iso*-butyle,
2-[(4-bromophényl)acétamido]butyrate d'*iso*-butyle,
2-[(3-chlorophényl)acétamido])butyrate d'*iso*-butyle,
2-[(3-fluorophényl)acétamido]butyrate d'*iso*-butyle,
2-[(benzothiazol-4-yl)acétamido]butyrate *d'iso*-butyle,
2-[(2-méthylphényl)acétamido]butyrate d'*iso*-butyle,
2-[(2-fluorophényl)acétamido]butyrate d'*iso*-butyle,
2-[(4-fluorophényl)acétamido]butyrate d'*iso*-butyle,
2-[(3-bromophényl)acétamido]butyrate d'*iso*-butyle
2-[(3-trifluorométhylphényl)acétamido]butyrate d'*iso*-butyle,
2-[(2-thiényl)acétamido]butyrate d'*iso*-butyle,
2-(phénylacétamido)butyrate d'*iso*-butyle,
N-(phénylacétyl)valine de 2-méthylbutyle,
N-(phénylacétyl)méthioninate d'*iso*-butyle,
N-(phénylacétyl)leucine d'*iso*-butyle,
N-[(3-chlorophényl)acétyl]alaninate de 3-méthylbut-2-ényle,
N-[(3-chlorophényl)acétyl]alaninate de cyclopropylméthyle,
N-[(3-chlorophényl)acétyl]alaninate de 2-thiénylméthyle,
N-[(3-chlorophényl)acétyl]alaninate de (1-méthylcyclopropyl)méthyle,
N-[(3-chlorophényl)acétyl]alaninate de 3-thiénylméthyle,
N-[(3-chlorophényl)acétyl]alaninate de 2-méthylcyclopentyle,
N-[(3-chlorophényl)acétyl]alaninate de 2-méthylprop-2-ényle,
N-[(3-chlorophényl)acétyl)alaninate de cyclohex-2-ényle,
N-[(2-phénylbenzoxazol-5-yl)acétyl]alaninate d'*iso*-butyle,
N-[(3-méthylthiophényl)acétyl]alaninate d'*iso*-butyle,
N-4-[(2-furyl)acétyl]alaninate d'*iso*-butyle,
N-[(benzofuran-2-yl)acétyl]alaninate d'*iso*-butyle,
N-[(benzothiophén-3-yl)acétyl]alaninate d'*iso*-butyle,
N-[(2-chloro-5-thiényl)acétyl]alaninate d'*iso*-butyle,
N-[(3-méthyl-isoxazol-5-yl)acétyl]alaninate d'*iso*-butyle,
N-[(2-phénylthiothiényl)acétyl]alaninate d'*iso*-butyle,
N-[(6-méthoxybenzothiophén-2-yl)acétyl]alaninate d'*iso*-butyle,
N-[(3-phényl-1,2,4-thiadiazol-5-yl)acétyl]alaninate d'*iso*-butyle,
N-[(2-phényloxazol-4-yl)acétyl]alaninate d'*iso*-butyle,
N-(3-méthylphényl)acétyl]alaninate d'*iso*-butyle,
N-[(2,5-difluorophényl)acétyl]alaninate d'*iso*-butyle,
N-[(3,5-difluorophényl)acétyl]alaninate d'*iso*-butyle,
N-[(3-thiényl)acétyl]alaninate d'*iso*-butyle,
N-[(4-méthylphényl)acétyl]alaninate d'*iso*-butyle,
N- (phénylacétyl) alaninate de (1-méthoxycarbonyl)iso-butyle,
N-[(3-nitrophényl)acétyl]alaninate d'*iso*-butyle
N-[(3,5-difluorophényl)acétyl]alaninate d'éthyle,
N-[(3-nitrophényl)acétyl]méthioninate d'éthyle,
N-[(3-chlorophényl)acétyl]alaninate d'*iso*-butyle,
N-[(3-chlorophényl)acétyl]alaninate de 2-(N,N-diméthylamino)-éthyle,
2-[(3,5-dichlorophényl)acétamido]hexanoate de méthyle,
N-[(3,5-dichlorophényl)acétyl]alaninate d'iso-butyle,

N-(cyclohexylacétyl)alaninate d'*iso*-butyle,
N-(cyclopentylacétyl)alaninate d'*iso*-butyle,
N-[(cyclohex-1-ényle)acétyl]alaninate d'*iso*-butyle,
N-[(3-chlorophényl)acétyl]alaninate de 3-méthylbut-2-ényle thioester,
N-[(2-phényl)-2-fluoroacétyl]alaninate d'éthyle,
N-(3,5-difluorophénylacétyl)phénylglycinate de méthyle,
N-(3,5-difluorophénylacétyl)phénylglycinate d'*iso*-butyle,
N-(cyclopentylacétyl)phénylglycinate de méthyle,
N-(cyclopentylacétyl)alaninate de méthyle,
N-(cyclopropylacétyl)phénylglycinate de méthyle,
N-(cyclopropylacétyl)alaninate de méthyle, et
N-[(3-nitrophényl)acétyl]méthioninate d'*iso*-butyle.

**70.** Procédé pour inhiber la libération du β-amyloïde et/ou sa synthèse dans une cellule in vitro, ledit procédé comprenant l'administration à une telle cellule d'une quantité d'un composé de formule I ou d'un mélange de composés de formule I' efficace à inhiber la libération et/ou la synthèse cellulaire du peptide β-amyloïde, où la formule I' est telle que définie dans l'une quelconque des revendications 45 à 69.

**71.** Composé pour l'utilisation comme médicament pris dans le groupe constitué par :

N-(phénylacétyl)alaninate d'*iso*-butyle,
N-(3-phénylpropionyl)alaninate d'*iso*-butyle,
2-(phénylacétamido)butyrate d'*iso*-butyle,
N-(phénylacétyl)valinate de 2-méthylbutyle,
N-(phénylacétyl)méthioninate d'*iso*-butyle,
N-(phénylacétyl)leucinate d'*iso*-butyle,
N-(phénylacétyl)alaninate de (1-méthoxycarbonyl)iso-butyle,
N-[(2-phényl)-2-fluoroacétyl]alaninate d'éthyle.

**72.** Composé pris dans le groupe constitué par :

N-(phénylacétyl)alaninate d'*iso*-butyle,
N-(3-phénylpropionyl)alaninate d'*iso*-butyle,
2-(phénylacétamido)butyrate d'*iso*-butyle,
N-(phénylacétyl)valinate de 2-méthylbutyle,
N-(phénylacétyl)méthioninate d'*iso*-butyle,
N-(phénylacétyl)leucine d'*iso*-butyle,
N-(phénylacétyl)alaninate de (1-méthoxycarbonyl) *iso*-butyle,
N-[(2-phényl)-2-fluoroacétyl]alaninate d'éthyle.

**73.** Composition pharmaceutique comprenant un véhicule inerte du point de vue pharmaceutique et une quantité à efficacité pharmaceutique d'un composé selon la revendication 71 ou 72.

SEQUENCE LISTING

(1) GENERAL INFORMATION:

(i) APPLICANTS:

ATHENA NEUROSCIENCES, INC.
ELI LILLY & COMPANY
JING WU
EUGENE D. THORSETT
JEFFREY S. NISSEN
THOMAS E. MABRY
LEE H. LATIMER
VARGHESE JOHN
LAWRENCE Y. FANG
JAMES E. AUDIA

(ii) TITLE OF INVENTION:

N-(ARYL/HETEROARYLACETYL)
AMINO ACID ESTERS, PHARMACEUTICAL
COMPOSITONS COMPRISING SAME, AND
METHODS FOR INHIBITING β-AMYLOID
PEPTIDE RELEASE AND/OR ITS
SYNTHESIS BY USE OF SUCH COMPOUNDS

(iii) NUMBER OF SEQUENCES: 1

(iv) CORRESPONDENCE ADDRESS:

(A) ADDRESSEE: Burns, Doane, Swecker & Mathis, LLP
(B) STREET: P.O. Box 1404
(C) CITY: Alexandria
(D) STATE: Virginia
(E) COUNTRY: U.S.A.
(F) ZIP: 22313-1404

(v) COMPUTER READABLE FORM:

(A) MEDIUM TYPE: Floppy disk

(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:

(A) APPLICATION NUMBER: Unassigned
(B) FILING DATE: Unassigned
(C) CLASSIFICATION:

(vii) PRIOR APPLICATION DATA:

(A) U.S. PATENT APPLICATION NO. 08/754,895
(B) FILING DATE: 22 November 1996

(viii) ATTORNEY/AGENT INFORMATION:

(A) NAME: Swiss, Gerald F.
(B) REGISTRATION NUMBER: 30,113
(C) REFERENCE/DOCKET NUMBER: 002010-051

(ix) TELECOMMUNICATION INFORMATION:

(A) TELEPHONE: 650-854-7400
(B) TELEFAX: 650-854-8275

(2) INFORMATION FOR SEQ ID NO:1:

(i) SEQUENCE CHARACTERISTICS:

(A) LENGTH: 43 amino acids
(B) TYPE: peptide
(D) TOPOLOGY: linear

(ii) MOLECULE TYPE: peptide

(xi) SEQUENCE DESCRIPTION: SEQ ID NO 1:

```
Asp Ala Glu Phe Arg His Asp Ser Gly Tyr Glu Val His His
1               5                   10

Gln Lys Leu Val Phe Phe Ala Glu Asp Val Gly Ser Asn Lys
15                  20                  25

Gly Ala Ile Ile Gly Leu Met Val Gly Gly Val Val Ile Ala
    30                  35                  40

Thr
```